# EUROPEAN PATENT APPLICATION

(11) **EP 4 667 468 A1**
(43) Date of publication of application: **24.12.2025**
(21) Application number: 24756268.9
(22) Date of filing: 07.02.2024
(51) Int. Cl.: C07D 471/04, C07D 487/04, C07D 519/00, A61K 31/437, A61P 29/00, A61P 37/00, A61P 13/12, A61P 3/10, A61P 9/00

(54) **COMPOUND FOR INHIBITING NLRP3, PREPARATION METHOD, AND USE**

(30) Priority: 14.02.2023 CN 202310139999; 31.05.2023 CN 202310644402; 14.06.2023 CN 202310705888; 12.01.2024 CN 202410056683
(71) Applicant: Shenzhen Zhongge Biological Technology Co., Ltd., Futian District Shenzhen, Guangdong 518017 (CN)
(72) Inventor: DU, Xinming, Shenzhen, Guangdong 518017 (CN); MA, Junjun, Shenzhen, Guangdong 518017 (CN); CHEN, Daoxing, Shenzhen, Guangdong 518017 (CN); WANG, Shaohui, Shenzhen, Guangdong 518017 (CN); CHEN, Bin, Shenzhen, Guangdong 518017 (CN); ZHANG, Peiyu, Shenzhen, Guangdong 518017 (CN)
(74) Representative: Rückerl, Florian
(86) International application number: PCT/CN2024/076764
(87) International publication number: WO 2024/169895

(57) **Abstract**

The present invention provides a compound as an NLRP3 inhibitor, which has a novel bicyclic fused ring core. The present invention further relates to a preparation method thereof, a pharmaceutical composition and a drug containing the same, and its use in the treatment of diseases and disorders mediated by NLRP3.

## Description

### Cross-Reference to Related Applications

The application claims the priorities of CN202310139999.7 which was filed on February 14, 2023, CN202310644402.4 which was filed on May 31, 2023, CN202310705888.8 which was filed on June 14, 2023, and CN202410056683.6 which was filed on January 12, 2024, the contents of which are herein incorporated by reference into the present application in their entireties and for all purposes.

### Technical Field

The present invention relates to a compound that inhibits NLRP3 and preparation method and use.

### Background

The NLRP3 inflammasome is a multi-protein polymer complex composed of NLRP3, apoptosis-associated speck-like protein (apoptosis-associated speck-like protein containing a CARD (ASC)), and caspase-1 precursor (Procaspase-1). The activation of the NLRP3 inflammasome requires two steps, namely initiation (first signal) and activation (second signal). First, the first signal activates the nuclear transcription factor NF-κB, which in turn upregulates the transcription of inflammasome-related components, including inactive NLRP3 and IL-1β precursor. The second signal promotes the conversion of inactive NLRP3 to activated NLRP3. After NLRP3 activation, ASC and pro-caspase-1 are recruited to form the inflammasome. The inflammasome promotes the self-cleavage of Procaspase-1 into the activated form of Caspase-1. Caspase-1 induces Gasdermin D (GSDMD) to cut and further form holes in the cell membrane, triggering cell death, namely pyroptosis. Caspase-1 induces cell pyroptosis while promoting the secretion of IL-1β and IL-18, regulating the body's inflammatory response.

Studies have shown that abnormal activation of the NLRP3 inflammasome can lead to the occurrence of a variety of diseases, such as cryopyrin-associated periodic syndrome (CAPS), a genetic disease caused by acquired mutations NLRP3, familial cold auto inflammatory syndrome (FCAS), Muckle-Wells syndrome, and neonatal-onset multisystem inflammatory disease (NOMID). In addition, NLRP3 inflammasomes are also involved in the occurrence of many complex diseases, such as diseases of the central nervous system (Alzheimer's disease, brain infection, multiple sclerosis, amyotrophic lateral sclerosis and Parkinson's disease), lung diseases (asthma, idiopathic pulmonary fibrosis); metabolic disorders (type II diabetes and related complications, atherosclerosis, obesity and gout), liver diseases (chronic liver disease, non-alcoholic steatohepatitis, viral hepatitis and cirrhosis), kidney diseases (acute and chronic kidney injury), inflammatory bowel disease (IBD), other autoimmune diseases (rheumatoid arthritis, suppurative hidradenitis, systemic lupus erythematosus) and tumors (colorectal cancer, lung cancer, myeloproliferative neoplasms, leukemia, myelodysplastic syndrome (MDS), and myelofibrosis). Therefore, NLRP3 inflammasomes, as an important potential target, have important therapeutic value for a variety of diseases.

Currently, there are no drug molecules targeting NLRP3 inflammasomes on the market, and there is a clear unmet market demand. So far, the compounds that have been developed and entered clinical trials include Dapansutrile (Olatec), IFM-2427 (IFM/Novartis), ZYIL-1 (Zydus Lifesciences), VTX-2735 (Zomagen Biosciences/Ventyx Biosciences), etc. Most of these compounds are derivatives of compound MCC950 which is the sulfonylurea small molecule NLRP3 inhibitor, which have the disadvantages of relatively single structural types, short half-life, and difficulty in penetrating the blood-cerebrospinal fluid barrier. Therefore, it is very necessary to develop a new generation of NLRP3 inflammasome small molecule inhibitors with higher activity and better druggability.

### Summary

In order to solve one of the above-mentioned technical problems existing in the prior art, the present invention provides a compound with a bicyclic fused ring core as an NLRP3 inhibitor, and the compound provided by the present invention has better druggability.

In one aspect, the present invention provides a compound represented by Formula I, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound, or prodrug thereof, 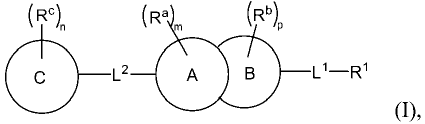 wherein, 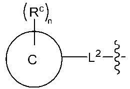 is connected to ring A, 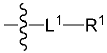 is connected to ring B;
ring A and ring B are each independently selected from: 5-6 membered heteroaryl, 5-6 membered heterocyclyl, and phenyl;
ring C is selected from: 6-10 membered aryl, 5-10 membered heteroaryl, phenyl fused 4-7 membered cycloalkyl, phenyl fused 4-7 membered heterocyclyl, 5-6 membered heteroaryl fused 4-7 membered cycloalkyl, 5-6 membered heteroaryl fused 4-7 membered heterocyclyl, 4-7 membered cycloalkyl, and 4-7 membered heterocyclyl;
each R^{c} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, R^{c1}S(O)₂-, -(CH₂)ₛ-N(R^{c1})(R^{c2}), 3-6 membered cycloalkyl, 3-6 membered heterocyclyl, phenyl, phenyl substituted with one or more halogens, phenyl substituted with one or more hydroxyl, and 5-6 membered heteroaryl; the 3-6 membered cycloalkyl, 3-6 membered heterocyclyl, phenyl, and 5-6 membered heteroaryl are optionally substituted with 1, 2, or 3 substituents selected from: deuterium, halogen, -OH, -NH₂, -CN, C₁₋₆ alkyl, C₁₋₆ haloalkyl, and C₁₋₆ alkoxy; or
two R^{c} together with the C atom to which they are attached may form a 3-6 membered cycloalkyl, 4-7 membered heterocyclyl, 6 membered aryl, or 5-6 membered heteroaryl; the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
each R^{a} and R^{b} are independently selected from: H, deuterium, halogen, -OH, -NH₂, -CN, oxo, C₁₋₆ alkyl, C₁₋₆ haloalkyl, C₁₋₆ alkoxy, C₁₋₆ alkoxy-C₁₋₆ alkyl, C₁₋₆ haloalkoxy, hydroxyl-C₁₋₆ alkyl, -NH(C₁₋₆ alkyl), -N(C₁₋₆ alkyl)(C₁₋₆ alkyl), -C(=O)-(C₁₋₆ alkyl), and 3-6 membered heterocyclyl optionally substituted by hydroxyl;
L¹ is selected from: bond, C₃₋₆ cycloalkylene, -NR²-, -O-, -S-, -C(=O)-, -(CR³R⁴)ₐ-, -NR²-(CR³R⁴)ₐ-, - (CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, - (CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, - NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, -C(=O)-(CR³R⁴)ₐ-NR²-, -NR²-(CR³R⁴)ₐ-C(=O)-NR⁵-, -NR²-C(=O)-(CR³R⁴)ₐ-NR⁵-, -C(=S)-NR²-, and -NR²-C(=S)-;
L² is selected from: bond, -NR⁶-, -O-, -S-, -NR⁶-(CR⁷R⁸)_{b}-, -(CR⁷R⁸)_{b}-NR⁶-, -O-(CR⁷R⁸)_{b}-, -(CR⁷R⁸)_{b}-O-, - S-(CR⁷R⁸)_{b}-, and -(CR⁷R⁸)_{b}-S-;
each R², R⁵, R⁶, R⁹ and R¹⁰ are each independently selected from: H, deuterium, and C₁₋₆ alkyl;
each R³, R⁴, R⁷, and R⁸ are each independently selected from: H, deuterium, halogen, oxo, -OH, -NH₂, C₁₋₆ alkyl;
R¹ is selected from: H, -OH, -NH₂, -COOH, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl (preferably 3-6 membered cycloalkyl), 3-6 membered monocyclic heterocyclyl, 6-11 membered bicyclic heterocyclyl, 6-10 membered aryl, and 5-10 membered heteroaryl; wherein the -NH₂, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl (preferably 3-6 membered cycloalkyl), 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: H, deuterium, halogen, -OH, oxo, -CN, C₁₋₆ alkyl, hydroxyl C₁₋₆ alkyl, C₁₋₆ haloalkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, 5-6 membered heteroaryl, -C(=O)-R^{c3}, -S(O)-R^{c3}, -S(O)₂-R^{c3}, -C(=O)O-R^{c3}, -CH₂-C(=O)O-R^{c4}, -N(R^{c4})-C(=O)-R^{c3}, -CH₂-N(R^{c4})-C(=O)-R^{c4}, -C(=O)-N(R^{c6})(R^{c5}), -CH₂-C(=O)-N(R^{c4})(R^{c4}), - N(R^{c4})-C(=O)-N(R^{c6})(R^{c5}), -P(=O)R^{c5} R^{c6}, -CR^{c7}R^{c8}-CN, and -(CH₂)ₛ-R^{c9};
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: H, deuterium, C₁₋₆ alkyl, C₁₋₆ haloalkyl, and C₃₋₆ cycloalkyl, wherein the C₁₋₆ alkyl and C₃₋₆ cycloalkyl are optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and - N(C₁₋₆ alkyl)(C₁₋₆ alkyl);
R^{c4} is selected from: H, deuterium, and C₁₋₆ alkyl;
R^{c5} and R^{c6} are selected from: H, deuterium, C₁₋₆ alkyl, and C₃₋₆ cycloalkyl, or R^{c5}, R^{c6} together with the N atom to which they are attached form a 4-6 membered heterocyclyl; wherein the C₁₋₆ alkyl, C₃₋₆ cycloalkyl, and 4-6 membered heterocyclyl are optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
R^{c7} and R^{c8} are selected from: H, deuterium, and C₁₋₆ alkyl, or R^{c7}, R^{c8} together with the C atom to which they are attached form a C₃₋₆ cycloalkyl;
R^{c9} is selected from 3-6 membered heterocyclyl, wherein the heterocyclyl is optionally substituted with 1, 2 or 3 substituents selected from: H, deuterium, and C₁₋₆ alkyl;
R¹¹ is selected from: H, deuterium, C₁₋₆ alkyl, and 3-6 monocyclic heterocyclyl;
each a and b are each independently 1, 2, 3, or 4;
each s, n, m, and p are each independently selected from: 0, 1, 2, 3, and 4;
provided that, 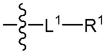 is not H, C₁₋₆ alkyl.

In some embodiments, 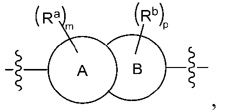 which relates to ring A and ring B, is selected from the following groups: 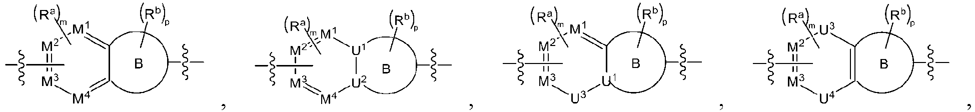 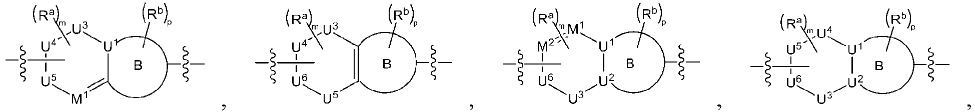 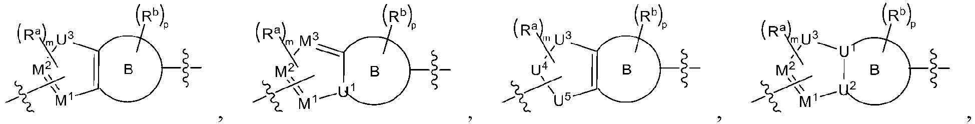 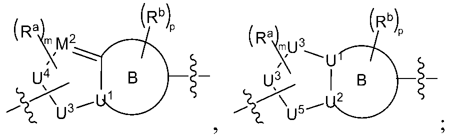
each M¹, M², M³, M⁴, U¹, and U² are each independently selected from: CH and N;
each U³, U⁴, U⁵, and U⁶ are each independently selected from: CH₂, NH, O and S.

In some embodiments, 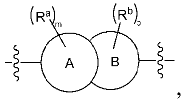 which relates to ring A and ring B, is selected from the following groups: 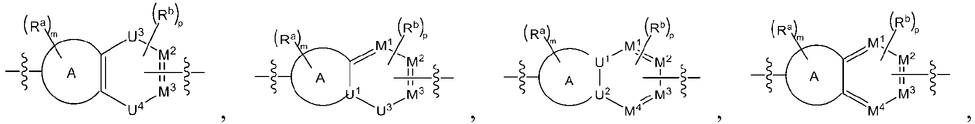 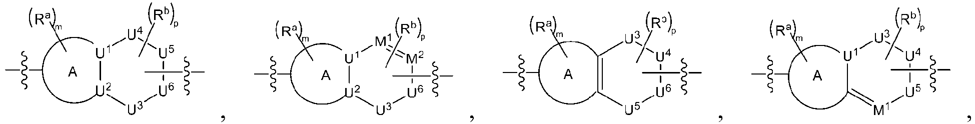 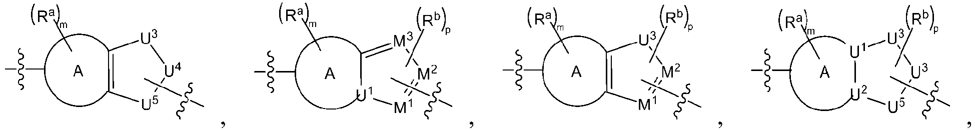 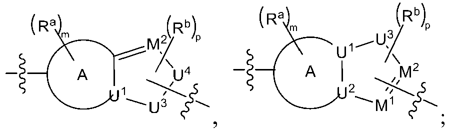
each M¹, M², M³, M⁴, U¹, and U² are each independently selected from: CH and N;
each U³, U⁴, U⁵, and U⁶ are each independently selected from: CH₂, NH, O and S.

In some embodiments, 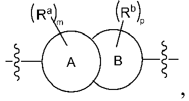 which relates to ring A and ring B, is selected from the following groups: 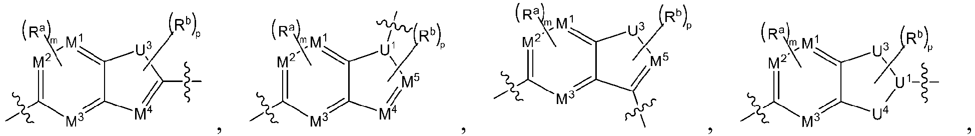 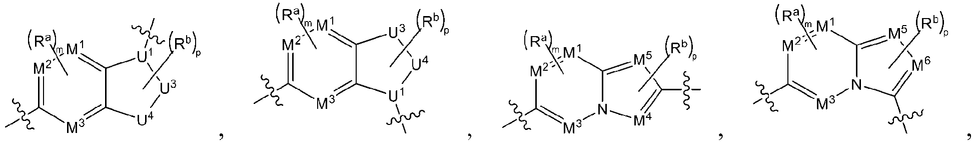 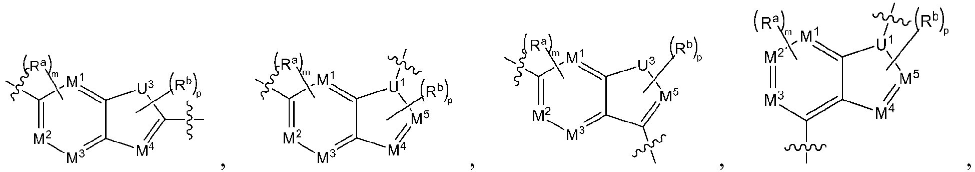 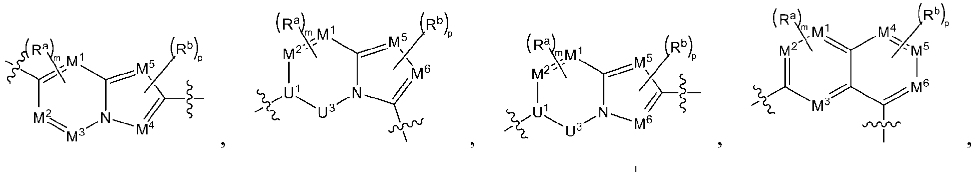 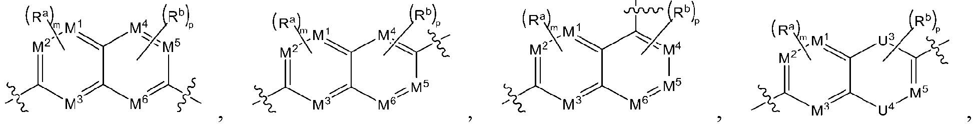 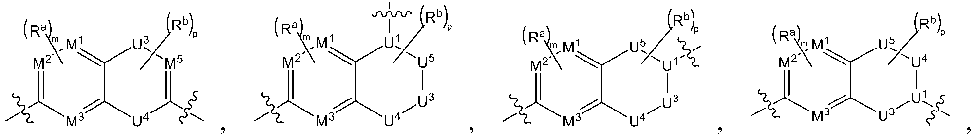 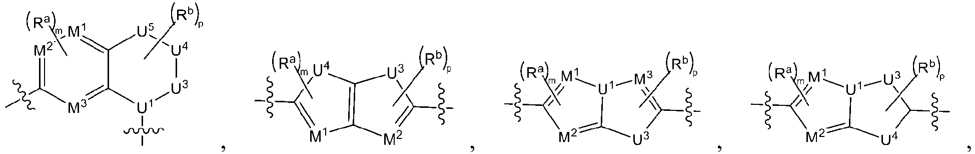 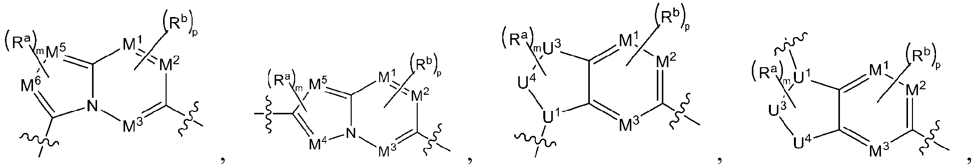 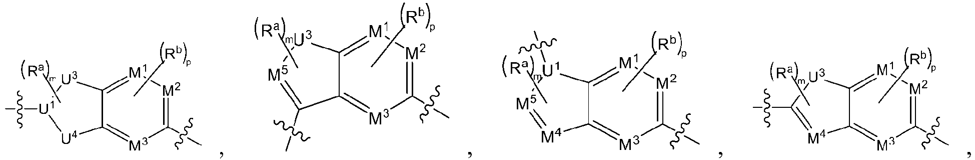 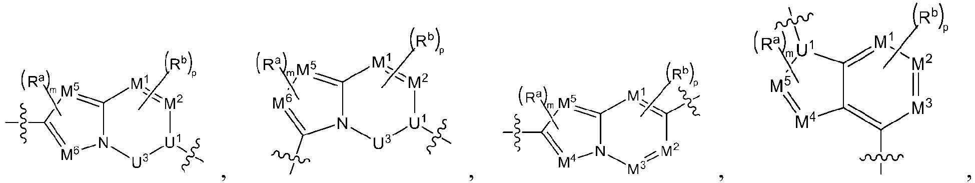 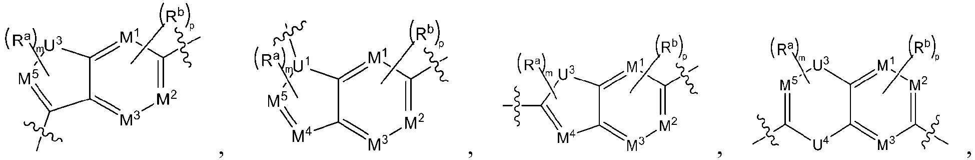 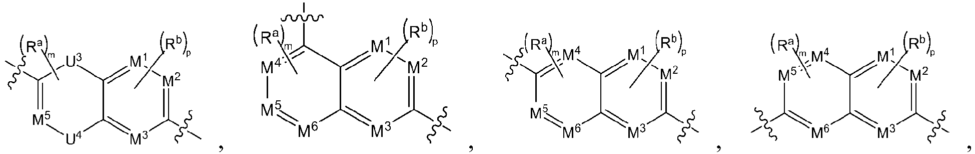 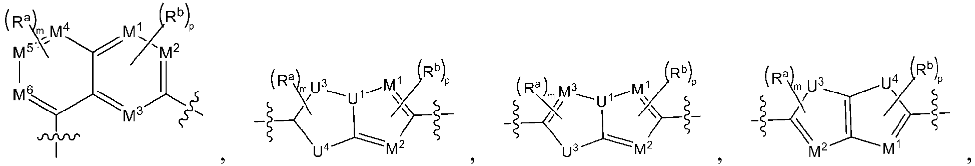 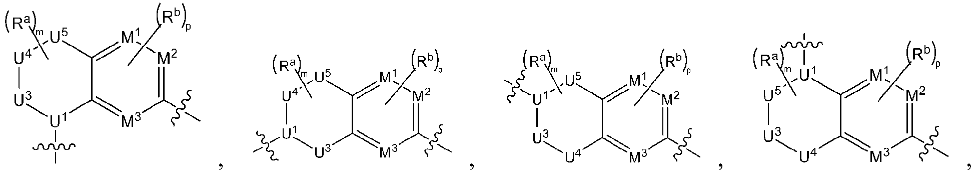 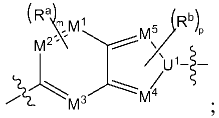
each M¹, M², M³, M⁴, M⁵, M⁶, U¹, and U² are each independently selected from: CH and N;
each U³, U⁴, U⁵, and U⁶ are each independently selected from: CH₂, NH, O and S.

In some embodiments, the compound has the structure represented by Formula VIII-3 orVIII-4: 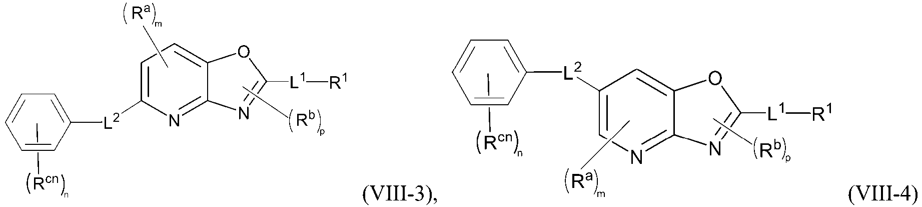
wherein, R¹ is selected from: -OH, -NH₂, -COOH, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the -NH₂, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: H, deuterium, halogen, -OH, oxo, -CN, C₁₋₆ alkyl, hydroxyl C₁₋₆ alkyl, C₁₋₆ haloalkyl, C₁₋₆ alkoxy, -(CH₂)ₛ-O(R^{c1}), - (CH₂)ₛ-N(R^{c1})(R^{c2}), C₃₋₆ cycloalkyl, 3-6 membered monocyclic heterocyclyl, 5-6 membered heteroaryl, -CR^{c7}R^{c8}-CN, and -(CH₂)ₛ-R^{c9};
R^{cn} is defined as R^{c} above;
provided that the following 1)-45) are met at the same time:
   1) when 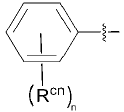 is 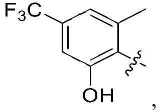 L² is bond, m=p=0 or R^{a}=R^{b}=H, and L¹ is -NH-, R¹ is not selected from the following groups: 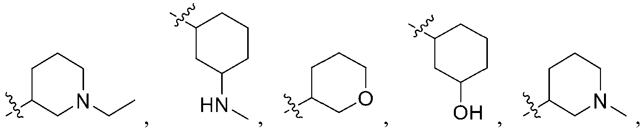 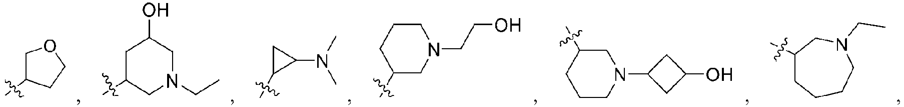 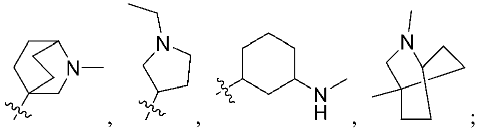
   2) when 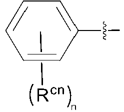 is 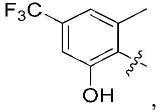 L² is bond, m=p=0 or R^{a}=R^{b}=H, and L¹ is -NH-CH₂-, R¹ is not selected from the following groups: 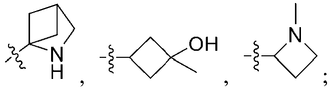
   3) when 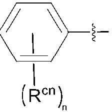 is 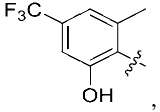 L² is bond, m=p=0 or R^{a}=R^{b}=H, and L¹ is bond, R¹ is not selected from the following groups: 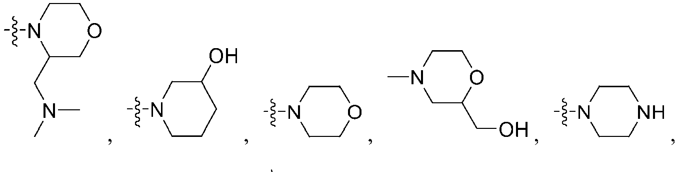 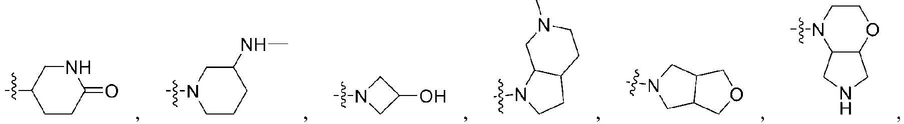 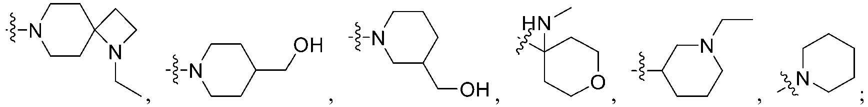
   4) when 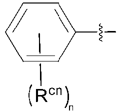 is 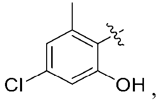 L² is bond, m=p=0 or R^{a}=R^{b}=H, and L¹ is -NH-, R¹ is not selected from the following groups: 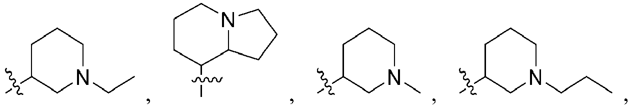 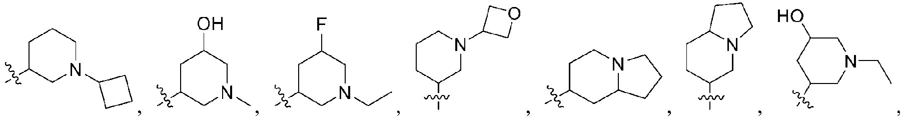 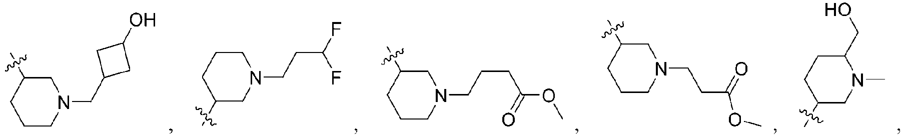 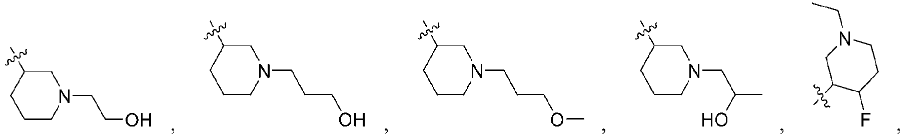 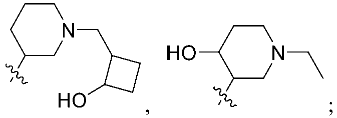
   5) when 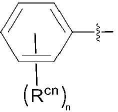 is 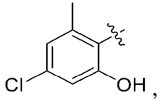 L² is bond, m=p=0 or R^{a}=R^{b}=H, and L¹ is bond, R¹ is not selected from the following groups: 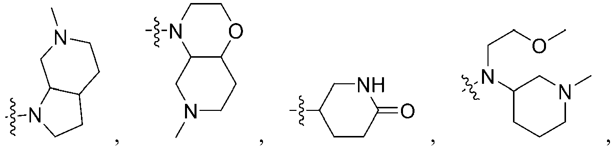 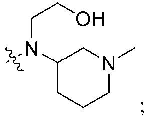
   6) when 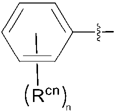 is 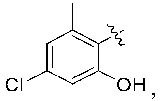 L² is bond, m=p=0 or R^{a}=R^{b}=H, and L¹ is -NH-CH₂-, R¹ is not selected from the following groups: 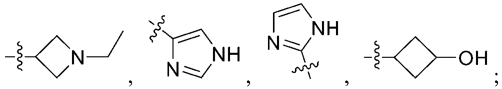
   7) when 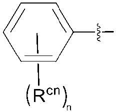 is 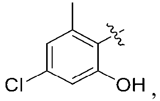 L² is bond, m=p=0 or R^{a}=R^{b}=H, and L¹ is -CH₂-, R¹ is not selected from the following groups: 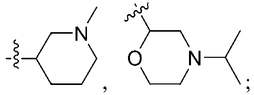
   8) when 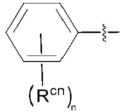 is 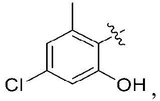 L² is bond, 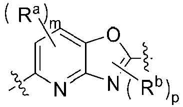 is 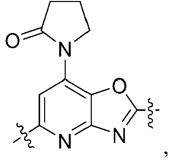 and L¹ is -NH-, R¹ is not 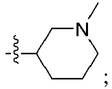
   9) when 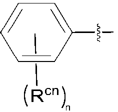 is 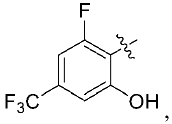 L² is bond, m=p=0 or R^{a}=R^{b}=H, and L¹ is -NH-, R¹ is not 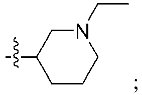
   10) when 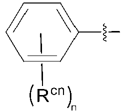 is 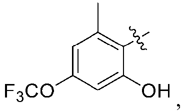 L² is bond, m=p=0 or R^{a}=R^{b}=H, and L¹ is -NH-, R¹ is not 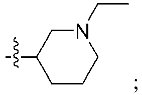
   11) when 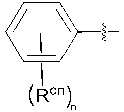 is 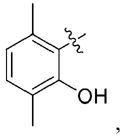 L² is bond, m=p=0 or R^{a}=R^{b}=H, and L¹ is -NH-, R¹ is not 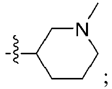
   12) when 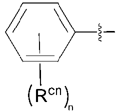 is 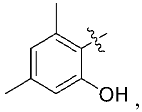 L² is bond, m=p=0 or R^{a}=R^{b}=H, and L¹ is -NH-, R¹ is not selected from the following groups: 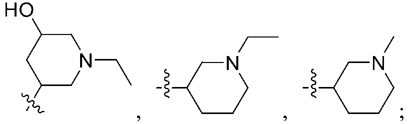
   13) when 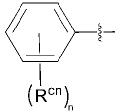 is 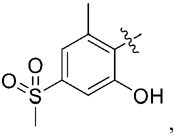 L² is bond, m=p=0 or R^{a}=R^{b}=H, and L¹ is -NH-, R¹ is not 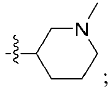
   14) when 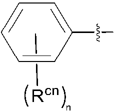 is 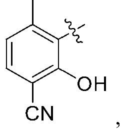 L² is bond, m=p=0 or R^{a}=R^{b}=H, and L¹ is -NH-, R¹ is not 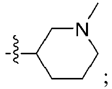
   15) when 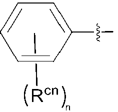 is 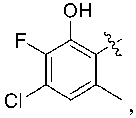 L² is bond, m=p=0 or R^{a}=R^{b}=H, and L¹ is -NH-, R¹ is not 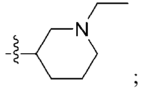
   16) when 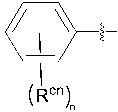 is 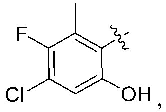 L² is bond, m=p=0 or R^{a}=R^{b}=H, and L¹ is -NH-, R¹ is not 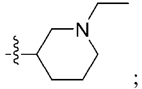
   17) when 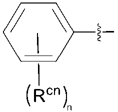 is 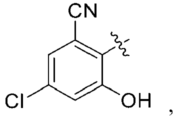 L² is bond, m=p=0 or R^{a}=R^{b}=H, and L¹ is -NH-, R¹ is not 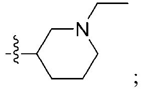
   18) when 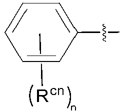 is 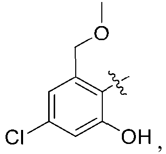 L² is bond, m=p=0 or R^{a}=R^{b}=H, and L¹ is -NH-, R¹ is not selected from the following groups: 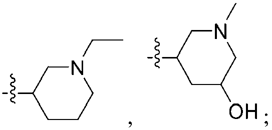
   19) when 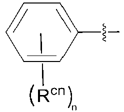 is 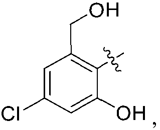 L² is bond, m=p=0 or R^{a}=R^{b}=H, and L¹ is -NH-, R¹ is not 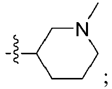
   20) when 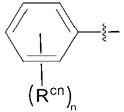 is 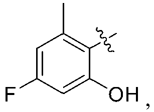 L² is bond, m=p=0 or R^{a}=R^{b}=H, and L¹ is -NH-, R¹ is not selected from the following groups: 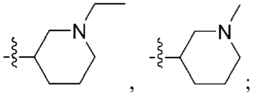
   21) when 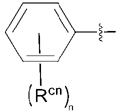 is 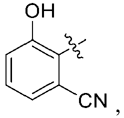 L² is bond, m=p=0 or R^{a}=R^{b}=H, and L¹ is -NH-, R¹ is not 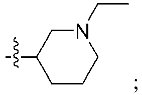
   22) when 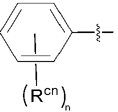 is 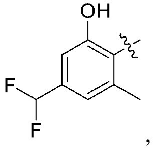 L² is bond, m=p=0 or R^{a}=R^{b}=H, and L¹ is -NH-, R¹ is not selected from the following groups: 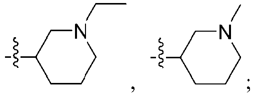
   23) when 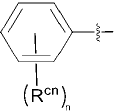 is 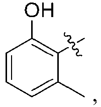 L² is bond, m=p=0 or R^{a}=R^{b}=H, and L¹ is -NH-, R¹ is not 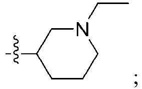
   24) when 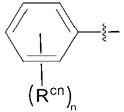 is 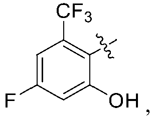 L² is bond, m=p=0 or R^{a}=R^{b}=H, and L¹ is -NH-, R¹ is not 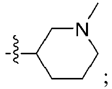
   25) when 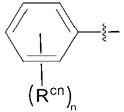 is 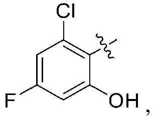 L² is bond, m=p=0 or R^{a}=R^{b}=H, and L¹ is -NH-, R¹ is not 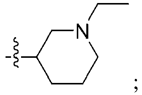
   26) when 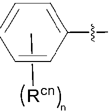 is 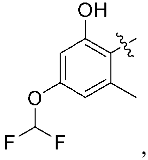 L² is bond, m=p=0 or R^{a}=R^{b}=H, and L¹ is -NH-, R¹ is not 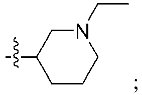
   27) when 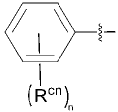 is 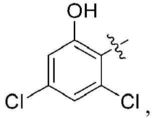 L² is bond, m=p=0 or R^{a}=R^{b}=H, and L¹ is -NH-, R¹ is not 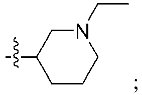
   28) when 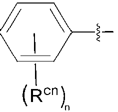 is 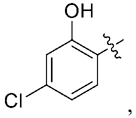 L² is bond, 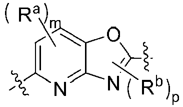 is 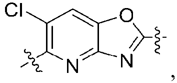 and L¹ is -NH-, R¹ is not 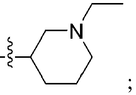
   29) when 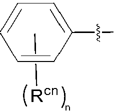 is 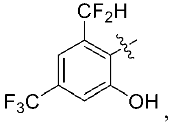 L² is bond, m=p=0 or R^{a}=R^{b}=H, and L¹ is -NH-, R¹ is not 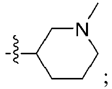
   30) when 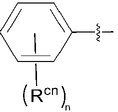 is 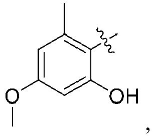 L² is bond, m=p=0 or R^{a}=R^{b}=H, and L¹ is -NH-, R¹ is not 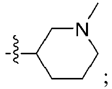
   31) when 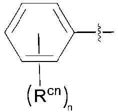 is 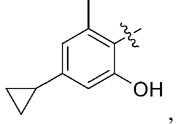 L² is bond, m=p=0 or R^{a}=R^{b}=H, and L¹ is -NH-, R¹ is not 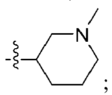
   32) when 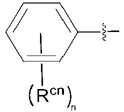 is 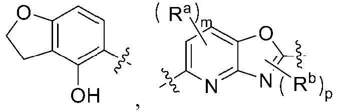 is 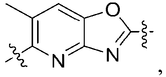 and L¹ is -NH-, R¹ is not 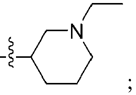
   33) when 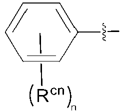 is 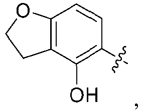 L² is bond, m=p=0 or R^{a}=R^{b}=H, and L¹ is -NH-, R¹ is not 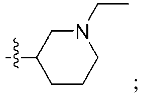
   34) when 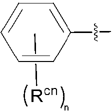 is 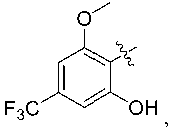 L² is bond, m=p=0 or R^{a}=R^{b}=H, and L¹ is -NH-, R¹ is not 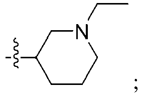
   35) when 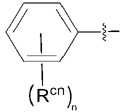 is 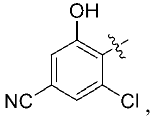 L² is bond, m=p=0 or R^{a}=R^{b}=H, and L¹ is -NH-, R¹ is not 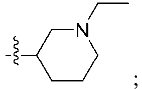
   36) when 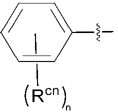 is 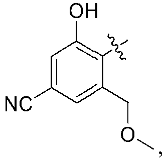 L² is bond, m=p=0 or R^{a}=R^{b}=H, and L¹ is -NH-, R¹ is not 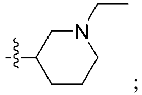
   37) when 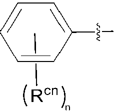 is 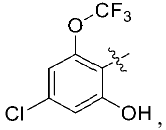 L² is bond, m=p=0 or R^{a}=R^{b}=H, and L¹ is -NH-, R¹ is not 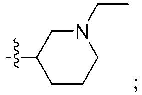
   38) when 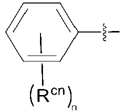 is 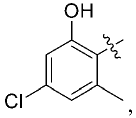 L² is bond, m=p=0 or R^{a}=R^{b}=H, and L¹ is -NH-, R¹ is not selected from the following groups: 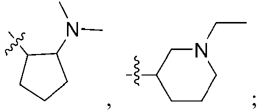
   39) when 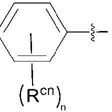 is 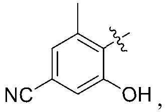 L² is bond, m=p=0 or R^{a}=R^{b}=H, and L¹ is -NH-, R¹ is not selected from the following groups: 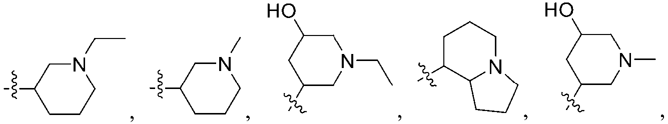 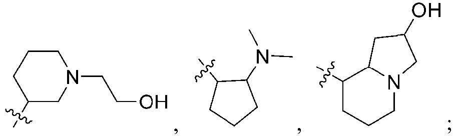
   40) when 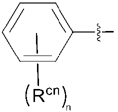 is 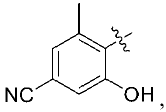 L² is bond, m=p=0 or R^{a}=R^{b}=H, and L¹ is bond, R¹ is not selected from the following groups: 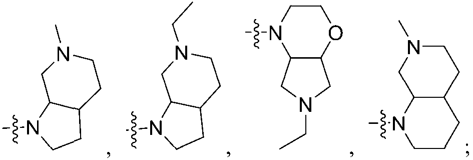
   41) when 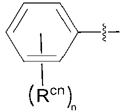 is 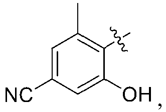 L² is bond, 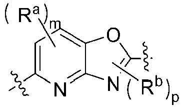 is 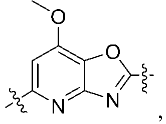 and L¹ is bond, R¹ is not 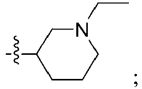
   42) when 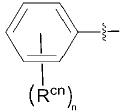 is 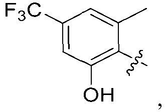 L² is bond, L¹ is -NH-, and R¹ is 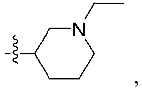 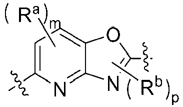 is not selected from the following groups: 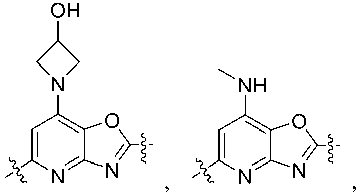 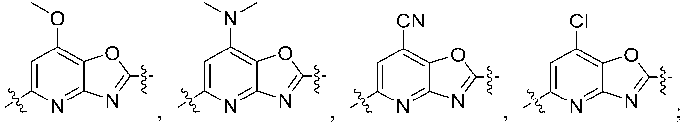
   43) when 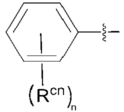 is 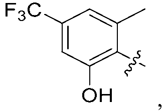 L² is bond, L¹ is -NH-, and R¹ is 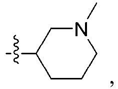 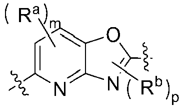 is not selected from the following group: 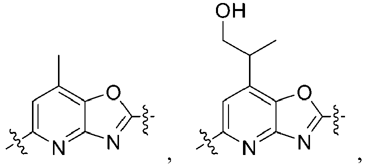 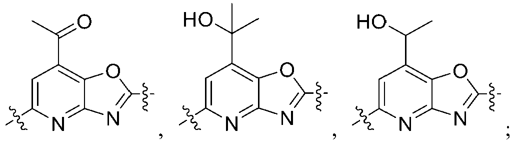
   44) when 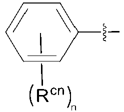 is 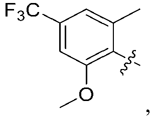 L² is bond, m=p=0 or R^{a}=R^{b}=H, and L¹ is bond, R¹ is not selected from the following groups: 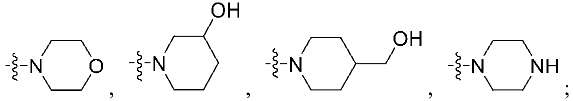
   45) the compound is not 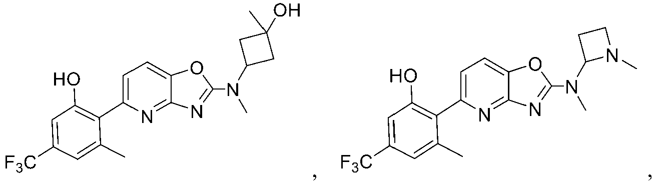 or

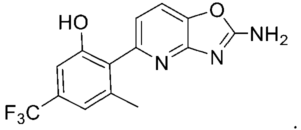

In some embodiments, 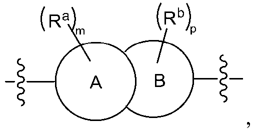 which relates to ring A and ring B, is selected from the following groups, wherein, the following bicyclic groups represent ring A and ring B from left to right or ring A and ring B from right to left; 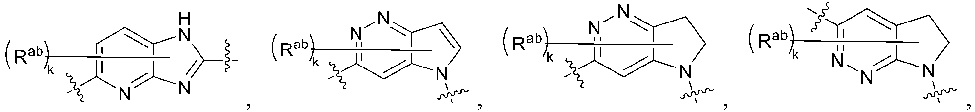 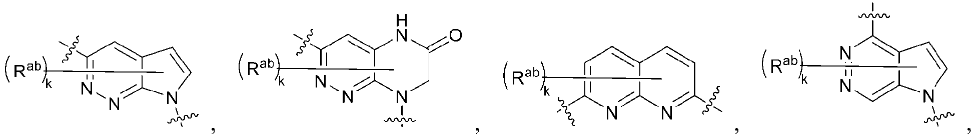 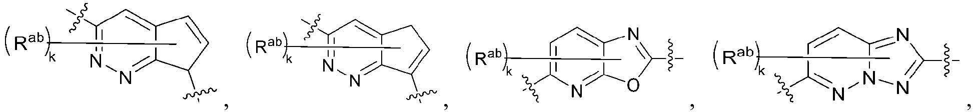 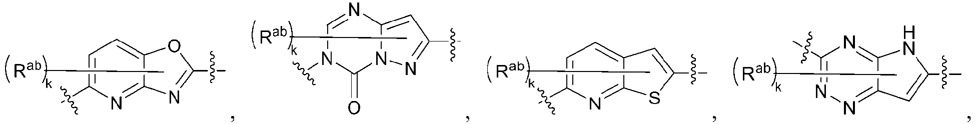 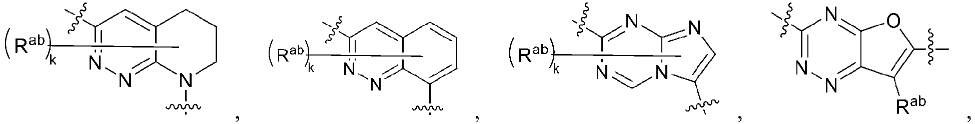 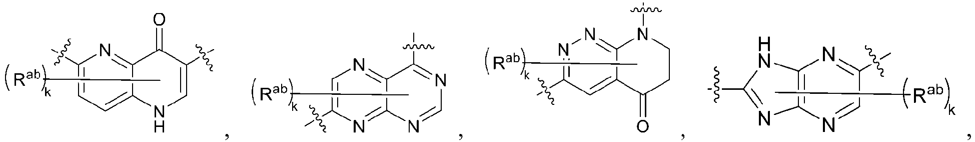 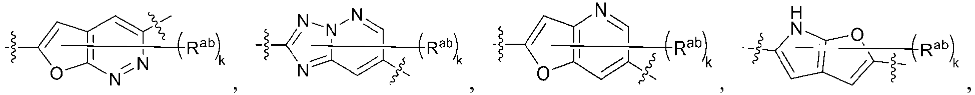 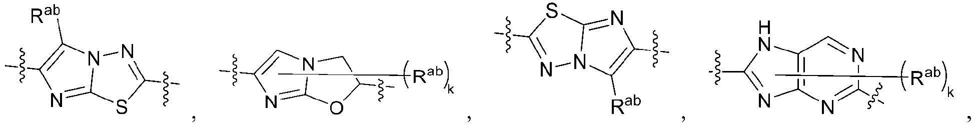 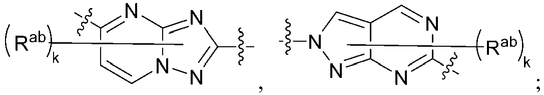
wherein, each R^{ab} is independently selected from: H, deuterium, halogen, -OH, -NH₂, -CN, oxo, methyl, ethyl, propyl, isopropyl, butyl;
each k is independently selected from: 0, 1, 2, 3, and 4.

In some embodiments, 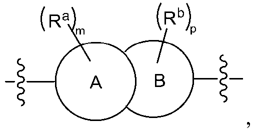 which relates to ring A and ring B, is selected from the following groups, wherein, the following bicyclic groups represent ring A and ring B from left to right or ring A and ring B from right to left; 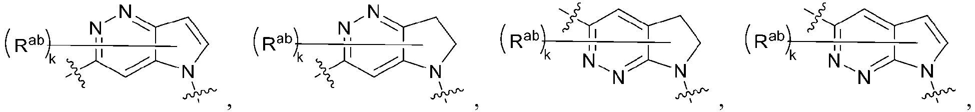 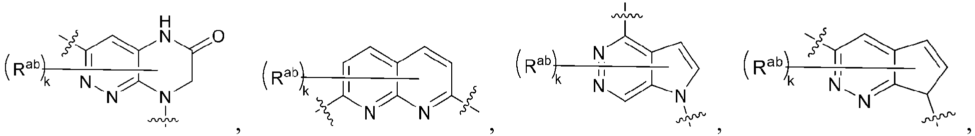 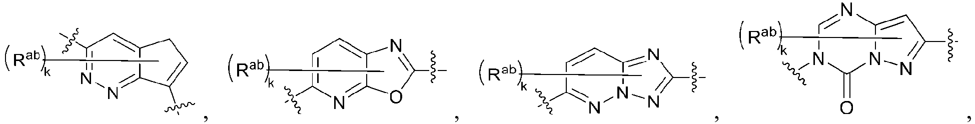 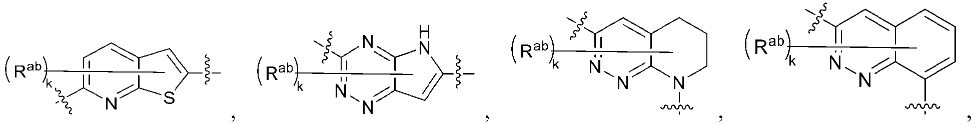 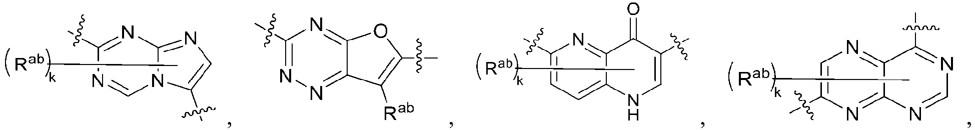 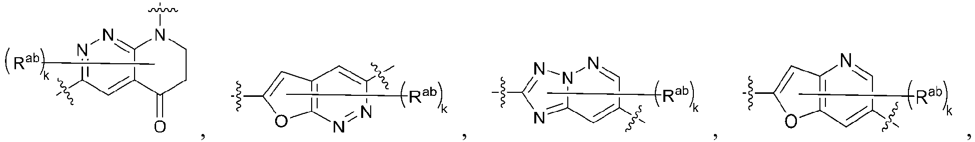 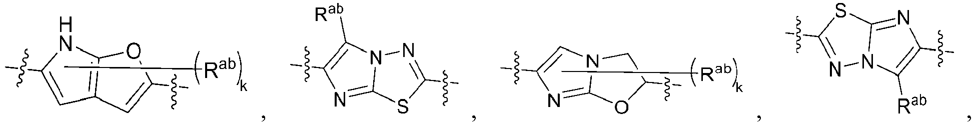 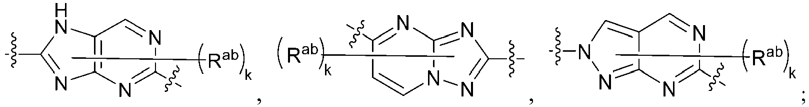
wherein, each R^{ab} is independently selected from: H, deuterium, halogen, -OH, -NH₂, -CN, oxo, methyl, ethyl, propyl, isopropyl, and butyl;
each k is independently selected from: 0, 1, 2, 3, and 4.

In some embodiments, 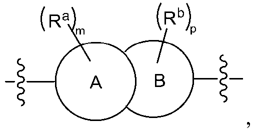 which relates to ring A and ring B, is selected from the following groups, wherein, the following bicyclic groups represent ring A and ring B from left to right or ring A and ring B from right to left;

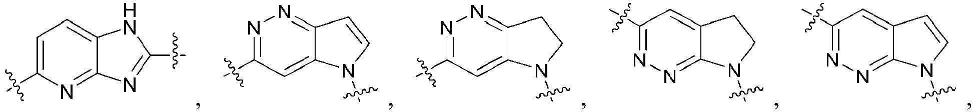

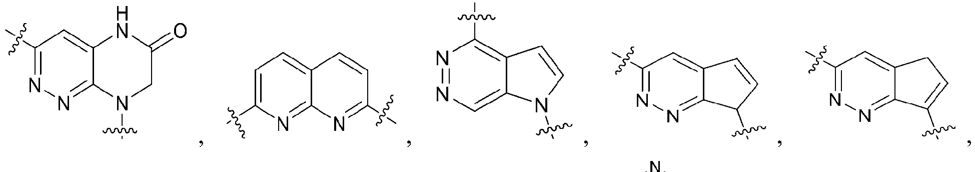

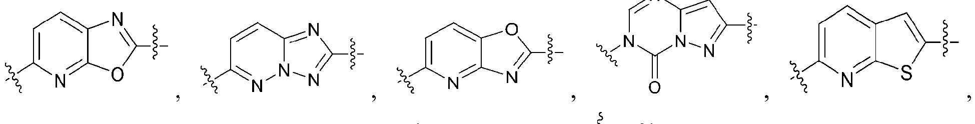

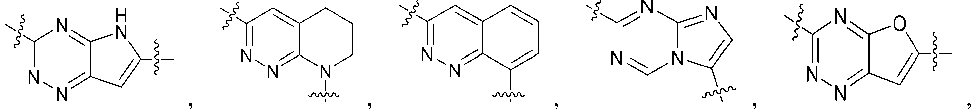

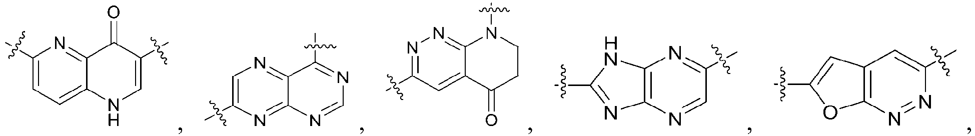

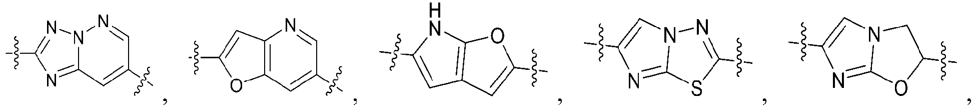

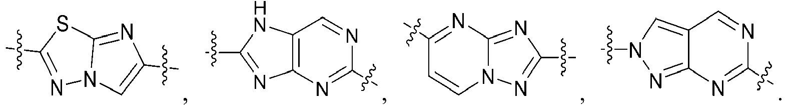

In some embodiments, 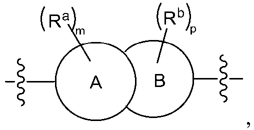 which relates to ring A and ring B, is selected from the following groups, wherein, the following bicyclic groups represent ring A and ring B from left to right or ring A and ring B from right to left;

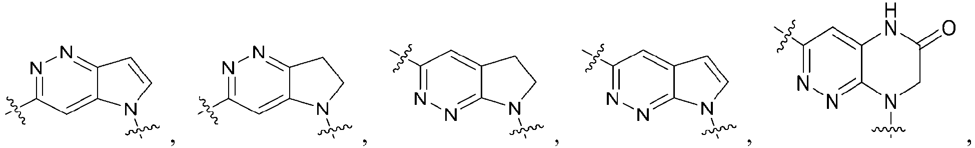

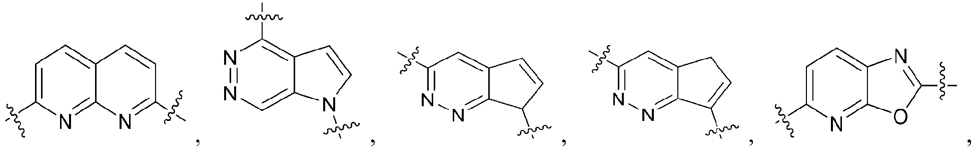

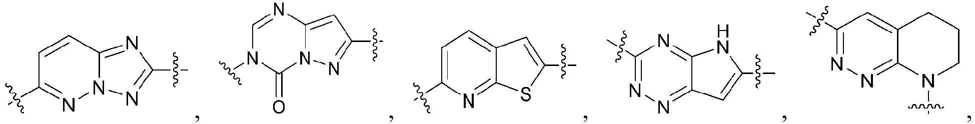

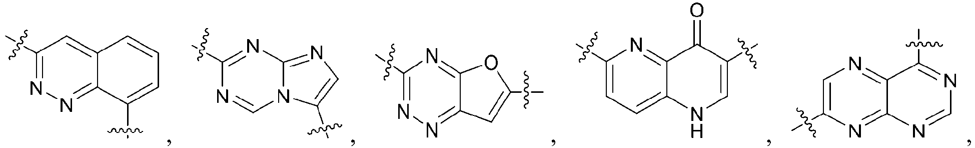

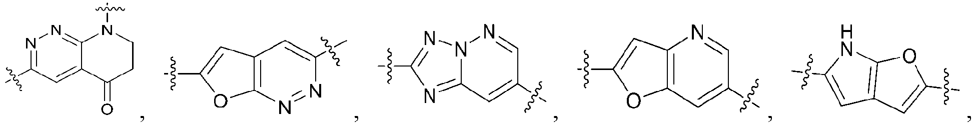

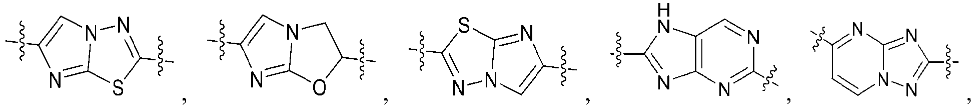

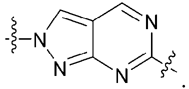

In some embodiments, ring C is selected from: 6-10 membered aryl, 5-6 membered heteroaryl, phenyl fused 5-6 membered heteroaryl, phenyl fused 4-6 membered cycloalkyl, phenyl fused 4-6 membered heterocyclyl, 5-6 membered heteroaryl fused 5-6 membered heteroaryl, 5-6 membered heteroaryl fused 4-6 membered cycloalkyl, 5-6 membered heteroaryl fused 4-6 membered heterocyclyl, 5-6 membered cycloalkyl, and 5-6 membered heterocyclyl.

In some embodiments, ring C is selected from: 6-10 membered aryl, 5-6 membered heteroaryl, phenyl fused 5-6 membered heteroaryl, phenyl fused 4-6 membered cycloalkyl, phenyl fused 4-6 membered heterocyclyl, nitrogen-containing 6-membered heteroaryl fused 5-6 membered heteroaryl, nitrogen-containing 6-membered heteroaryl fused 4-6 membered cycloalkyl, nitrogen-containing 6-membered heteroaryl fused 4-6 membered heterocyclyl, 5-6 membered cycloalkyl, and 5-6 membered heterocyclyl.

In some embodiments, 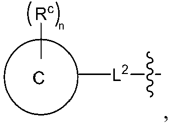 which relates to ring C, is selected from the following groups: 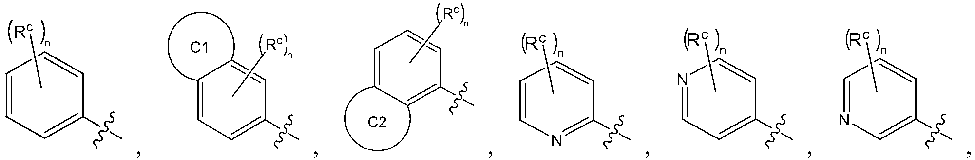 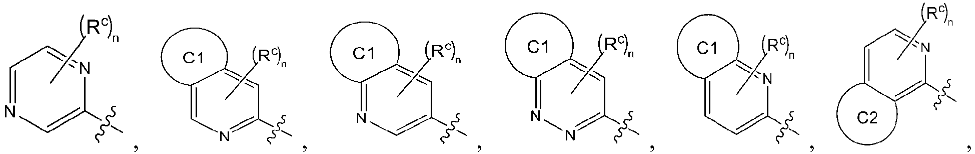 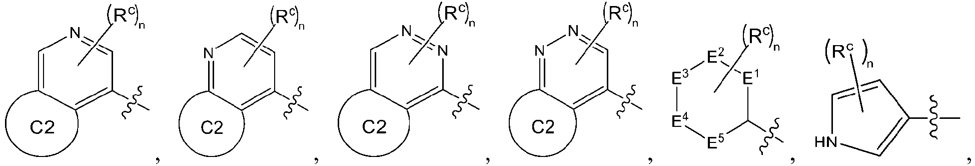 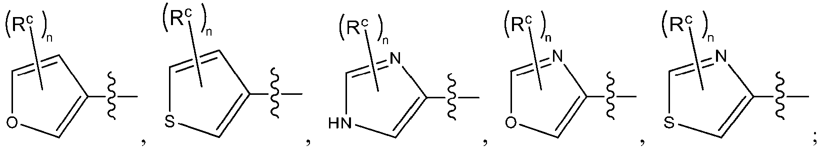
wherein, ring C1 and ring C2 are each independently selected from: phenyl, 5-membered heteroaryl, 6-membered heteroaryl, 4-membered heterocyclyl, 5-membered heterocyclyl, 6-membered heterocyclyl, 4-membered cycloalkyl, 5-membered cycloalkyl, 6-membered cycloalkyl; preferably, ring C1 and ring C2 are each independently selected from: phenyl, pyridyl, pyrimidinyl, pyrazinyl, pyrrolyl, furyl, thienyl, imidazolyl, oxazolyl, thiazolyl, triazolyl, 2,3-dihydrofuranyl, 2,3-dihydrothienyl, 2,3-dihydropyrrolyl, 2,3-dihydrodioxinyl, cyclobutyl, cyclopentyl, cyclohexyl;
E¹, E², E³, E⁴, and E⁵ are each independently selected from: CH₂, O, S, and NH, provides that, at least one of E¹ E², E³, E⁴, and E⁵ is NH, and they form a stable ring.

In some embodiments, 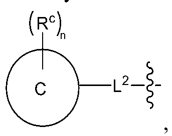 which relates to ring C, is selected from the following groups: 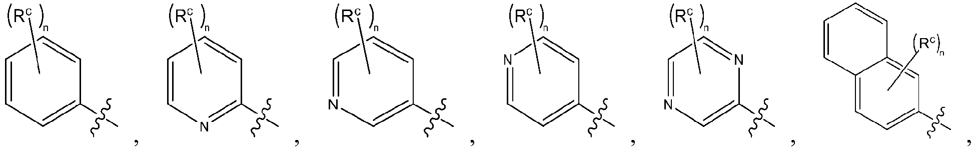 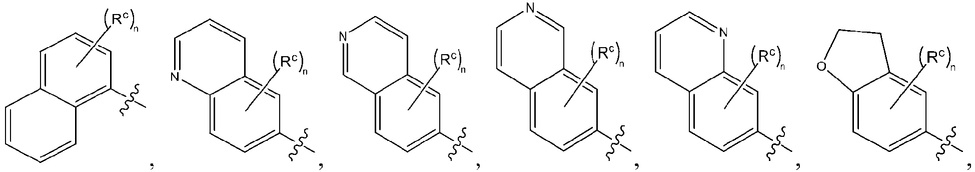 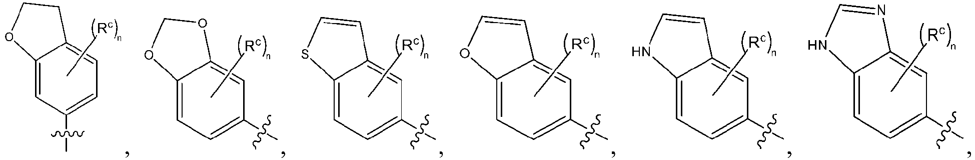 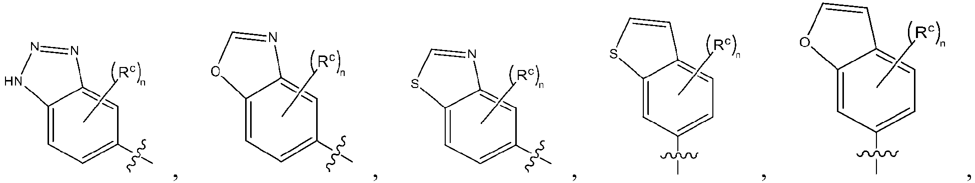 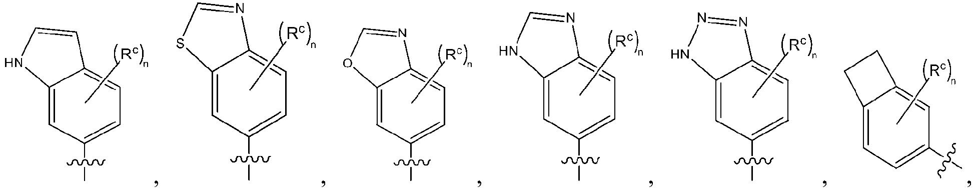 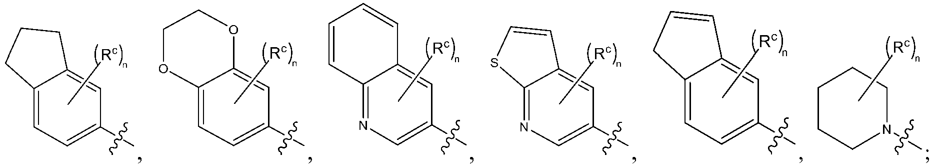 wherein, R^{c} and n have the meanings as defined in the present invention.

In some embodiments, 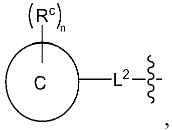 which relates to ring C, is selected from the following groups:

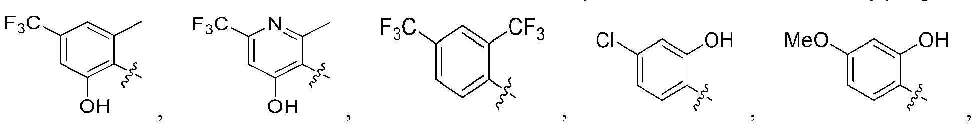

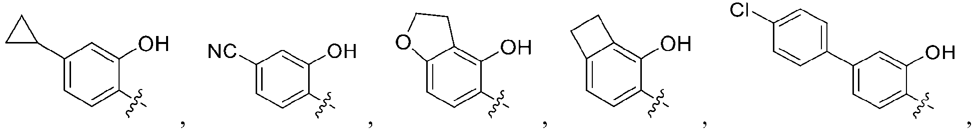

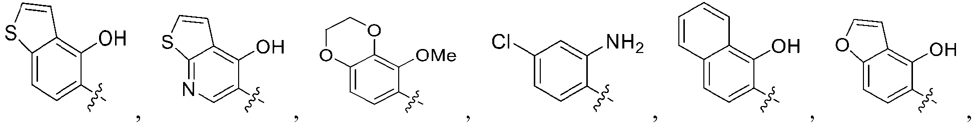

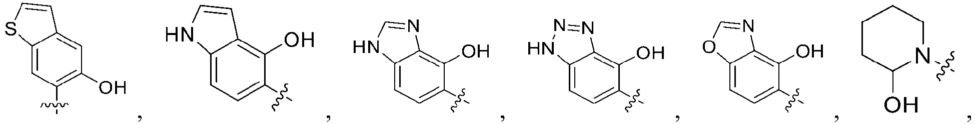

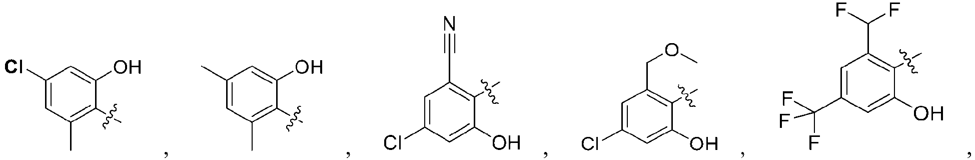

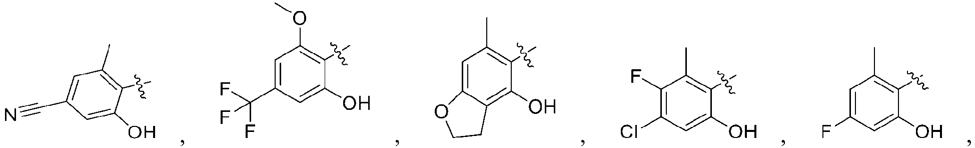

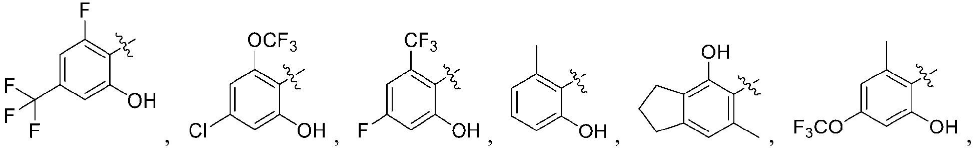

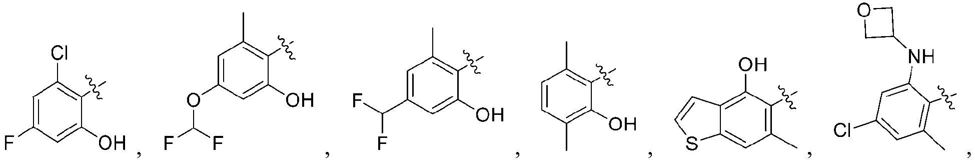

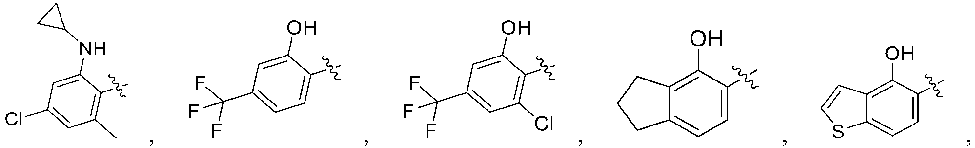

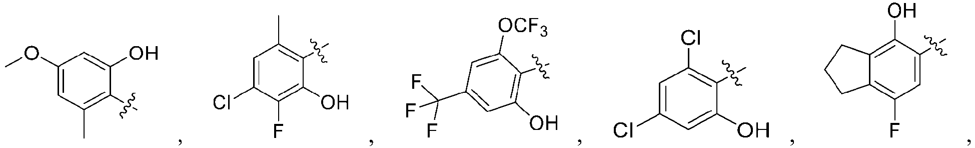

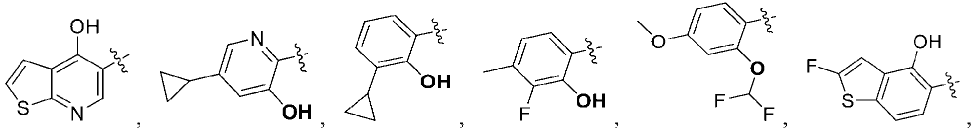

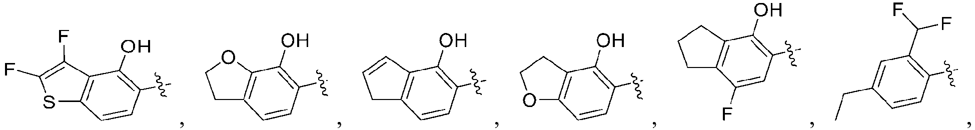

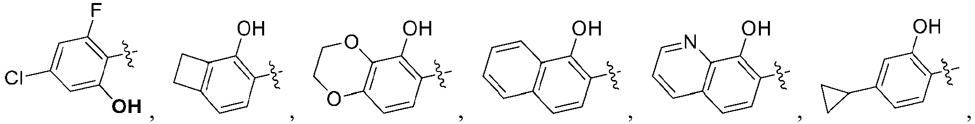

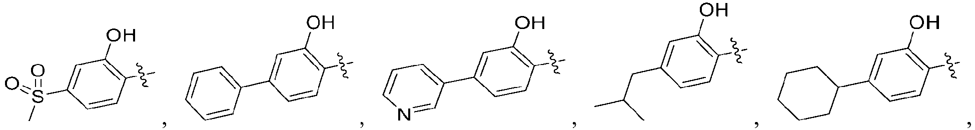

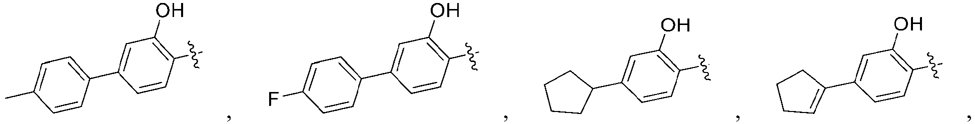

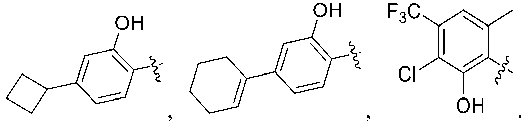

In some embodiments, each R^{a} and R^{b} are independently selected from: H, deuterium, F, Cl, Br, -OH, -NH₂, -CN, oxo, methyl, ethyl, trifluoromethyl, methoxy, n-propyl, isopropyl, dimethylamino, and acetyl.

In some embodiments, each R^{c} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, hydroxyl C₁₋₄ alkyl, C₁₋₄ alkoxy-C₁₋₂ alkyl, R^{c1}S(O)₂-, -(CH₂)ₛ-N(R^{c1})(R^{c2}), 3-6 membered cycloalkyl, 3-6 membered heterocyclyl, and phenyl, the phenyl is optionally substituted with 1, 2, or 3 substituents selected from: deuterium, halogen, -OH, -NH₂, -CN, C₁₋₆ alkyl, C₁₋₆ haloalkyl, C₁₋₆ alkoxy; or
two R^{c} together with the C atom to which they are attached may form a 3-6 membered cycloalkyl, 4-7 membered heterocyclyl, 6 membered aryl, or 5-6 membered heteroaryl; the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, C₁₋₆ haloalkyl.

In some embodiments, each R^{c} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, C₁₋₄ hydroxyalkyl, C₁₋₄ alkoxy-C₁₋₂ alkyl, -(CH₂)ₛ-N(R^{c1})(R^{c2}), 3-6 membered cycloalkyl, 3-6 membered heterocyclyl, phenyl, phenyl substituted with one or more halogens, and phenyl substituted with one or more hydroxyl; or two R^{c} together with the C atom to which they are attached may form a 3-6 membered cycloalkyl, 4-7 membered heterocyclyl, 6 membered aryl, or 5-6 membered heteroaryl; the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, C₁₋₆ haloalkyl.

In some embodiments, each R^{c} is independently selected from: F, Cl, Br, -OH, -NH₂, -CN, methoxy, ethoxy, isopropoxy, methylamino, ethylamino, isopropylamino, methyl, ethyl, n-propyl, isopropyl, n-butyl, isobutyl, trifluoromethyl, difluoromethyl, 2,2,2-trifluoroethyl, pentafluoroethyl, trifluoromethoxy, difluoromethoxy, hydroxymethyl, aminoethyl, methoxymethyl, methoxyethyl, ethoxymethyl, cyclopropyl, cyclobutyl, cyclopentyl, cyclohexyl, cyclopentenyl, cyclobutenyl, cyclohexenyl, oxetanyl, phenyl, 4-chloro-phenyl, 2-chloro-4-fluoro-phenyl, 4-hydroxyphenyl, 4-methyl-phenyl, 4-fluoro-phenyl, pyridyl, cyclobutylamino, 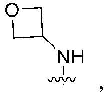 and methanesulfonyl; or two R^{c} together with the C atom to which they are attached form a cyclopentenyl, 2,3-dihydrofuranyl, cyclobutenyl, thienyl, 1,4-dioxanyl, pyridyl, cyclopenta-1,3-dienyl, phenyl, or dioxenyl.

In some embodiments,
each R², R⁵, and R⁶ are each independently selected from: H, deuterium, methyl, ethyl, propyl, and isopropyl;
each R³, R⁴, R⁷, and R⁸ are each independently selected from: H, deuterium, oxo, -OH, -NH₂, methyl, ethyl, propyl, and isopropyl;
each a and b are each independently1 and 2.

In some embodiments,
L¹ is selected from: bond, -NR²-, -O-, -S-, -C(=O)-, -NR²-(CR³R⁴)ₐ-, -(CR³R⁴)ₐ-NR²-, -O-(CR³R⁴)ₐ-, - (CR³R⁴)ₐ-O-, -S-(CR³R⁴)ₐ-, and -(CR³R⁴)ₐ-S-; wherein a is 1 or 2;
L² is selected from: bond, -NR⁶-, -NR⁶-(CR⁷R⁸)_{b}-, -O-, and -S-.

In some embodiments, each s, n, m, and p are each independently selected from: 0, 1, 2, and 3.

In some embodiments,
R¹ is selected from: H, -OH, -NH₂, -COOH, C₁₋₄ alkyl, C₁₋₄ alkoxy, NHR¹¹-C(=O)-, C₁₋₄ alkyl-C(=O)-O-, C₁₋₄ alkyl-O-C(=O)-, 3-6 membered cycloalkyl, 3-6 membered monocyclic heterocyclyl, 6-11 membered spiro bicyclic heterocyclyl, 6-11 membered bridged bicyclic heterocyclyl, 6-11 membered fused bicyclic heterocyclyl, phenyl, naphthyl, and 5-10 membered heteroaryl; wherein the -NH₂, C₁₋₄ alkyl, C₁₋₄ alkoxy, NHR¹¹-C(=O)-, C₁₋₄ alkyl-C(=O)-O-, C₁₋₄ alkyl-O-C(=O)-, 3-6 membered cycloalkyl, 3-6 membered monocyclic heterocyclyl, 6-11 membered spiro bicyclic heterocyclyl, 6-11 membered bridged bicyclic heterocyclyl, 6-11 membered fused bicyclic heterocyclyl, phenyl, naphthyl, and 5-10 membered heteroaryl are each independently optionally further substituted with 1, 2, or 3 substituents selected from: H, deuterium, halogen, -OH, -NH₂, oxo, C₁₋₄ alkyl, hydroxyl C₁₋₄ alkyl, C₁₋₄ haloalkyl, C₁₋₄ alkoxy, C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -CH₂-C(=O)O-R^{c4}, -N(R^{c4})-C(=O)-R^{c3}, -CH₂-N(R^{c4})-C(=O)-R^{c4}, -C(=O)-N(R^{c6})(R^{c5}), -CH₂-C(=O)-N(R^{c4})(R^{c4}), -N(R^{c4})-C(=O)-N(R^{c6})(R^{c5}), -P(=O)R^{c5}R^{c6}, -CR^{c7}R^{c8}-CN, and -(CH₂)ₛ-R^{c9}; wherein, R¹¹ is selected from: H, deuterium, C₁₋₄ alkyl, and 3-6 membered monocyclic heterocyclyl.

In some embodiments,
R¹ is selected from: H, -OH, -NH₂, -COOH, methyl, ethyl, n-propyl, isopropyl, methoxy, ethoxy, n-propoxy, isopropoxy, NH₂-C(=O)-, CH₃NH-C(=O)-, CH₃CH₂NH-C(=O)-, CH₃-C(=O)-O-, CH₃CH₂-C(=O)-O-, CH₃-O-C(=O)-, CH₃CH₂-O-C(=O)-, cyclopropyl, cyclobutyl, cyclopentyl, cyclohexyl, azetidinyl, oxetanyl, tetrahydrofuranyl, pyrrolidinyl, piperidinyl, piperazinyl, morpholinyl, 1,4-dioxanyl, tetrahydro-2H-pyranyl, phenyl, naphthyl, pyridyl, pyrimidinyl, pyrazinyl, pyrrolyl, furyl, thienyl, imidazolyl, pyrazolyl, oxazolyl, isoxazolyl, thiazolyl, isothiazolyl, thiadiazolyl, triazolyl, quinolyl, and isoquinolyl, or selected from the following groups: 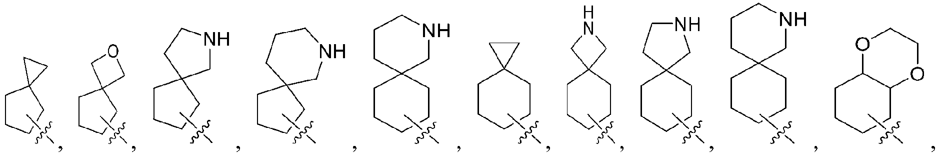 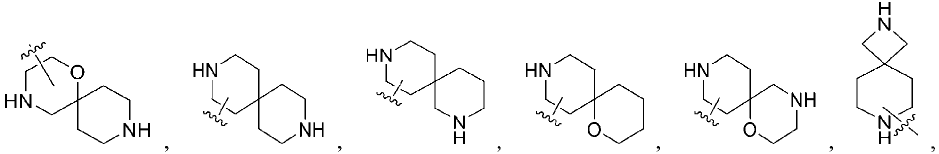 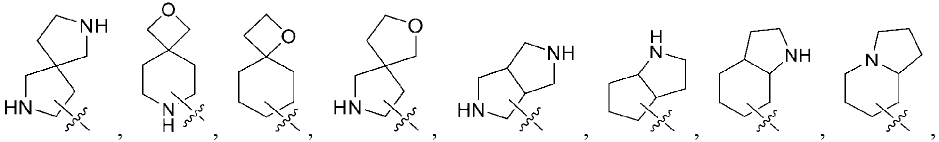 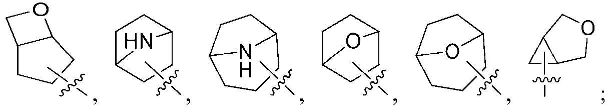
wherein, the methyl, ethyl, n-propyl, isopropyl, methoxy, ethoxy, n-propoxy, isopropoxy, NH₂-C(=O)-, CH₃NH-C(=O)-, CH₃CH₂NH-C(=O)-, CH₃-C(=O)-O-, CH₃CH₂-C(=O)-O-, CH₃-O-C(=O)-, CH₃CH₂-O-C(=O)-, cyclopropyl, cyclobutyl, cyclopentyl, cyclohexyl, azetidinyl, oxetanyl, tetrahydrofuranyl, pyrrolidinyl, piperidinyl, piperazinyl, morpholinyl, 1,4-dioxanyl, tetrahydro-2H-pyranyl, phenyl, naphthyl, pyridyl, pyrimidinyl, pyrazinyl, pyrrolyl, furyl, thienyl, imidazolyl, pyrazolyl, oxazolyl, isoxazolyl, thiazolyl, isothiazolyl, thiadiazolyl, triazolyl, quinolyl, isoquinolyl, 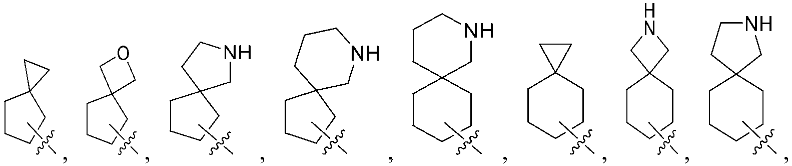 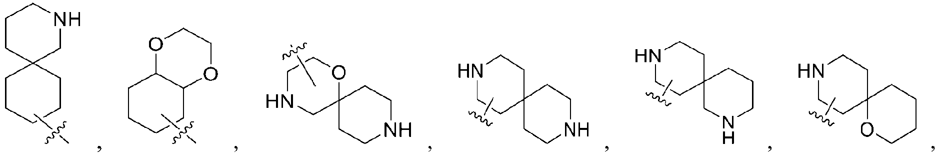 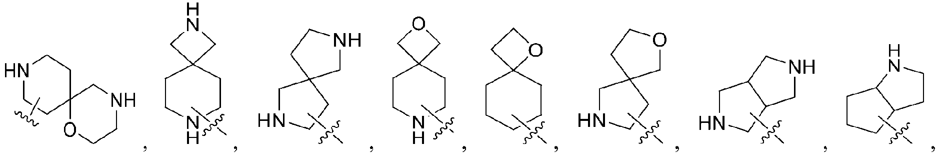 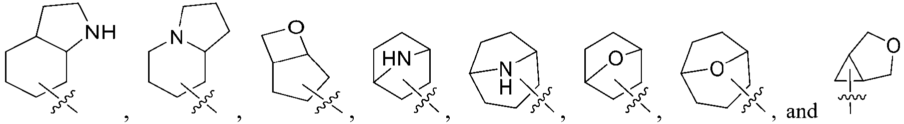 are each independently optionally further substituted with 1, 2, or 3 substituents selected from: H, deuterium, halogen, -OH, -NH₂, oxo, methyl, ethyl, n-propyl, isopropyl, 3-6 membered monocyclic heterocyclyl, -C(=O)-R^{c3}, -S(O)-R^{c3}, - S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), -P(=O)R^{c5}R^{c6}, and -CR^{c7}R^{c8}-CN.

In some embodiments, R¹ is selected from: 3-6 membered cycloalkyl, 3-6 membered monocyclic heterocyclyl, and 6-11 membered bicyclic heterocyclyl; wherein the 3-6 membered cycloalkyl, 3-6 membered monocyclic heterocyclyl, and 6-11 membered bicyclic heterocyclyl are substituted with 1 or 2 substituents selected from: - C(=O)-R^{c3}, -S(O)-R^{c3}, -S(O)₂-R^{c3}, -C(=O)-N(R^{c6})(R^{c5}), and -P(=O)R^{c5}R^{c6}; and the 3-6 membered cycloalkyl, 3-6 membered monocyclic heterocyclyl, and 6-11 membered bicyclic heterocyclyl are optionally substituted with 1, 2 or 3 substituents selected from: H, deuterium, halogen, -OH, oxo, C₁₋₆ alkyl, C₁₋₆ alkoxy, C₁₋₆ alkoxy-C₁₋₆ alkyl (preferably C₁₋₆ alkoxy-C₁₋₄ alkyl), -(CH₂)ₛ₋N(R^{c1})(R^{c2}), C₃₋₆ cycloalkyl, and 3-6 membered monocyclic heterocyclyl;
R^{c3} is selected from: C₁₋₆ alkyl and C₃₋₆ cycloalkyl, wherein the C₁₋₆ alkyl and C₃₋₆ cycloalkyl are further optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
R^{c5} and R^{c6} are selected from: H, deuterium, C₁₋₆ alkyl, and C₃₋₆ cycloalkyl; wherein the C₁₋₆ alkyl and C₃₋₆ cycloalkyl are further optionally substituted with 1, 2, or 3 substituents selected from: deuterium, halogen, -OH, - NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl).

In some embodiments, R¹ is selected from: cyclopentyl, cyclohexyl, azetidinyl, tetrahydropyrrolyl, piperidinyl, morpholinyl, piperazinyl, 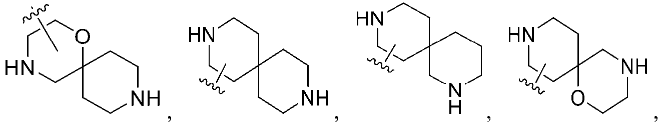 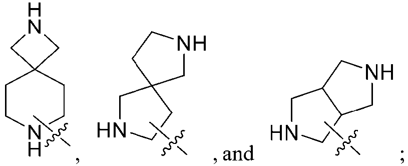 wherein the cyclopentyl, cyclohexyl, azetidinyl, tetrahydropyrrolyl, piperidinyl, morpholinyl, piperazinyl, 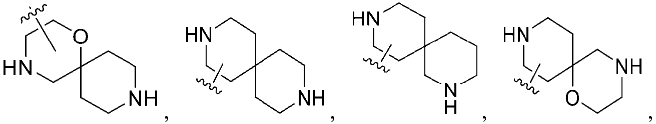 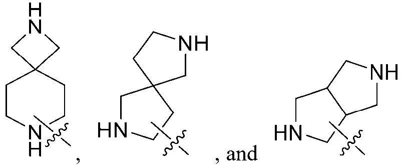 are substituted with 1 or 2 substituents selected from: -C(=O)-R^{c3}, -S(O)-R^{c3}, -S(O)₂-R^{c3}, -C(=O)-N(R^{c6})(R^{c5}), and -P(=O)R^{c5}R^{c6}, and the cyclopentyl, cyclohexyl, azetidinyl, tetrahydropyrrolyl, piperidinyl, morpholinyl, piperazinyl, 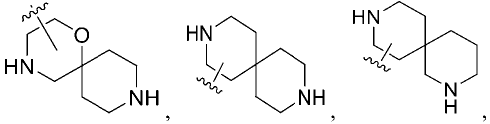 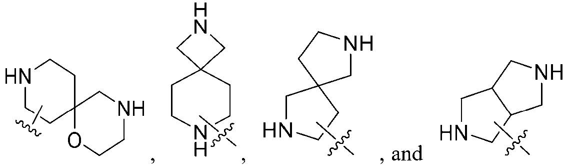 are further optionally substituted with 1, 2 or 3 substituents selected from: H, deuterium, halogen, -OH, oxo, C₁₋₆ alkyl, C₁₋₆ alkoxy, C₁₋₆ alkoxy-C₁₋₆ alkyl (preferably C₁₋₆ alkoxy-C₁₋₄ alkyl), -(CH₂)ₛ-N(R^{c1})(R^{c2}), C₃₋₆ cycloalkyl, and 3-6 membered monocyclic heterocyclyl.

In some embodiments, R¹ is selected from: tetrahydropyrrolyl, piperidinyl, morpholinyl, piperazinyl, 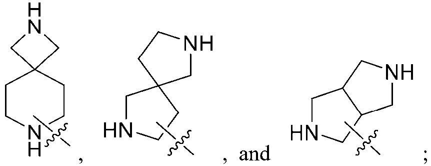 the tetrahydropyrrolyl, piperidinyl, morpholinyl, piperazinyl, 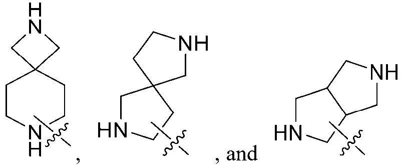 are substituted with 1 or 2 substituents selected from: -C(=O)-R^{c3}, -S(O)-R^{c3}, -S(O)₂-R^{c3}, -C(=O)-N(R^{c6})(R^{c5}), and -P(=O)R^{c5}R^{c6}, and the tetrahydropyrrolyl, piperidinyl, morpholinyl, and piperazinyl are further optionally substituted with 1, 2 or 3 substituents selected from: H, deuterium, halogen, -OH, oxo, C₁₋₆ alkyl, C₁₋₆ alkoxy, -(CH₂)ₛ-N(R^{c1})(R^{c2}), C₃₋₆ cycloalkyl, C₁₋₆ alkoxy-C₁₋₆ alkyl (preferably C₁₋₆ alkoxy-C₁₋₄ alkyl), and 3-6 membered monocyclic heterocyclyl.

In some embodiments, R¹ is selected from: tetrahydropyrrolyl, piperidinyl, morpholinyl, and piperazinyl; wherein the tetrahydropyrrolyl, piperidinyl, morpholinyl, and piperazinyl are substituted with 1 substituent selected from: -C(=O)-R^{c3}, -S(O)₂-R^{c3}, and -P(=O)R^{c5}R^{c6}, and the tetrahydropyrrolyl, piperidinyl, morpholinyl, and piperazinyl are further optionally substituted with 1 or 2 substituents selected from: deuterium, halogen, -OH, C₁₋₆ alkyl, C₁₋₆ alkoxy, and C₁₋₆ alkoxy-C₁₋₆ alkyl (preferably C₁₋₆ alkoxy-C₁₋₄ alkyl).

In some embodiments, R¹ is selected from:

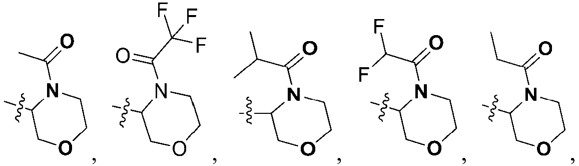

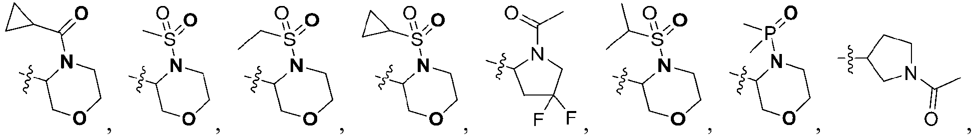

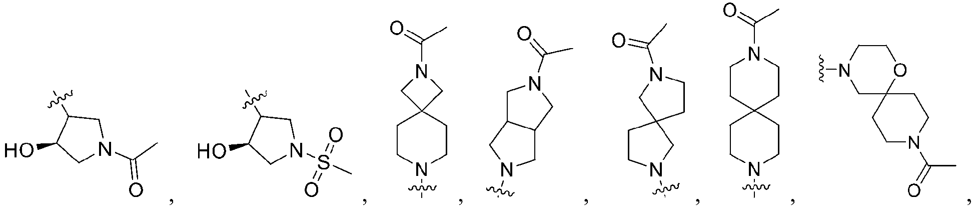

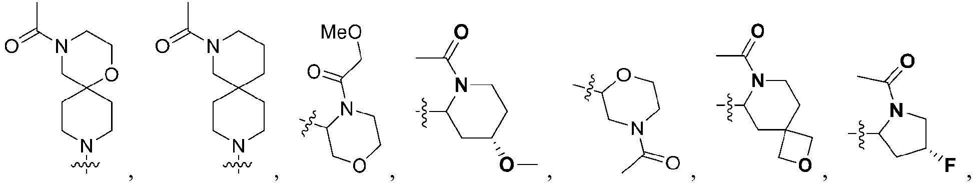

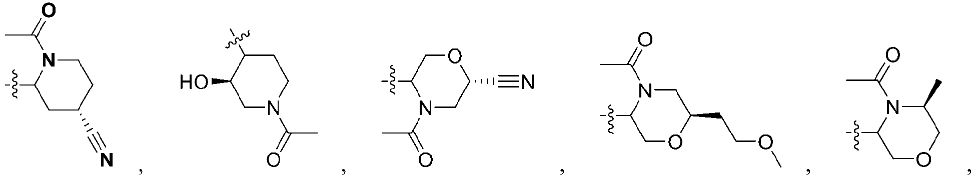

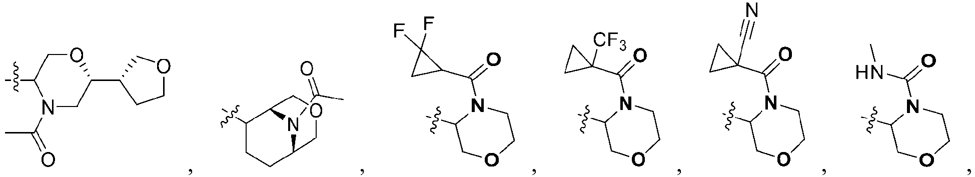

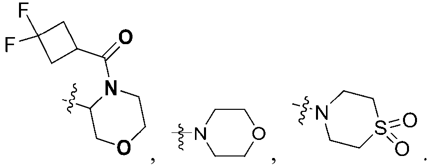

In some embodiments, the compound has the structure represented by Formula II, 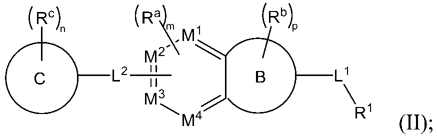 wherein, ring B, ring C, L¹, L², R^{a}, R^{b}, R^{c}, n, m, p, R¹, M¹, M², M³, and M⁴ have the meanings as defined in the present invention.

In some embodiments, the compound has the structure represented by Formula III, 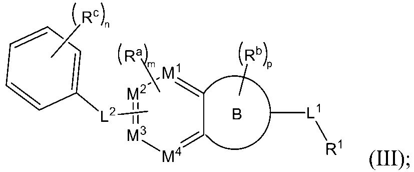 wherein, ring B, L¹, L², R^{a}, R^{b}, R^{c}, n, m, p, R¹, M¹, M², M³, and M⁴ have the meanings as defined in the present invention.

In some embodiments, the compound has the structure represented by Formula IV-1, IV-2, IV-3, IV-4, IV-5, IV-6, IV-7, IV-8, IV-9 or IV-10, 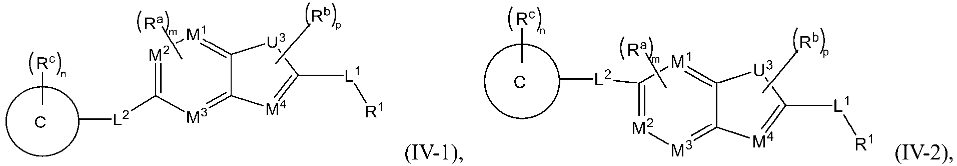 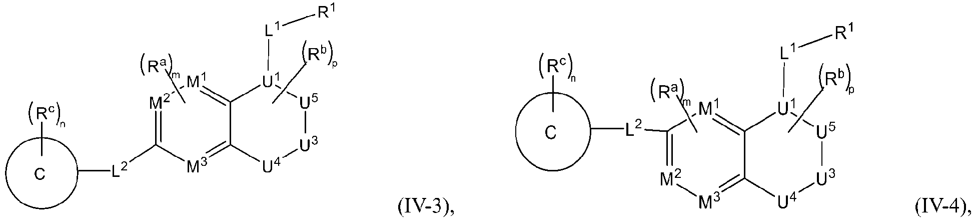 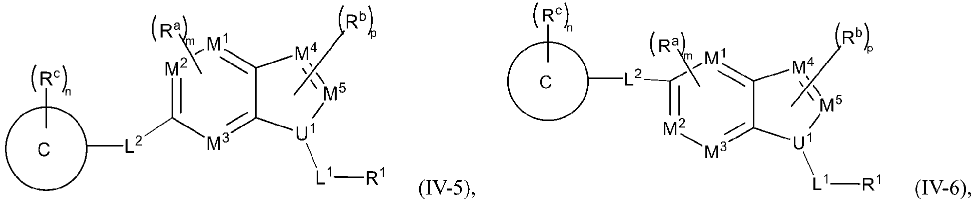 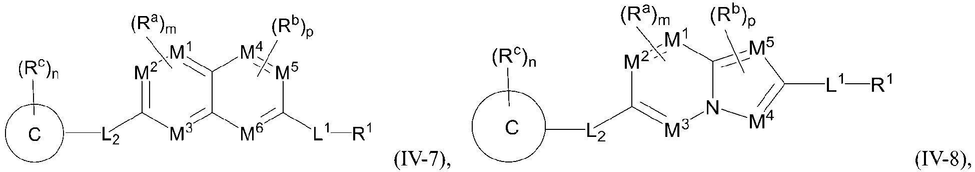 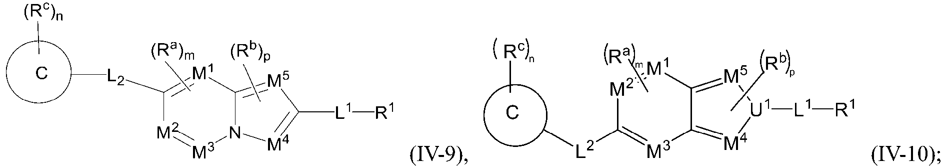 wherein, ring C, L¹, L², R^{a}, R^{b}, R^{c}, n, m, p, R¹, M¹, M², M³, M⁴, M⁵, M⁶, U¹, U³, U⁴, and U⁵ have the meanings as defined in the present invention.

In some embodiments, the compound has the structure represented by Formula IV-1.

In some embodiments, the compound has the structure represented by Formula IV-2.

In some embodiments, the compound has the structure represented by Formula IV-3.

In some embodiments, the compound has the structure represented by Formula IV-4.

In some embodiments, the compound has the structure represented by Formula IV-5.

In some embodiments, the compound has the structure represented by Formula IV-6.

In some embodiments, the compound has the structure represented by Formula IV-7.

In some embodiments, the compound has the structure represented by Formula IV-8.

In some embodiments, the compound has the structure represented by Formula IV-9.

In some embodiments, the compound has the structure represented by Formula IV-10.

In some embodiments, the compound has the structure represented by Formula V-1, V-2, V-3, V-4, V-5, V-6, V-7, V-8, V-9 or V-10, 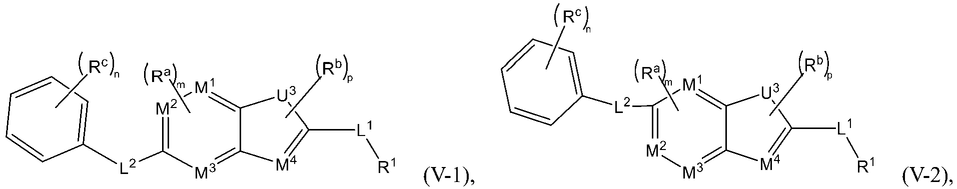 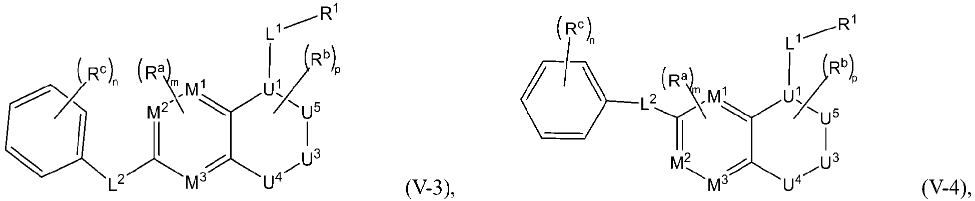 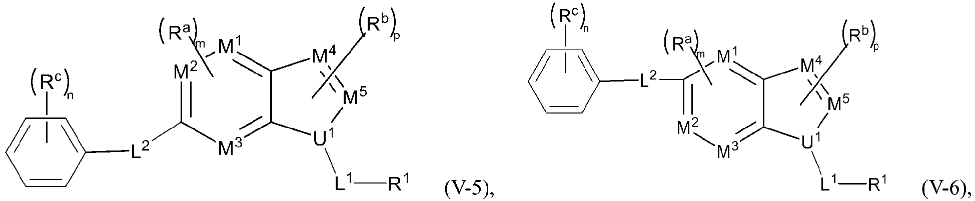 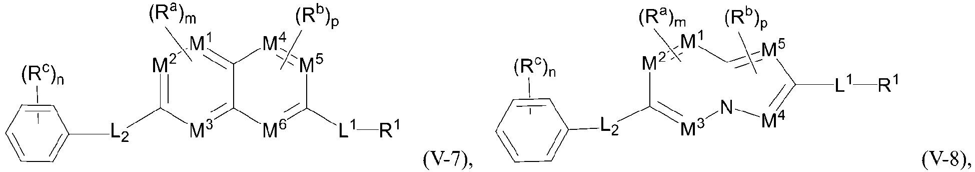 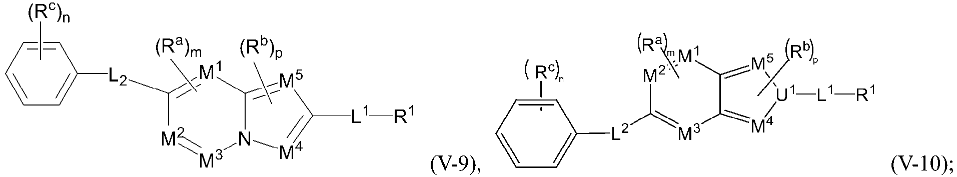 wherein, L¹, L², R^{a}, R^{b}, R^{c}, n, m, p, R¹, M¹, M², M³, M⁴, M⁵, M⁶, U¹, U³, U⁴, and U⁵ have the meanings as defined in the present invention.

In some embodiments, the compound has the structure represented by Formula V-1.

In some embodiments, the compound has the structure represented by Formula V-2.

In some embodiments, the compound has the structure represented by Formula V-3.

In some embodiments, the compound has the structure represented by Formula V-4.

In some embodiments, the compound has the structure represented by Formula V-5.

In some embodiments, the compound has the structure represented by Formula V-6.

In some embodiments, the compound has the structure represented by Formula V-7.

In some embodiments, the compound has the structure represented by Formula V-8.

In some embodiments, the compound has the structure represented by Formula V-9.

In some embodiments, the compound has the structure represented by Formula V-10.

In some embodiments, the compound has the structure represented by Formula VI-1, VI-2, VI-3, VI-4, VI-5, VI-6, VI-7, VI-8, VI-9 or VI-10, 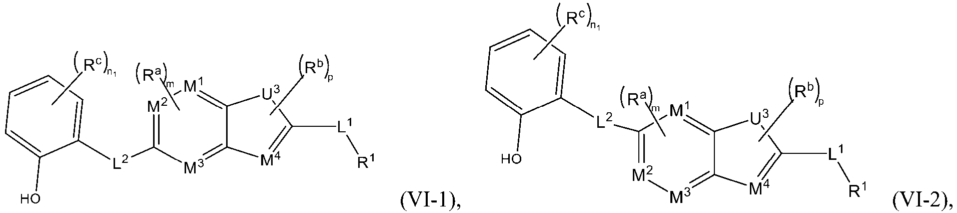 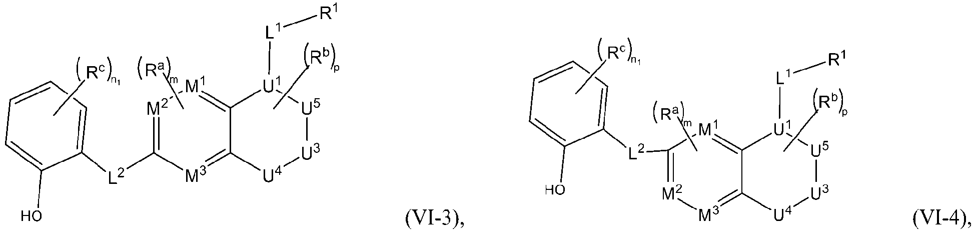 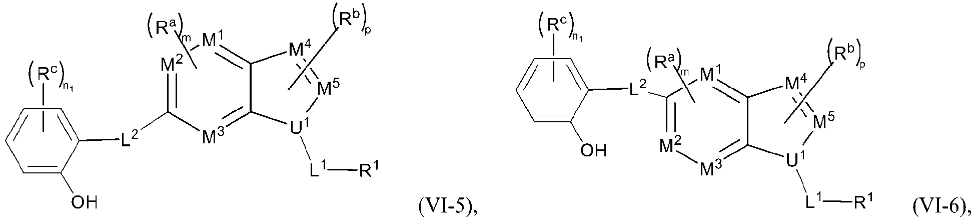 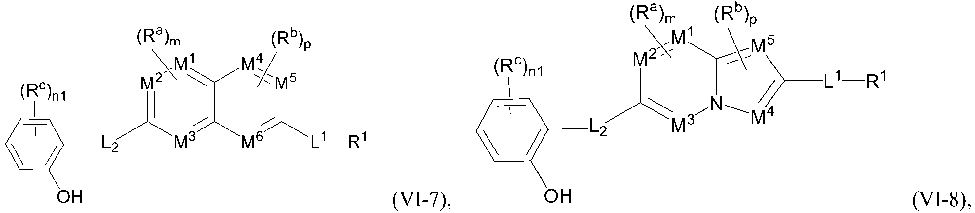 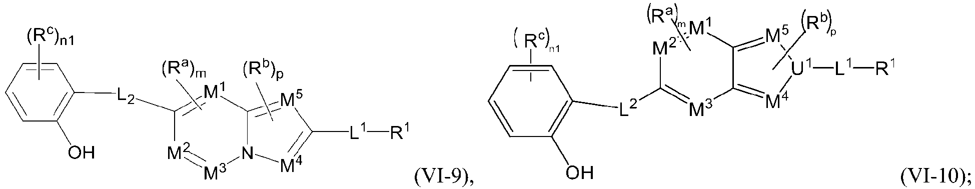 wherein, n₁ is 0, 1, 2 or 3; L¹, L², R^{a}, R^{b}, R^{c}, m, p, R¹, M¹, M², M³, M⁴, M⁵, M⁶, U¹, U³, U⁴, and U⁵ have the meanings as defined in the present invention.

In some embodiments, the compound has the structure represented by Formula VI-1.

In some embodiments, the compound has the structure represented by Formula VI-2.

In some embodiments, the compound has the structure represented by Formula VI-3.

In some embodiments, the compound has the structure represented by Formula VI-4.

In some embodiments, the compound has the structure represented by Formula VI-5.

In some embodiments, the compound has the structure represented by Formula VI-6.

In some embodiments, the compound has the structure represented by Formula VI-7.

In some embodiments, the compound has the structure represented by Formula VI-8.

In some embodiments, the compound has the structure represented by Formula VI-9.

In some embodiments, the compound has the structure represented by Formula VI-10.

In some embodiments, the compound has the structure represented by Formula VII-1, VII-2, VII-3, VII-4, VII-5, VII-6, VII-7, VII-8, VII-9 or VII-10, 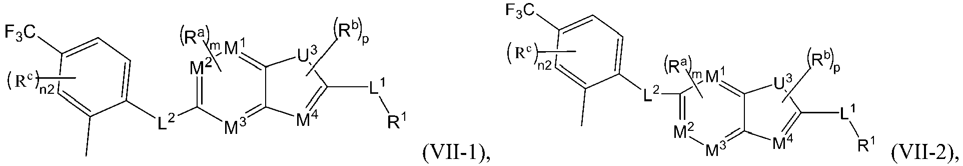 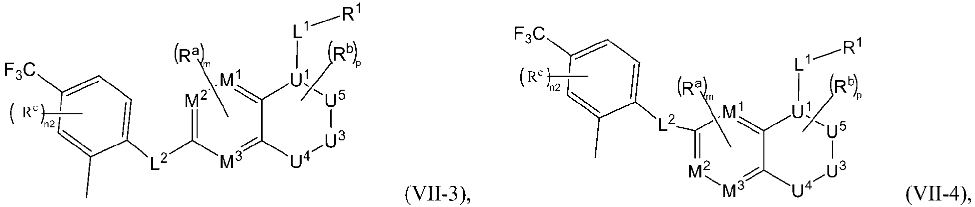 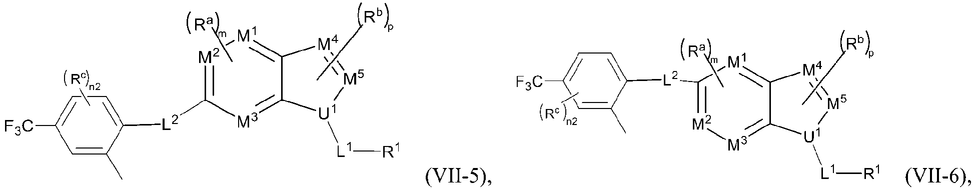 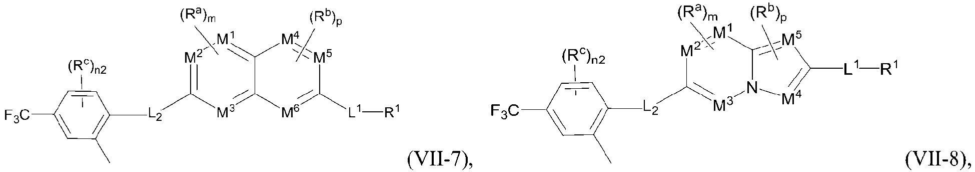 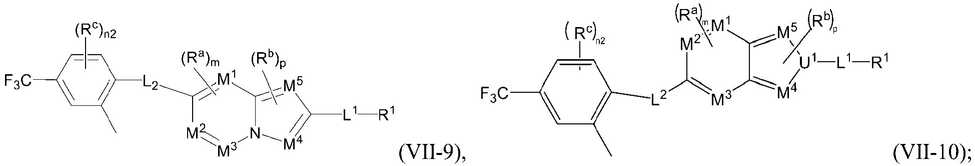 wherein, n₂ is 0, 1 or 2; L¹, L², R^{a}, R^{b}, R^{c}, m, p, R¹, M¹, M², M³, M⁴, U¹, U³, U⁴, and U⁵ have the meaning as defined in the present invention.

In some embodiments, the compound has the structure represented by Formula VII-1.

In some embodiments, the compound has the structure represented by Formula VII-2.

In some embodiments, the compound has the structure represented by Formula VII-3.

In some embodiments, the compound has the structure represented by Formula VII-4.

In some embodiments, the compound has the structure represented by Formula VII-5.

In some embodiments, the compound has the structure represented by Formula VII-6.

In some embodiments, the compound has the structure represented by Formula VII-7.

In some embodiments, the compound has the structure represented by Formula VII-8.

In some embodiments, the compound has the structure represented by Formula VII-9.

In some embodiments, the compound has the structure represented by Formula VII-10.

In some embodiments, the compound has the structure represented by Formula VIII-1, VIII-2, VIII-3, VIII-4, VIII-5, VIII-6, VIII-7, VIII-8, VIII-9, VIII-10, VIII-11 or VIII-12, 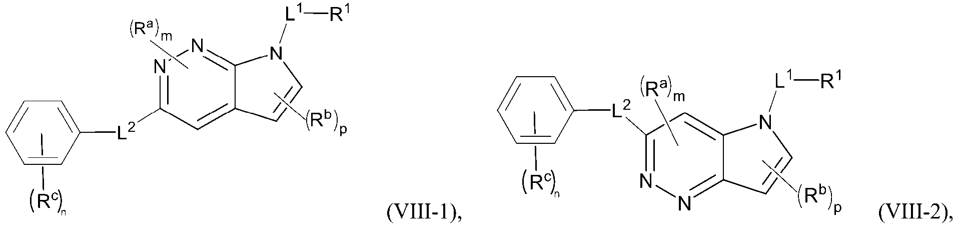 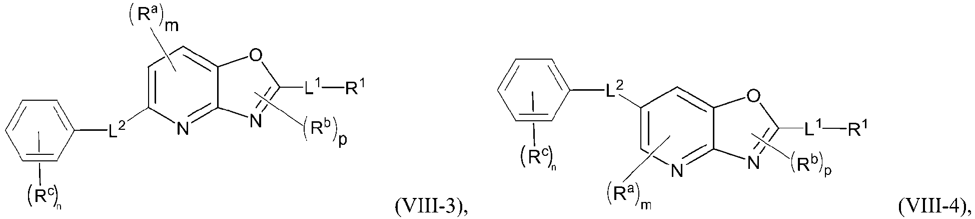 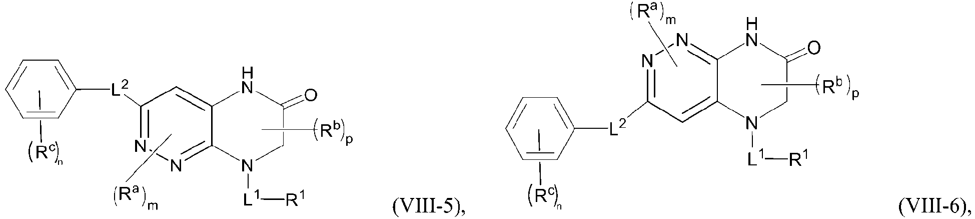 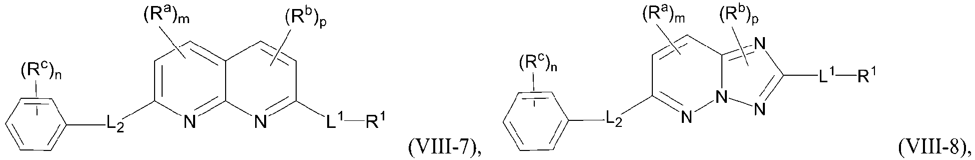 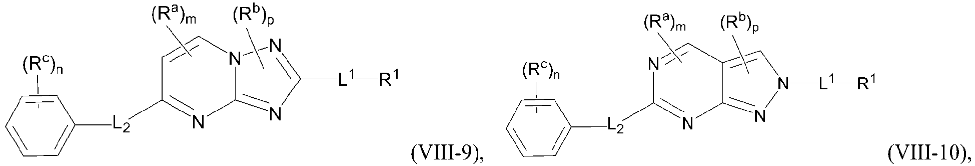 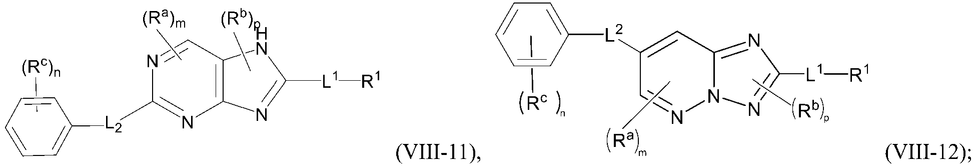 wherein, R^{a}, R^{b}, R^{c}, m, n, p, L¹, L², and R¹ have the meanings as defined in the present invention.

In some embodiments, the compound has the structure represented by Formula VIII-1.

In some embodiments, the compound has the structure represented by Formula VIII-2.

In some embodiments, the compound has the structure represented by Formula VIII-3.

In some embodiments, the compound has the structure represented by Formula VIII-4.

In some embodiments, the compound has the structure represented by Formula VIII-5.

In some embodiments, the compound has the structure represented by Formula VIII-6.

In some embodiments, the compound has the structure represented by Formula VIII-7.

In some embodiments, the compound has the structure represented by Formula VIII-8.

In some embodiments, the compound has the structure represented by Formula VIII-9.

In some embodiments, the compound has the structure represented by Formula VIII-10.

In some embodiments, the compound has the structure represented by Formula VIII-11.

In some embodiments, the compound has the structure represented by Formula VIII-12.

In some embodiments, the compound has the structure represented by Formula IX-1, IX-2, IX-3, IX-4, IX-5 or IX-6, 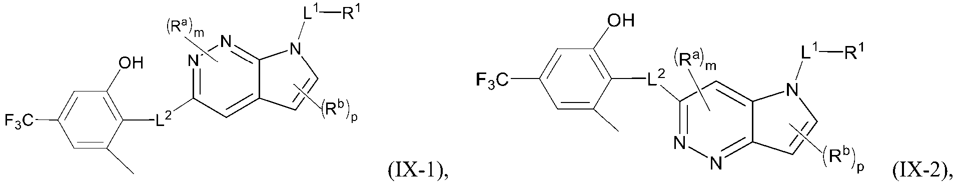 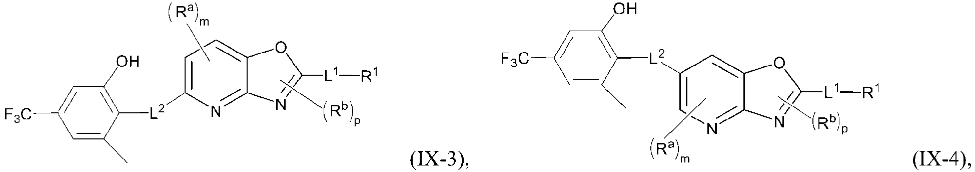 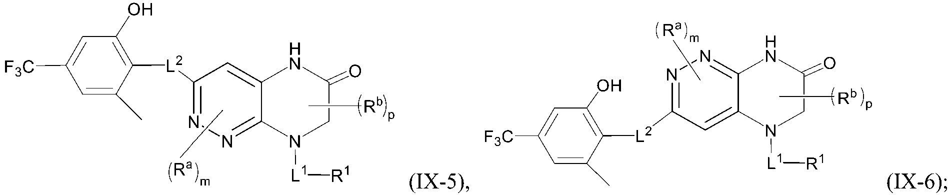 wherein, R^{a}, R^{b}, L¹, L², R¹, m, and p have the meanings as defined in the present invention.

In some embodiments, the compound has the structure represented by Formula IX-1.

In some embodiments, the compound has the structure represented by Formula IX-2.

In some embodiments, the compound has the structure represented by Formula IX-3.

In some embodiments, the compound has the structure represented by Formula IX-4.

In some embodiments, the compound has the structure represented by Formula IX-5.

In some embodiments, the compound has the structure represented by Formula IX-6.

In some embodiments, the compound has the structure represented by Formula VIII-1, or VIII-2: 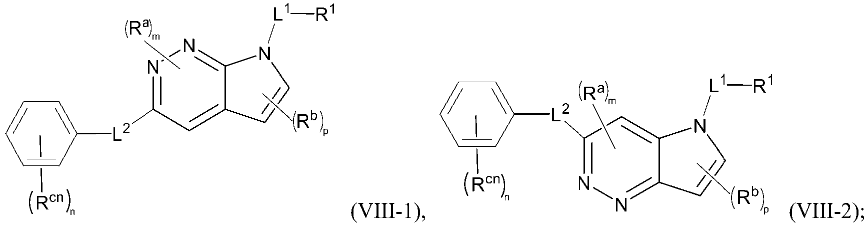
each R^{cn} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, -(CH₂)ₛ-N(R^{c1})(R^{c2}), 3-6 membered cycloalkyl, 3-6 membered heterocyclyl, phenyl, and 5-6 membered heteroaryl; wherein the 3-6 membered cycloalkyl, 3-6 membered heterocyclyl, phenyl, and 5-6 membered heteroaryl are optionally substituted with 1, 2 or 3 substituents selected from: deuterium, halogen, -OH, -NH₂, -CN, C₁₋₆ alkyl, C₁₋₆ haloalkyl, and C₁₋₆ alkoxy; or
two R^{cn} together with the C atom to which they are attached may form a 3-6 membered cycloalkyl, 4-7 membered heterocyclyl, 6-membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
preferably, each R^{cn} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, -(CH₂)ₛ-N(R^{c1})(R^{c2}), 3-6 membered cycloalkyl, 3-6 membered heterocyclyl, phenyl, phenyl substituted with one or more halogens, and phenyl substituted with one or more hydroxyl; or
two R^{cn} together with the C atom to which they are attached may form a 3-6 membered cycloalkyl, or 4-7 membered heterocyclyl;
each R^{a} and R^{b} are independently selected from: H, deuterium, halogen, -OH, -NH₂, -CN, C₁₋₆ alkyl, C₁₋₆ haloalkyl, C₁₋₆ alkoxy, C₁₋₆ alkoxy-C₁₋₆ alkyl, C₁₋₆ haloalkoxy, hydroxyl-C₁₋₆ alkyl, -NH(C₁₋₆ alkyl), -N(C₁₋₆ alkyl)(C₁₋₆ alkyl), -C(=O)-(C₁₋₆ alkyl), and 3-6 membered heterocyclyl optionally substituted by hydroxyl;
preferably, each R^{a} and R^{b} are independently selected from: H, deuterium, halogen, -OH, -NH₂, -CN, C₁₋₆ alkyl, C₁₋₆ haloalkyl, C₁₋₆ alkoxy, -NH(C₁₋₆ alkyl), -N(C₁₋₆ alkyl)(C₁₋₆ alkyl), and -C(=O)-(C₁₋₆ alkyl);
L¹ is selected from: bond, C₃₋₆ cycloalkylene, -NR²-, -O-, -S-, -C(=O)-, -(CR³R⁴)ₐ-, -NR²-(CR³R⁴)ₐ-, - (CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, - (CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, - NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, -C(=O)-(CR³R⁴)ₐ-NR²-, -NR²-(CR³R⁴)ₐ-C(=O)-NR⁵-, -NR²-C(=O)-(CR³R⁴)ₐ-NR⁵-, -C(=S)-NR²-, and -NR²-C(=S)-;
L² is selected from: bond, -NR⁶-, -O-, -S-, -NR⁶-(CR⁷R⁸)_{b}-, -(CR⁷R⁸)_{b}-NR⁶-, -O-(CR⁷R⁸)_{b}-, -(CR⁷R⁸)_{b}-O-, - S-(CR⁷R⁸)_{b}-, and -(CR⁷R⁸)_{b}-S-;
each R², R⁵, R⁶, R⁹ and R¹⁰ are each independently selected from: H, deuterium, and C₁₋₆ alkyl;
each R³, R⁴, R⁷, and R⁸ are each independently selected from: H, deuterium, halogen, oxo, -OH, -NH₂, and C₁₋₆ alkyl;
R¹ is selected from: H, -OH, -NH₂, -COOH, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the -NH₂, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: H, deuterium, halogen, -OH, oxo, -CN, C₁₋₆ alkyl, hydroxyl C₁₋₆ alkyl, C₁₋₆ haloalkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, 5-6 membered heteroaryl, -C(=O)-R^{c3}, -S(O)-R^{c3}, -S(O)₂-R^{c3}, -C(=O)O-R^{c3}, -N(R^{c4})-C(=O)-R^{c3}, -C(=O)-N(R^{c6})(R^{c5}), -CH₂-C(=O)-N(R^{c4})(R^{c4}), and -N(R^{c4})-C(=O)-N(R^{c6})(R^{c5});
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: H, deuterium, C₁₋₆ alkyl, C₁₋₆ haloalkyl, and C₃₋₆ cycloalkyl, wherein the C₃₋₆ cycloalkyl is further optionally substituted with 1, 2, or 3 substituents selected from: deuterium, -OH, halogen, and C₁₋₆ alkyl;
R^{c4} is selected from: H, deuterium, and C₁₋₆ alkyl;
R^{c5} and R^{c6} are selected from: H, deuterium, C₁₋₆ alkyl, C₁₋₆ haloalkyl, and C₃₋₆ cycloalkyl, or R^{c5}, R^{c6} together with the N atom to which they are attached form a 4-6 membered heterocyclyl; wherein the C₃₋₆ cycloalkyl and 4-6 membered heterocyclyl are further optionally substituted with 1, 2 or 3 substituents selected from: deuterium, - OH, halogen, and C₁₋₆ alkyl;
R¹¹ is selected from: H, deuterium, C₁₋₆ alkyl, and 3-6 monocyclic heterocyclyl;
each a and b are each independently 1, 2, 3, or 4;
each m is 0 or 1;
each p is 0, 1 or 2;
each s and n are each independently selected from: 0, 1, 2, 3, and 4;
provided that both 1) and 2) below are met:
   1) 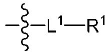 is not H, methyl, ethyl, CHF₂ or Boc;
   2) the compound is not

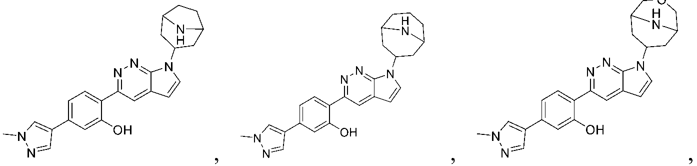

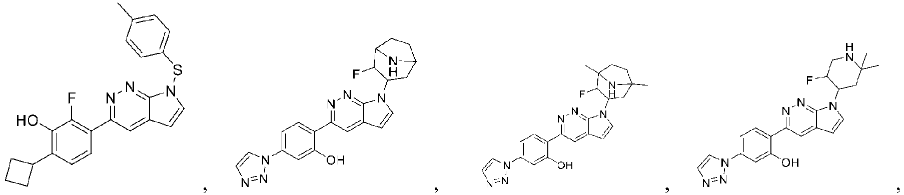

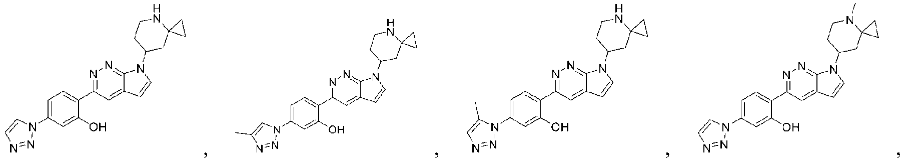

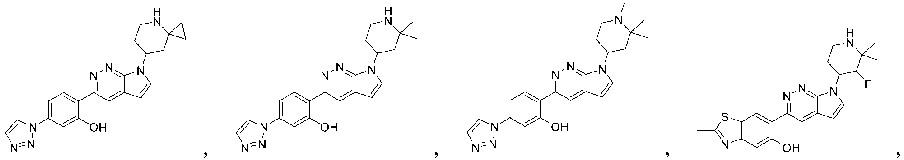

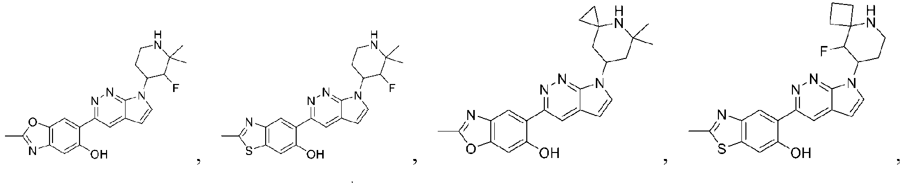

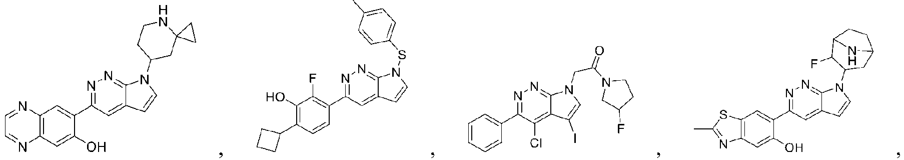

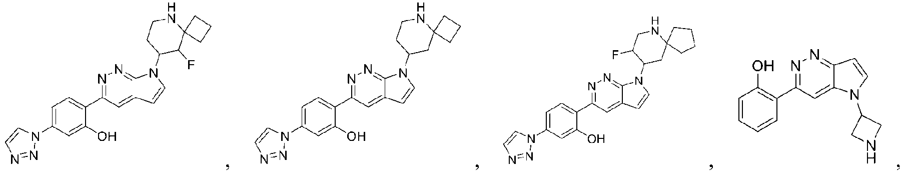

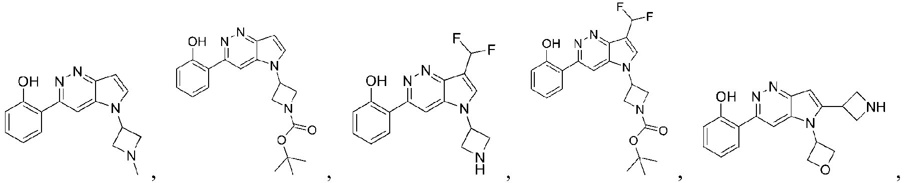

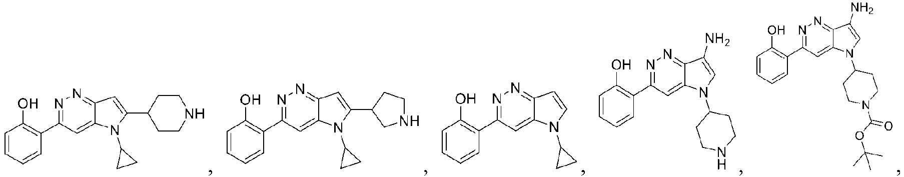

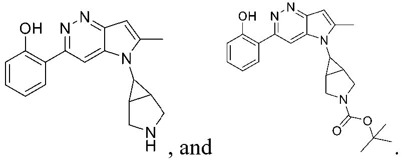

In some embodiments, the compound has the structure represented by Formula VIII-1,

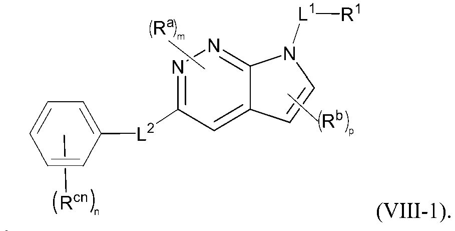

In some embodiments, L² is bond.

In some embodiments, R^{a} and R^{b} are each independently selected from: H, deuterium, F, Cl, Br, -CN, methyl, ethyl, trifluoromethyl, methoxy, dimethylamino, and acetyl; preferably, R^{a} and R^{b} are each independently selected from: H, deuterium, F, Cl, Br, methyl, and ethyl; more preferably, R^{a} and R^{b} are each independently selected from: H, deuterium, and methyl.

In some embodiments, each R^{cn} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, hydroxyl C₁₋₄ alkyl, C₁₋₄ alkoxy-C₁₋₂ alkyl, -(CH₂)ₛ-N(R^{c1})(R^{c2}), 3-6 membered cycloalkyl, 3-6 membered heterocyclyl, and phenyl; wherein the 3-6 membered cycloalkyl, 3-6 membered heterocyclyl, and phenyl are optionally substituted with 1, 2 or 3 substituents selected from: deuterium, halogen, -OH, -NH₂, -CN, C₁₋₆ alkyl, C₁₋₆ haloalkyl, and C₁₋₆ alkoxy; or two R^{cn} together with the C atom to which they are attached may form a 3-6 membered cycloalkyl, 4-7 membered heterocyclyl, or 5-6 membered heteroaryl.

In some embodiments, each R^{cn} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, C₁₋₄ hydroxyalkyl, C₁₋₄ alkoxy-C₁₋₂ alkyl, -(CH₂)ₛ-N(R^{c1})(R^{c2}), 3-6 membered cycloalkyl, 3-6 membered heterocyclyl, phenyl, phenyl substituted with one or more halogens, and phenyl substituted with one or more hydroxyl; or two R^{cn} together with the C atom to which they are attached may form a 3-6 membered cycloalkyl, or 4-7 membered heterocyclyl.

In some embodiments, each R^{cn} is independently selected from: F, Cl, Br, -OH, -NH₂, -CN, methoxy, ethoxy, isopropoxy, methylamino, ethylamino, isopropylamino, methyl, ethyl, n-propyl, isopropyl, trifluoromethyl, difluoromethyl, 2,2,2-trifluoroethyl, pentafluoroethyl, trifluoromethoxy, difluoromethoxy, hydroxymethyl, aminoethyl, methoxymethyl, methoxyethyl, ethoxymethyl, cyclopropyl, cyclobutyl, cyclopentyl, oxetanyl, phenyl, 4-chloro-phenyl, 2-chloro-4-fluoro-phenyl, 4-hydroxyphenyl, cyclobutylamino, and 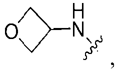 **or two** R^{cn} together with the C atom to which they are attached form a cyclopentyl or tetrahydrofuranyl.

In some embodiments, each R^{cn} is independently selected from: F, Cl, Br, -OH, methoxy, ethoxy, methyl, ethyl, trifluoromethyl, difluoromethyl, trifluoromethoxy, and difluoromethoxy.

In some embodiments, 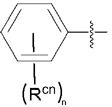 is selected from:

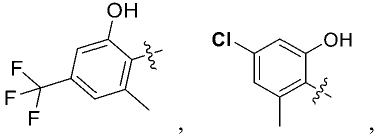

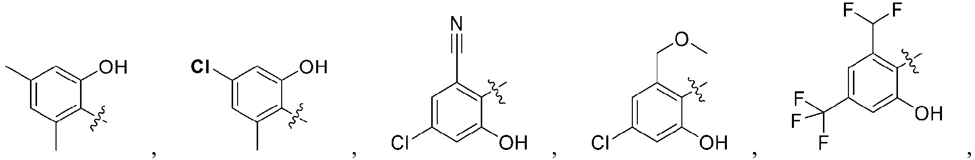

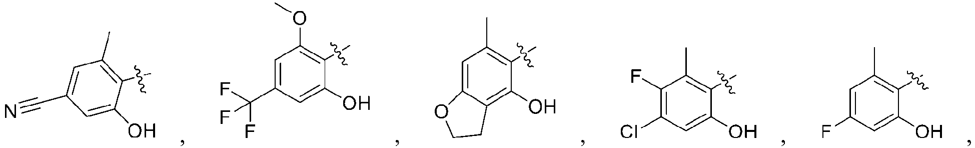

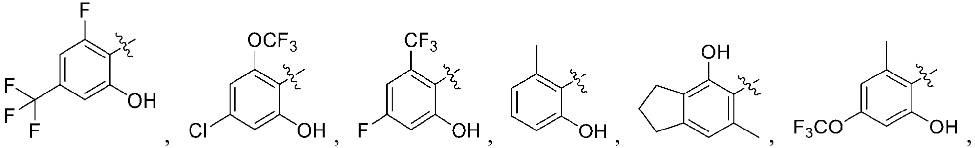

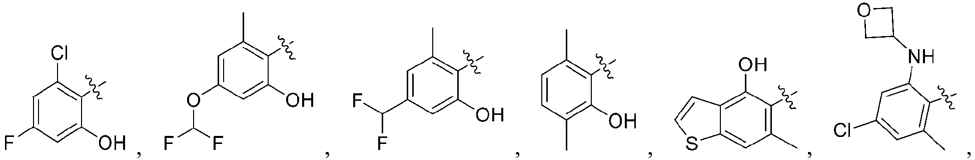

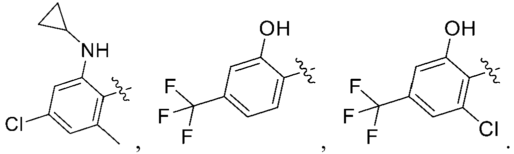

In some embodiments, 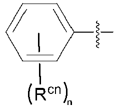 is selected from: 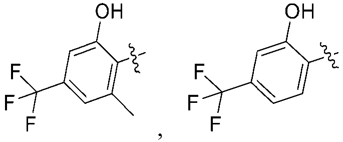 and

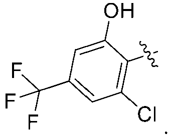

In some embodiments, L¹ is selected from: bond, C₃₋₆ cycloalkylene, -NR²-, -O-, -S-, -C(=O)-, -(CR³R⁴)ₐ-, - NR²-(CR³R⁴)ₐ-, -(CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, - (CR³R⁴)ₐ-C(=O)-, -(CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, - (CR³R⁴)ₐ-C(=O)-NR²-, -NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, and -C(=O)-(CR³R⁴)ₐ-NR²-.

In some embodiments, L¹ is selected from: bond, C₃₋₆ cycloalkylene, -C(=O)-, -(CR³R⁴)ₐ-, -(CR³R⁴)ₐ-(CR⁹=CR¹⁰)-, -(CR³R⁴)ₐ-C(=O)-NR²-, and -(CR³R⁴)ₐ-NR²-C(=O)-.

In some embodiments, each R² is each independently selected from: H, deuterium, and C₁₋₄ alkyl; each R³ and R⁴ are each independently selected from: H, deuterium, halogen, -OH, and C₁₋₄ alkyl.

In some embodiments, L¹ is selected from: bond, methylene, -C(=O)-, cyclopropylene, 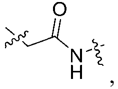 n-propylene, n-butylene, ethylene, 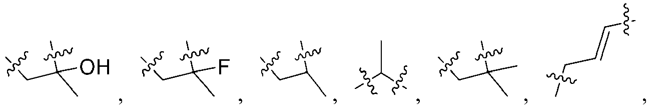 and

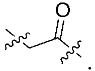

In some embodiments, L¹ is selected from: bond, methylene, -C(=O)-, and cyclopropylene.

In some embodiments, R¹ is selected from: C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the C₁₋₆ alkyl, C₁₋₆ alkoxy, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: H, deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -N(R^{c4})-C(=O)-R^{c3}, -C(=O)-N(R^{c6})(R^{c5}), and -N(R^{c4})-C(=O)-N(R^{c6})(R^{c5}).

In some embodiments, R¹ is selected from: C₁₋₃ alkyl, NHR¹¹-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the C₁₋₃ alkyl, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, - C(=O)-R^{c3}, -S(O)₂-R^{c3}, -C(=O)O-R^{c3}, -N(R^{c4})-C(=O)-R^{c3}, -C(=O)-N(R^{c6})(R^{c5}), and -N(R^{c4})-C(=O)-N(R^{c6})(R^{c5}).

In some embodiments, R¹ is selected from: methyl, ethyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, triazolyl, -C(=O)-NH₂, 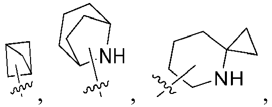 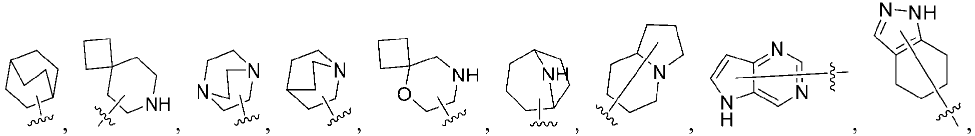 and 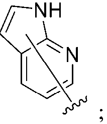 wherein the methyl, ethyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, triazolyl, 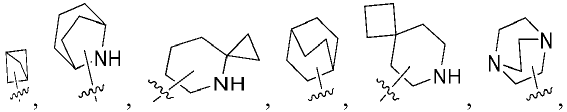 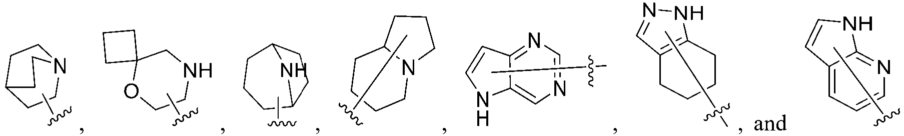 are each independently optionally further substituted with 1, 2, or 3 substituents selected from: deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, -C(=O)-R^{c3}, -S(O)₂-R^{c3}, -C(=O)O-R^{c3}, -N(R^{c4})-C(=O)-R^{c3}, -C(=O)-N(R^{c6})(R^{c5}), and -N(R^{c4})-C(=O)-N(R^{c6})(R^{c5}).

In some embodiments, R¹ is selected from: methyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, and triazolyl; wherein, the methyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, and triazolyl are each independently optionally further substituted with 1, 2, or 3 substituents selected from: deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, -C(=O)-R^{c3}, -S(O)₂-R^{c3}, -C(=O)O-R^{c3}, -N(R^{c4})-C(=O)-R^{c3}, -C(=O)-N(R^{c6})(R^{c5}), and -N(R^{c4})-C(=O)-N(R^{c6})(R^{c5}).

In some embodiments, -L¹-R¹ is selected from: 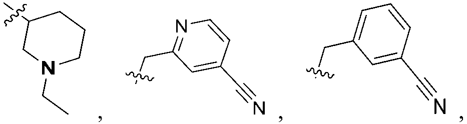 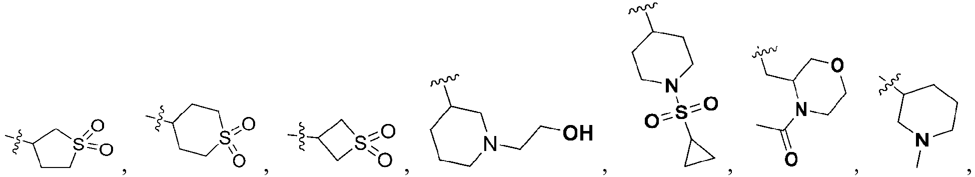 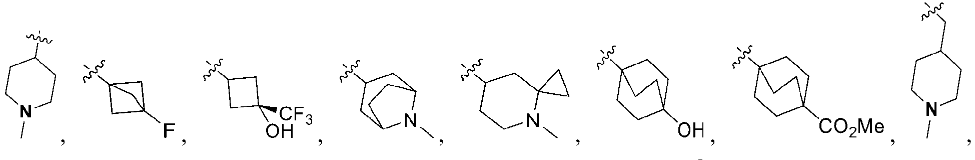 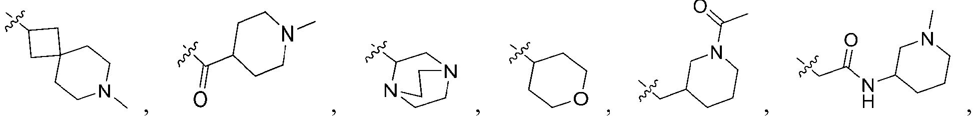 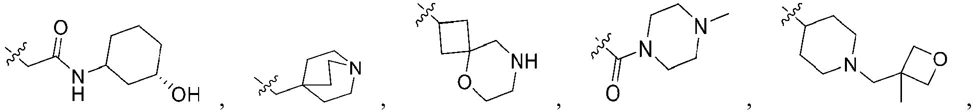 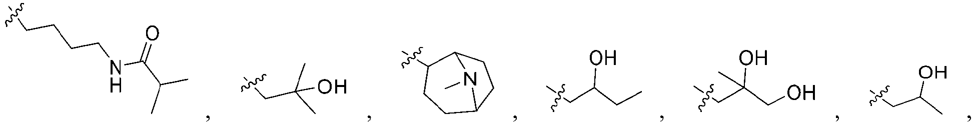 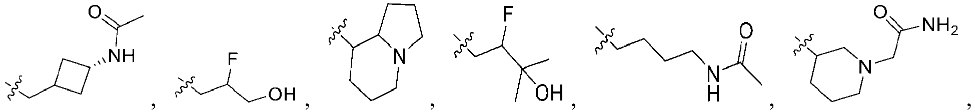 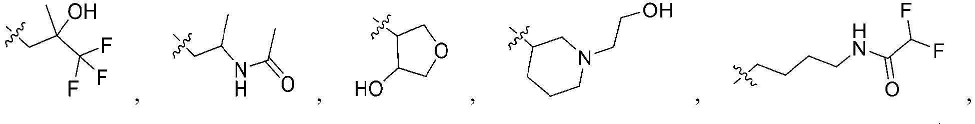 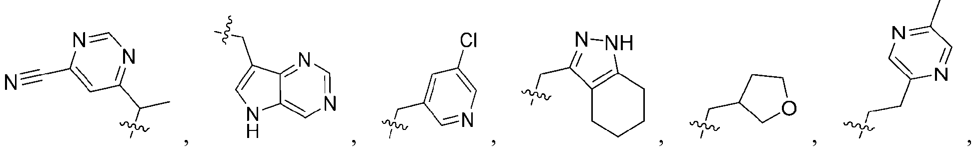 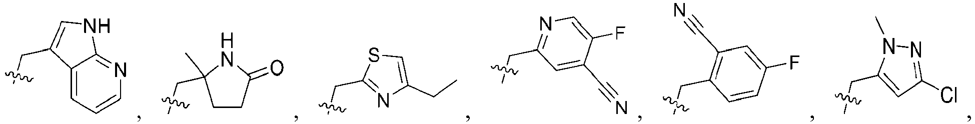 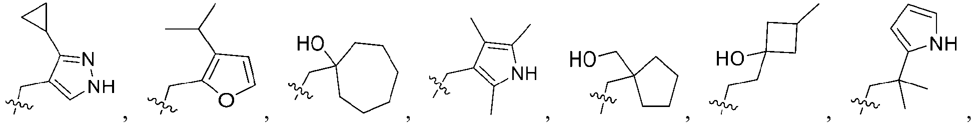 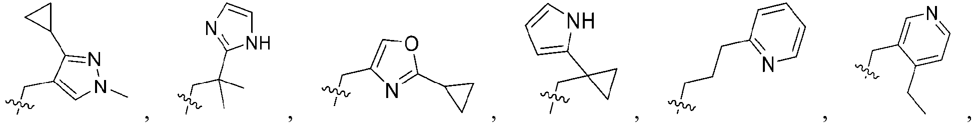 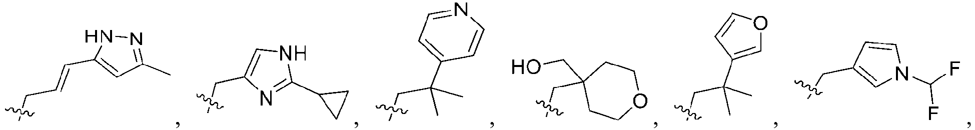 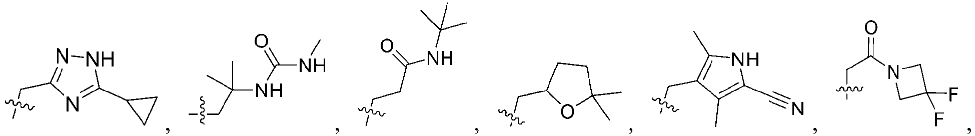 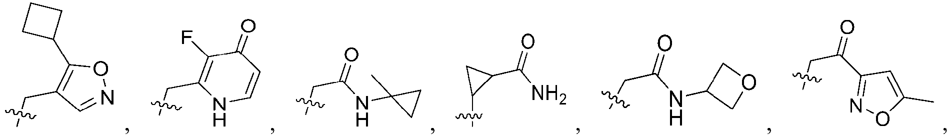 and

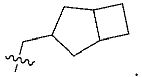

In some embodiments, -L¹-R¹ is selected from:

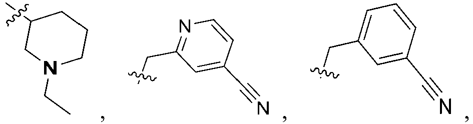

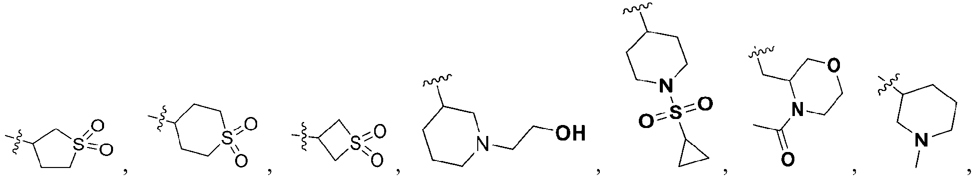

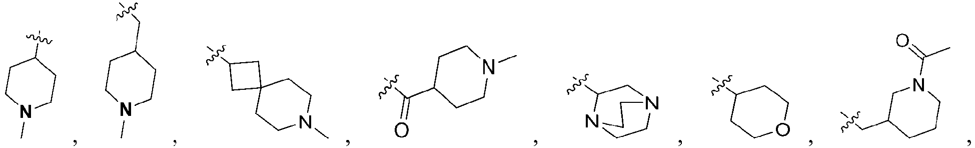

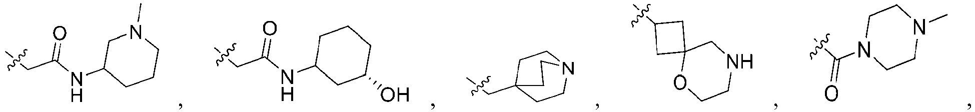

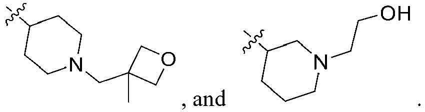

In some embodiments, -L¹-R¹ is selected from:

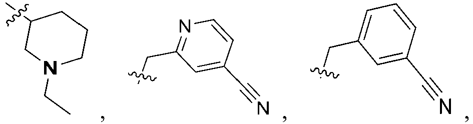

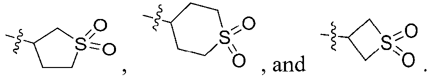

In some embodiments, the compound has the structure represented by Formula X-1 or X-2, 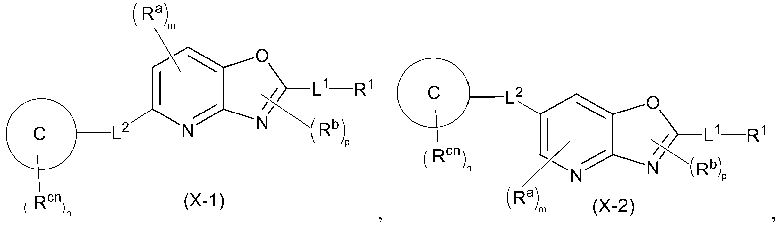
ring C selected from: 6-10 membered aryl, 5-10 membered heteroaryl, phenyl fused 4-7 membered cycloalkyl, phenyl fused 4-7 membered heterocyclyl, 5-6 membered heteroaryl fused 4-7 membered cycloalkyl, 5-6 membered heteroaryl fused 4-7 membered heterocyclyl, 4-7 membered cycloalkyl, and 4-7 membered heterocyclyl;
each R^{cn} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, R^{c1}S(O)₂-, -(CH₂)ₛ-N(R^{c1})(R^{c2}), 3-6 membered cycloalkyl, 3-6 membered heterocyclyl, phenyl, and 5-6 membered heteroaryl; wherein the 3-6 membered cycloalkyl, 3-6 membered heterocyclyl, phenyl, and 5-6 membered heteroaryl are optionally substituted with 1, 2 or 3 substituents selected from: deuterium, halogen, -OH, -NH₂, -CN, C₁₋₆ alkyl, C₁₋₆ haloalkyl, and C₁₋₆ alkoxy; or
two R^{cn} together with the C atom to which they are attached may form a 3-6 membered cycloalkyl, 4-7 membered heterocyclyl, 6-membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
each R^{a} and R^{b} are independently selected from: H, deuterium, halogen, -OH, -NH₂, -CN, C₁₋₆ alkyl, C₁₋₆ haloalkyl, C₁₋₆ alkoxy, C₁₋₆ alkoxy-C₁₋₆ alkyl, C₁₋₆ haloalkoxy, hydroxyl-C₁₋₆ alkyl, -NH(C₁₋₆ alkyl), -N(C₁₋₆ alkyl)(C₁₋₆ alkyl), -C(=O)-(C₁₋₆ alkyl), and 3-6 membered heterocycloalkyl optionally substituted by hydroxyl;
L¹ is selected from: bond, C₃₋₆ cycloalkylene, -NR²-, -O-, -S-, -C(=O)-, -(CR³R⁴)ₐ-, -NR²-(CR³R⁴)ₐ-, - (CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, - (CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, - NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, -C(=O)-(CR³R⁴)ₐ-NR²-, -NR²-(CR³R⁴)ₐ-C(=O)-NR⁵-, -NR²-C(=O)-(CR³R⁴)ₐ-NR⁵-, -C(=S)-NR²-, and -NR²-C(=S)-;
L² is selected from: bond, -NR⁶-, -O-, -S-, -NR⁶-(CR⁷R⁸)_{b}-, -(CR⁷R⁸)_{b}-NR⁶-, -O-(CR⁷R⁸)_{b}-, -(CR⁷R⁸)_{b}-O-, - S-(CR⁷R⁸)_{b}-, and -(CR⁷R⁸)_{b}-S-;
each R², R⁵, R⁶, R⁹ and R¹⁰ are each independently selected from: H, deuterium, and C₁₋₆ alkyl;
each R³, R⁴, R⁷, and R⁸ are each independently selected from: H, deuterium, halogen, oxo, -OH, -NH₂, C₁₋₆ alkyl;
R¹ is selected from: 3-10 membered cycloalkyl, 3-6 membered monocyclic heterocyclyl, 6-11 membered bicyclic heterocyclyl, 6-10 membered aryl, and 5-10 membered heteroaryl; wherein the 3-10 membered cycloalkyl, 3-6 membered monocyclic heterocyclyl, 6-11 membered bicyclic heterocyclyl, 6-10 membered aryl, and 5-10 membered heteroaryl are substituted with 1 or 2 substituents selected from: -C(=O)-R^{c3}, -S(O)-R^{c3}, -S(O)₂-R^{c3}, - C(=O)-N(R^{c5})(R^{c6}), and -P(=O)R^{c5}R^{c6}; and the 3-10 membered cycloalkyl, 3-6 membered monocyclic heterocyclyl, 6-11 membered bicyclic heterocyclyl, 6-10 membered aryl, and 5-10 membered heteroaryl are each optionally further substituted with 1, 2, or 3 substituents selected from: H, deuterium, halogen, -OH, oxo, -CN, C₁₋₆ alkyl, hydroxyl C₁₋₆ alkyl, C₁₋₆ haloalkyl, C₁₋₆ alkoxy, C₁₋₆ alkoxy-C₁₋₆ alkyl, -(CH₂)ₛ-N(R^{c1})(R^{c2}), C₃₋₆ cycloalkyl, and 3-6 membered monocyclic heterocyclyl;
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: C₁₋₆ alkyl, and C₃₋₆ cycloalkyl, wherein the C₁₋₆ alkyl and C₃₋₆ cycloalkyl are further optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
R^{c5} and R^{c6} are selected from: H, deuterium, C₁₋₆ alkyl, and C₃₋₆ cycloalkyl, or R^{c5}, R^{c6} together with the N atom to which they are attached form a 4-6 membered heterocyclyl; wherein the C₁₋₆ alkyl, C₃₋₆ cycloalkyl, and 4-6 membered heterocyclyl are optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
each a and b are each independently 1, 2, 3, or 4;
each m is 0 or 1;
each p is 0, 1 or 2;
each s and n are each independently selected from: 0, 1, 2, 3, and 4;
provided that, the compound is not

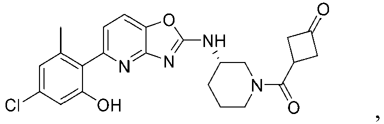

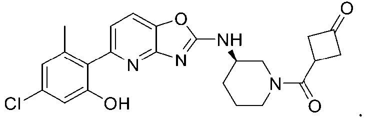

In some embodiments, the compound has the structure represented by Formula VIII-3 or Formula VIII-4, 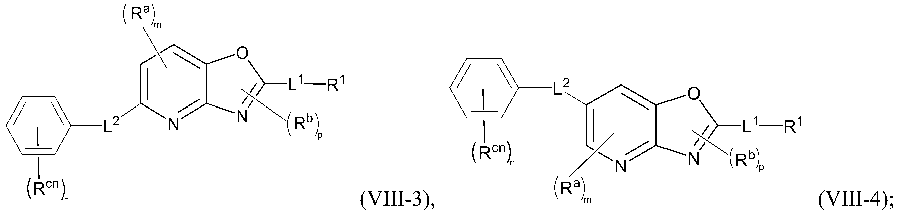
each R^{cn} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, R^{c1}S(O)₂-, -(CH₂)ₛ-N(R^{c1})(R^{c2}), 3-6 membered cycloalkyl, 3-6 membered heterocyclyl, phenyl, and 5-6 membered heteroaryl; wherein the 3-6 membered cycloalkyl, 3-6 membered heterocyclyl, phenyl, and 5-6 membered heteroaryl are optionally substituted with 1, 2 or 3 substituents selected from: deuterium, halogen, -OH, -NH₂, -CN, C₁₋₆ alkyl, C₁₋₆ haloalkyl, and C₁₋₆ alkoxy; or
two R^{cn} together with the C atom to which they are attached may form a 3-6 membered cycloalkyl, 4-7 membered heterocyclyl, 6-membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
each R^{a} and R^{b} are independently selected from: H, deuterium, halogen, -OH, -NH₂, -CN, C₁₋₆ alkyl, C₁₋₆ haloalkyl, C₁₋₆ alkoxy, C₁₋₆ alkoxy-C₁₋₆ alkyl, C₁₋₆ haloalkoxy, hydroxyl-C₁₋₆ alkyl, -NH(C₁₋₆ alkyl), -N(C₁₋₆ alkyl)(C₁₋₆ alkyl), -C(=O)-(C₁₋₆ alkyl), and 3-6 membered heterocycloalkyl optionally substituted by hydroxyl;
L¹ is selected from: bond, C₃₋₆ cycloalkylene, -NR²-, -O-, -S-, -C(=O)-, -(CR³R⁴)ₐ-, -NR²-(CR³R⁴)ₐ-, - (CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, - (CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, - NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, -C(=O)-(CR³R⁴)ₐ-NR²-, -NR²-(CR³R⁴)ₐ-C(=O)-NR⁵-, -NR²-C(=O)-(CR³R⁴)ₐ-NR⁵-, -C(=S)-NR²-, and -NR²-C(=S)-;
L² is selected from: bond, -NR⁶-, -O-, -S-, -NR⁶-(CR⁷R⁸)_{b}-, -(CR⁷R⁸)_{b}-NR⁶-, -O-(CR⁷R⁸)_{b}-, -(CR⁷R⁸)_{b}-O-, - S-(CR⁷R⁸)_{b}-, and -(CR⁷R⁸)_{b}-S-;
each R², R⁵, R⁶, R⁹ and R¹⁰ are each independently selected from: H, deuterium, and C₁₋₆ alkyl;
each R³, R⁴, R⁷, and R⁸ are each independently selected from: H, deuterium, halogen, oxo, -OH, -NH₂, C₁₋₆ alkyl;
R¹ is selected from: 3-6 membered cycloalkyl, 3-6 membered monocyclic heterocyclyl, and 6-11 membered bicyclic heterocyclyl; wherein the 3-6 membered cycloalkyl, 3-6 membered monocyclic heterocyclyl, and 6-11 membered bicyclic heterocyclyl are substituted with 1 or 2 substituents selected from: -C(=O)-R^{c3}, -S(O)-R^{c3}, - S(O)₂-R^{c3}, -C(=O)-N(R^{c5})(R^{c6}), and -P(=O)R^{c5}R^{c6}; and the 3-6 membered cycloalkyl, 3-6 membered monocyclic heterocyclyl, and 6-11 membered bicyclic heterocyclyl are further optionally substituted with 1, 2, or 3 substituents selected from: H, deuterium, halogen, -OH, oxo, -CN, C₁₋₆ alkyl, C₁₋₆ haloalkyl, C₁₋₆ alkoxy, C₁₋₆ alkoxy-C₁₋₆ alkyl, -(CH₂)ₛ-N(R^{c1})(R^{c2}), C₃₋₆ cycloalkyl, and 3-6 membered monocyclic heterocyclyl;
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: C₁₋₆ alkyl, and C₃₋₆ cycloalkyl, wherein the C₁₋₆ alkyl and C₃₋₆ cycloalkyl are further optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
R^{c5} and R^{c6} are selected from: H, deuterium, C₁₋₆ alkyl, and C₃₋₆ cycloalkyl, or R^{c5} and R^{c6} together with the N atom to which they are attached form a 4-6 membered heterocyclyl; wherein the C₁₋₆ alkyl, C₃₋₆ cycloalkyl, and 4-6 membered heterocyclyl are further optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, - NH(C₁₋₆ alkyl), and -N(C₁₋₆ alkyl)(C₁₋₆ alkyl);
R¹¹ is selected from: H, deuterium, C₁₋₆ alkyl, and 3-6 membered monocyclic heterocyclyl;
each a and b are each independently 1, 2, 3, or 4;
each m is 0 or 1;
each p is 0, 1 or 2;
each s and n are each independently selected from: 0, 1, 2, 3, and 4.

In some embodiments, the compound has the structure represented by Formula VIII-3:

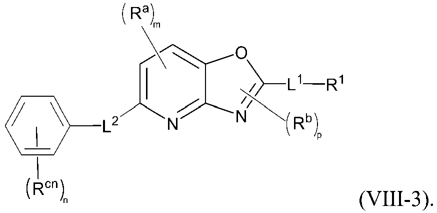

In some embodiments, L² is bond.

In some embodiments, R^{a} and R^{b} are each independently selected from: H, deuterium, F, Cl, Br, -CN, methyl, ethyl, trifluoromethyl, methoxy, 1-hydroxyethyl-1-yl, methylamino, dimethylamino, acetyl, and 3-hydroxyazetidinyl; preferably, R^{a} and R^{b} are each independently selected from: H, deuterium, F, Cl, Br, methyl, and ethyl; more preferably, R^{a} and R^{b} are each independently selected from: H, deuterium, and methyl.

In some embodiments, each R^{cn} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, hydroxyl C₁₋₄ alkyl, C₁₋₄ alkoxy-C₁₋₂ alkyl, R^{c1}S(O)₂-, -(CH₂)ₛ-N(R^{c1})(R^{c2}), 3-6 membered cycloalkyl, 3-6 membered heterocyclyl, phenyl and 5-6 membered heteroaryl; wherein the 3-6 membered cycloalkyl, 3-6 membered heterocyclyl, phenyl and 5-6 membered heteroaryl are optionally substituted with 1, 2, or 3 substituents selected from: deuterium, halogen, -OH, -NH₂, -CN, C₁₋₆ alkyl, C₁₋₆ haloalkyl, and C₁₋₆ alkoxy; or two R^{cn} together with the C atom to which they are attached may form a 3-6 membered cycloalkyl, 4-7 membered heterocyclyl, 6 membered aryl or 5-6 membered heteroaryl; wherein the 3-6 membered cycloalkyl, 4-7 membered heterocyclyl, 6 membered aryl or 5-6 membered heteroaryl may be optionally substituted with 1-3 substituents selected from: deuterium, halogen, -OH, -NH₂, -CN, C₁₋₆ alkoxy, C₁₋₆ alkyl, and C₁₋₆ haloalkyl.

In some embodiments, each R^{cn} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, hydroxyl C₁₋₄ alkyl, C₁₋₄ alkoxy-C₁₋₂ alkyl, R^{c1}S(O)₂-, -(CH₂)ₛ-N(R^{c1})(R^{c2}), 3-6 membered cycloalkyl, 3-6 membered heterocyclyl, phenyl, phenyl substituted with one or more halogens, and phenyl substituted with one or more hydroxyl; or two R^{cn} together with the C atom to which they are attached may form a 3-6 membered cycloalkyl, 4-7 membered heterocyclyl, 6 membered aryl or 5-6 membered heteroaryl; wherein the 3-6 membered cycloalkyl, 4-7 membered heterocyclyl, 6 membered aryl or 5-6 membered heteroaryl may be optionally substituted with 1-3 substituents selected from: deuterium, halogen, -OH, -NH₂, -CN, C₁₋₆ alkoxy, C₁₋₆ alkyl, and C₁₋₆ haloalkyl.

In some embodiments, each R^{cn} is independently selected from: F, Cl, Br, -OH, -NH₂, -CN, methoxy, ethoxy, isopropoxy, methylamino, ethylamino, isopropylamino, methyl, ethyl, n-propyl, isopropyl, n-butyl, isobutyl, trifluoromethyl, difluoromethyl, 2,2,2-trifluoroethyl, pentafluoroethyl, trifluoromethoxy, difluoromethoxy, hydroxymethyl, aminoethyl, methoxymethyl, methoxyethyl, ethoxymethyl, cyclopropyl, cyclobutyl, cyclopentyl, cyclohexyl, cyclopentenyl, cyclobutenyl, cyclohexenyl, oxetanyl, phenyl, 4-chloro-phenyl, 2-chloro-4-fluoro-phenyl, 4-hydroxyphenyl, 4-methyl-phenyl, 4-fluoro-phenyl, pyridyl, cyclobutylamino, and 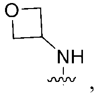 or two R^{cn} together with the C atom to which they are attached form a cyclopentenyl, 2,3-dihydrofuranyl, cyclobutenyl, thienyl, 1,4-dioxanyl, pyridyl, cyclopenta-1,3-dienyl, phenyl, or 1,4-dioxa-2-hexenyl.

In some embodiments, each R^{cn} is independently selected from: F, Cl, Br, -OH, -CN, methoxy, ethoxy, methyl, ethyl, trifluoromethyl, difluoromethyl, trifluoromethoxy, and difluoromethoxy.

In some embodiments, 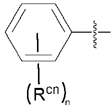 is selected from:

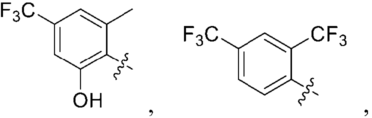

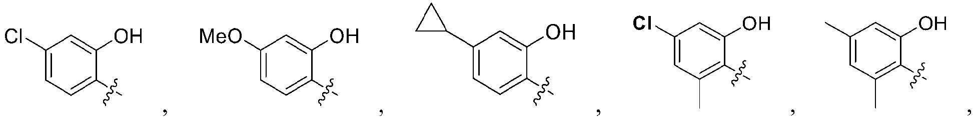

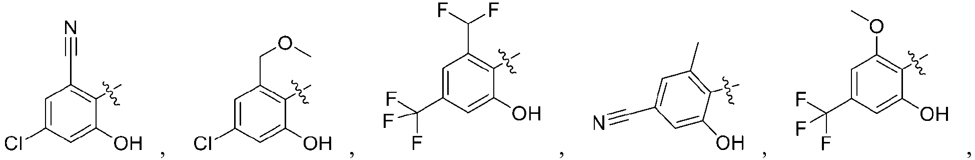

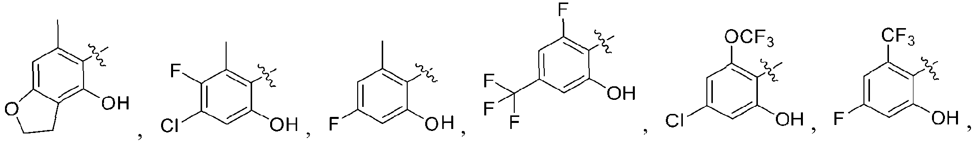

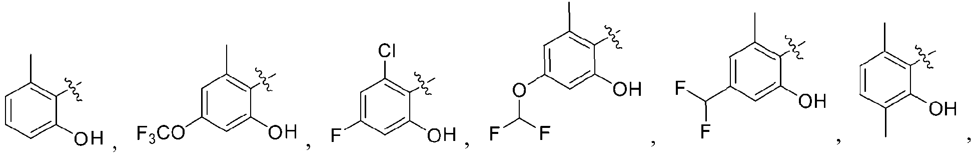

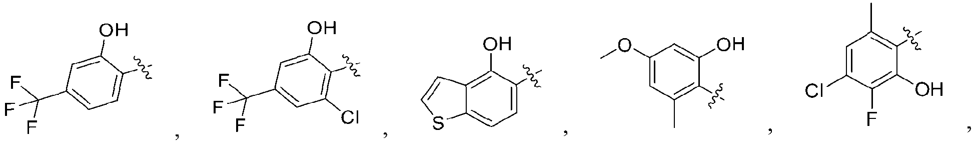

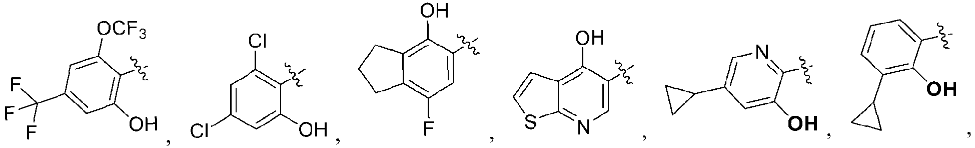

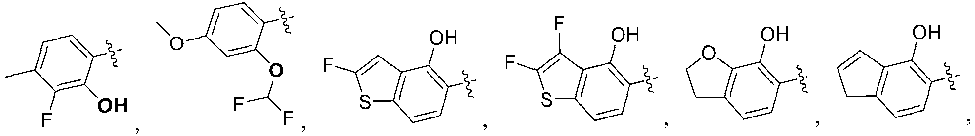

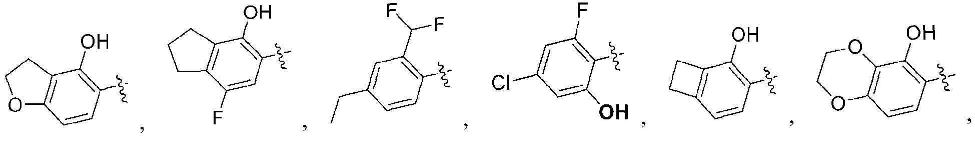

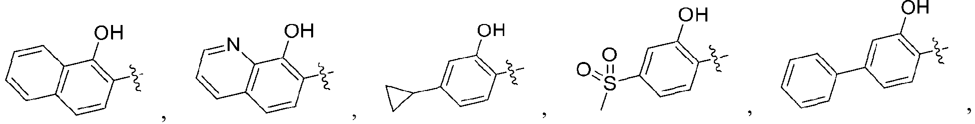

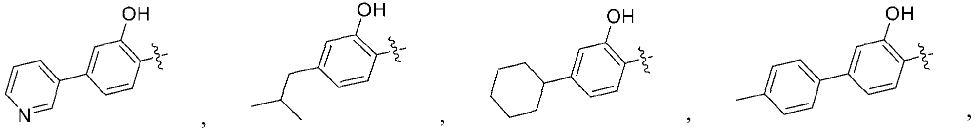

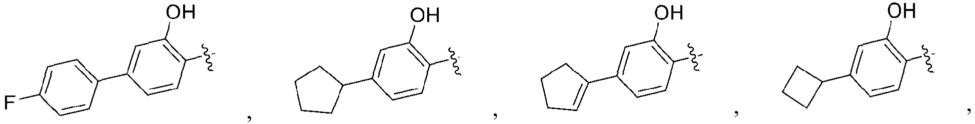

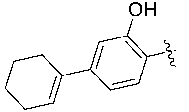

In some embodiments, 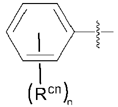 is selected from:

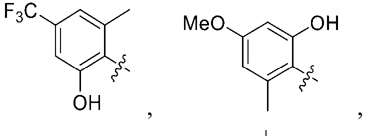

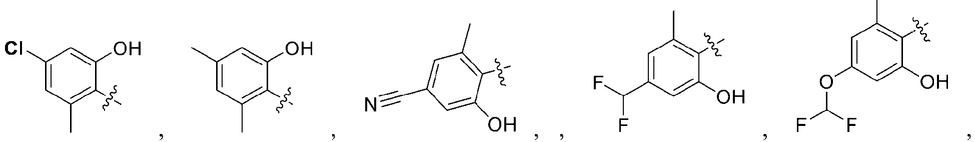

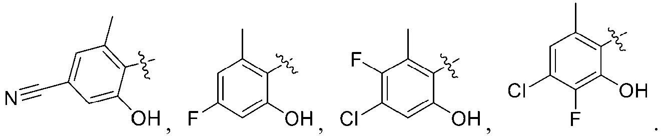

In some embodiments, L² is selected from: bond, -NR⁶-, -O-, -S-, -NR⁶-(CR⁷R⁸)_{b1}-, -(CR⁷R⁸)_{b1}-NR⁶-, -O-(CR⁷R⁸)_{b1}-, -(CR⁷R⁸)_{b1}-O-, -S-(CR⁷R⁸)_{b1}-, and -(CR⁷R⁸)_{b1}-S-; wherein b1 is 1 or 2.

In some embodiments, L² is selected from: bond, -NR⁶-, -NR⁶-(CR⁷R⁸)_{b1}-, and -(CR⁷R⁸)_{b1}-NR⁶-; wherein b1 is 1 or 2.

In some embodiments, L² is selected from: -NR⁶-, -NR⁶-(CR⁷R⁸)_{b1}-, and -(CR⁷R⁸)_{b1}-NR⁶-; wherein b1 is 1.

In some embodiments, L² is selected from: -NH- and -NH-(CR⁷R⁸)-.

In some embodiments, L² is selected from: -NH- and -NH-CH₂-.

In some embodiments, R¹ is selected from: cyclopentyl, cyclohexyl, azetidinyl, tetrahydropyrrolyl, piperidinyl, morpholinyl, piperazinyl, 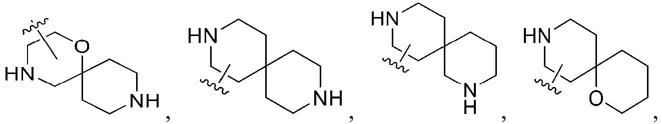 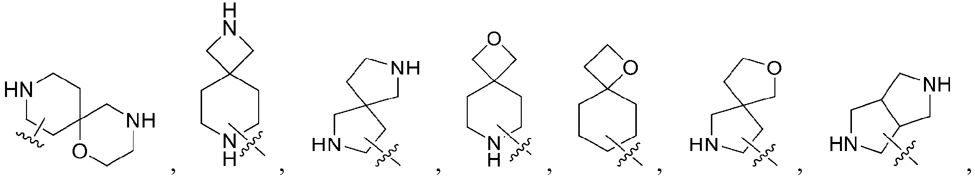 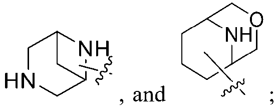 wherein the cyclopentyl, cyclohexyl, azetidinyl, tetrahydropyrrolyl, piperidinyl, morpholinyl, piperazinyl, 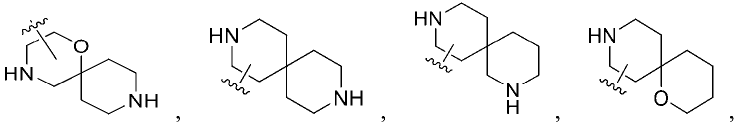 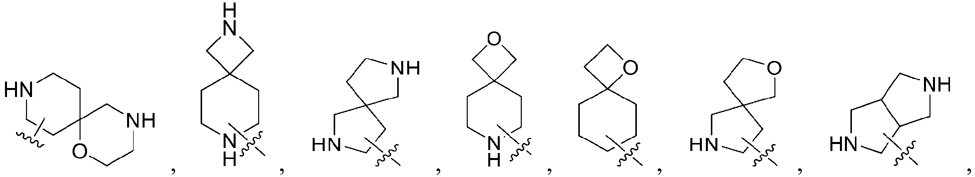 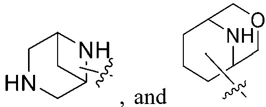 are substituted with 1 or 2 substituents selected from: -C(=O)-R^{c3}, -S(O)-R^{c3}, - S(O)₂-R^{c3}, -C(=O)-N(R^{c5})(R^{c6}), and -P(=O)R^{c5}R^{c6}; and the cyclopentyl, cyclohexyl, azetidinyl, tetrahydropyrrolyl, piperidinyl, morpholinyl, piperazinyl, 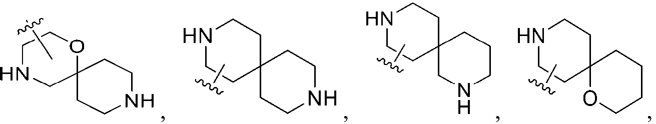 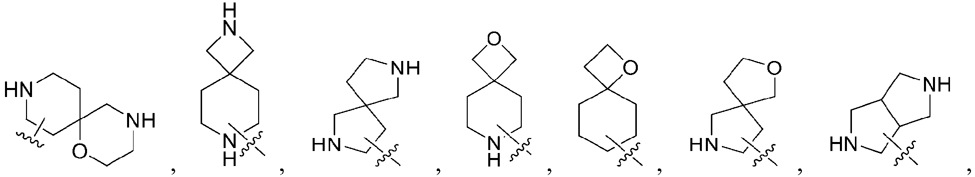 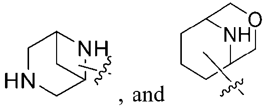 are further optionally substituted with 1, 2, or 3 substituents selected from: H, deuterium, halogen, -OH, oxo, C₁₋₆ alkyl, C₁₋₆ alkoxy, C₁₋₆ alkoxy-C₁₋₆ alkyl, -(CH₂)ₛ-N(R^{c1})(R^{c2}), C₃₋₆ cycloalkyl, and 3-6 membered monocyclic heterocyclyl.

In some embodiments, R¹ is selected from: tetrahydropyrrolyl, piperidinyl, morpholinyl, piperazinyl, 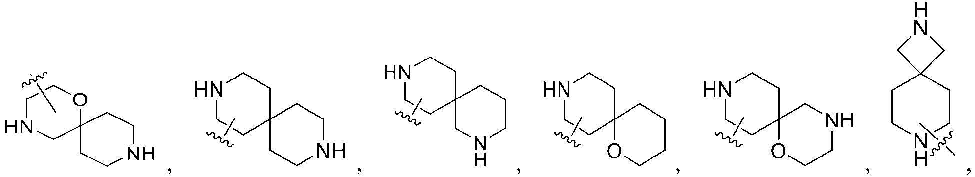 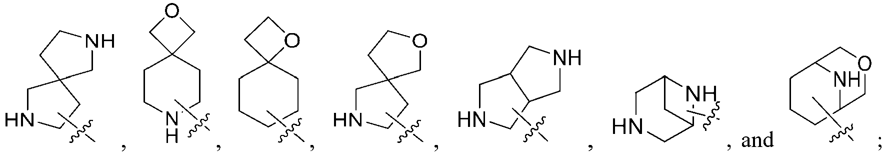 wherein the tetrahydropyrrolyl, piperidinyl, morpholinyl, piperazinyl, 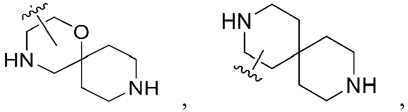 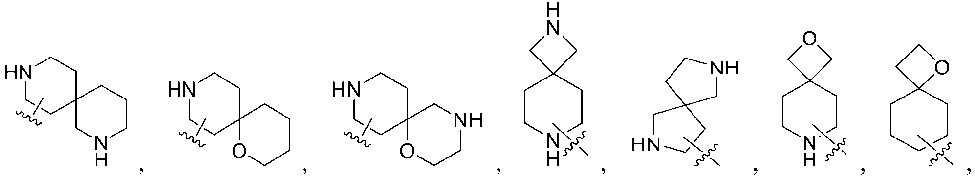 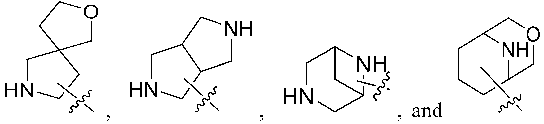 are substituted with 1 or 2 substituents selected from: -C(=O)-R^{c3}, -S(O)-R^{c3}, -S(O)₂-R^{c3}, -C(=O)-N(R^{c5})(R^{c6}), and -P(=O)R^{c5}R^{c6}; and the tetrahydropyrrolyl, piperidinyl, morpholinyl, piperazinyl, 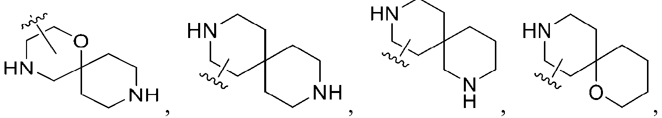 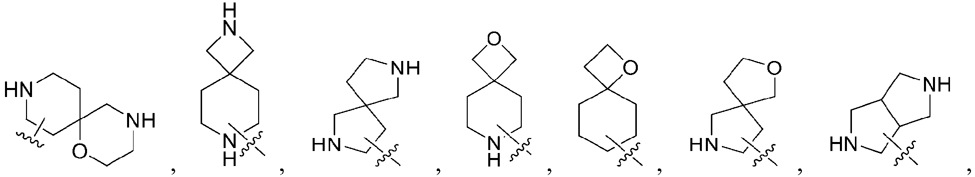 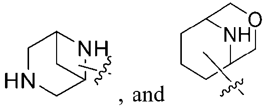 are further optionally substituted with 1, 2, or 3 substituents selected from: H, deuterium, halogen, -OH, oxo, C₁₋₆ alkyl, C₁₋₆ alkoxy, -(CH₂)ₛ-N(R^{c1})(R^{c2}), C₃₋₆ cycloalkyl, C₁₋₆ alkoxy-C₁₋₆ alkyl, and 3-6 membered monocyclic heterocyclyl.

In some embodiments, R¹ is selected from: tetrahydropyrrolyl, piperidinyl, morpholinyl, piperazinyl, 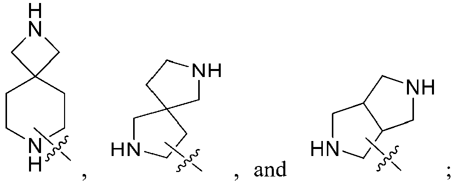 wherein the tetrahydropyrrolyl, piperidinyl, morpholinyl, piperazinyl, 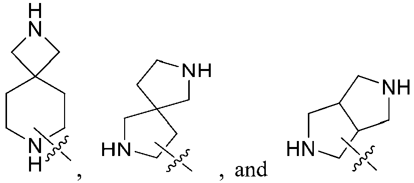 are substituted with 1 substituent selected from: - C(=O)-R^{c3}, -S(O)₂-R^{c3}, and -P(=O)R^{c5}R^{c6}; and the tetrahydropyrrolyl, piperidinyl, morpholinyl, piperazinyl, 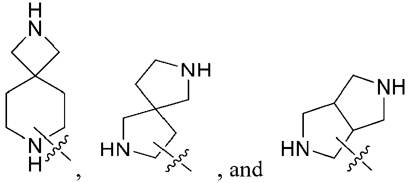 are further optionally substituted with 1 or 2 substituents selected from: deuterium, halogen, -OH, C₁₋₆ alkyl, C₁₋₆ alkoxy, and C₁₋₆ alkoxy-C₁₋₆ alkyl.

In some embodiments, R¹ is selected from:

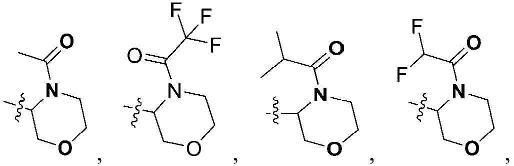

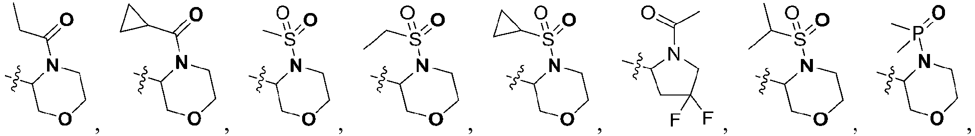

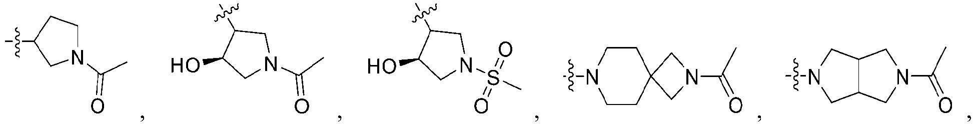

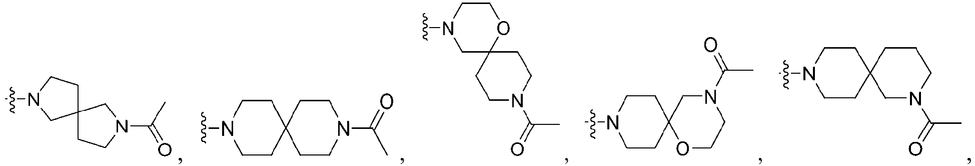

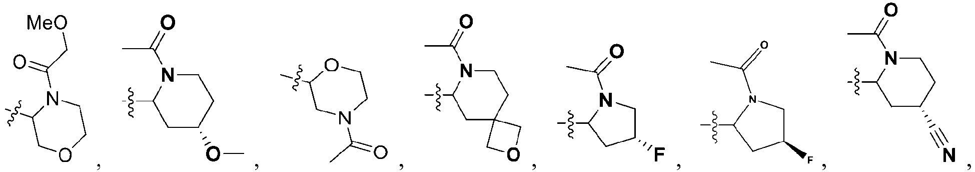

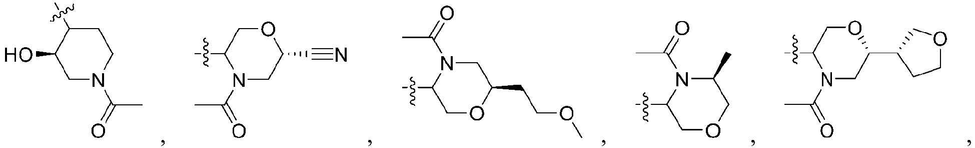

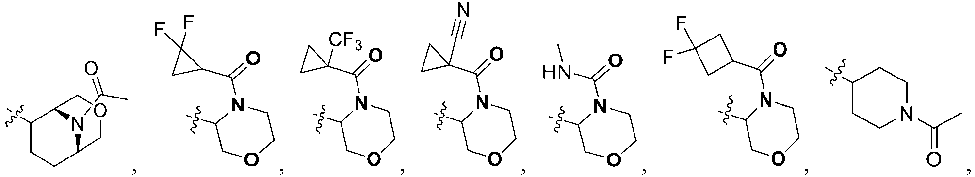

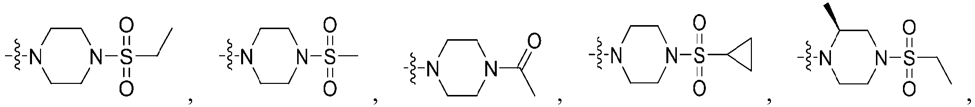

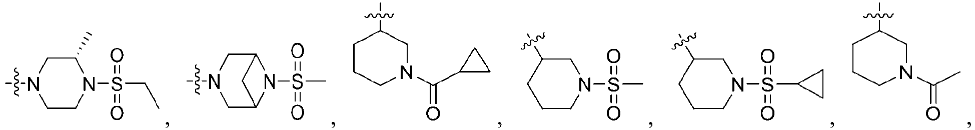

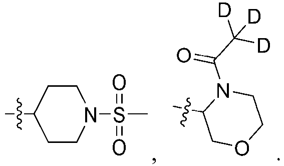

In some embodiments, R¹ is selected from:

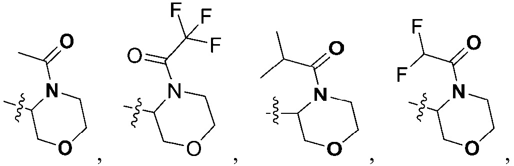

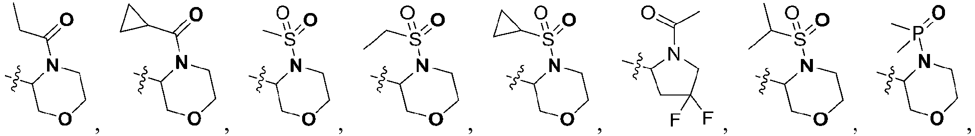

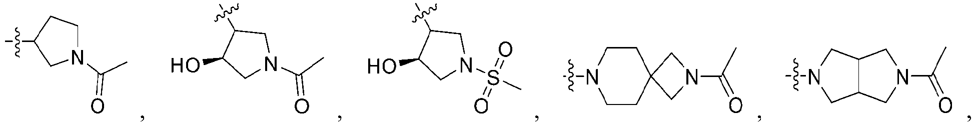

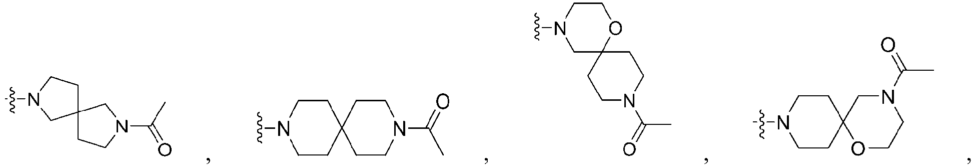

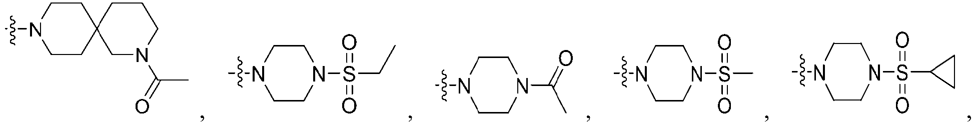

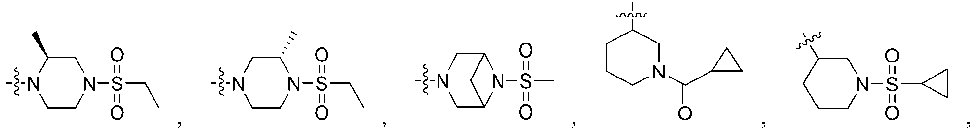

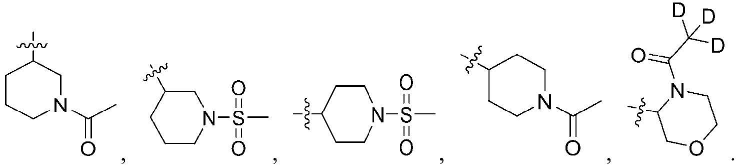

In some embodiments, the compound has the structure represented by Formula VIII-8 or VIII-12, 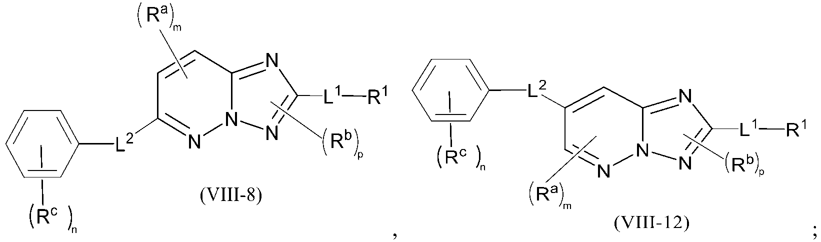 wherein, R¹, L¹, R^{a}, R^{b}, R^{c}, L², m, n, and p are defined as above.

In some embodiments, each R^{c} is independently selected from: H, deuterium, halogen, -OH, -CN, C₁₋₆ alkoxy, C₁₋₆ alkyl, C₁₋₆ haloalkyl, C₁₋₆ haloalkoxy, C₁₋₆ alkoxy-C₁₋₆ alkyl, R^{c1}S(O)₂-, 3-6 membered cycloalkyl, 3-6 membered heterocyclyl, phenyl, and 5-6 membered heteroaryl; the 3-6 membered cycloalkyl, 3-6 membered heterocyclyl, phenyl, and 5-6 membered heteroaryl are optionally substituted with 1, 2, or 3 substituents selected from: deuterium, halogen, -OH, -NH₂, -CN, C₁₋₆ alkyl, C₁₋₆ haloalkyl, and C₁₋₆ alkoxy; or
two R^{c} together with the C atom to which they are attached may form a 3-6 membered cycloalkyl, 4-7 membered heterocyclyl, 6 membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
L² is selected from: bond, -NR⁶-, -O-, and -S-;
R², R^{b}, R^{c}, R^{c1}, R⁶, R¹, L¹, n, m, and p are defined as above.

In some embodiments, the compound has the structure represented by Formula VIII-8: 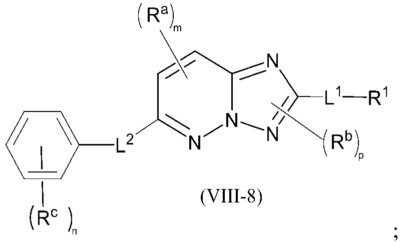 wherein, R¹, L¹, R^{a}, R^{b}, R^{c}, L², m, n, and p are defined as above.

In some embodiments, R¹ is 3-6 membered monocyclic heterocyclyl, the 3-6 membered monocyclic heterocyclyl is optionally further substituted with 1, 2, or 3 substituents selected from: deuterium, halogen, -OH, oxo, -CN, C₁₋₆ alkyl, C₁₋₆ haloalkyl, hydroxyl C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, 5-6 membered heteroaryl, -C(=O)-R^{c3}, -S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6}.

In some embodiments, the R¹ is selected from: tetrahydropyrrolyl, piperidinyl, morpholinyl, piperazinyl, and 1,4-oxathianyl; the tetrahydropyrrolyl, piperidinyl, morpholinyl, piperazinyl, and 1,4-oxathianyl are each independently optionally further substituted with 1, 2, or 3 substituents selected from: deuterium, halogen, -OH, - NH₂, oxo, C₁₋₄ alkyl, C₁₋₄ haloalkyl, hydroxyl C₁₋₄ alkyl, C₁₋₄ alkoxy, C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, - C(=O)-N(R^{c6})(R^{c5}), -N(R^{c4})-C(=O)-N(R^{c6})(R^{c5}), and -P(=O)R^{c5}R^{c6}; preferably, the R¹ is selected from morpholinyl, the morpholinyl is optionally further substituted with 1, 2, or 3 substituents selected from: deuterium, halogen, - OH, -NH₂, oxo, C₁₋₄ alkyl, C₁₋₄ alkoxy, C₃₋₆ cycloalkyl, 3-6 membered monocyclic heterocyclyl, -C(=O)-R^{c3}, - S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6}.

In some embodiments, R¹ is selected from:

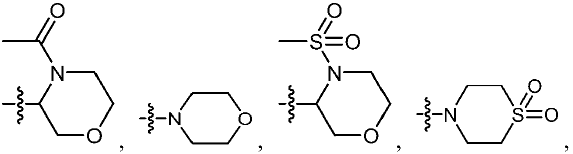

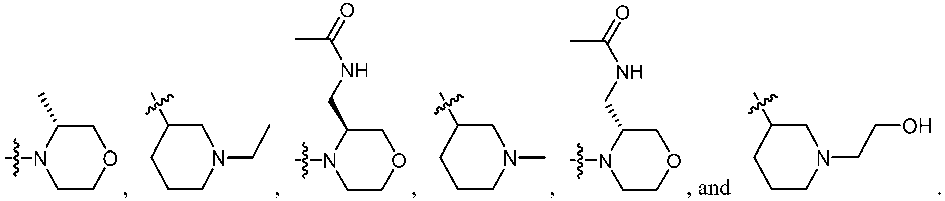

In some embodiments, the compound has the structure represented by Formula VIII-9: 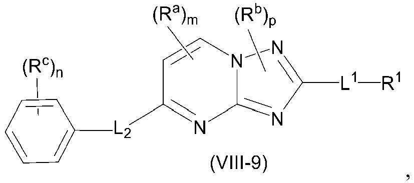 wherein, R¹, L¹, R^{a}, R^{b}, R^{c}, L², m, n, and p are defined as above; provided that, the compound is not

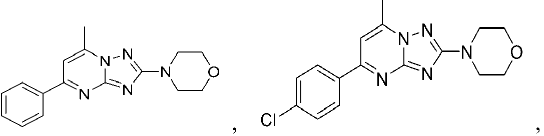

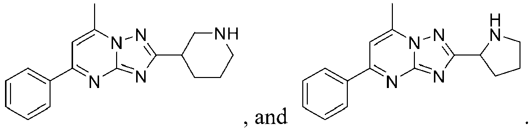

In some embodiments, R¹ is 3-6 membered monocyclic heterocyclyl, the 3-6 membered monocyclic heterocyclyl is optionally further substituted with 1, 2, or 3 substituents selected from: deuterium, halogen, -OH, - CN, C₁₋₆ alkyl, C₁₋₆ haloalkyl, hydroxyl C₁₋₆ alkyl, C₁₋₆ alkoxy, -(CH₂)ₛ-N(R^{c1})(R^{c2}), C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6}.

In some embodiments, R¹ is selected from: morpholinyl and piperidinyl, the morpholinyl and piperidinyl are each independently optionally further substituted with 1, 2, or 3 substituents selected from: deuterium, halogen, - OH, -NH₂, C₁₋₄ alkyl, C₁₋₄ alkoxy, C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6}.

In some embodiments, the R¹ is selected from:

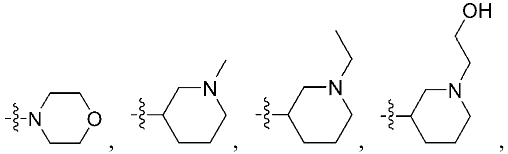

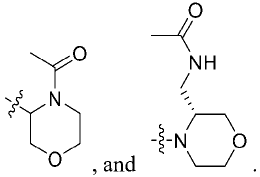

In some embodiments, the compound has the following structures:

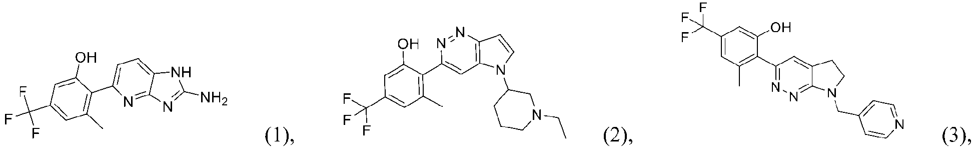

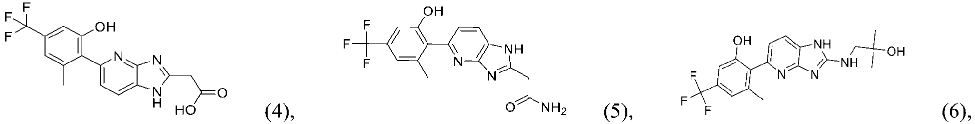

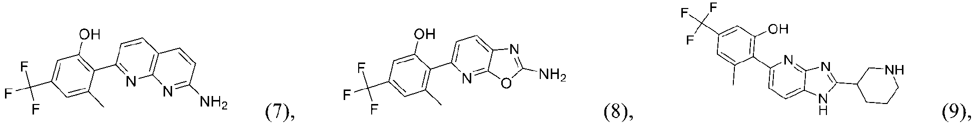

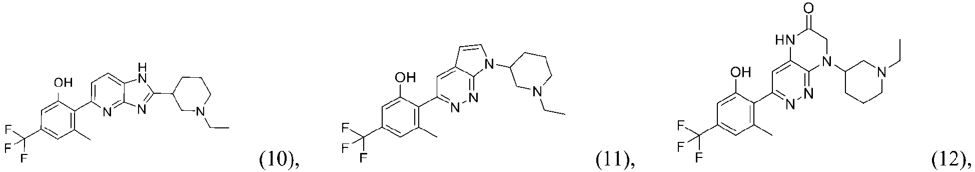

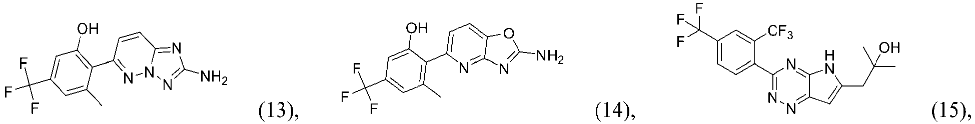

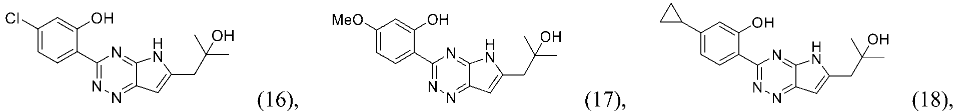

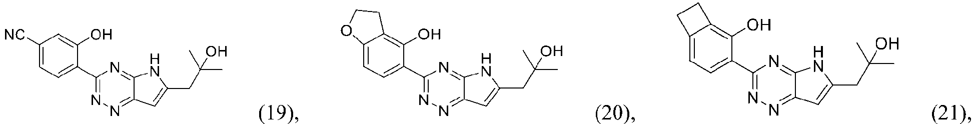

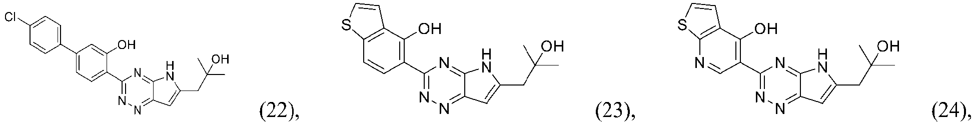

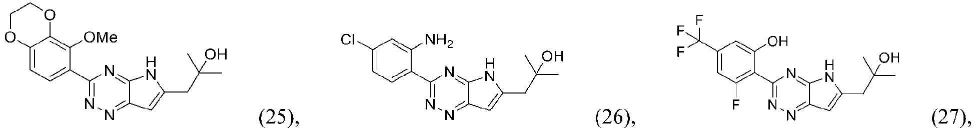

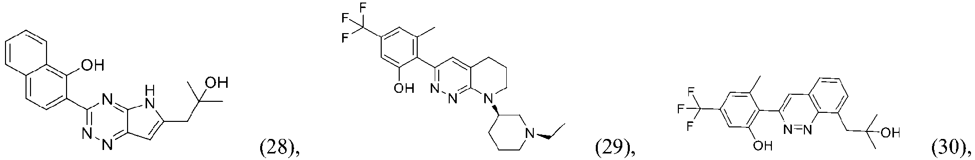

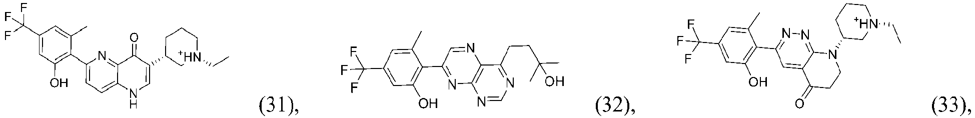

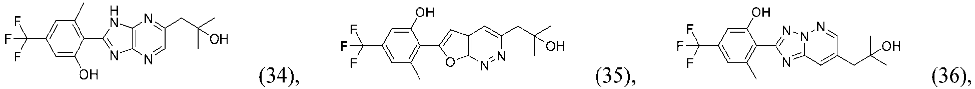

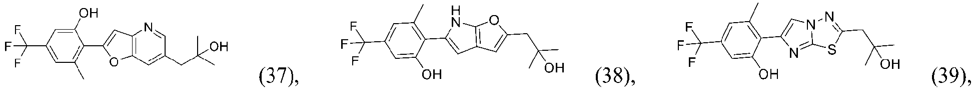

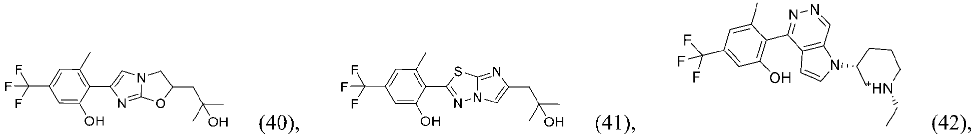

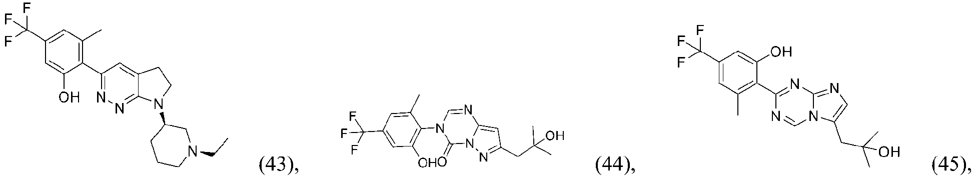

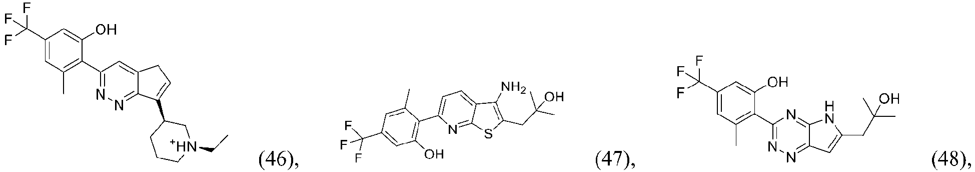

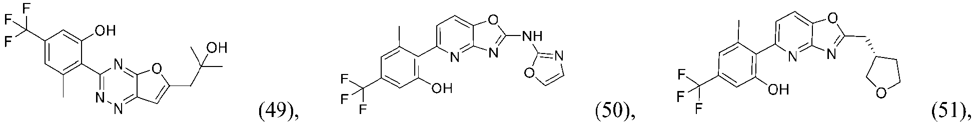

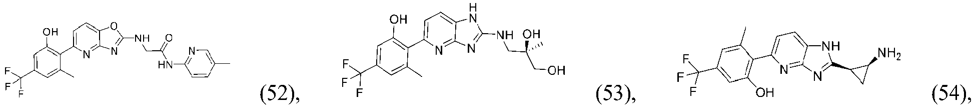

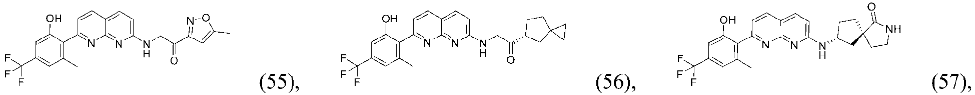

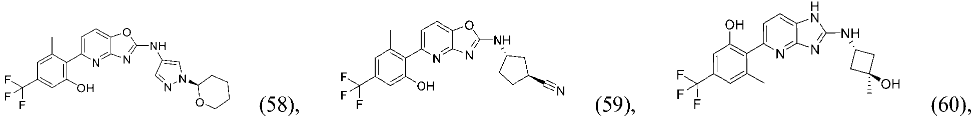

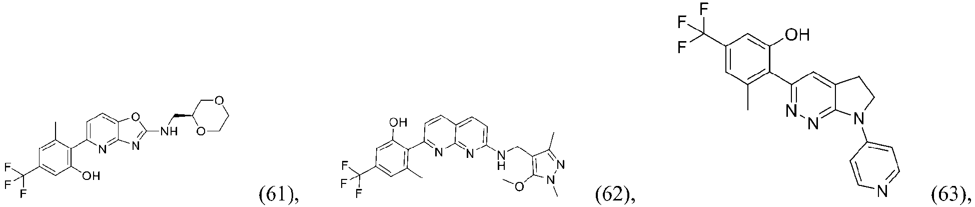

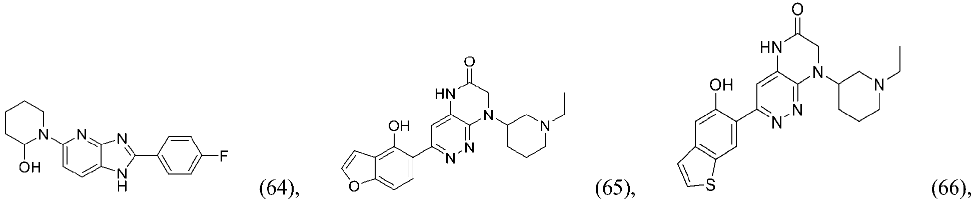

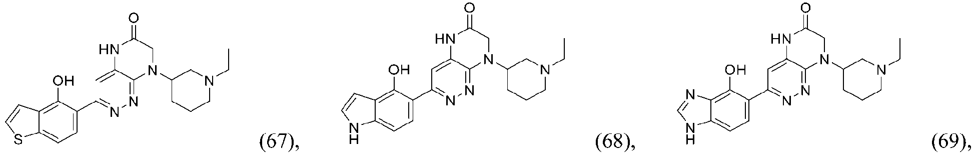

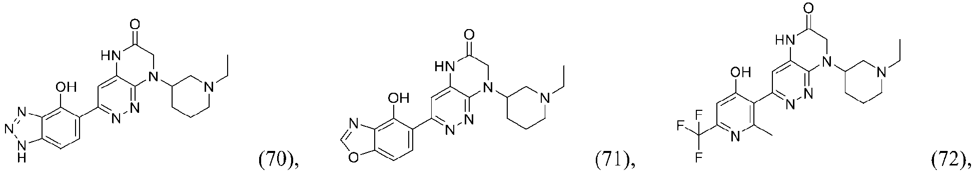

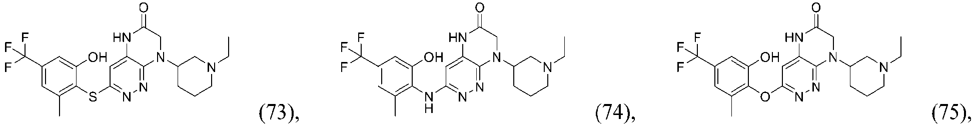

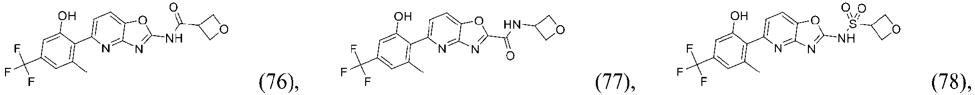

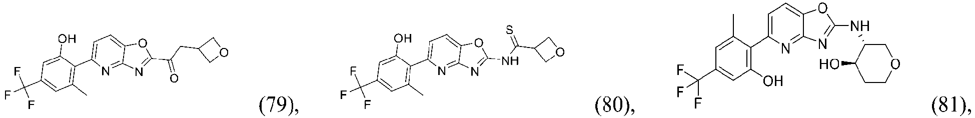

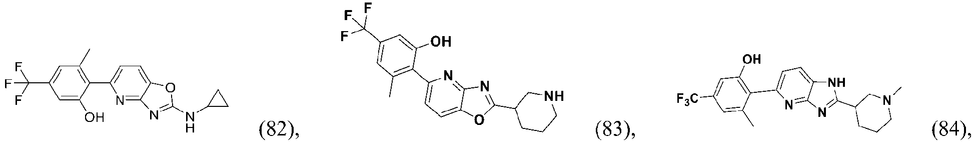

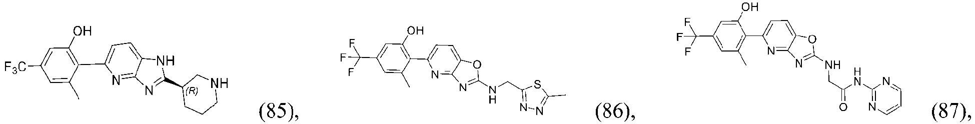

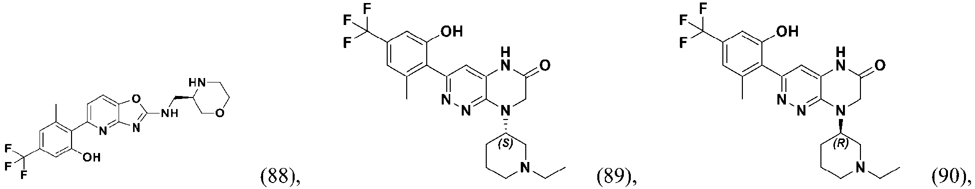

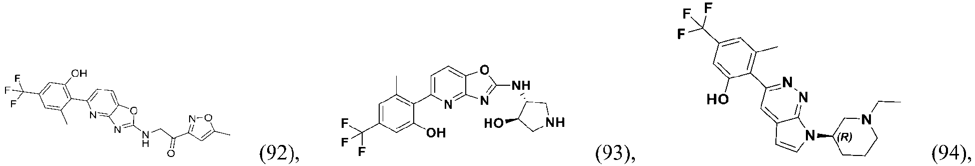

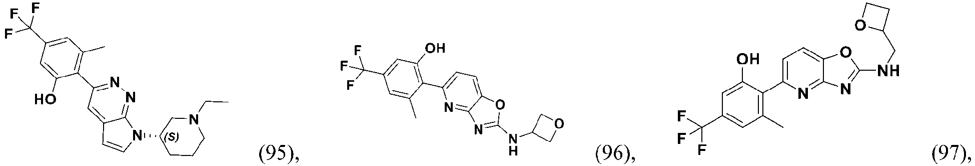

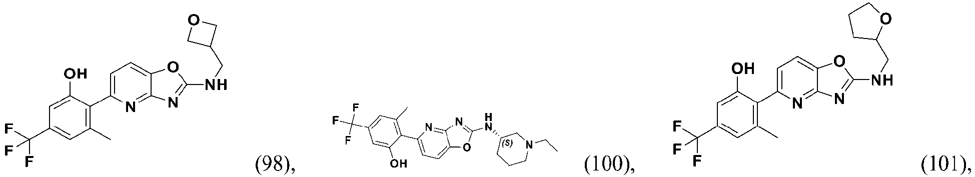

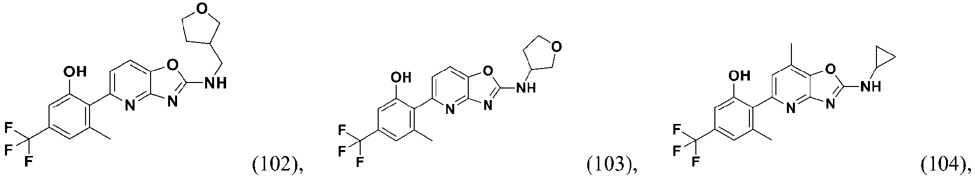

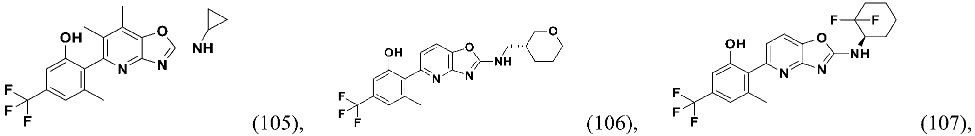

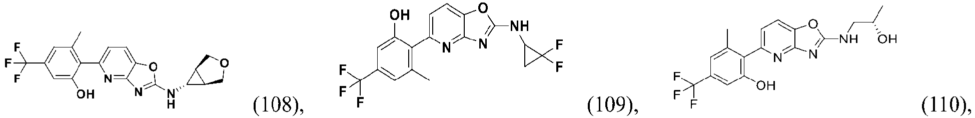

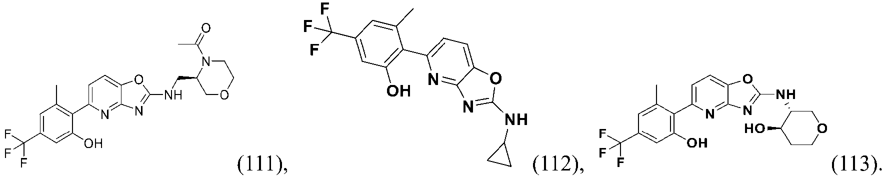

In some embodiments, the compound has the following structures:

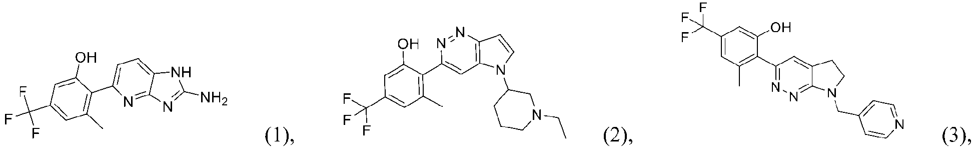

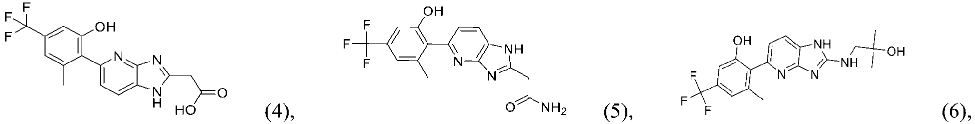

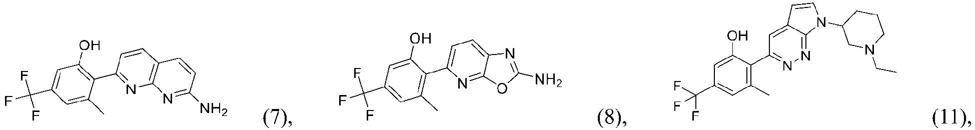

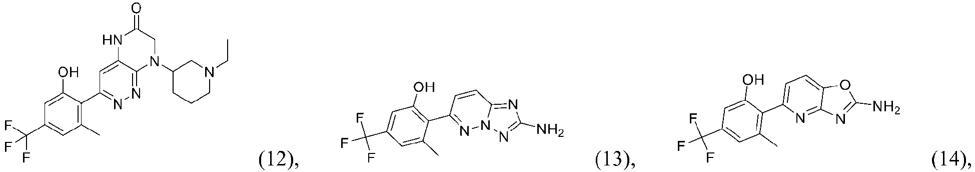

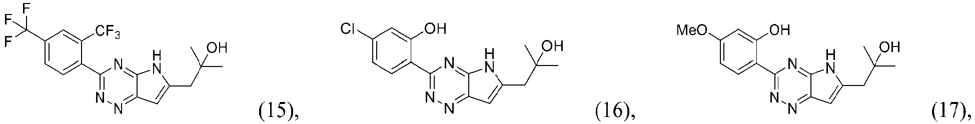

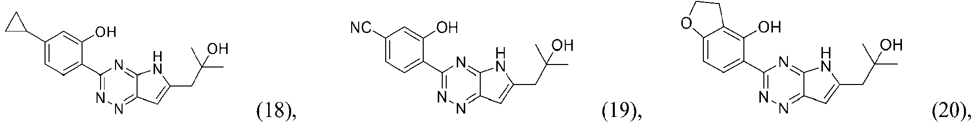

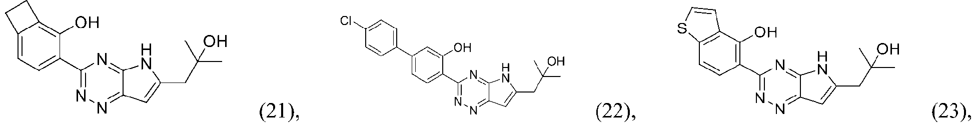

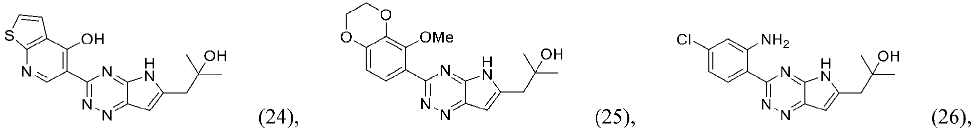

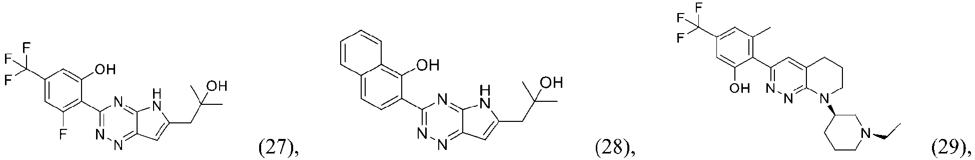

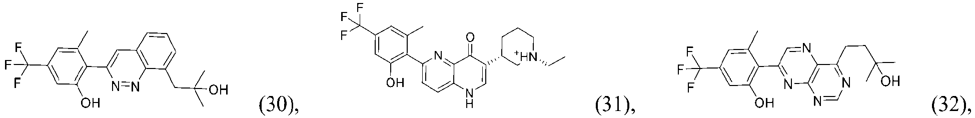

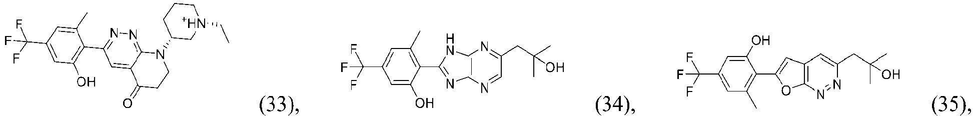

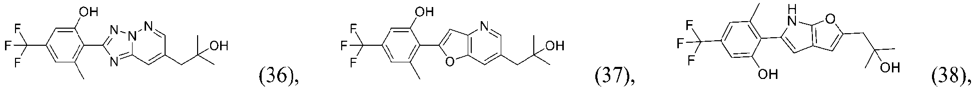

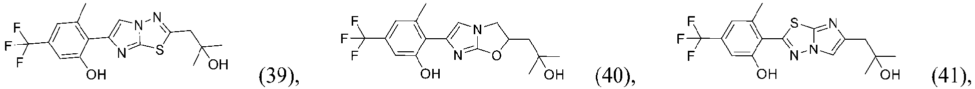

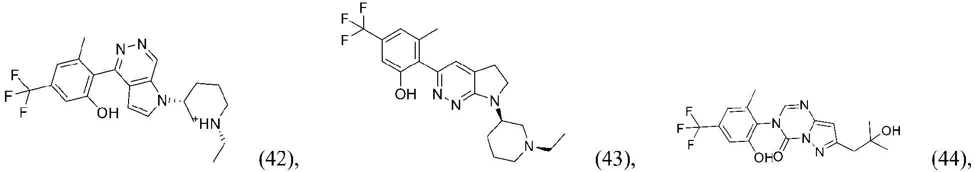

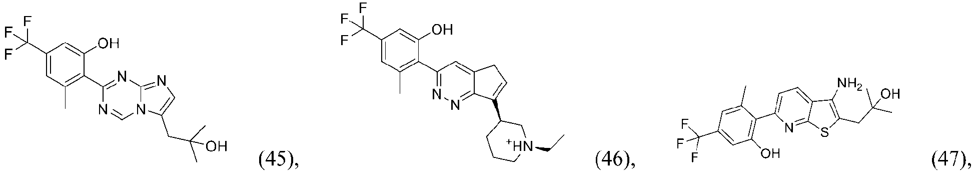

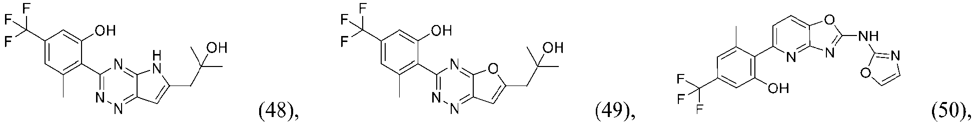

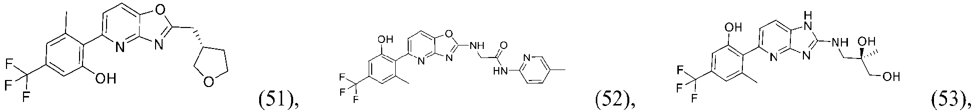

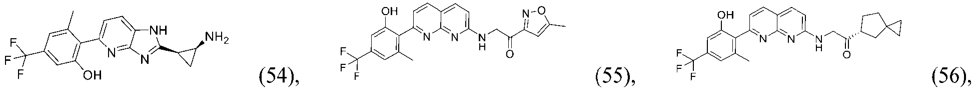

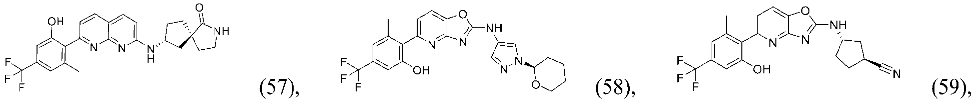

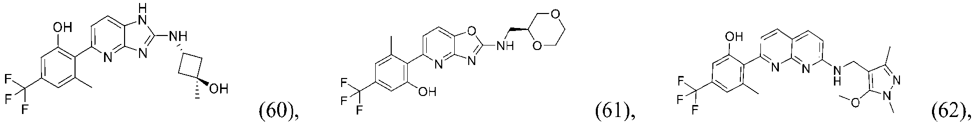

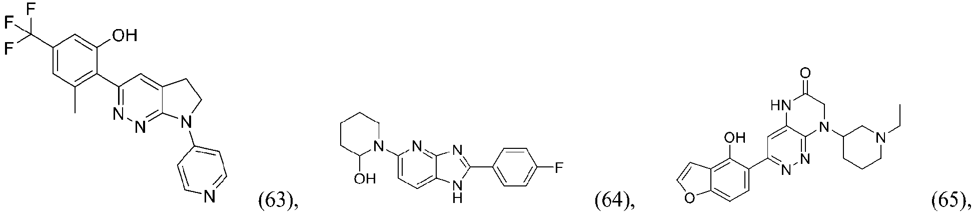

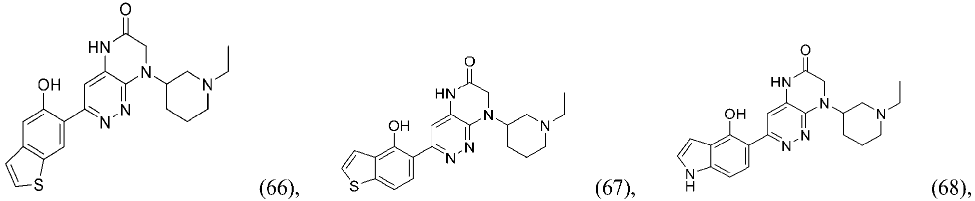

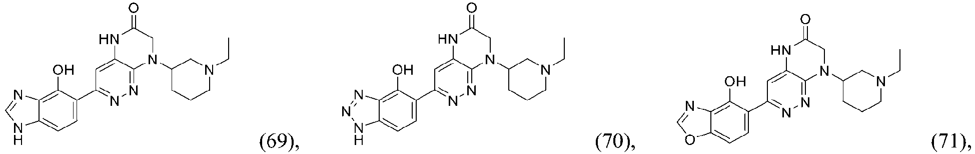

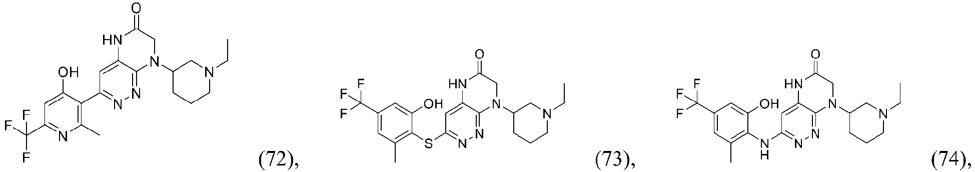

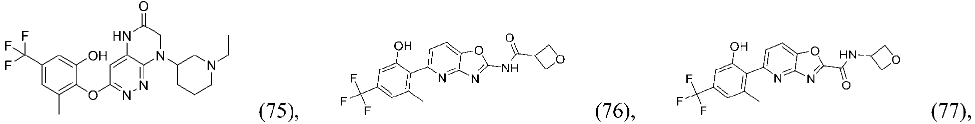

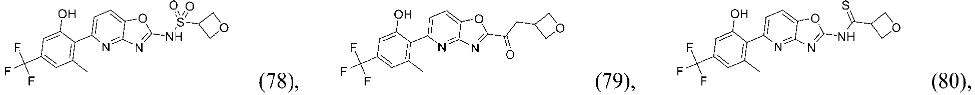

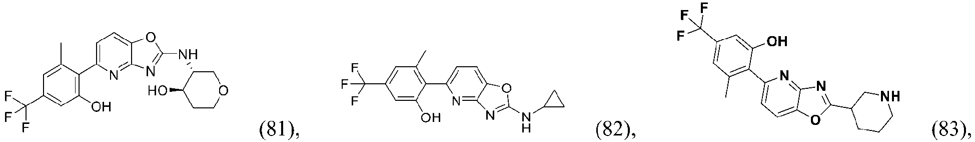

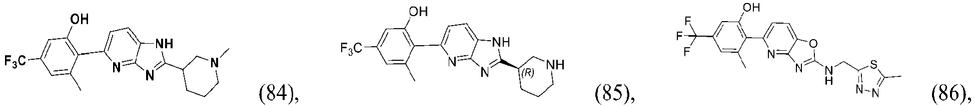

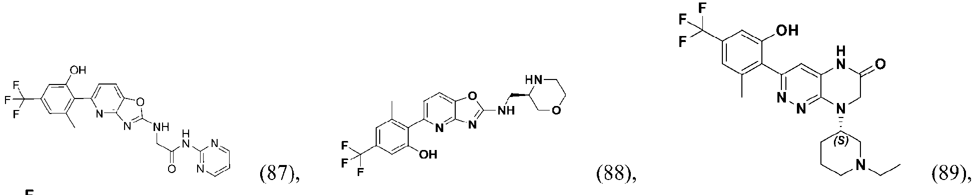

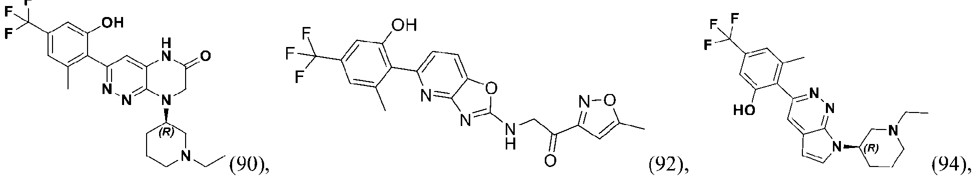

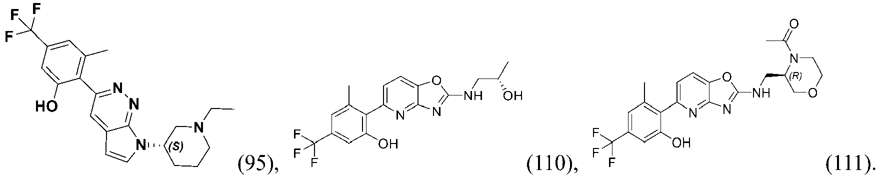

In some embodiments, the compound has the following structures:

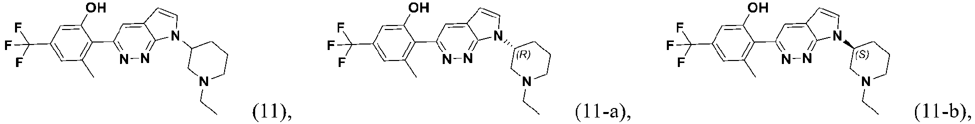

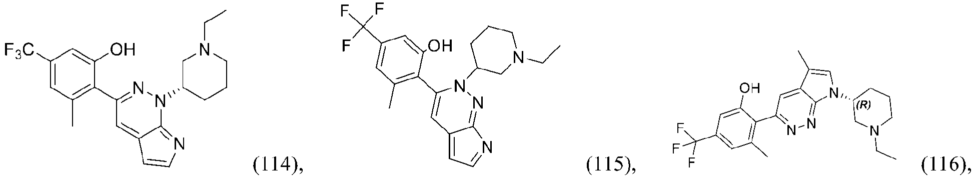

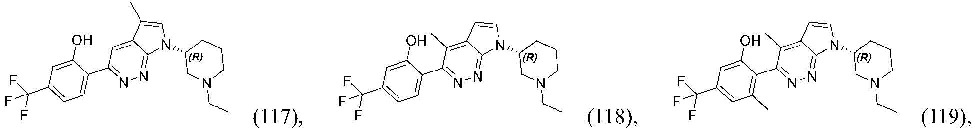

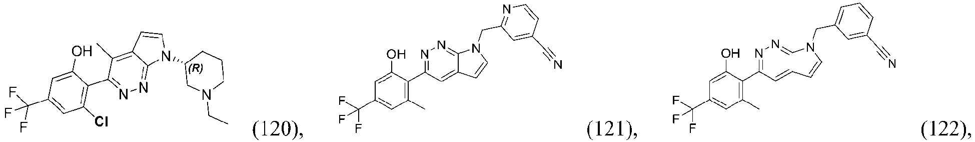

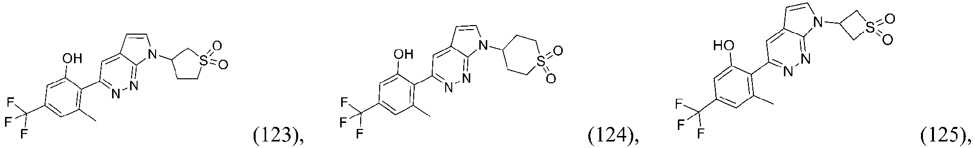

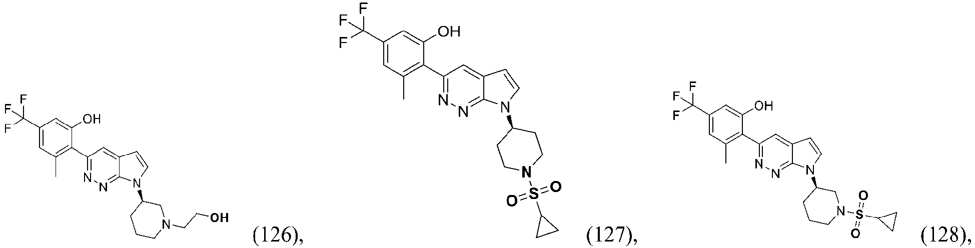

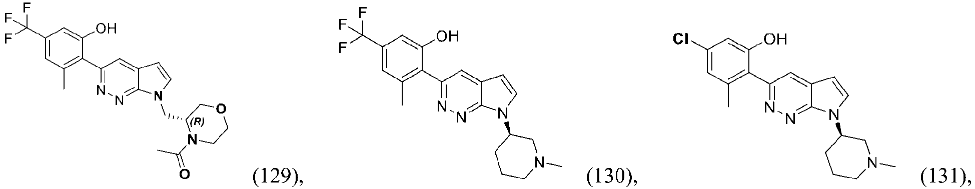

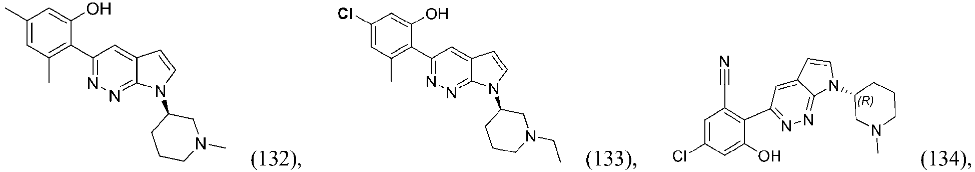

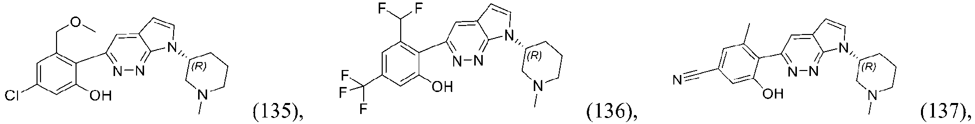

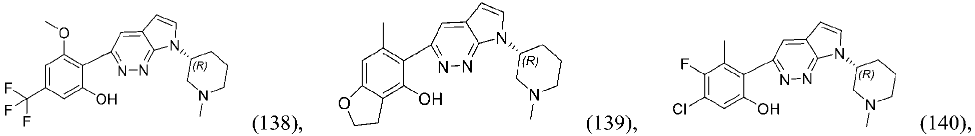

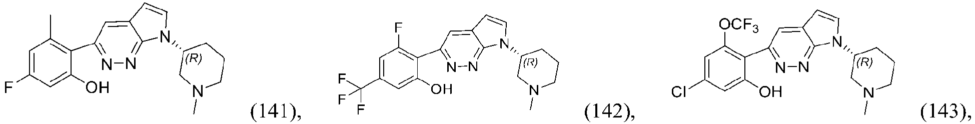

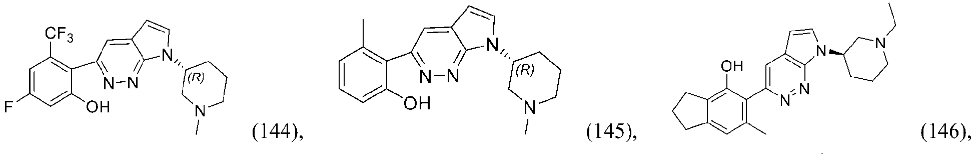

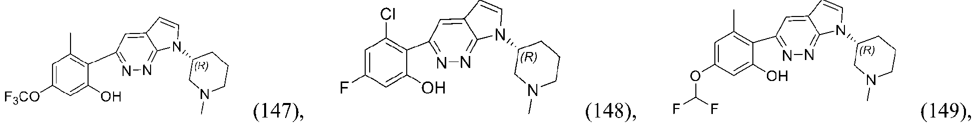

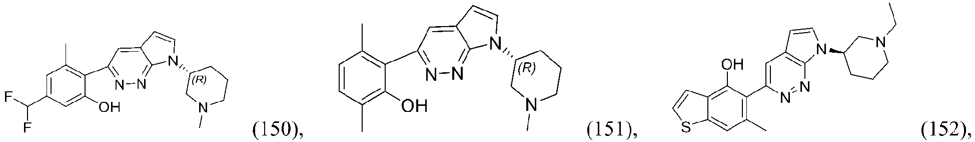

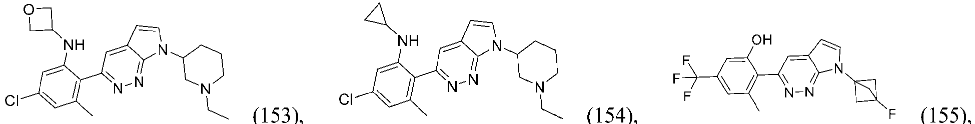

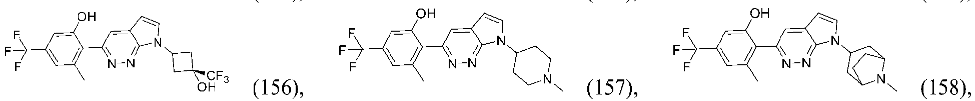

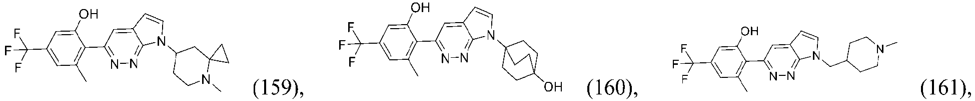

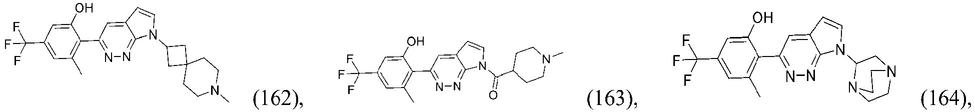

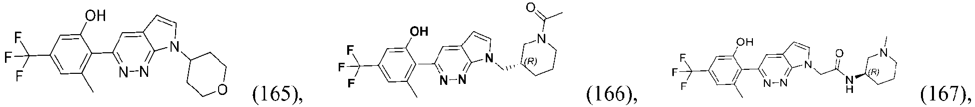

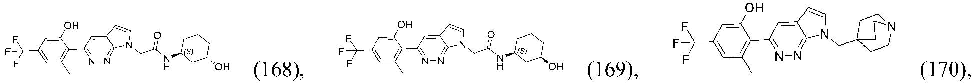

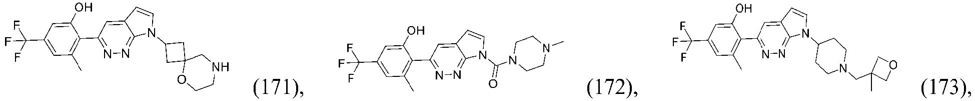

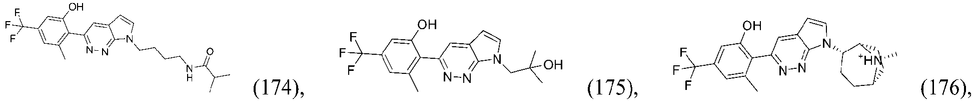

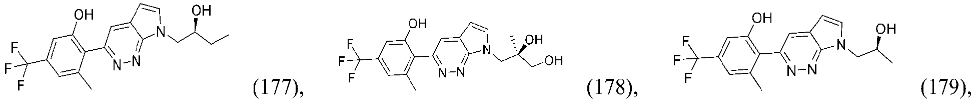

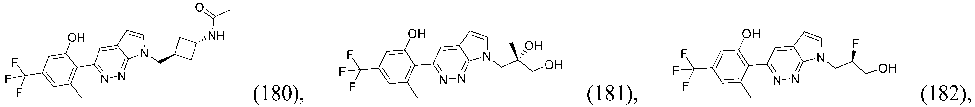

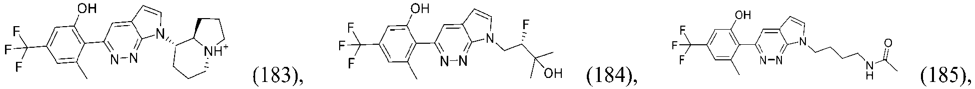

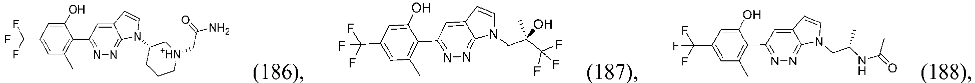

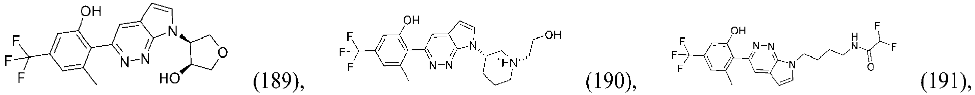

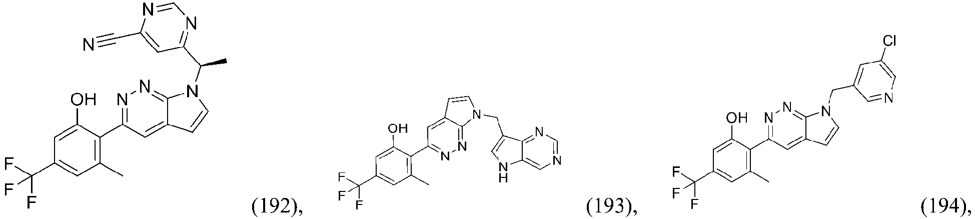

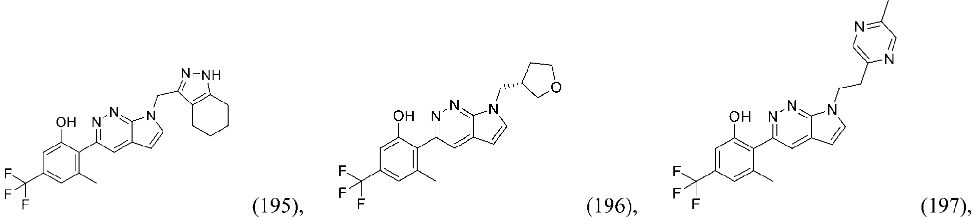

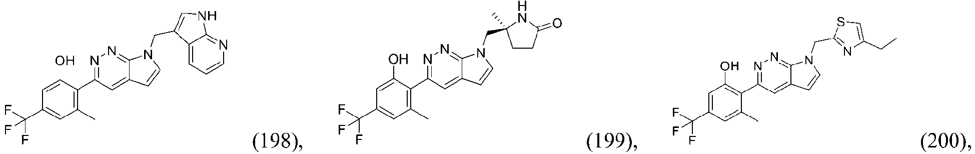

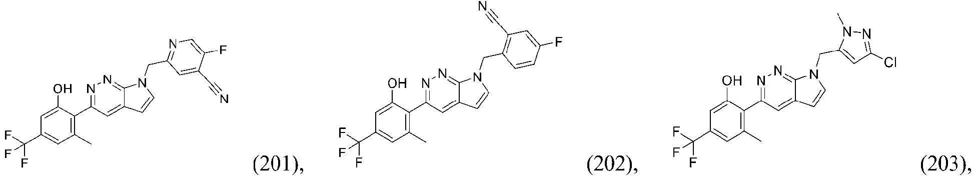

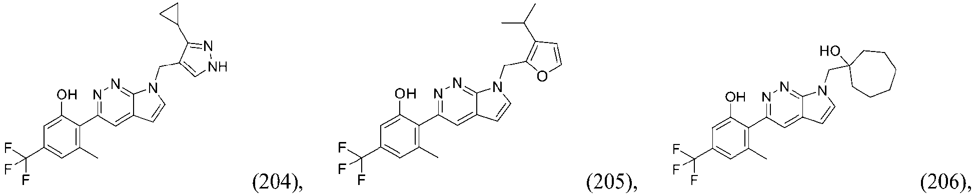

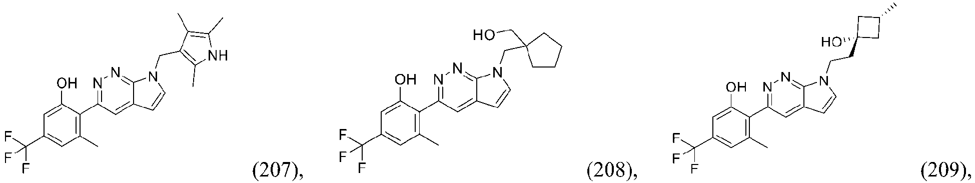

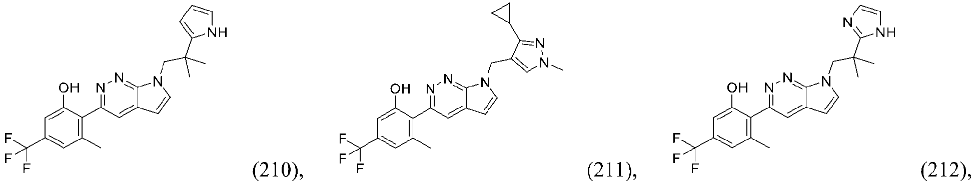

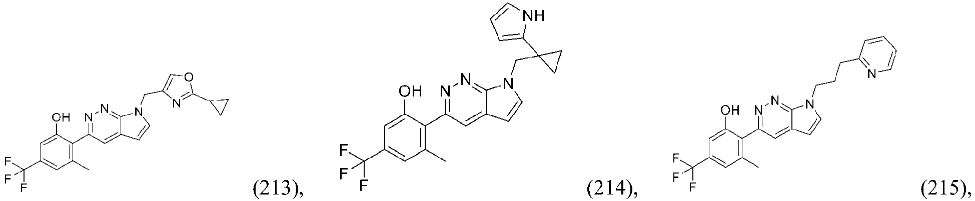

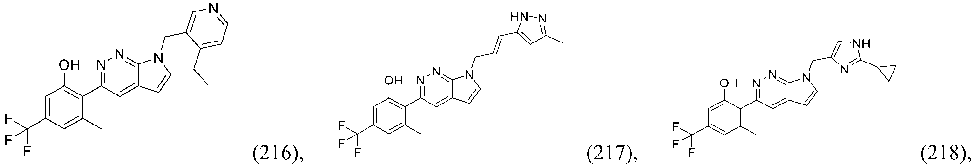

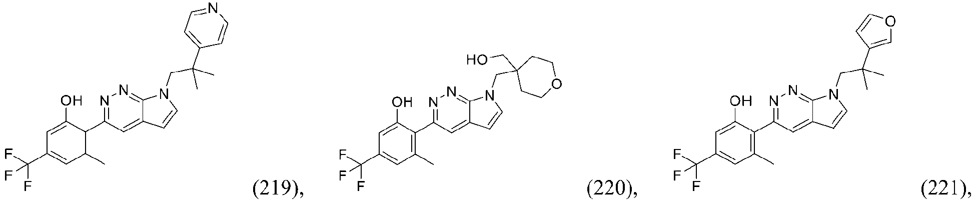

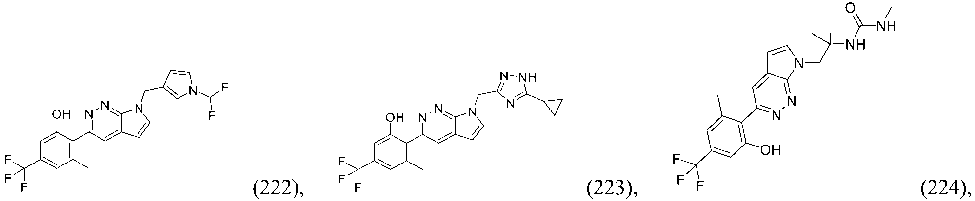

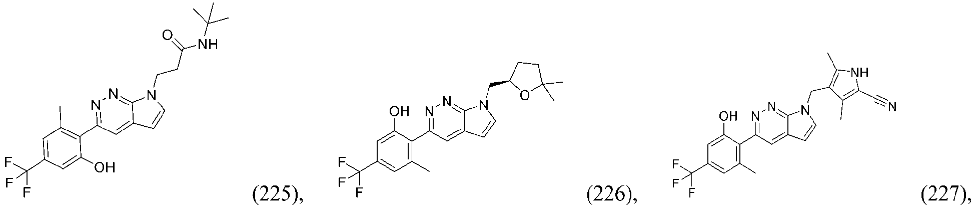

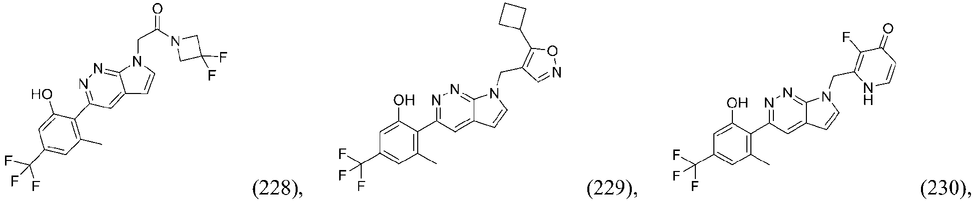

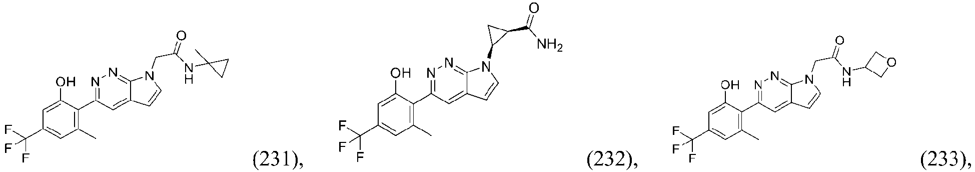

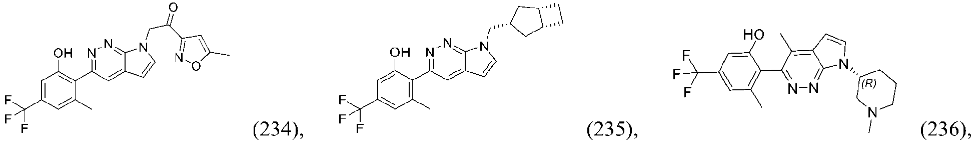

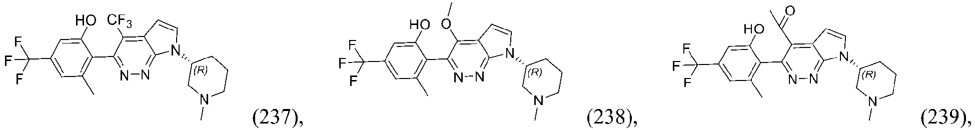

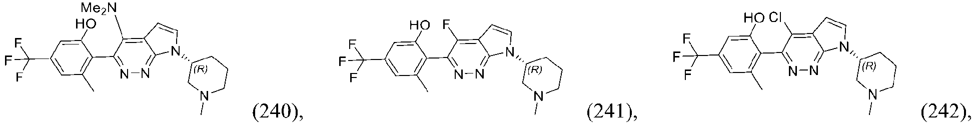

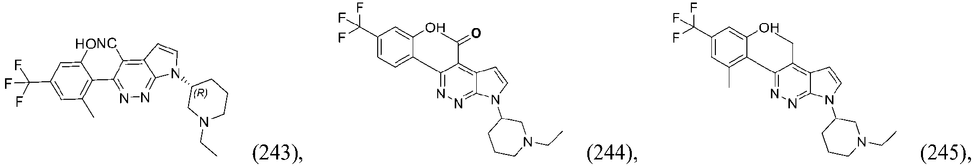

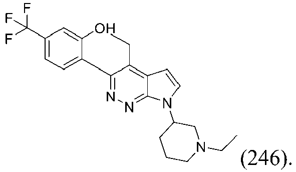

In some embodiments, the compound has the following structures:

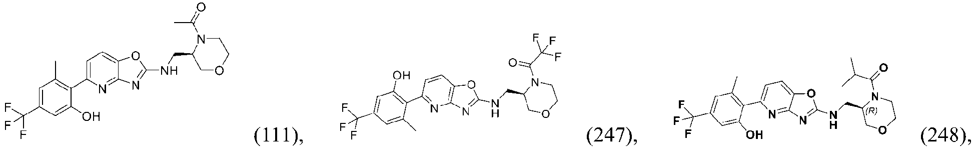

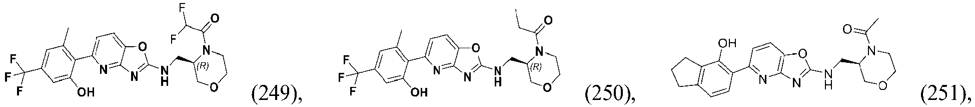

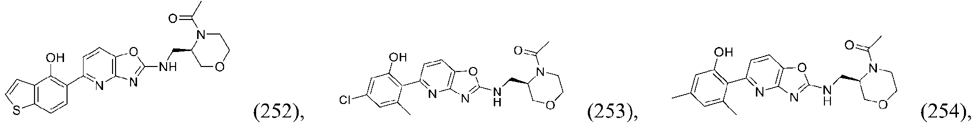

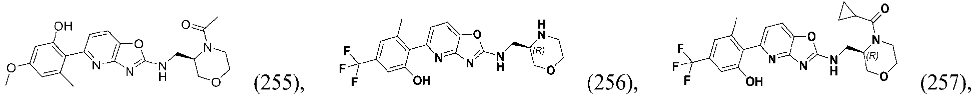

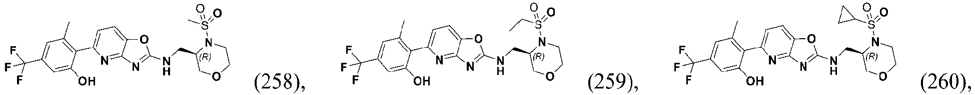

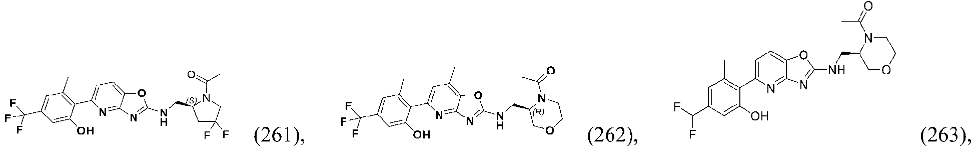

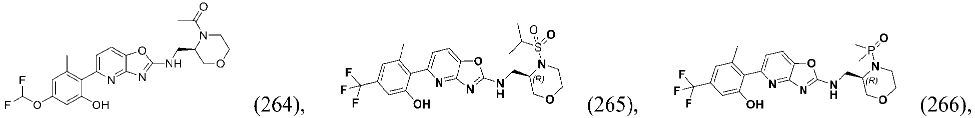

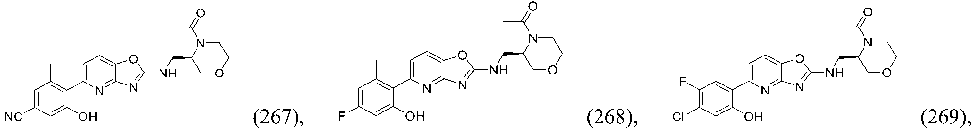

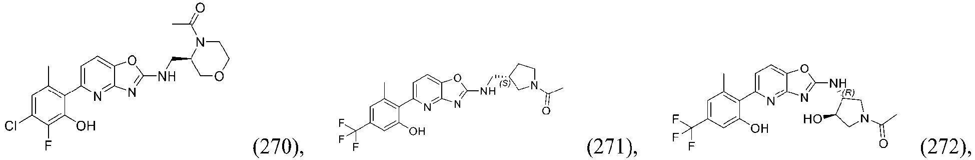

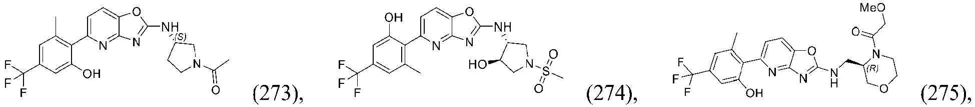

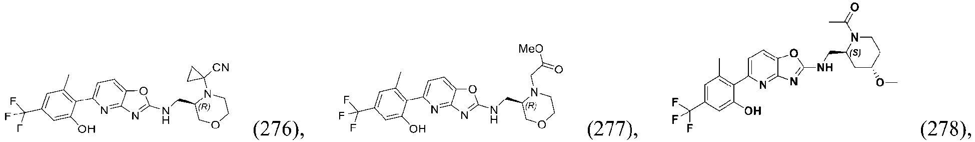

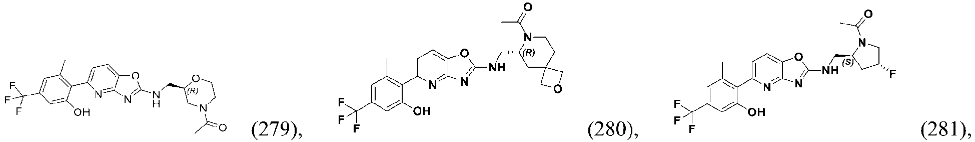

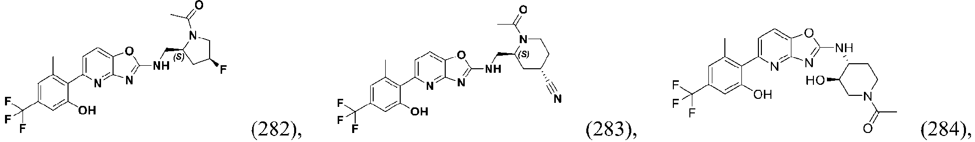

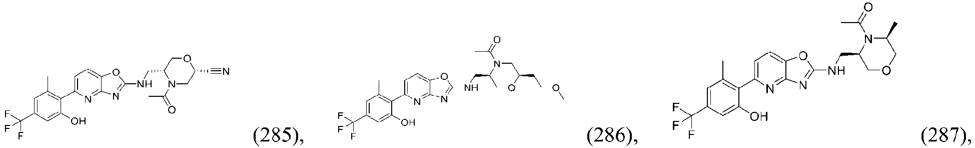

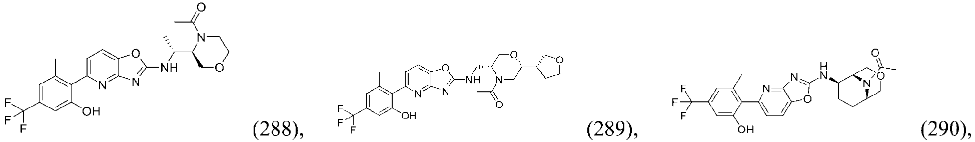

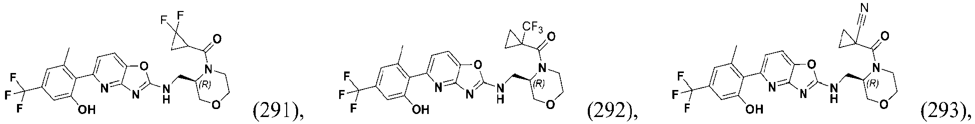

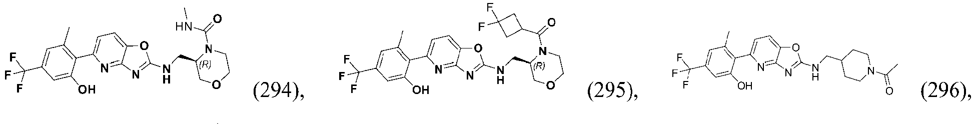

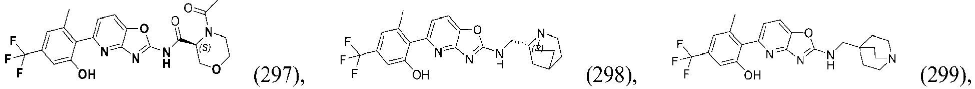

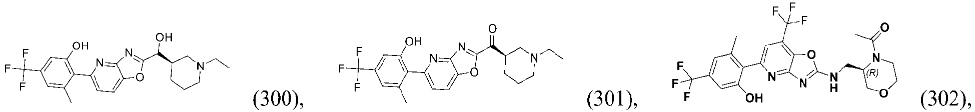

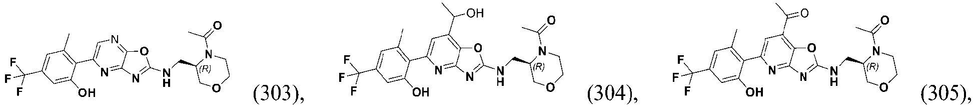

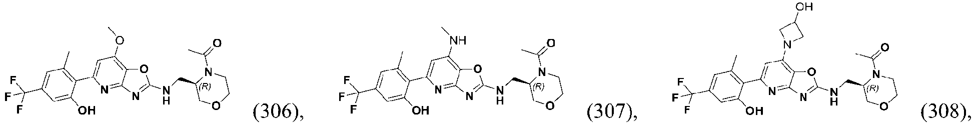

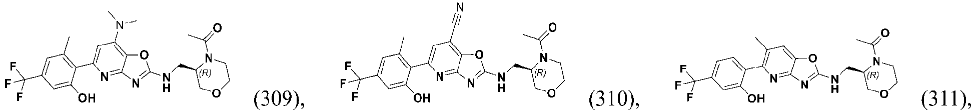

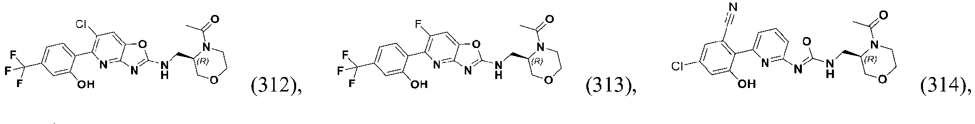

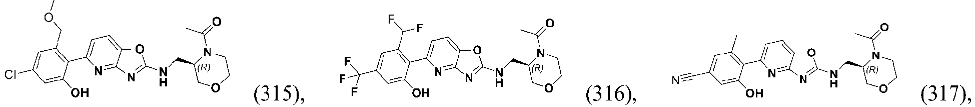

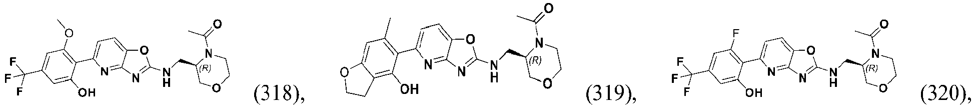

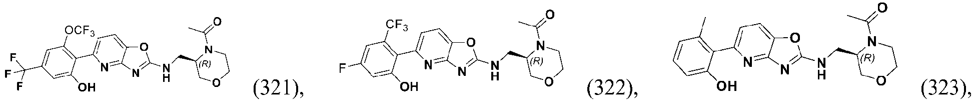

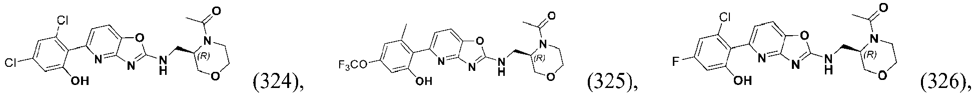

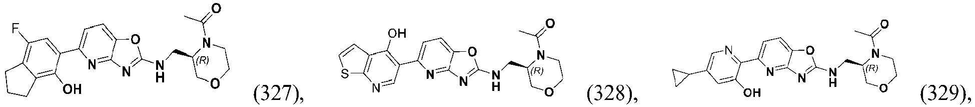

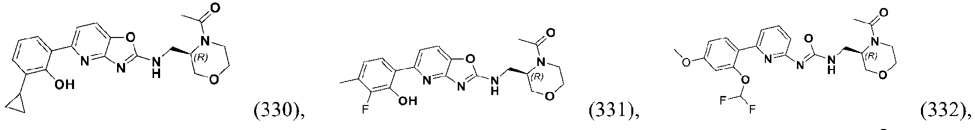

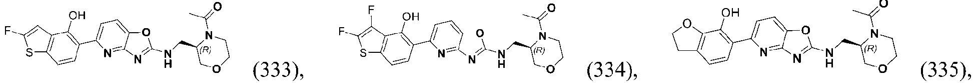

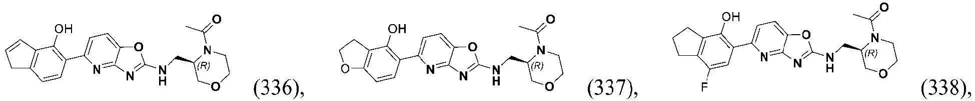

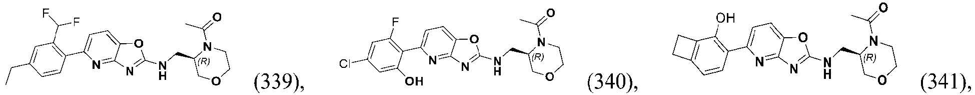

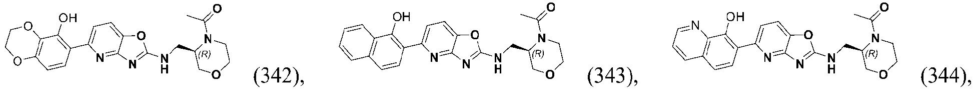

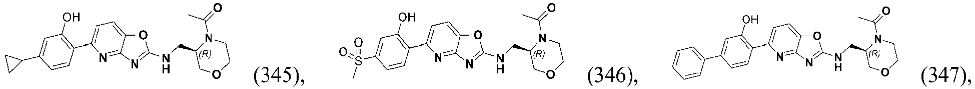

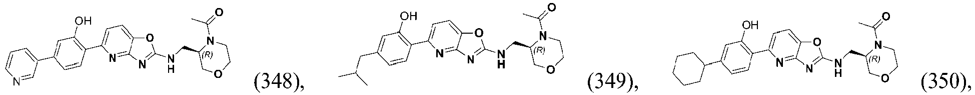

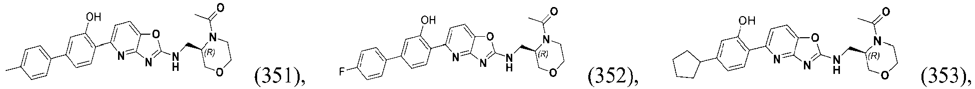

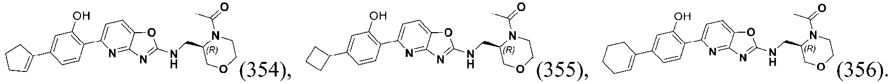

In some embodiments, the compound has the following structures:

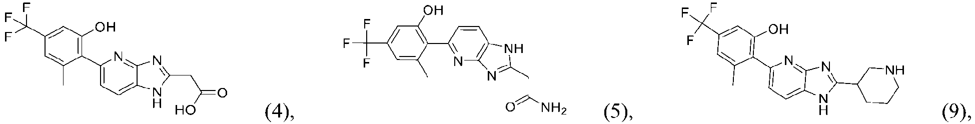

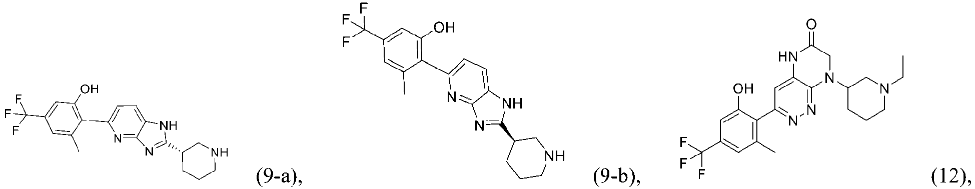

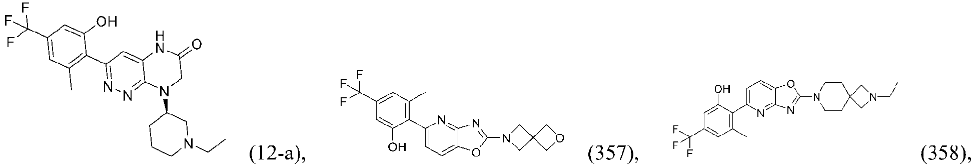

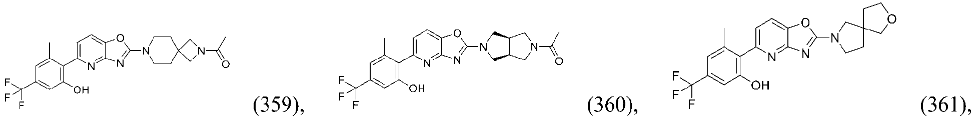

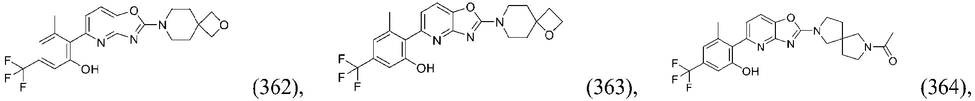

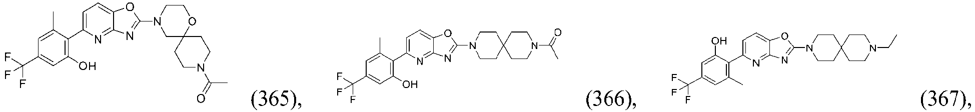

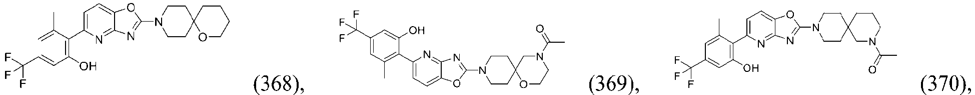

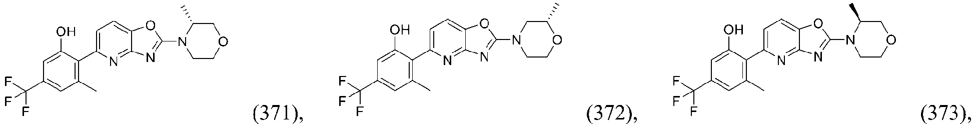

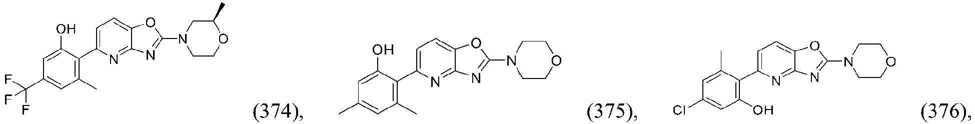

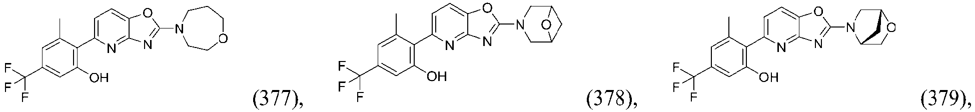

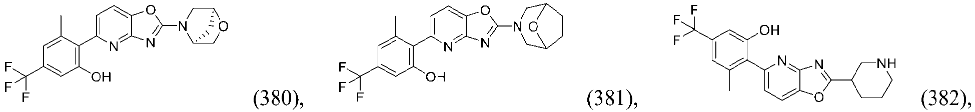

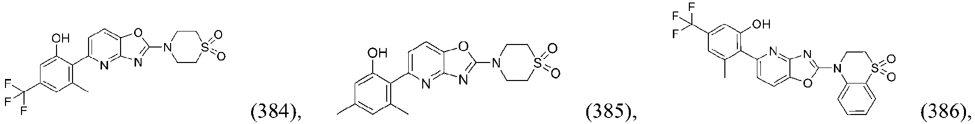

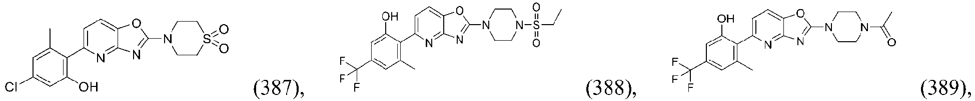

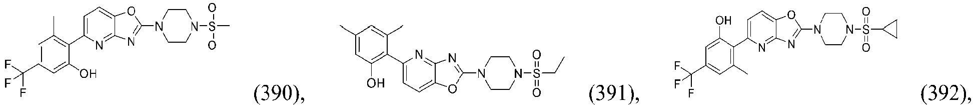

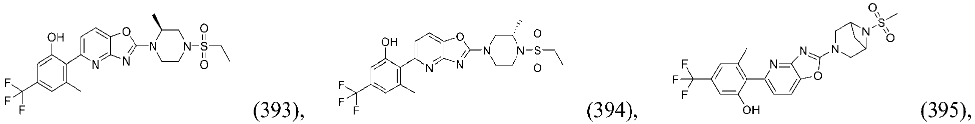

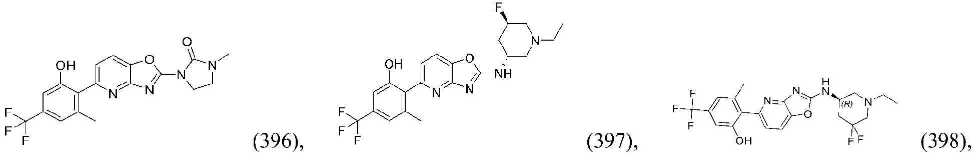

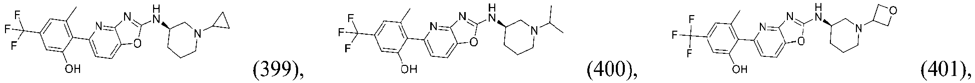

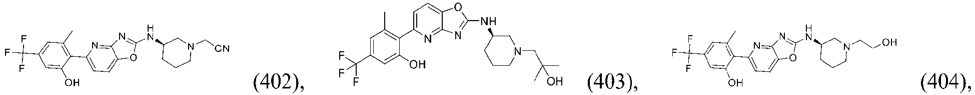

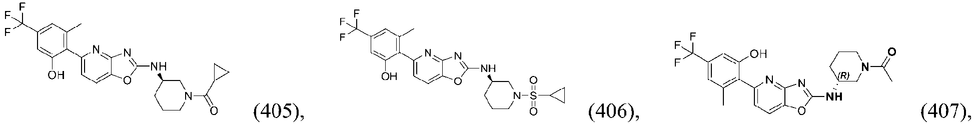

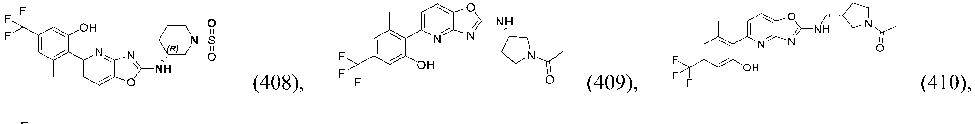

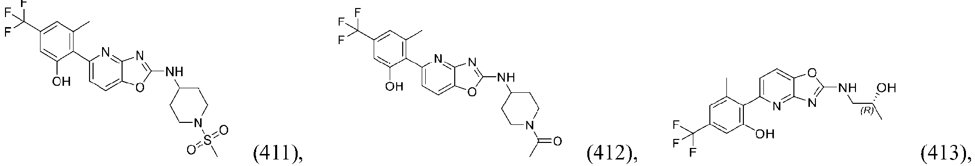

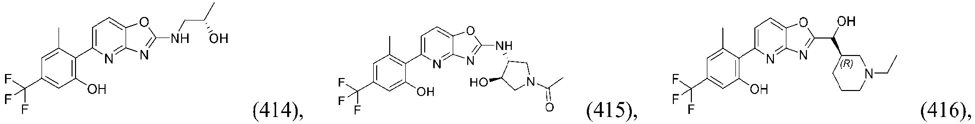

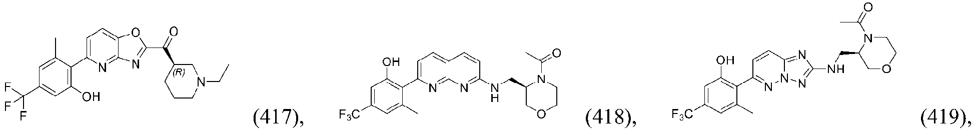

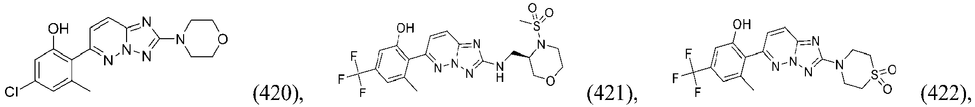

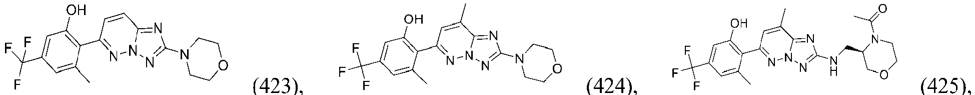

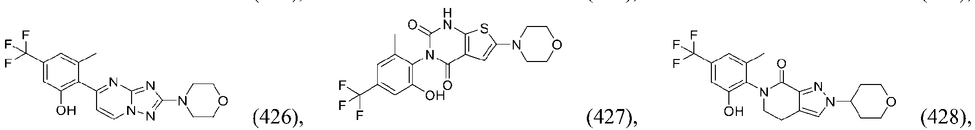

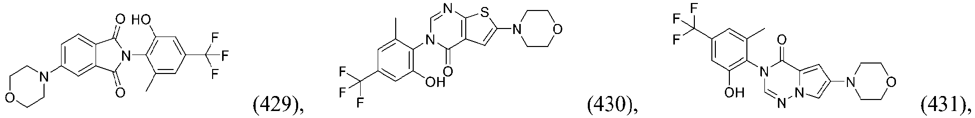

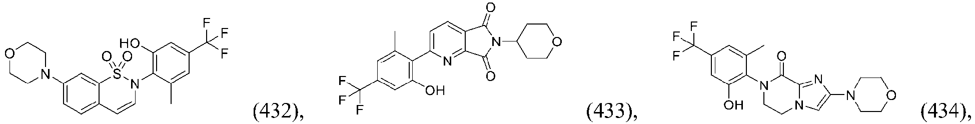

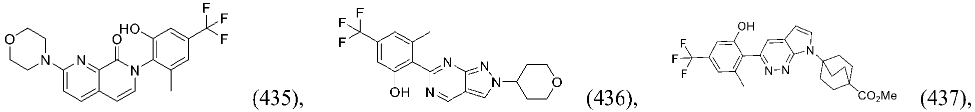

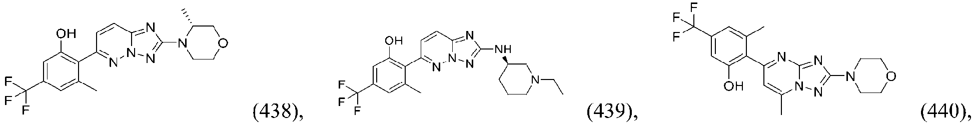

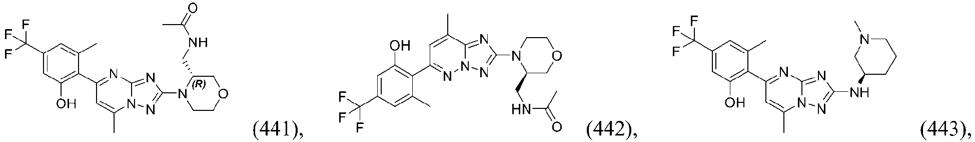

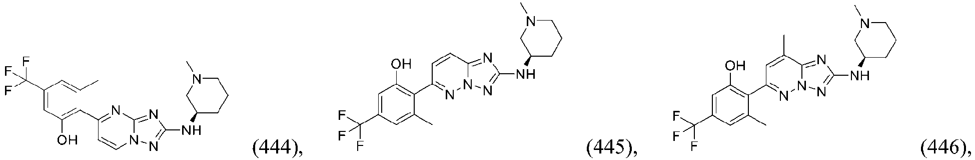

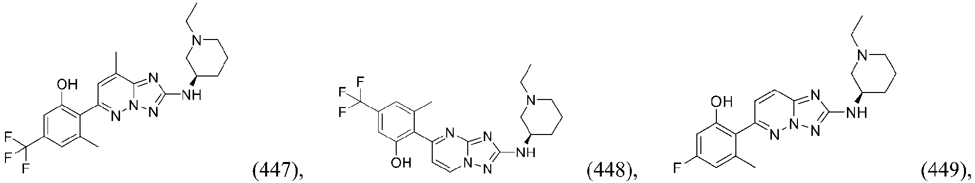

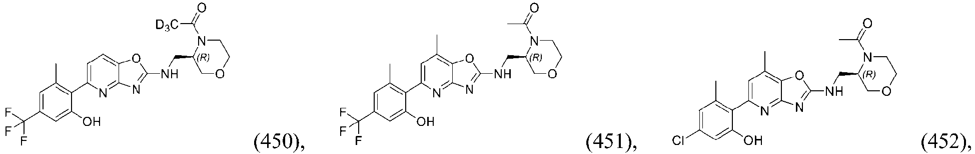

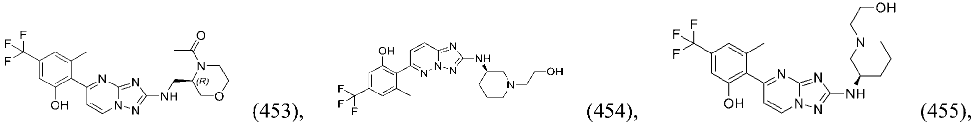

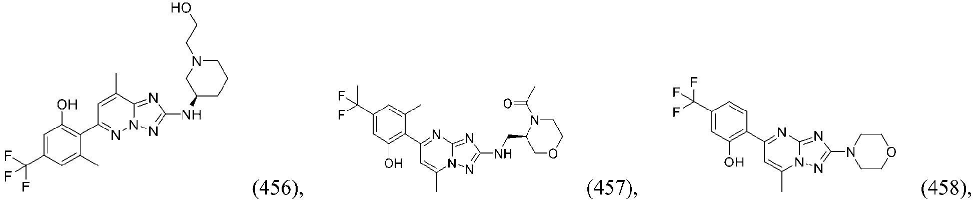

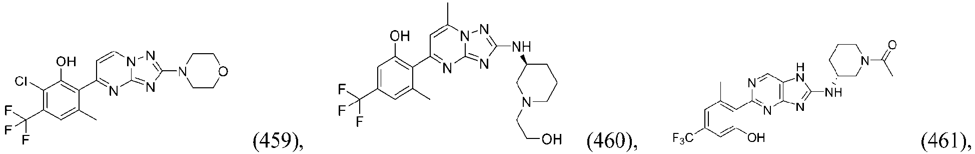

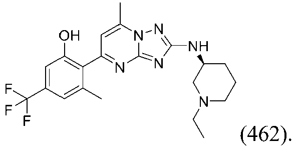

In another aspect, the present invention provides a pharmaceutical composition, which comprises a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to the present invention.

In another aspect, the present invention provides a combination, which comprises a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to the present invention, and one or more therapeutic agents.

In some embodiments of the combination of the present invention, the one or more therapeutic agents are independently selected from farnesoid X receptor (FXR) agonists; anti-steatosis agents; anti-fibrotic agents; JAK inhibitors; checkpoint inhibitors; chemotherapy, radiation therapy and surgery; urate-lowering therapy; anabolic agents and cartilage regeneration therapy; blockers of IL 17; complement inhibitors; Bruton's tyrosine kinase inhibitors (BTK inhibitors); Toll-like receptor inhibitors (TLR7/8 inhibitors); CAR T therapy; antihypertensive agents; cholesterol-lowering agents; leukotriene A4 hydrolase (LTAH4) inhibitors; SGLT2 inhibitors; β2 agonists; anti-inflammatory agents; nonsteroidal anti-inflammatory drugs ("NSAIDs"); acetylsalicylic acid drugs (ASAs); regenerative therapy treatments; cystic fibrosis treatments; and atherosclerosis treatments.

In another aspect, the present invention provides the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to the present invention, or the combination according to the present invention, for use as a medicament.

In another aspect, the present invention provides the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to the present invention, for use in the treatment of the following diseases or disorders, in which NLRP3 signaling contributes to the pathology, and/or symptoms, and/or progression of the disease or disorder. In another aspect, the present invention provides a method of treating the following disease or disorder, in which NLRP3 signaling contributes to the pathology, and/or symptoms, and/or progression of the disease or disorder, comprising administering a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to the present invention.

In another aspect, the present invention provides the use of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to the present invention, in the manufacture of the medicament for the treatment of the following disease or disorder, in which NLRP3 signaling contributes to the pathology, and/or symptoms, and/or progression of the disease or disorder.

In another aspect, the present invention provides the compound for use according to the present invention, or the treatment method of the present invention, wherein the disease or disorder is selected from: inflammasome-related disease/disorder, immune disease, inflammatory disease, autoimmune disease, or auto inflammatory disease, for example, auto inflammatory fever syndrome (e.g., cryptopyrin-associated periodic syndrome), liver-related disease/disorder (e.g., chronic liver disease, viral hepatitis, nonalcoholic steatohepatitis (NASH), alcoholic steatohepatitis, and alcoholic liver disease), inflammatory arthritis-related disorder (e.g., gout, pseudogout (chondrocalcinosis), osteoarthritis, rheumatoid arthritis, joint diseases such as acute and chronic joint diseases), kidney-related disease (e.g., hyperoxaluria, lupus nephritis, type I/type II diabetes and related complications (e.g., nephropathy, retinopathy, hypertensive nephropathy, hemodialysis-related inflammation), neuroinflammation-related diseases (e.g., multiple sclerosis, brain infection, acute injury, neurodegenerative diseases, Alzheimer's disease), cardiovascular/metabolic diseases/disorders (e.g., cardiovascular risk reduction (CvRR), hypertension, atherosclerosis, type I and type II diabetes and related complications, peripheral arterial disease (PAD), acute heart failure), inflammatory skin diseases (e.g., hidradenitis suppurativa, acne), wound healing and scarring, asthma, sarcoidosis, age-related macular degeneration, and cancer-related diseases/disorders (e.g., colon cancer, lung cancer, myeloproliferative neoplasms, leukemia, myelodysplastic syndrome (MDS), myelofibrosis).

In another aspect, the present invention provides a method of inhibiting NLRP3 inflammasome activity in a subject in need thereof, comprising administering to a subject in need thereof a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to the present invention.

Another aspect of the present invention is related to a methods for preparing, isolating and purifying the compound represented by Formula I, II, III, IV-1, IV-2, IV-3, IV-4, IV-5, IV-6, IV-7, IV-8, V-1, V-2, V-3, V-4, V-5, V-6, V-7, V-8, VI-1, VI-2, VI-3, VI-4, VI-5, VI-6, VI-7, VI-8, VII-1, VII-2, VII-3, VII-4, VII-5, VII-6, VII-7, VII-8, VIII-1, VIII-2, VIII-3, VIII-4, VIII-5, VIII-6, VIII-7, VIII-8, IX-1, IX-2, IX-3, IX-4, IX-5, or IX-6.

Any embodiment of any aspect of the present invention can be combined with other embodiments as long as they do not conflict. In addition, in any embodiment of any aspect of the present invention, any technical feature is applicable to the technical feature in other embodiments as long as they do not conflict.

The aforementioned contents only summarize certain aspects of the present invention, but the present invention is not limited to these aspects. These aspects and other aspects will be described in more detail and complete below. All references in this specification are incorporated herein by reference in their entirety.

### Detailed description

In order to make the purpose, technical scheme and advantages of the present invention clearer, the present invention is further described in detail below in conjunction with examples. The specific examples described herein are only used to explain the present invention and are not intended to constitute any limitation of the present invention. In addition, in the following description, the description of known structures and technologies is omitted to avoid unnecessary confusion of the concepts of the present invention. Such structures and technologies are also described in many publications.

### Definition

Certain embodiments of the present invention are now described in detail, examples of which are illustrated by the accompanying structural formulas and chemical formulas. The present invention is intended to encompass all alternatives, modifications, and equivalent technical solutions, all of which are included within the scope of the present invention as defined in the claims. It should be appreciated by those skilled in the art that many methods and materials similar or equivalent to those described herein can be used to practice the present invention. The present invention is in no way limited to the methods and materials described herein. In the event that one or more of the combined literature, patents, and similar materials differ or contradict the present application (including but not limited to defined terms, term applications, described technologies, etc.), the present application shall prevail.

It should be further appreciated that certain features of the invention, which for clarity are described in multiple independent embodiments, may also be provided in combination in a single embodiment. Conversely, various features of the invention, which for brevity are described in a single embodiment, may also be provided individually or in any suitable sub-combination.

Unless otherwise stated, all technical terms used in the present invention have the same meaning as commonly understood by those skilled in the art to which the present invention belongs. All patents and publications related to the present invention are incorporated herein by reference in their entirety.

Unless otherwise stated, the following definitions used herein should be applied. For purposes of the present invention, chemical elements are consistent with the Periodic Table of the Elements, CAS version, and Handbook of Chemistry and Physics, 75th edition, 1994. In addition, general principles of organic chemistry can be found in "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

Unless otherwise stated or there is an apparent conflict in the context, the articles "a", "an", and "the" as used herein are intended to include "at least one" or "one or more". Therefore, these articles as used herein refer to articles that refer to one or more than one (i.e., at least one) object. For example, "a component" refers to one or more components, i.e., there may be more than one component contemplated for employment or use in the implementation of the described embodiment.

The term "subject" refers to an animal. Typically, the animal is a mammal. Subjects, for example, also refer to primates (e.g., humans, male or female), cattle, sheep, goats, horses, dogs, cats, rabbits, rats, mice, fish, birds, etc. In certain embodiments, the subject is a primate. In other embodiments, the subject is a human.

The term "patient" refers to a human (including adults and children) or other animals. In some embodiments, "patient" refers to a human.

The term "comprise(s)" is an open expression, that is, including the contents specified in the present invention but not excluding other contents.

When substituents are described by conventional chemical formulas written from left to right, the substituents also include chemically equivalent substituents that would result if the formula were written from right to left. For example, -CH₂O- is equivalent to -OCH₂-.

The term "enantiomers" refers to two non-superimposable isomers of a compound that are mirror images of each other.

The term "diastereomers" refers to stereoisomers that have two or more chiral centers and the molecules are not mirror images of each other. Diastereomers have different physical properties, such as melting points, boiling points, spectral properties and reactivity. Diastereomeric mixtures can be separated by high-resolution analytical procedures such as electrophoresis and chromatography, such as HPLC.

The term "racemate", "raceme" or "racemic mixture" refers to an equimolar mixture of two enantiomers that lacks optical activity.

The term "tautomers" or "tautomeric forms" refers to structural isomers with different energies that can be converted into each other through a low energy barrier. If tautomerism is possible (e.g., in solution), a chemical equilibrium of tautomers can be achieved. For example, proton tautomers (also called prototropic tautomers) involve in interconversion that occurs by proton migration, such as keto-enol isomerization and imine-enamine isomerization. Valence tautomers involve in interconversion that occurs by reconfiguration of some of the bonding electrons. A specific example of keto-enol tautomerism is the interconversion of pentane-2,4-dione and 4-hydroxypent-3-en-2-one tautomers. Another example of tautomerism is phenol-keto tautomerism. A specific example of phenol-keto tautomerism is the interconversion of pyridin-4-ol and pyridin-4(1H)-one tautomers. For another example, the hydrogen on the 1-nitrogen atom on the imidazole ring can be transferred to the 3-nitrogen atom, and the two forms of the imidazole ring are tautomers, that is, the group containing the imidazole ring herein has tautomerism, such as 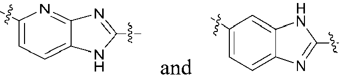 are tautomers; and 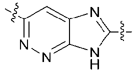 and 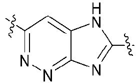 are tautomers. Unless otherwise indicated, all tautomeric forms of the compound of the present invention are within the scope of the present invention.

The term "stereoisomers" refers to compounds that have the same chemical constitution, but differ in the way the atoms or groups are arranged in space. Stereoisomers include enantiomers, diastereomers, conformers (rotamers), geometric isomers (cis/trans isomers), atropisomers, and the like.

The term "geometric isomer" is also called "cis-trans isomer", which is an isomer caused by double bond (including olefin double bond, C=N double bond, and N=N double bond) or a single bond of ring carbon atom that cannot rotate freely.

The stereochemical definitions and rules used in the present invention generally follow those described by S. P. Parker, Ed., McGraw-Hill Dictionary of Chemical Terms (1984) McGraw-Hill Book Company, New York; and Eliel, E. and Wilen, S, "Stereochemistry of Organic Compounds", John Wiley & Sons, Inc, New York, 1994. Many organic compounds exist in optically active forms, that is, they have the ability to rotate the plane of plane polarized light. When describing optically active compounds, the prefixes D and L or R and S are used to indicate the absolute configuration of the molecule about its one or more chiral centers. The prefixes d and 1 or (+) and (-) are the symbols used to specify the rotation of plane polarized light caused by the specific compound, wherein (-) or 1 indicates that the compound is left-handed, and the prefix (+) or d indicates that the compound is right-handed. A specific stereoisomer is an enantiomer, and a mixture of such isomers is called an enantiomeric mixture. A mixture of enantiomers at ratio of 50:50 is called a racemic mixture or racemate, which can occur when there is no stereo selectivity or stereospecificity in a chemical reaction or process.

Any asymmetric atom (e.g., carbon, etc.) of the compound disclosed herein can exist in a racemic or enantiomerically enriched form, such as in form of (R)-, (S)-, or (R,S)-configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess, in terms of (R)- or (S)-configuration.

Depending on the choice of starting materials and process, the compound of the present invention can exist in the form of one of its possible isomers or a mixture thereof, such as a racemate and a diastereomeric mixture (depending on the number of asymmetric carbon atoms). Optically active (R)- or (S)-isomer may be prepared using a chiral synthon or chiral reagent, or resolved using conventional techniques. If the compound contains a double bond, the substituents may be in the E or Z configuration; if the compound contains a disubstituted cycloalkyl, the cycloalkyl substituents may be in the cis or trans configuration.

Any mixture of stereoisomers obtained may be separated into pure or substantially pure geometric isomers, enantiomers, diastereomers, on the basis of differences in the physicochemical properties of components, for example, by chromatography and/or fractional crystallization.

Any racemate of the resulting final product or intermediate may be resolved into the optical enantiomers by methods familiar to those skilled in the art, for example, by separation of the diastereomeric salts thereof as obtained. The racemic product may also be separated by chiral chromatography, for example, by high performance liquid chromatography (HPLC) using a chiral adsorbent. In particular, enantiomers can be prepared by asymmetric synthesis, for example, by referring to Jacques, et al., Enantiomers, Racemates and Resolutions (Wiley Interscience, New York, 1981); Principles of Asymmetric Synthesis (2nd Ed. Robert E. Gawley, Jeffrey Aube, Elsevier, Oxford, UK, 2012); Eliel, E. L. Stereochemistry of Carbon Compounds (McGraw-Hill, NY, 1962); Wilen, S. H. Tables of Resolving Agents and Optical Resolutions p. 268 (E. L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "nitrogen oxide" indicates that when a compound contains several amino groups, one or more nitrogen atoms may be oxidized to form a nitrogen oxide. Specific examples of nitrogen oxides are nitrogen oxides of tertiary amines or nitrogen oxides of nitrogen atoms of nitrogen-containing heterocyclic rings. The corresponding amines can be treated with oxidants such as hydrogen peroxide or peracids (e.g., peroxycarboxylic acids) to form nitrogen oxides (see, Advanced Organic Chemistry, Wiley Interscience, 4th edition, Jerry March, pages). In particular, nitrogen oxides can be prepared by the method of L.W. Deady (Syn. Comm. 1977, 7, 509-514), wherein, for example, the amine compound is reacted with meta-chloroperbenzoic acid (MCPBA) in an inert solvent such as dichloromethane.

The term "metabolite" refers to a product obtained by the metabolism of a specific compound or its salt in vivo. The metabolite of a compound can be identified by techniques known in the art, and its activity can be characterized by experimental methods as described in the present invention. Such products can be obtained by administering the compound through oxidation, reduction, hydrolysis, amidation, deamidation, esterification, defatting, enzymatic cleavage, etc. Accordingly, the present invention includes metabolites of compounds, including metabolites produced by contacting the compounds of the present invention with mammals for a period of time.

The term "pharmaceutically acceptable" means that a substance or composition must be chemically and/or toxicologically compatible with other ingredients of a formulation and/or a mammal treated therewith. Preferably, the "pharmaceutically acceptable" described in the present invention refers to that it is approved by federal regulatory agencies or national governments or listed in the United States Pharmacopoeia or other generally recognized pharmacopoeias for use in animals, especially humans.

The term "pharmaceutically acceptable salt" refers to organic salts and inorganic salts of the compounds of the present invention. Pharmaceutically acceptable salts are well known in the art, as described in S. M. Berge et al., J. Pharmaceutical Sciences, 66: 1-19, 1977. Pharmaceutically acceptable salts include salts formed by compounds with acids, including but not limited to inorganic acid salts (such as hydrochloride, hydrobromide, phosphate, sulfate, nitrate, perchlorate) and organic acid salts (such as acetate, glycolate, oxalate, maleate, tartrate, citrate, succinate, fumarate, mandelate, sulfosalicylate), or other methods described in books and literature, such as ion exchange methods, to obtain these salts. More pharmaceutically acceptable salts include adipate, alginate, ascorbate, aspartate, benzenesulfonate, benzoate, bisulfate, borate, butyrate, camphorate, camphorsulfonate, cyclopentylpropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, malonate, methanesulfonate, 2-naphthalenesulfonate, nicotinate, oleate, palmitate, pamoate, pectinate, persulfate, 3-phenylpropionate, picrate, pivalate, propionate, stearate, thiocyanate, p-toluenesulfonate, undecanoate, valerate, and the like. Pharmaceutically acceptable salts also include salts formed from compounds and bases, including but not limited to, inorganic base salts (such as alkali metal salt, alkaline earth metal salt, ammonium salt and N+(C1-4 alkyl)4 salt), alkali metal or alkaline earth metal salts include sodium, lithium, potassium, calcium, magnesium, and the like. The present invention also contemplates quaternary ammonium salts formed by compounds of any group containing N. Water-soluble or oil-soluble or dispersed products can be obtained by quaternization. Pharmaceutically acceptable salts further include appropriate, non-toxic ammonium, quaternary ammonium salts and amine cations formed by counter ions, such as halides, hydroxides, carboxylates, sulfates, phosphates, nitrates, C1-8 sulfonates and aromatic sulfonates. Organic base salts (e.g., primary, secondary, and tertiary amine salts, substituted amines (including naturally occurring substituted amines, cyclic amines, basic ion exchange resins) salts), certain organic amine salts include, for example, isopropylamine salts, benzathine salts, cholinate salts, diethanolamine salts, diethylamine salts, lysine salts, meglumine salts, piperazine salts, and tromethamine salts.

Pharmaceutically acceptable acid addition salts can be formed by the compounds of the present invention and inorganic or organic acid, and pharmaceutically acceptable base addition salts can be formed by the compounds of the present invention and inorganic or organic base. Pharmaceutically acceptable salts of the present invention can be synthesized by conventional chemical methods from parent compounds, alkaline or acidic moieties. Generally, such salts can be prepared by reacting the free acid forms of these compounds with a stoichiometric amount of a suitable base (such as hydroxides, carbonates, bicarbonates, etc. of Na, Ca, Mg or K), or by reacting the free base forms of these compounds with a stoichiometric amount of a suitable acid. Such reactions are usually carried out in water or organic solvents or mixtures of the two. Generally, in appropriate cases, it is necessary to use a nonaqueous medium such as ether, ethyl acetate, ethanol, isopropanol or acetonitrile. Additional lists of suitable salts can be found, for example, in "Remington's Pharmaceutical Sciences", 20th edition, Mack Publishing Company, Easton, Pa., (1985); and in "Handbook of Pharmaceutical Salts: Properties, Selection, and Use", Stahl and Wermuth (Wiley-VCH, Weinheim, Germany, 2002).

The term "solvate" refers to an associated matter formed by one or more solvent molecules and the compound of the present invention. The solvent can be water, acetic acid, ether, isopropyl ether, petroleum ether, ethyl formate, ethyl acetate, isopropyl acetate, n-propyl acetate, isobutyl acetate, n-butyl acetate, methyl tert-butyl ether (MTBE), n-heptane, a mixed solvent of ethanol and water in a volume ratio of 10:90 to 90:10, acetone, methyl isobutyl ketone, acetonitrile, benzene, chloroform, carbon tetrachloride, dichloromethane, dimethyl sulfoxide, 1,4-dioxane, ethanol, ethyl acetate, ethylene glycol, n-butanol, tert-butanol, sec-butyl alcohol, N,N-dimethylacetamide, N,N-dimethylformamide, formamide, formic acid, n-hexane, cyclohexane, n-heptane, a mixed solvent of n-heptane and ethyl acetate in a volume ratio of 1:5 to 5:1, isopropanol, methanol, butanone, 1-methyl-2-pyrrolidone, mesitylene, nitromethane, polyethylene glycol, n-propanol, isopropanol, 2-acetone, 4-methyl-2-pentanone, pyridine, tetrahydrofuran, methyl ethyl ketone, toluene, xylene, isopropylbenzene or a mixture thereof, etc.

The term "hydrate" refers to an associated matter formed by one or more water molecules and the compound of the present invention.

In addition, the compounds disclosed in the present invention, including their salts, can also be obtained in the form of their hydrates or in the form of containing their solvents (such as ethanol, DMSO, etc.) for their crystallization. The compounds disclosed in the present invention can form solvates with pharmaceutically acceptable solvents (including water) inherently or by design; therefore, the present invention is intended to include both solvated and unsolvated forms.

The term "ester" is represented by the formula -OC(O)R or -C(O)OR, wherein R can be an alkyl, cycloalkyl, alkenyl, cycloalkenyl, alkynyl, cycloalkynyl, aryl or heteroaryl as described herein.

The term "isotope-labeled" compound refers to a compound of the present invention is labeled with an isotope. They are identical to those compounds described herein except for the fact that one or more atoms are replaced by atoms having an atomic mass or mass number different from the natural common atomic mass or mass number. Exemplary isotopes that may also be introduced into the compounds of the present invention include isotopes of hydrogen, carbon, nitrogen, oxygen, phosphorus, sulfur, fluorine, and chlorine, such as ²H, ³H, ¹³C, ¹⁴C, ¹⁵N, ¹⁶O, ¹⁷O, ³¹P, ³²P, ³⁶S, ¹⁸F and ³⁷Cl.

The compounds of the present invention comprising the aforementioned isotope and/or other isotope of other atoms and pharmaceutically acceptable salts of the compounds are all included within the scope of the present invention. Isotope-labeled compounds of the present invention, such as radioisotopes, such as ³H and ¹⁴C are incorporated into the compounds of the present invention and can be used for drug and/or substrate tissue distribution analysis. Due to ease of preparation as well as detection, deuterated, i.e., ³H, and carbon-14, i.e., ¹⁴C, isotopes are particularly preferred. In addition, substitution with isotopes of larger mass numbers, such as deuterium, i.e., ²H, can provide some greater therapeutic advantages of metabolic stability, such as increased in vivo half-life or reduced dosage requirements. Therefore, it may be preferred in some cases.

In addition, the substitution of heavier isotopes, particularly deuterium (i.e., ²H or D) can provide certain therapeutic advantages, which are brought about by higher metabolic stability. For example, an increased in vivo half-life or a reduction in dosage requirements or an improved therapeutic index. It should be understood that deuterium in the present invention is regarded as a substituent of compounds of formula (I) to (IV). The concentration of such heavier isotopes, particularly deuterium, can be defined by an isotopic enrichment factor. The term "isotopic enrichment factor" used in the present invention refers to the ratio between the isotopic abundance and the natural abundance of a specified isotope. Where a substituent of a compound of the invention is designated as deuterium, the compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). Pharmaceutically acceptable solvates according to the invention include those wherein the solvent of crystallization may be isotopically substituted, for example D₂O, acetone-d6, DMSO-d6.

The term "prodrug", as used in the present invention, represents the in vivo conversion of a compound to a compound of formula (I). Such conversion is affected by the hydrolysis of the prodrug in the blood or the enzymatic conversion of the prodrug in the blood or tissues into the parent structure. The prodrug compound of the present invention can be an ester, and among the existing invention, esters that can be used as prodrugs include phenyl esters, aliphatic (C1-24) esters, acyloxymethyl esters, carbonates, carbamates and amino acid esters. For example, a compound in the present invention contains a hydroxyl group, which can be acylated to obtain a compound in the form of a prodrug. Other prodrug forms include phosphate esters, such as these phosphate ester compounds obtained by phosphorylation of the hydroxyl group on the parent. For a complete discussion of prodrugs, please refer to the following literature: Higuchi et al., Pro-drugs as Novel Delivery Systems, Vol. 14, A.C.S. Symposium Series; Roche et al., Bioreversible Carriers in Drug Design, American Pharmaceutical Association and Pergamon Press, 1987; Rautio et al., Prodrugs: Design and Clinical Applications, Nature Reviews Drug Discovery, 2008, 7, 255-270, and Hecker et al., Prodrugs of Phosphates and Phosphonates, J. Med. Chem., 2008, 51, 2328-2345.

Unless otherwise explicitly stated, the descriptions used in the present invention such as "each...independently", "...each independently" and "...independently" are interchangeable and should be understood broadly, Which can mean that the specific options expressed by the same symbols in different groups do not affect each other, and can also mean that the specific options expressed by the same symbols in the same group do not affect each other.

The terms "optional", "optionally" or "arbitrary", "arbitrarily" means that the event or situation described subsequently may but need not occur, and the description includes instances where the event or situation occurs and instances where it does not occur. For example, "optionally substituted with ..." means that the substitution may or may not be present.

The term "unsaturation" or "unsaturated" refers to the moiety containing one or more degrees of unsaturation.

In various parts of this specification, the substituents of the compounds of the present invention are disclosed in terms of group types or ranges. It is particularly noted that the present invention comprises each independent secondary combination of the individual members of these group types and ranges. For example, the term "C₁₋₆ alkyl" specifically refers to independently disclosed methyl, ethyl, C₃ alkyl, C₄ alkyl, C₅ alkyl and C₆ alkyl.

In various parts of the present invention, linking substituents are described. When the structure clearly requires a linking group, the Markush variable listed for that group should be understood as a linking group. For example, if the structure requires a linking group and the Markush group definition for that variable lists "alkyl" or "aryl", it should be understood that the "alkyl" or "aryl" represents a linked alkylene group or arylene group, respectively.

The term "heteroatom" refers to O, S, N, P and Si, including any oxidation state of S, N and P; primary, secondary, tertiary amines and quaternary ammonium salts; or the hydrogen on the nitrogen atom in the heterocyclic ring is substituted, for example, N (such as N in 3,4-dihydro-2H-pyrrolyl), NH (such as NH in pyrrolidinyl) or NRT (such as NRT in N-substituted pyrrolidinyl, RT is a substituent on N). Among the compounds involved in the present invention, when containing multiple heteroatoms, the compounds composed thereof conform to the covalent rules and composition rules of organic compounds, that is, the compounds containing multiple heteroatoms should exclude compounds that do not conform to the covalent rules and composition rules of organic compounds.

The term "heterocyclyl" or "heterocycle" refers to a monovalent or polyvalent monocyclic, bicyclic or tricyclic ring system containing carbon atoms and heteroatoms. The heteroatoms have the meanings as described herein. The bicyclic or tricyclic ring system can be a spirocyclic, bridged or fused ring, for example, a bicyclic system includes a spirobicyclic, a bridged bicyclic or a fused bicyclic. Saturated or partially unsaturated heterocyclyl can be fully saturated or contain one or more degrees of unsaturation, but none of the aromatic rings. In one embodiment, the heterocyclyl group is a 3-6 membered ring, such as a 3-6 membered saturated or partially unsaturated heterocyclyl (2-6 carbon atoms and 1-3 heteroatoms selected from N, O, P, S, wherein S or P is optionally substituted by one or more oxygen atoms to obtain groups such as SO, SO₂, PO, PO₂). In one embodiment, the saturated or partially unsaturated heterocyclyl is selected from: saturated monocyclic heterocyclyl, saturated bicyclic heterocyclyl, saturated tricyclic heterocyclyl, partially unsaturated monocyclic heterocyclyl, partially unsaturated bicyclic heterocyclyl, partially unsaturated tricyclic heterocyclyl. Bicyclic heterocyclyl refers to heterocyclyl of bicyclic system, such as bicyclic heterocyclic systems including spiro bicyclic heterocyclic rings, bridged bicyclic heterocyclic rings, and fused bicyclic heterocyclic rings. Monocyclic heterocyclyl refer to heterocyclyl of monocyclic systems. 5-6 membered heterocyclyl refer to heterocyclyl with 5-6 ring atoms. 4-7 membered heterocyclyl refer to heterocyclyl with 4-7 ring atoms. 3-6 membered heterocyclyl refer to heterocyclyl with 3-6 ring atoms.

The term "cycloalkyl" refers to a monocyclic, bicyclic or tricyclic ring system containing carbon atoms, which is monovalent or polyvalent. Saturated or partially unsaturated cycloalkyl may be fully saturated or contain one or more degrees of unsaturation, without an aromatic ring. In one embodiment, the cycloalkyl contains 3-6 carbon atoms, such as C₃-C₆ saturated or partially unsaturated cycloalkyl. Examples of cycloalkyl groups include, but are not limited to, cyclopropyl, cyclobutyl, cyclopentyl, cyclohexyl, cyclopentenyl, cyclohexenyl, and the like. In one embodiment, the saturated or partially unsaturated cycloalkyl is selected from: saturated monocyclic cycloalkyl, saturated bicyclic cycloalkyl, saturated tricyclic cycloalkyl, partially unsaturated monocyclic cycloalkyl, partially unsaturated bicyclic cycloalkyl, partially unsaturated tricyclic cycloalkyl. Bicyclic cycloalkyl refers to cycloalkyl of bicyclic ring systems, for example, bicyclic cycloalkyl system include spiro bicyclic cycloalkyl, bridged bicyclic cycloalkyl, and fused bicyclic cycloalkyl. Monocyclic cycloalkyl refers to cycloalkyl of monocyclic ring systems. The 4-7-membered cycloalkyl refers to a cycloalkyl having 4 to 7 ring atoms. The 3-6-membered cycloalkyl refers to a cycloalkyl having 3 to 6 ring atoms.

The term "heteroaryl" refers to a monocyclic, bicyclic and tricyclic aromatic system containing 5-10 ring atoms. The term "heteroaryl" can be used interchangeably with the term "heteroaromatic ring" or "heteroaromatic compound". In some embodiments, the heteroaryl is a heteroaryl consisting of 5 to 10 atoms containing 1, 2, 3 or 4 heteroatoms independently selected from O, S and N, i.e., a 5-10 membered heteroaryl; the heteroaryl is a heteroaryl consisting of 5 to 8 atoms containing 1, 2, 3 or 4 heteroatoms independently selected from O, S and N, i.e., a 5-8 membered heteroaryl; in some embodiments, the heteroaryl is a heteroaryl consisting of 5 to 7 atoms containing 1, 2, 3 or 4 heteroatoms independently selected from O, S and N, i.e., a 5-7 membered heteroaryl; in some embodiments, the heteroaryl is a heteroaryl consisting of 5-6 atoms containing 1, 2, 3 or 4 heteroatoms independently selected from O, S and N, i.e., a 5-6 membered heteroaryl; in some embodiments, the heteroaryl is a heteroaryl consisting of 5 atoms containing 1, 2, 3 or 4 heteroatoms independently selected from O, S and N, i.e., a 5-membered heteroaryl; in some embodiments, the heteroaryl is a heteroaryl consisting of 6 atoms containing 1, 2, 3 or 4 heteroatoms independently selected from O, S and N, i.e., a 6-membered heteroaryl.

The term "aryl" refers to a monocyclic, bicyclic, or tricyclic aromatic carbocyclic system. The term "aryl" can be used interchangeably with the term "aryl ring" or "aromatic ring". 6-10 membered aryl refers to an aryl group containing 6-10 ring atoms. Examples include, but are not limited to, phenyl, naphthyl, etc.

The term "hydrogen" refers to ¹H; "deuterium" refers to ²H.

The terms "halogen" and "halo" refer to fluorine (F), chlorine (Cl), bromine (Br), or iodine (I).

The term "amino" refers to -NH₂.

The term "hydroxy" refers to -OH.

The term "cyano" refers to -CN.

The term "nitro" refers to -NO₂.

The term "carboxyl" refers to HO(C=O)-.

The term "O=" refers to oxo, i.e., when the substituent is O=, the O is connected to the substituted group by a double bond.

The term "alkyl" or "alkyl group" refers to a saturated, straight or branched chain monovalent hydrocarbon group containing carbon atoms. In one embodiment, the alkyl group contains 1-6 carbon atoms, i.e., C₁-C₆ alkyl; in another embodiment, the alkyl group contains 1-4 carbon atoms, i.e., C₁-C₄ alkyl; in another embodiment, the alkyl group contains 1-3 carbon atoms, i.e., C₁-C₃ alkyl. Examples of alkyl include, but are not limited to, methyl, ethyl, n-propyl, isopropyl, n-butyl, tert-butyl, isobutyl, sec-butyl, n-pentyl, n-hexyl, and the like.

The term "alkoxy" refers to an alkyl group attached to the rest of the molecule via an oxygen atom, wherein the alkyl group has the meaning as described herein. In one embodiment, the alkoxy group contains 1-6 carbon atoms, i.e., C₁-C₆ alkoxy; in another embodiment, the alkoxy group contains 1-4 carbon atoms, i.e., C₁-C₄ alkoxy; in yet another embodiment, the alkoxy group contains 1-3 carbon atoms, i.e., C₁-C₃ alkoxy.

The term "alkylamino" refers to an alkyl group attached to the rest of the molecule via a nitrogen atom, wherein the alkyl group has the meaning as described herein. In one embodiment, the alkylamino is a monoalkylamino, represented by "alkyl-NH-". In one embodiment, the alkylamino is a dialkylamino, represented by "(alkyl)₂N-". In one embodiment, the alkylamino group contains 1-6 carbon atoms, i.e., C₁-C₆ alkylamino; in another embodiment, the alkylamino group contains 1-4 carbon atoms, i.e., C₁-C₄ alkylamino; in yet another embodiment, the alkylamino group contains 1-3 carbon atoms, i.e., C₁-C₃ alkylamino. The term "haloalkyl" indicates that the hydrogen on the alkyl is substituted by one or more halogens (F, Cl, Br). The term "hydroxyalkyl" indicates that the hydrogen on the alkyl is substituted by one or more hydroxyl (OH).

The term "aminoalkyl" indicates that the hydrogen on the alkyl is substituted by one or more amino (NH₂).

One or more halogenated phenyl indicates that the phenyl is substituted with 1, 2, 3, 4 or 5 halogens. The halogens have the definition as described herein. One or more halogenated phenyl is also represented as phenyl substituted with one or more halogens.

One or more hydroxyl-substituted phenyl refers to that phenyl is substituted with 1, 2, 3, 4 or 5 hydroxyls. The hydroxyl has the definition as described herein.

When two ring system groups are connected by "fused", it means that the two ring systems are in a fused relationship, with each of the two corresponding rings having its own definition as described herein. For example, phenyl fused 4-7-membered cycloalkyl means that the phenyl is fused with a cycloalkyl having 4-7 ring atoms as described herein; 5-6 membered heteroaryl fused 4-7 membered cycloalkyl means that the heteroaryl having 5-6 ring atoms as described herein is fused with a cycloalkyl having 4-7 ring atoms as described herein.

When two groups are used in combination, it means that the group written first is connected to the rest of the molecule through the group written behind. For example, C₁₋₆ alkoxy-C₁₋₆ alkyl means that the C₁₋₆ alkoxy is connected to the rest of the molecule through the C₁₋₆ alkylene, and hydroxy C₁₋₆ alkyl means that the hydroxyl is connected to the rest of the molecule through the C₁₋₆ alkylene.

As described in the present invention, a ring system (as shown in the figure below) formed by connecting the substituent R to the center of the ring by a bond means that the substituent R is limited to substitution at any substitutable or any reasonable position on the ring A. For example, formula f represents any possible position on the ring A that can be substituted, as shown in formulas f¹-f⁴:

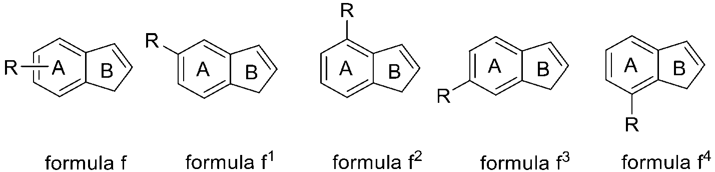

As described in the present invention, the ring system formed by connecting the substituent R to the ring system by a bond through the multiple rings (as shown in the figure below) means that the substituent R can be substituted at any substitutable or any reasonable position on the ring A and the ring B. For example, formula e represents any possible substituted position on the ring A and the ring B, as shown in formula e¹-e⁷:

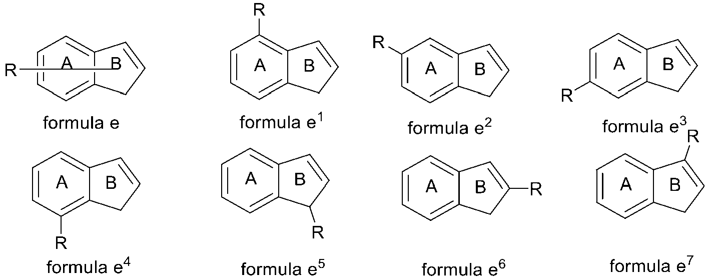

As described in the present invention, the ring system formed by connecting the substituent to the center of the ring by a bond such as (R^{x})ₙ means that n substituents R^{x} can be substituted at any substitutable position on the ring. For example, formula a represents that the benzene ring can be substituted with n R^{x}.

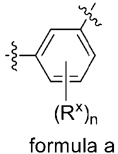

The term "substituted" indicates that one or more hydrogen atoms on a specific group are substituted by a specific substituent. The specific substituent is a substituent described above, or a substituent appearing in the embodiments. Unless otherwise specified, a substituted group may have a substituent selected from a specific group at any substitutable position of the group, and the substituent may be the same or different at each position, i.e., each substitution is independent of each other. It will be understood by those skilled in the art that the combinations of substituents contemplated by the present invention are those that are stable or chemically feasible.

In the present invention, 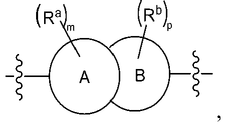 which relates to ring A and ring B, refers to a fused structure formed by ring A and ring B sharing a common ring edge (i.e., ring A and ring B form a fused bicyclic ring), for example

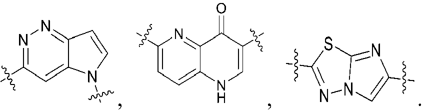

In the present invention, the definition of substituents means connecting to the corresponding groups from left to right. For example, in the structure of Formula I 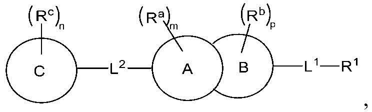 when R^{c} is selected from C₁₋₆ alkoxy-C₁₋₆ alkyl, it means that the C₁₋₆ alkoxy is connected to the C₁₋₆ alkyl, and the C₁₋₆ alkyl is further connected to the ring C.

### Description of the compounds of the present invention

The present invention provides a compound having the structure represented by Formula I, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof, 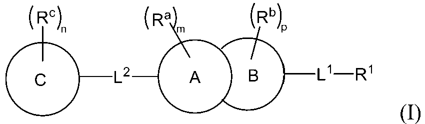
wherein, 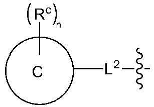 is connected to ring A, 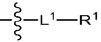 is connected to ring B;
wherein, ring A, ring B, ring C, L¹, L², R^{a}, R^{b}, R^{c}, n, m, p, and R¹ have the definition as defined in the present invention.

In some embodiments, the present invention provides a compound having the structure represented by Formula II, 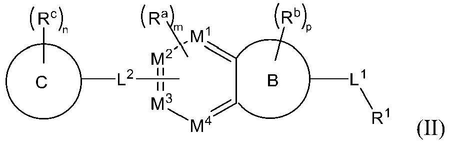 wherein, ring B, ring C, L¹, L², R^{a}, R^{b}, R^{c}, n, m, p, R¹, M¹, M², M³, and M⁴ have the definition as defined in the present invention.

In some embodiments, the present invention provides a compound having the structure represented by Formula III, 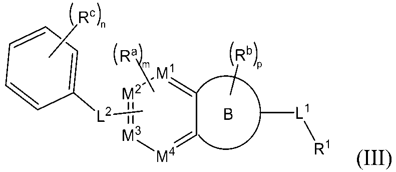 wherein, ring B, L¹, L², R^{a}, R^{b}, R^{c}, n, m, p, R¹, M¹, M², M³, and M⁴ have the definition as defined in the present invention.

In some embodiments, the present invention provides a compound having the structure represented by Formula IV-1, IV-2, IV-3, IV-4, IV-5, IV-6, IV-7 or IV-8; 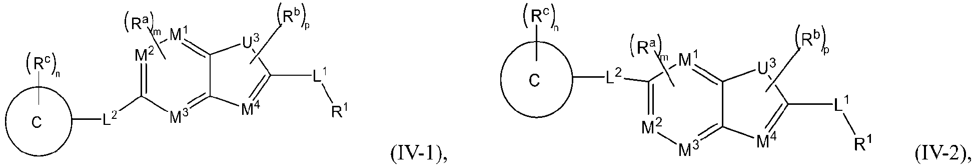 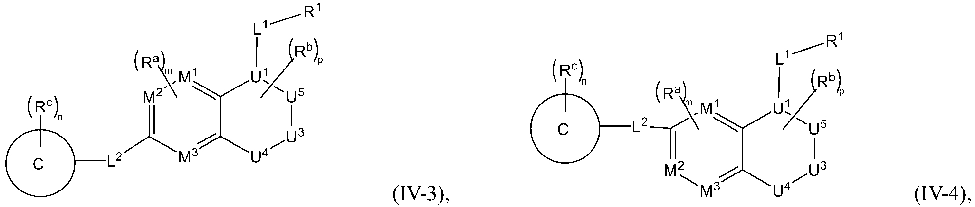 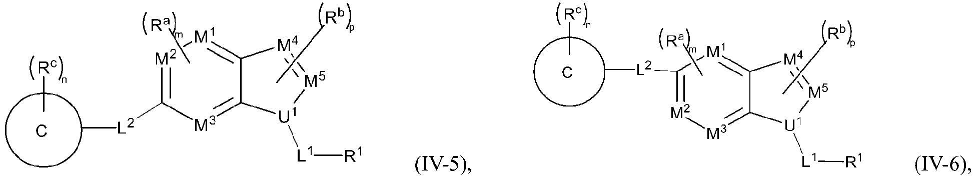 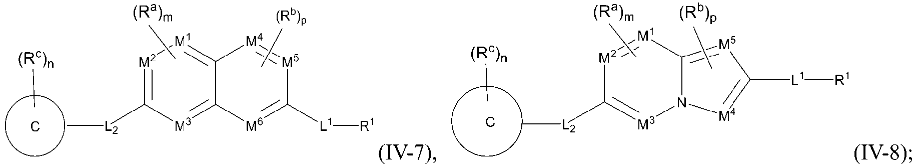 wherein, ring C, L¹, L², R^{a}, R^{b}, R^{c}, n, m, p, R¹, M¹, M², M³, M⁴, M³, M⁶, U¹, U³, U⁴, and U⁵ have the definition as defined in the present invention.

In some embodiments, the present invention provides a compound having the structure represented by Formula V-1, V-2, V-3, V-4, V-5, V-6, V-7 or V-8; 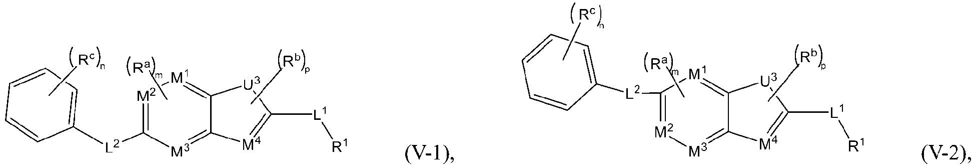 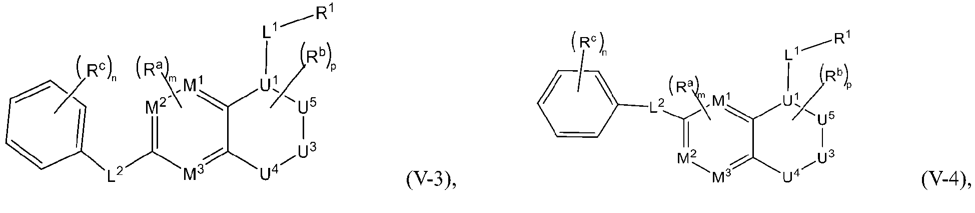 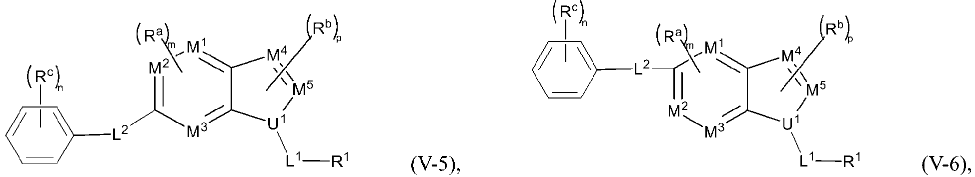 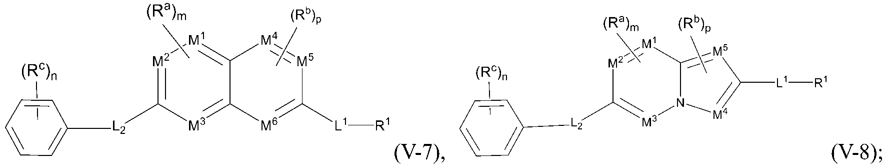 wherein, L¹, L², R^{a}, R^{b}, R^{c}, n, m, p, R¹, M¹, M², M³, M⁴, M³, M⁶, U¹, U³, U⁴, and U⁵ have the definition as defined in the present invention.

In some embodiments, the present invention provides a compound having the structure represented by Formula VI-1, VI-2, VI-3, VI-4, VI-5, VI-6, VI-7 or VI-8; 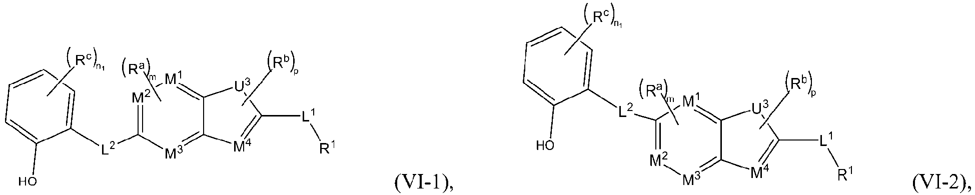 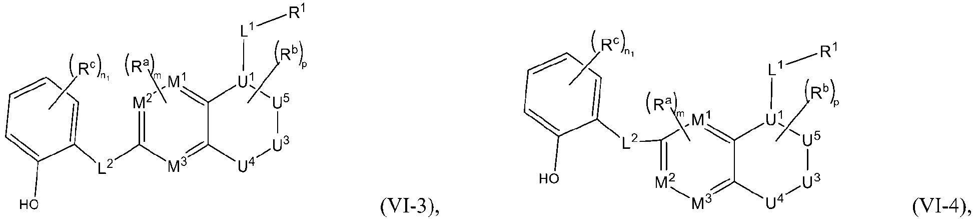 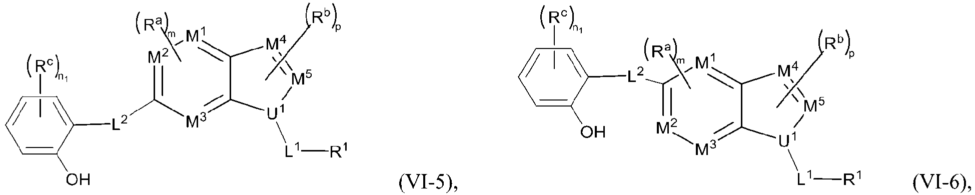 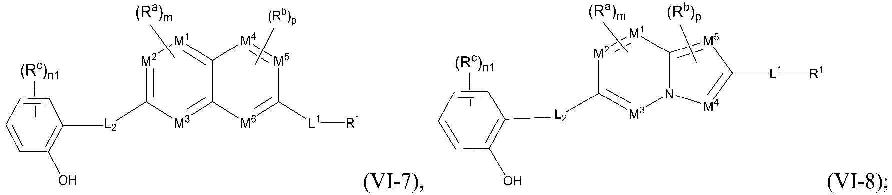 wherein, n1 is 0, 1, 2, or 3, L¹, L², R^{a}, R^{b}, R^{c}, n, m, p, R¹, M¹, M², M³, M⁴, M⁵, M⁶, U¹, U³, U⁴, and U⁵ have the definition as defined in the present invention.

In some embodiments, the compound of the present invention has the structure represented by Formula VII-1, VII-2, VII-3, VII-4, VII-5 or VII-6; 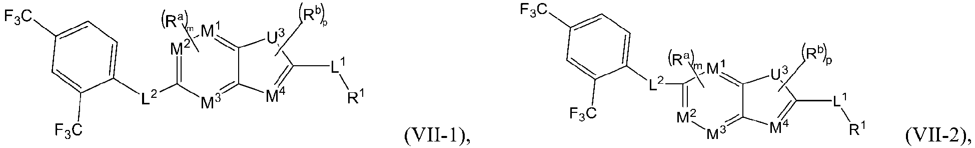 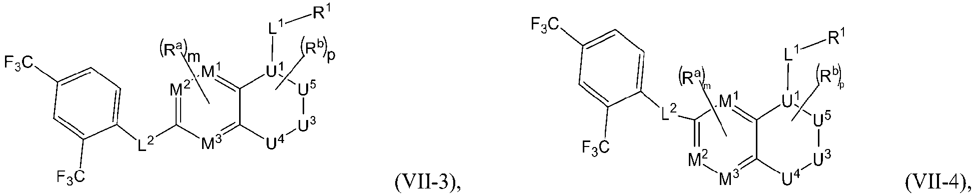 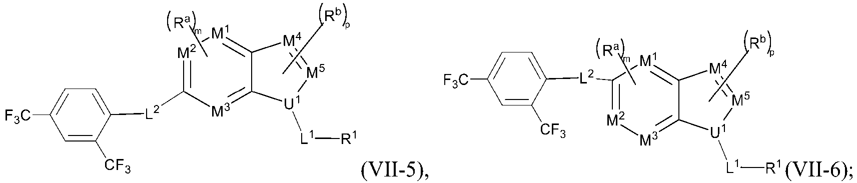 wherein, L¹, L², R^{a}, R^{b}, m, p, R¹, M¹, M², M³, M⁴, M⁵, and U¹ have the definition as defined in the present invention.

In some embodiments, the compound of the present invention has the structure represented by Formula VIII-1, VIII-2, VIII-3, VIII-4, VIII-5, VIII-6, VIII-7 or VIII-8: 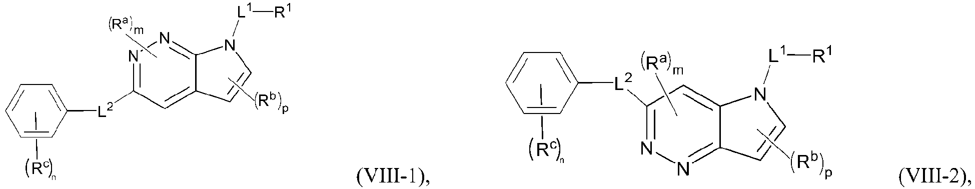 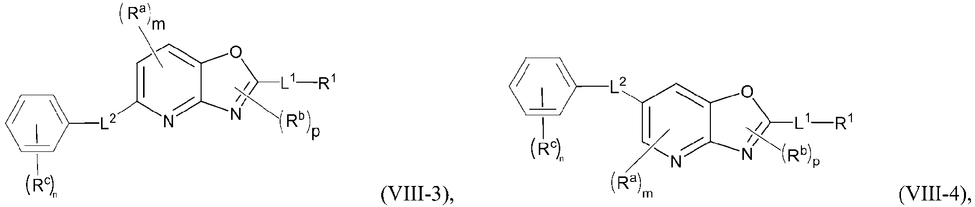 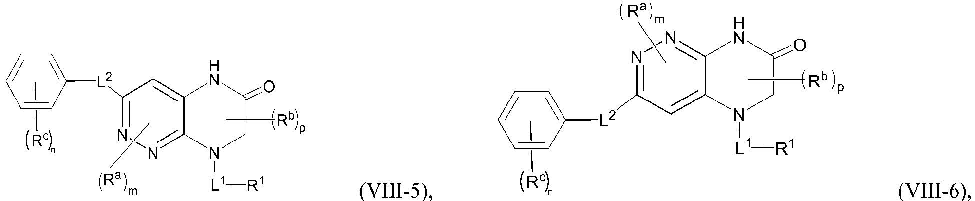 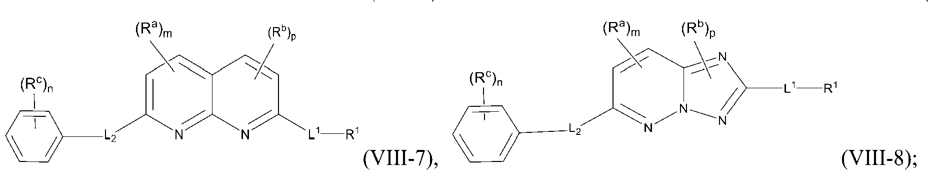 wherein, R^{c}, n, L¹, L², R¹, R^{a}, R^{b}, m, and n have the definition as defined in the present invention.

In some embodiments, the compound of the present invention has the structure represented by Formula IX-1, IX-2, IX-3, IX-4, IX-5 or IX-6: 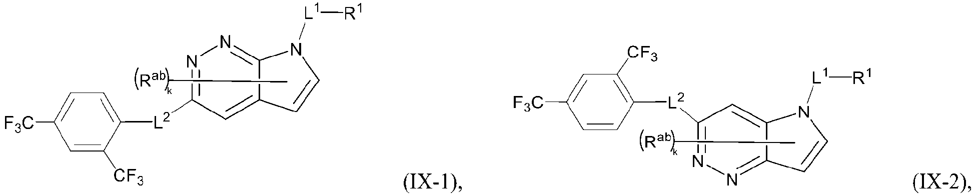 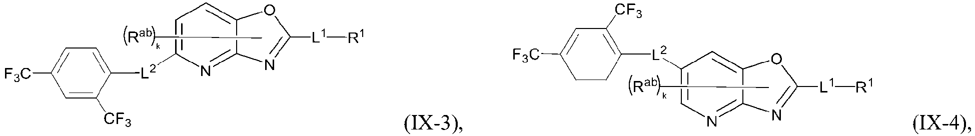 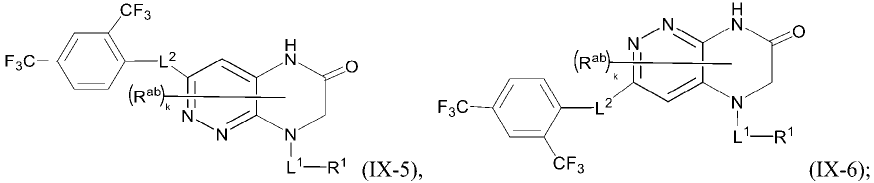
wherein, L¹, L², R¹ have the definition as defined in the present invention.
R^{ab} is each independently selected from hydrogen, deuterium, halogen, -OH, -NH₂, -CN, oxo, methyl, ethyl, propyl, isopropyl, and butyl;
k is each independently selected from 0, 1, 2, and 3.

### Pharmaceutical compositions and administration methods

The present invention relates to a pharmaceutical composition, which comprises the compound of the present invention, or enantiomer, diastereomer, racemate, tautomer, stereoisomer, geometric isomer, nitrogen oxide, metabolite thereof, or pharmaceutically acceptable salt, ester, solvate, hydrate, isotope-labeled compound or prodrug thereof; and a pharmaceutically acceptable carrier or diluent.

The term "pharmaceutical composition" refers to a mixture of one or more compounds described herein or physiologically/pharmaceutically acceptable salts or prodrugs with other chemical components, such as physiologically/pharmaceutically acceptable carriers and diluents, and further adjuvants such as excipients, binders, fillers, etc., and additional therapeutic agents such as antidiabetic agents, antihyperglycemic agents, antiobesity agents, antihypertensive agents, antiplatelet agents, antiatherosclerotic agents, or lipid-lowering agents. The purpose of a pharmaceutical composition is to facilitate the administration of the compound to an organism.

The term "carrier" includes any solvent, dispersion medium, coating material, surfactant, antioxidant, preservative (e.g., antibacterial agent, antifungal agent), isotonic agent, salt, drug stabilizer, binder, excipient, dispersant, lubricant, sweetener, flavoring agent, colorant, or combination thereof, which are known to those skilled in the art (such as Remington's Pharmaceutical Sciences, 18th Ed. Mack Printing Company, 1990, pp. 1289-1329). The use thereof in therapeutic or pharmaceutical composition is covered except for the case where any conventional carrier is incompatible with the active ingredient.

The present invention also relates to a pharmaceutical compositions comprising a compound represented by formula I, II, III, IV-1, IV-2, IV-3, IV-4, IV-5, IV-6, IV-7, IV-8, V-1, V-2, V-3, V-4, V-5, V-6, V-7, V-8, VI-1, VI-2, VI-3, VI-4, VI-5, VI-6, VI-7, VI-8, VII-1, VII-2, VII-3, VII-4, VII-5, VII-6, VII-7, VII-8, VIII-1, VIII-2, VIII-3, VIII-4, VIII-5, VIII-6, VIII-7, VIII-8, IX-1, IX-2, IX-3, IX-4, IX-5, or IX-6, or a pharmaceutically acceptable salt thereof, as an active ingredient, which can be used, in particular, for the treatment of neoplastic diseases, in particular cancer, as described herein. The composition can be formulated for non-parenteral administration, such as nasal, oral, rectal, pulmonary, vaginal, sublingual, topical, transdermal, ophthalmic, or especially for oral administration, for example in the form of oral solid dosage forms, such as granules, pills, powders, tablets, film-coated tablets or sugar-coated tablets, effervescent tablets, hard and soft capsules or hydroxypropylmethylcellulose (HPMC) capsules (suitably coated), orally disintegrating tablets, oral solutions, lipid emulsions or suspensions, or for parenteral administration, such as intravenous, intramuscular or subcutaneous, intrathecal, intradermal or epidural administration to mammals, especially humans, for example in the form of solutions, lipid emulsions or suspensions containing microparticles or nanoparticles. These compositions can contain the active ingredient alone, or preferably, together with a pharmaceutically acceptable carrier.

The compound represented by formula I, II, III, IV-1, IV-2, IV-3, IV-4, IV-5, IV-6, IV-7, IV-8, V-1, V-2, V- 3. V-4, V-5, V-6, V-7, V-8, VI-1 , VI-2, VI-3, VI-4, VI-5, VI-6, VI-7, VI-8, VII-1, VII-2, VII-3, VII-4, VII-5, VII -6, VII-7, VII-8, VIII -1, VIII-2, VIII-3, VIII-4, VIII-5, VIII-6, VIII-7, VIII-8, IX-1, IX-2, IX-3, IX-4, IX-5, or IX-6, or a pharmaceutically acceptable salt thereof, can be processed with a pharmaceutically inert inorganic or organic excipient to produce an oral solid dosage form, such as granules, pills, powders, tablets, film-coated tablets or sugar-coated tablets, effervescent tablets, hard capsules or HPMC capsules or orally disintegrating tablets. Fillers such as lactose, cellulose, mannitol, sorbitol, calcium phosphate, starch or its derivatives, binders such as cellulose, starch, polyvinyl pyrrolidone or its derivatives, glidants such as talc, stearic acid or its salts, flow agents such as fumed silica, can be used as such excipients for the preparation and manufacture of oral solid dosage forms, such as granules, pills, powders, tablets, film-coated tablets or sugar-coated tablets, effervescent tablets, hard capsules or HPMC capsules or orally disintegrating tablets. Suitable excipients for soft capsules are, for example, vegetable oils, waxes, fats, semisolid and liquid polyols, etc.

Suitable excipients for the manufacture of oral solutions, lipid emulsions or suspensions are, for example, water, alcohols, polyols, sucrose, invert sugar, glucose and the like.

Suitable excipients for parenteral formulation are, for example, water, alcohols, polyols, glycerol, vegetable oils, lecithin, surfactants and the like.

In addition, the pharmaceutical preparations may contain preservatives, solubilizers, stabilizers, wetting agents, emulsifiers, sweeteners, colorants, flavorings, salts for varying the osmotic pressure, buffers, masking agents or antioxidants. The pharmaceutical preparations may also contain other therapeutically valuable substances.

The dosage can vary within a wide range and, of course, meets individual requirements in each particular case. In general, in the case of oral administration, a daily dosage of about 1 to 1000 mg of a compound represented by formula (I) per person should be appropriate, although the above lower or upper limits may also be exceeded if necessary.

The compound represented by formula I, II, III, IV-1, IV-2, IV-3, IV-4, IV-5, IV-6, IV-7, IV-8, V-1, V-2, V-3, V-4, V-5, V-6, V-7, V-8, VI-1, VI-2, VI-3, VI-4, VI-5, VI-6, VI-7, VI-8, VII-1, VII-2, VII-3, VII-4, VII-5, VII-6, VII-7, VII-8, VIII-1, VIII-2, VIII-3, VIII-4, VIII-5, VIII-6, VIII-7, VIII-8, IX-1, IX-2, IX-3, IX-4, IX-5, or IX-6 can also be used in combination with one or more other pharmacologically active compounds, these other compounds are also effective against the same disease, preferably using a different mode of action, or reduce or prevent possible undesirable side effects of compound represented by formula I, II, III, IV-1, IV-2, IV-3, IV-4, IV-5, IV-6, IV-7, IV-8, V-1, V-2, V-3, V-4, V-5, V-6, V-7, V-8, VI-1, VI-2, VI-3, VI-4, VI-5, VI-6, VI-7, VI-8, VII-1, VII-2, VII-3, VII-4, VII-5, VII-6, VII-7, VII-8, VIII-1, VIII-2, VIII-3, VIII-4, VIII-5, VIII-6, VIII-7, VIII-8, IX-1, IX-2, IX-3, IX-4, IX-5, or IX-6. The combination partners can be administered simultaneously in such treatment, for example by incorporating them into a single pharmaceutical formulation, or sequentially by administering two or more different dosage forms, each containing one or more than one combination partner.

As used herein, the term "pharmaceutically acceptable carrier" refers to a substance that can be used to prepare or use a pharmaceutical composition, and includes, for example, suitable diluents, solvents, dispersion media, surfactants, antioxidants, preservatives, isotonic agents, buffers, emulsifiers, absorption delaying agents, salts, drug stabilizers, binders, excipients, disintegrants, lubricants, wetting agents, sweeteners, flavoring agents, dyes, and combinations thereof, as known to those skilled in the art (see, for example, Remington The Science and Practice of Pharmacy, 22nd ed., Pharmaceutical Press, 2013, pp. 1049-1070).

The term "a therapeutically effective amount" of the compound of the present invention refers to an amount of compound of the present invention that will cause a biological or medical response in a subject (e.g., reduction or inhibition of enzyme or protein activity, or improvement in symptoms, relief of symptoms, slowing or delaying disease progression, or prevention of disease, etc.). In one non-limiting embodiment, the term "therapeutically effective amount" refers to an amount of the compound of the present invention that, when administered to a subject, effectively (1) at least partially alleviates, inhibits, prevents and/or improves a condition, disorder or disease (i) mediated by NLRP3, or (ii) associated with NLRP3 activity, or (iii) characterized by the activity (normal or abnormal) of NLRP3; or (2) reduces or inhibits the activity of NLRP3; or (3) reduces or inhibits the expression of NLRP3. In another non-limiting embodiment, the term "therapeutically effective amount" of a compound of the present invention refers to an amount that, when administered to a cell, or tissue, or non-cellular biological material or medium, effectively at least partially reduces or inhibits the activity of NLRP3; or at least partially reduces or inhibits the expression of NLRP3.

The term "treatment" or "treating" as used herein in the context of treating a disease or disorder generally relates to treatment and therapy of humans or animals (e.g., in veterinary applications), wherein some desired therapeutic effects are obtained, e.g., inhibiting the progression of a disease or disorder, and including reducing the rate of progression, stopping the rate of progression, alleviating the symptoms of a disease or disorder, improving a disease or disorder, and curing a disease or disorder. Treatment as a preventive measure (i.e., prevention) is also included. For example, patients who have not yet developed the disease or disorder but are at risk of developing the disease or disorder are covered by the term "treatment". For example, treatment includes prevention of cancer, reducing the incidence of cancer, alleviating cancer symptoms, etc.

As used herein, the term "inhibit", "inhibition" or "inhibiting" refers to reducing or inhibiting a given condition, symptom, disorder, or disease, or significantly reducing the baseline activity of a biological activity or process. Specifically, inhibiting NLRP3 or inhibiting the NLRP3 inflammasome pathway comprises reducing the ability of NLRP3 or the NLRP3 inflammasome pathway to induce the production of IL-1β and/or IL-18. This can be achieved by mechanisms including, but not limited to, inactivating, destabilizing NLRP3, and/or altering the distribution of NLRP3.

As used herein, the terms "prevent," "preventing," or "prevention" of any disease or disorder refers to prophylactic treatment of the disease or disorder; or delaying the onset or progression of the disease or disorder.

As used herein, the term "NLRP3" refers to included, but not limited to, nucleic acids, polynucleotides, oligonucleotides, sense and antisense polynucleotide chains, complementary sequences, peptides, polypeptides, proteins, homologous and/or orthologous NLRP molecules, isoforms, precursors, mutants, variants, derivatives, splice variants, alleles, different species, and active fragments thereof.

### Synthesis method

Compounds represented by formula I, II, III, IV-1, IV-2, IV-3, IV-4, IV-5, IV-6, IV-7, IV-8, V-1, V-2, V-3, V-4, V-5, V-6, V-7, V-8, VI-1, VI-2, VI-3, VI-4, VI-5, VI-6, VI-7, VI-8, VII-1, VII-2, VII-3, VII-4, VII-5, VII-6, VII-7, VII-8, VIII-1, VIII-2, VIII-3, VIII-4, VIII-5, VIII-6, VIII-7, VIII-8, IX-1, IX-2, IX-3, IX-4, IX-5, or IX-6 can be synthesized by the methods given below, by the methods given in the experimental part below or by similar methods. The schemes described herein are not intended to present an exhaustive list of methods for preparing compounds represented by formula I, II, III, IV-1, IV-2, IV-3, IV-4, IV-5, IV-6, IV-7, IV-8, V-1, V-2, V-3, V-4, V-5, V-6, V-7, V-8, VI-1, VI-2, VI-3, VI-4, VI-5, VI-6, VI-7, VI-8, VII-1, VII-2, VII-3, VII-4, VII-5, VII-6, VII-7, VII-8, VIII-1, VIII-2, VIII-3, VIII-4, VIII-5, VIII-6, VIII-7, VIII-8, IX-1, IX-2, IX-3, IX-4, IX-5, or IX-6; instead, other techniques known to the skilled chemist may also be used for the synthesis of the compounds.

The structures of the compounds were determined by nuclear magnetic resonance (¹H-NMR, ¹³C-NMR or/and ¹⁹F-NMR). ¹H-NMR, ¹³C-NMR, ¹⁹F-NMR chemical shifts (δ) are given in parts per million (ppm). ¹H-NMR, ¹³C-NMR, ¹⁹F-NMR were measured using a Bruker Ultrashield-400 NMR spectrometer and a Bruker Avance III HD 600 NMR spectrometer, and the measurement solvents were deuterated chloroform (CDCl₃), deuterated methanol (CD₃OD or MeOH-d₄) or deuterated dimethyl sulfoxide (DMSO-d₆). TMS (0 ppm) or chloroform (7.25 ppm) was used as a reference standard. When multiple peaks appear, the following abbreviations are used: s (singlet,), d (doublet), t (triplet), m (multiplet), br (broadened), dd (doublet of doublets), dt (doublet of triplets), td (triplet of doublets), brs (broadened singlet). The coupling constant J is expressed in Hertz (Hz).

Liquid chromatography-mass spectrometry (LC-MS) was detected using an Agilent 1260 mass spectrometer. HPLC was determined using an Agilent 1100 high pressure chromatograph (Microsorb 5micron C18 100 x 3.0mm column).

The thin layer chromatography silica gel plate used was Qingdao GF254 silica gel plate, TLC used 0.15-0.20mm, and preparative thin layer chromatography used 0.4mm-0.5mm. Column chromatography generally uses Qingdao silica gel 200-300 mesh silica gel as a carrier.

The starting materials in the examples of the present invention are all known and commercially available, or can be synthesized using or according to literature data reported in the art.

Unless otherwise specified, all reactions of the present invention are carried out under the protection of dry inert gas (such as nitrogen or argon) with continuous magnetic stirring, and the reaction temperatures are all degrees Celsius.

It will be understood by those skilled in the art of organic synthesis that optimal reaction conditions may vary with the specific reactants or solvents used, but these conditions may be determined by conventional optimization procedures. In some cases, the order of the following reaction scheme and/or reaction steps may be changed to facilitate reaction or avoid forming unwanted by-products. In addition, the functional groups present at each position of the molecule must be compatible with the proposed reagent and reaction. This limitation of substituents compatible with reaction conditions is obvious to those skilled in the art, and then alternative methods must be used. Furthermore, in some reactions mentioned herein, it may be necessary or desirable to protect any sensitive groups in the compounds, and it is assumed that such a protecting group (PG) is in the appropriate position if necessary. Conventional protecting groups can be used according to standard practices well known in the art (for explanation, see Greene T.W, Wuts P.G.M, Protective Groups in Organic Synthesis, 5th edition, publisher: John Wiley & Sons, 2014). The protecting groups may be removed at any convenient stage in the synthesis using conventional techniques well known in the art, or may be removed in a subsequent reaction step or work-up.

The following abbreviations are used throughout this invention:
°C: Celsius
h, hr: Hour
MW: Microwave
TEA: Triethylamine
TFA: Trifluoroacetic acid
DCM: Dichloromethane
MeOH: Methanol
EtOH: Ethanol
DMF: N,N-Dimethylformamide
DMSO: Dimethyl sulfoxide
DIPEA: N,N-Diisopropylethylamine
THF: Tetrahydrofuran
ACN: Acetonitrile
BrCN: Cyanogen bromide
DCM/TFA = 1:1: A mixed solution of dichloromethane and trifluoroacetic acid in a volume ratio of 1:1
K₃PO₄: Potassium phosphate tribasic
NaIO₄: Sodium periodate
NH₄OAc: Ammonium acetate
TsCl: 4-Toluenesulfonyl chloride
Cs₂CO₃: Cesium carbonate
K₂CO₃: Potassium carbonate
NH₃H₂O: Ammonia water
Pd(dppf)Cl₂: 1,1'-bis(diphenylphosphino)ferrocene palladium(II) dichloride
XPhos Pd G3: Methanesulfonato(2-dicyclohexylphosphino-2',4',6'-tri-isopropyl-1,1'-biphenyl)(2'-amino-1,1'-biphenyl-2-yl)palladium(II)
SPhos Pd G3: Methanesulfonato(2-dicyclohexylphosphino-2',6'-dimethoxy-1,1'-biphenyl)(2'-amino-1,1'-biphenyl-2-yl)palladium(II)
NaBH₃CN: Sodium cyanoborohydride
-OMOM: Methoxymethoxy-
Boc-: tert-Butyloxycarbonyl-
Boc₂O: Di-tert-butyl dicarbonate
LHMDS: Lithium bis(trimethylsilyl)amide
CDI: N,N-Carbonyldiimidazole
POCl₃: Phosphorus oxychloride
NMP: N-Methylpyrrolidone
DMAP: 4-Dimethylaminopyridine

### General Synthesis Methods

The typical synthesis steps for preparing the compounds disclosed in the present invention are shown in the following synthesis scheme.

### Scheme 1

The following compounds represented by formula I-1 were synthesized by the following methods

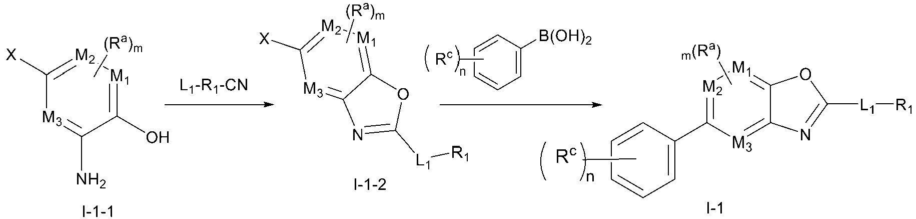

Formula I-1-1 undergoes cyclization reaction to obtain compound formula I-1-2, and formula I-1-2 undergoes coupling reaction to obtain compound I-1, wherein X is halogen, and the definitions of M₁, M₂, M₃, R^{a}, R^{c}, L₁, R₁, m, and n are as described in the embodiments above.

### Description of the Drawings

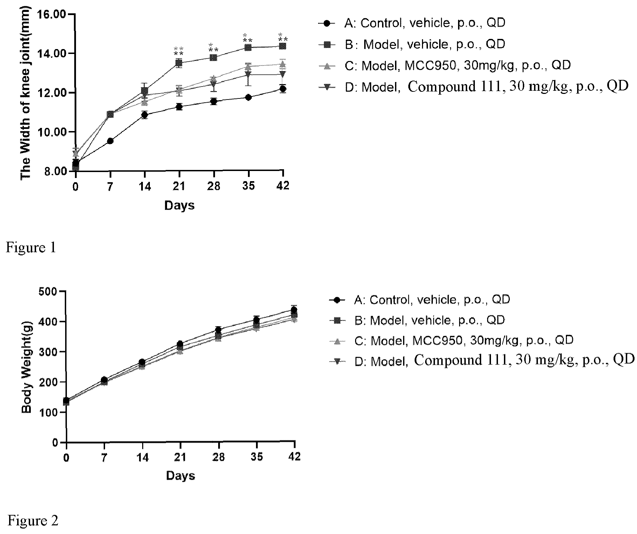

Figures 1 and 2 show the efficacy of compound 111 of the present application and the positive compound on the iodoacetic acid-induced SD rat knee osteoarthritis model.

Examples and figures are provided below to assist in understanding the present invention. However, it should be understood that these examples and figures are only used to illustrate the present invention, but do not constitute any limitation. The actual protection scope of the present invention is set forth in the claims. It should be understood that any modifications and changes can be made without departing from the spirit of the present invention.

### Preparation of intermediate M-1

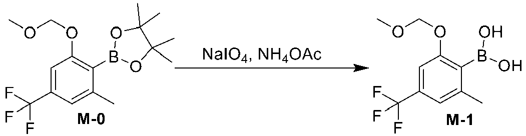

Ammonium acetate (935 mg, 12.1 mmol) and sodium periodate (2.59 g, 12.1 mmol) were added to compound **M-0** (700 mg, 2.02 mmol) in a mixture of acetone (10 mL) and water (10 mL), stirred at 50 °C and allowed to react for 18 hours. The reaction solution was filtered, the filter cake was washed 3 times with acetone (2 mL), the filtrate was dried by rotary evaporation, diluted with water (10 mL), and extracted 3 times with dichloromethane (5 mL), the organic phases were combined and dried over anhydrous sodium sulfate, the crude product was filtered and dried by rotary evaporation and was directly used in the next step without purification, to obtain intermediate **M-1.**

### Example 1: Preparation of Compound 12

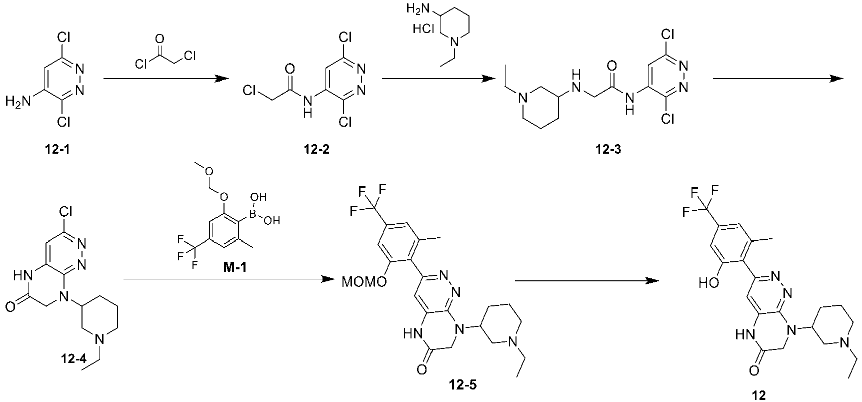

**Step 1):** Compound **12-1** (3.0 g, 18.29 mmol, Bide), triethylamine (5.07 mL, 36.59 mmol), and anhydrous dichloromethane (15 mL) were sequentially added to a 100 mL three-necked bottle at room temperature, and mixed thoroughly, and the mixture was cooled to 0 °C in an ice-water bath, and chloroacetyl chloride (1.60 mL, 20.12 mmol) in dichloromethane (15 mL) was slowly added dropwise. The reaction solution was stirred at 20 °C for 16 hours. After the raw material was completely reacted monitored by LC-MS, the reaction solution was poured into water, extracted with dichloromethane, washed with saturated sodium chloride solution, dried over anhydrous sodium sulfate, and concentrated under reduced pressure to obtain a solid. The solid was purified by column chromatography (petroleum ether/ethyl acetate/ammonia water = 1:1:0.1) to obtain compound **12-2.** LC-MS: m/z :240.2[M+H]⁺.

**Step 2):** Compound **12-2** (600 mg, 2.50 mmol), N, N-dimethylformamide (6 mL) were sequentially added to a 40 mL IKA bottle at room temperature, then triethylamine (1.04 mL, 7.49 mmol), 1-ethylpiperidin-3-amine hydrochloride (534.16 mg, 3.24 mmol, Leyan) were added to the reaction bottle. The reaction solution was stirred at 60 °C for 5 hours. After the raw material was completely reacted monitored by LC-MS , 10 mL of saturated sodium chloride solution was added to the reaction solution, then extracted twice with 10 mL of tetrahydrofuran, and the organic phase was concentrated under reduced pressure, and purified by column chromatography (petroleum ether/ethyl acetate/ammonia water = 1:1:0.1) to obtain compound **12-3.** LC-MS: m/z :332.1[M+H]⁺.

**Step 3):** Compound **12-3** (400 mg, 2.50 mmol) and dimethyl sulfoxide (4 mL) were sequentially added to a 40 mL IKA bottle at room temperature, then DIPEA (0.60 mL, 3.61 mmol) was added to the reaction bottle, and the reaction solution was stirred at 130 °C for 3 hours. After the raw material was completely reacted monitored by LC-MS, 10 mL of saturated sodium chloride solution was added to the reaction solution after cooling to room temperature, and extracted twice with 10 mL of tetrahydrofuran, the organic phase was concentrated under reduced pressure, and purified by column chromatography (petroleum ether/ethyl acetate/ammonia water = 1:1:0.1) to obtain compound **12-4.** LC-MS: m/z :296.1[M+H]⁺.

¹H NMR (400MHz, CDCl₃) δ 6.70 (s, 1H), 4.86 - 4.78 (m, 1H), 4.33 - 4.16 (m, 2H), 3.06 - 3.04 (m, 1H), 2.92 - 2.89 (m, 1H), 2.51 - 2.46 (m, 2H), 2.30 - 2.24 (m, 1H), 2.02 - 1.92 (m, 1H), 1.90 - 1.89 (m, 1H), 1.82 - 1.71 (m, 4H), 1.11 (t, J=7.2 Hz, 3H).

**Step 4):** Compound **12-4** (90.0 mg, 0.300 mmol), intermediate **M-1** (137 mg, 0.520 mmol), Xphos Pd G3 (25.8 mg, 0.030 mmol), potassium phosphate (258 mg, 1.22 mmol), dioxane (2 mL), and water (0.2 mL) were added to an 8 mL single-necked bottle at room temperature. The reaction was carried out at 80 °C for 1 hour, the reaction solution was diluted with water (3 mL), and extracted 3 times with dichloromethane (6 mL), the organic phases were combined, dried over anhydrous sodium sulfate, filtered, and dried by rotary evaporation. The residue was purified by preparative thin layer chromatography on silica gel (dichloromethane / amine methanol (7 M) = 20 / 1) to obtain compound **12-5.** LC-MS: m/z : 480.2[M+H]⁺.

**Step 5):** Compound **12-5,** dichloromethane (1 mL), and trifluoroacetic acid (1 mL) were added to an 8 mL single-necked bottle at room temperature. The reaction was carried out at 25°C for 3 hours. The reaction solution was dried by rotary evaporation. The residue was purified by high performance liquid chromatography (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **12.** LC-MS: m/z : 436.1[M+H]⁺.

¹H NMR (400 MHz, DMSO-*d₆*) δ 11.01 (s, 1H), 10.22 (s, 1H), 7.11 (s, 1H), 7.05 (s, 1H), 6.68 (s, 1H), 4.81 - 4.77 (m, 1H), 4.21 - 4.22 (m, 2H), 2.98 (d, *J =* 8.8 Hz, 1H), 2.88 (d, *J* = 11.2 Hz, 1H), 2.48 - 2.44 (m, 1H), 2.30 - 2.20 (m, 1H), 2.14 (s, 3H), 2.02 - 1.92 (m, 2H), 1.78 - 1.70 (m, 2H), 1.71 - 1.56 (m, 2H), 1.03 (t, *J =* 7.1 Hz, 3H).

### Example 2: Preparation of Compound 11

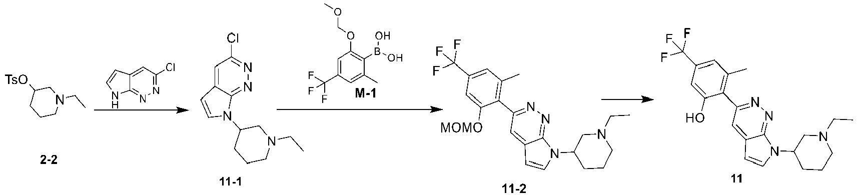

**Step 1):** Compound **2-2** (200.0 mg, 1.30 mmol) and *N*, *N*-dimethylformamide (5 mL) were sequentially added to a 40 mL IKA bottle at room temperature, then cesium carbonate (636.0 mg, 1.95 mmol) and compound 3-chloro-7H-pyrrolo[2,3-c]pyridazine (442.88 mg, 1.56 mmol, Bide) were added to the reaction bottle. The reaction solution was stirred at 100°C for 2 hours. After the raw material was completely reacted monitored by LC-MS, 20 mL of water was added to the reaction solution, then extracted twice with 50 mL of ethyl acetate. The organic phase was concentrated under reduced pressure, and the residue was purified by silica gel column (tetrahydrofuran/dichloromethane = 0~10%) to obtain compound **11-1.** LC-MS: m/z :265.3 [M+H]⁺.

¹H NMR (400 MHz, CDCl₃) δ 7.69 (d, *J =* 3.4 Hz, 1H), 7.66 (s, 1H), 6.39 (d, *J =* 3.4 Hz, 1H), 4.60 (dd, *J =* 4.8, 13.9 Hz, 1H), 4.25 (dd, *J =* 5.8, 14.0 Hz, 1H), 3.18 - 3.16 (m, 1H), 3.03 - 3.02 (m, 1H), 2.74 - 2.69 (m, 1H), 2.35 - 2.31 (m, 1H), 2.23 - 2.21 (m, 1H), 1.82 - 1.80 (m, 1H), 1.69 - 1.62 (m, 1H), 1.56 - 1.52 (m, 2H), 1.10 (t, *J =* 7.3 Hz, 3H).

**Step 2):** Compound **11-1** (100 mg, 0.380 mmol), intermediate **M-1** (169 mg, 0.640 mmol), XPhos Pd G3 (31.9 mg, 0.040 mmol), potassium phosphate (320 mg, 1.51 mmol), water (0.2 mL) and dioxane (2.0 mL) were added to a 25 mL single-necked bottle at 25 °C. The mixture was purged with nitrogen three times, and the reaction solution was stirred at 80°C for 1 hour. Saturated sodium chloride solution (3 mL) was added to the reaction solution for dilution, extracted 3 times with dichloromethane (5 mL). The organic phases were combined, dried over anhydrous sodium sulfate, and dried by rotary evaporation. The residue was purified by column chromatography on a silica gel column (dichloromethane/methanol = 15/1) to obtain compound **11-2.** LC-MS: m/z 449.2 [M+H]⁺.

**Step 3):** Compound **11-2** (45.0 mg, 0.100 mmol) and the dioxane solution of hydrochloric acid (2.0 mL, 4.0 M) were added to a 25 mL single-necked bottle at 25 °C, and the reaction solution was stirred at 25°C for 1 hour. The reaction solution was dried by rotary evaporation. The crude product was purified by preparation column (Waters-SunFire-C18-10µm-19×250mm; mobile phase: water (containing 10mmol/L formic acid) and acetonitrile, gradient ratio: acetonitrile 23%-48%, flow rate: 30 mL/min) to obtain compound **11.** LC-MS: m/z 405.1 [M+H]⁺.

¹H NMR (400 MHz, DMSO-*d*₆) δ 10.05 (s, 1H), 8.15 (s, 1H), 7.80 (s, 1H), 7.15 (s, 1H), 7.10 (s, 1H), 6.60 (d, *J =* 3.4 Hz, 1H), 5.14 - 5.05 (m, 1H), 3.15 - 3.11 (m, 1H), 2.88 (d, *J* = 11.1 Hz, 1H), 2.63 - 2.52 (m, 2H), 2.24 - 2.16 (m, 1H), 2.15 - 1.95 (m, 6H), 1.90 - 1.80 (m, 1H), 1.77 - 1.67 (m, 1H), 1.05 (t, *J =* 7.2 Hz, 3H).

### Example 3: Preparation of Compound 5

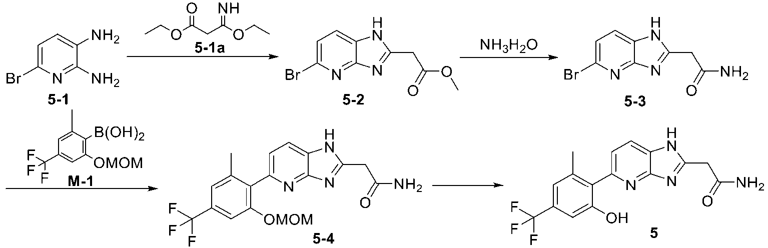

**Step 1):** Compound **5-1** (1.00 g, 6.28 mmol) and compound **5-1a** (1.14 g, 5.85 mmol) were added to EtOH (10 mL) at room temperature and refluxed with stirring overnight. The reaction system was directly concentrated, and the residue was purified by column chromatography (0-3% methanol/dichloromethane) to obtain compound **5-2.** LC-MS: m/z : 283.8(M+H)⁺.

**Step 2):** Compound 5-2 (250 mg, 1.04 mmol) in ammonia water (10 mL, 28%) was stirred at 50 °C for 5 hours. The reaction solution was evaporated to dryness under reduced pressure, ethyl acetate (3 mL) and petroleum ether (6 mL) were added to the residue, and stirred for 2 hours, then the mixture was filtered to obtain compound **5-3** (150 mg). LC-MS: m/z : 254.9(M+H)⁺.

**Step 3):** Potassium carbonate (162 mg, 1.18 mmol) and 1,1'-bis(diphenylphosphino)ferrocene palladium(II) dichloride (28.69 mg, 0.04 mmol) were added to compound **5-3** (100 mg, 0.39 mmol) and intermediate M-1 (207 mg, 0.78 mmol) in a mixture of dioxane (3 mL) and water (0.5 mL). The reaction solution was stirred at 80 °C for 2 hours under a nitrogen protection. The reaction solution was filtered and concentrated under reduced pressure, and the crude product was purified by silica gel chromatography (eluent: dichloromethane/methanol = 20/1) to obtain compound **5-4.** LC-MS: m/z: 395.0(M+H)⁺.

**Step 4):** Trifluoroacetic acid (1 mL, 13.42 mmol) was added to compound **5-4** (120 mg, 0.30 mmol) in dichloromethane (1 mL) at room temperature. The reaction solution was stirred at 25 °C for 2 hours. The reaction solution was concentrated under reduced pressure, and the residue was purified by high performance liquid chromatography (Waters-SunFire-C18-10 µm-19×250 mm; mobile phase: water (containing 10 mmol/L formic acid) and acetonitrile, gradient ratio: acetonitrile 23%-48%, flow rate: 30 mL/min) to obtain compound 5. LC-MS:m/z :351.1(M+H)⁺.

### Example 4: Preparation of Compound 9

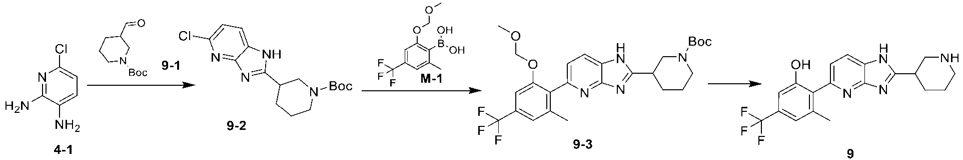

**Step 1):** Compound **9-1** (3.86 g, 18.1 mmol, Bide) and ammonium acetate (1.61 g, 20.90 mmol) were added to compound **4-1** (2.00 g, 13.9 mmol) in ethanol (15 mL). The reaction mixture was stirred at 70 °C for 18 hours. The solvent was removed from the reaction solution. The residue was purified by C18 reversed phase column (methanol/water = 62%) to obtain compound **9-2.** LC-MS: m/z :337.5 [M+H]⁺.

**Step 2)**: Compound **9-2** (100 mg, 0.300 mmol), intermediate **M-1** (195 mg, 0.740mmol), dioxane (2 mL), water (0.3 mL), potassium phosphate (126 mg, 0.590 mmol), and XPhos Pd G3 (25.1 mg, 0.03 mmol) were added to a single-necked bottle at 25 °C. The reaction solution was stirred at 80 °C for 2 hours. Water (3 mL) was added to the reaction solution for dilution, and extracted with ethyl acetate (3 mL) 3 times. The organic phases were combined, dried over anhydrous sodium sulfate, filtered, and dried by rotary evaporation. The residue was purified by column chromatography on a silica gel column (MeOH/DCM = 1/10) to obtain compound **9-3.** LC-MS: m/z 521.1 [M+H]⁺.

**Step 3):** Compound **9-3** (70 mg, 0.13 mmol), dichloromethane (1 mL), and trifluoroacetic acid (1 mL, 0.670 mmol) were added to a 25 mL single-necked bottle at 25 °C, and the reaction solution was stirred at room temperature for 1 hour. The reaction solution was dried by rotary evaporation and purified by preparation column (Waters-SunFire-C18-10 µm-19×250 mm; mobile phase: water (containing 10 mmol/L formic acid) and acetonitrile, gradient ratio: acetonitrile 23%-48%, flow rate: 30 mL/min) to obtain compound **9.** LC-MS: m/z 377.1 [M+H]⁺.

### Example 5: Preparation of Compound 10

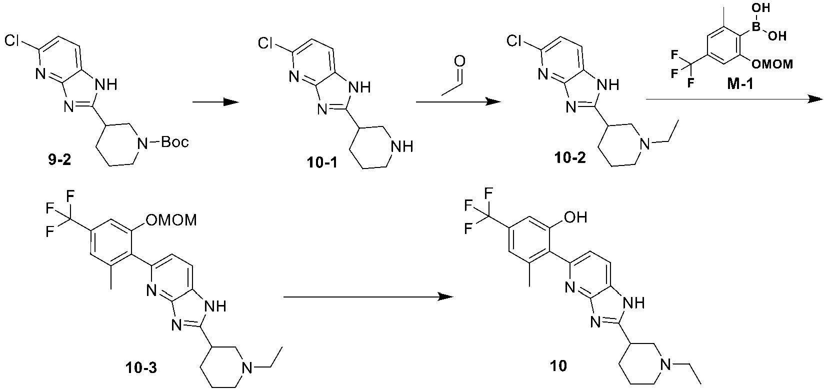

**Step 1):** Trifluoroacetic acid (1 mL, 13.42 mmol) was added to compound **9-2** (360 mg, 1.07 mmol) in dichloromethane (5 mL). The reaction solution was stirred at 25 °C for 3 hours, and the reaction solution was concentrated to obtain compound **10-1.** LC-MS: m/z :237.1 [M+H]⁺.

**Step 2):** The compound 10-1 (360 mg, 1.44 mmol) and acetaldehyde (0.54 mL, 3.06 mmol) in methanol (1 mL) was stirred at 25 °C for 1 hour. Then sodium cyanoborohydride (191.2 mg, 3.06 mmol) was added and the reaction solution was stirred at 25 °C for 18 hours. Ethyl acetate (50 ml) and water (50 ml) were added to the reaction solution for extraction. The organic phase was washed with saturated brine (50 mL), dried over anhydrous sodium sulfate, filtered and concentrated, and the crude product was purified by silica gel chromatography (eluent: petroleum ether/ethyl acetate = 1/1) to obtain compound **10-2.** LC-MS:m/z :265.1 [M+H]⁺.

**Step 3):** Compound **10-2** (150 mg, 0.57 mmol), intermediate **M-1** (226 mg, 0.86 mmol), potassium phosphate (481 mg, 2.27 mmol), dioxane (2 mL) and water (0.2 mL) were added to a three-necked bottle at room temperature, the mixture was purged with nitrogen three times, then XPhos PdG3 (48 mg, 0.06 mmol) was added, and purged with nitrogen three times. The reaction solution was heated to 80 °C, stirred for 1 hour, and then slowly cooled to room temperature. Water (15 mL) was added to the reaction solution, extracted with dichloromethane (6 mL×3), the organic phases were separated and combined, dried over anhydrous sodium sulfate, concentrated under reduced pressure, and the residue was purified by column chromatography (methanol/dichloromethane = 0-10%) to obtain compound **10-3.** LC-MS: m/z :449.1 [M+H]⁺.

**Step 4):** Trifluoroacetic acid (1.0 mL) was added to compound **10-3** (60.0 mg, 0.130 mmol) in dichloromethane (1.0 mL) at 25 °C, and the reaction solution was stirred at room temperature for 2 hours. The reaction solution was dried by rotary evaporation, and the crude product was purified by preparation column (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **10.** LC-MS: m/z 405.1 [M+H]⁺.

¹H NMR (400 MHz, CDCl₃) δ 7.69 (d, *J* = 3.4 Hz, 1H), 7.66 (s, 1H), 6.39 (d, *J* = 3.4 Hz, 1H), 4.60 (dd, *J* = 4.8, 13.9 Hz, 1H), 4.25 (dd, *J =* 5.8, 14.0 Hz, 1H), 3.18 - 3.16 (m, 1H), 3.03 - 3.02 (m, 1H), 2.74 - 2.69 (m, 1H), 2.35 - 2.31 (m, 1H), 2.23 - 2.21 (m, 1H), 1.82 - 1.80 (m, 1H), 1.69 - 1.62 (m, 1H), 1.56 - 1.52 (m, 2H), 1.10 (t, *J =* 7.3 Hz, 3H).

### Example 6: Preparation of Compound 14

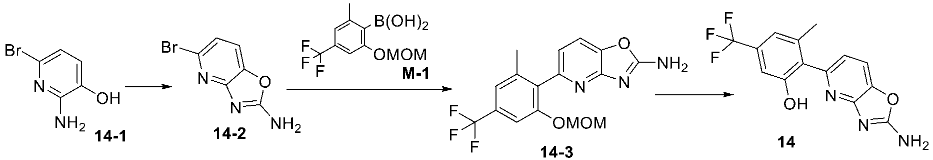

**Step 1):** Compound **14-1** (2.00 g, 10.6 mmol), methanol (10 mL) and water (10 mL) were added to a 250 mL single-necked bottle at room temperature and stirred. Cyanogen bromide (2.33 mL, 31.74 mmol) was added. The reaction solution was stirred at 70 °C for 3 hours under a nitrogen atmosphere. Water was added to the reaction solution, extracted with ethyl acetate (50 mL×3),washed with saturated brine (20 mL×1), dried over anhydrous sodium sulfate, and concentrated under reduced pressure. The residue was purified by column chromatography (0-5% methanol/dichloromethane) to obtain compound **14-2.** LC-MS: m/z :213.9(M+H)⁺.

**Step 2):** Compound **14-2** (100 mg, 0.47 mmol), 1,4-dioxane (5 mL) and water (1 mL) were added to a 50 mL single-necked bottle at room temperature and stirred, intermediate **M-1** (123 mg, 0.470 mmol) was added, potassium carbonate (193 mg, 1.40 mmol) and 1,1'-bis(diphenylphosphino)ferrocene palladium(II) dichloride dichloromethane complex (38.2 mg, 0.05 mmol) were added, and the reaction solution was stirred at 80 °C for 2 hours under a nitrogen atmosphere, and water was added to the reaction solution, extracted with ethyl acetate (50 mL×3),washed with saturated brine (20 mL×1), dried over anhydrous sodium sulfate, and concentrated under reduced pressure. The residue was purified by column chromatography (0-5% methanol/dichloromethane) to obtain compound **14-3.** LC-MS: m/z :354.0(M+H)⁺.

**Step 3):** Compound **14-3** (100 mg, 0.28 mmol) was added to a 50 mL single-necked bottle at room temperature, and dichloromethane (2 mL) and TFA (2 mL) were added and stirred. The reaction solution was stirred at room temperature for 2 hours. The crude product was purified by high performance liquid chromatography (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **14.** LC-MS: m/z :310.0(M+H)⁺.

¹H NMR (400 MHz, DMSO-*d₆*) δ 7.90 (s, 1H), 7.71 (d, *J =* 8.0 Hz, 1H), 7.06 (d, *J =* 12.7 Hz, 2H), 6.90 (d, *J* = 8.0 Hz, 1H), 3.36 (s, 2H), 2.07 (s, 3H).

### Example 7: Preparation of Compound 3

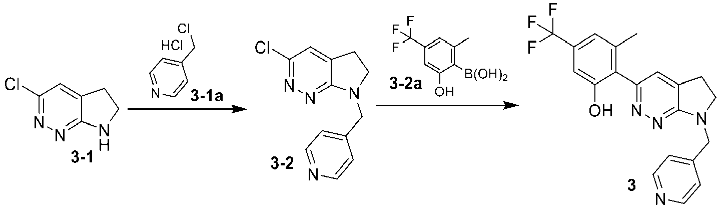

**Step 1):** Lithium bis(trimethylsilyl)amide (0.64 mL, 0.64 mmol, 1M) was slowly added to compound **3-1** (50.0 mg, 0.320 mmol, synthesis method with reference to: WO2021018858 A1) in THF (1 mL) at 0 °C, the reaction solution was stirred at 0°C for 1 hour. Compound **3-1a** (40.8 mg, 0.320 mmol, Bide) was added to the reaction solution, and the reaction solution was stirred at room temperature for 3 hours. After the reaction was completed detected by LCMS, saturated ammonium chloride solution (2 mL) was added to the reaction solution to quench the reaction, and extracted with ethyl acetate (2 mL) 3 times, the organic phases were combined, dried over anhydrous sodium sulfate, filtered, and dried by rotary evaporation. The crude product was purified by column chromatography (ammonia/methanol/dichloromethane = 1/10/100) to obtain compound **3-2.** LC-MS: m/z 247.1 [M+H]⁺.

**Step 2):** Compound **3-2a** (26.7 mg, 0.120 mmol, synthesis method with reference to: US20200361898A1, synthesis of intermediate B005 on page 39), potassium carbonate (33.6 mg, 0.240 mmol), Pd(dtbpf)Cl₂ (7.85 mg, 0.01 mmol) were added to compound **3-2** (30.0 mg, 0.120 mmol) in acetone (0.5 mL) and water (0.5 mL) at room temperature, the reaction was carried out in a microwave reactor at 120 °C for 30 min. The reaction solution was evaporated to dryness under reduced pressure, and the residue was extracted 3 times with dichloromethane (1 mL), the organic phases were combined, dried over anhydrous sodium sulfate, filtered, and dried by rotary evaporation. The crude product was purified by preparation column (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **3.** LC-MS: m/z 387.3 [M+H]⁺.

### Example 8: Preparation of Compound 2

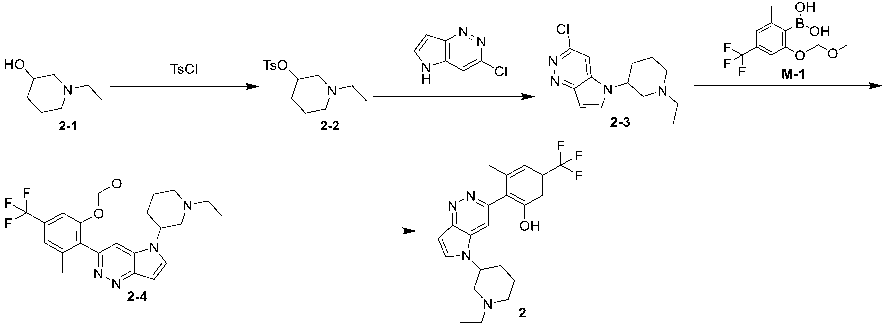

**Step 1):** Compound **2-1** (1.03 mL, 7.74 mmol, Bide) and pyridine (10 mL) were sequentially added to a 40 mL IKA bottle at room temperature, and p-toluenesulfonyl chloride (1.62 g, 8.51 mmol) was added after mixed thoroughly. The reaction solution was stirred at 25 °C for 16 hours. After the raw material was completely reacted monitored by LC-MS, the reaction solution was concentrated into a dark red mud by rotary evaporation under reduced pressure, then 10 mL of ethanol was added and shaken evenly to obtain a dark red suspension, which was concentrated again by rotary evaporation under reduced pressure. Ethanol was added, concentrated, and repeated three times to obtain a slightly reddish white solid. as little ethanol as possible was added and shake evenly to obtain a suspension with a large amount of white solid. The suspension was filtered under reduced pressure, and the filter residue was washed twice with a small amount of ethanol to obtain a solid. The solid was dried by rotary evaporation under reduced pressure to obtain compound **2-2.** LC-MS: m/z :284.3 [M+H]⁺.

¹H NMR (400 MHz, CDCl₃) δ 7.87 (d, *J =* 8.3 Hz, 2H), 7.40 (d, *J =* 8.3 Hz, 2H), 5.08 - 5.00 (m, 1H), 3.60 - 3.56 (m, 1H), 3.43 - 3.40 (m, 1H), 3.12 - 3.09 (m, 2H), 2.69 - 2.60 (m, 2H), 2.47 (s, 3H), 2.30 - 2.20 (m, 1H), 2.13 - 2.05 (m, 1H), 1.96 - 1.90 (m, 1H), 1.66 - 1.57 (m, 1H), 1.44 (t, *J =* 7.4 Hz, 3H).

**Step 2):** Compound 3-chloro-5H-pyrrolo[3,2-c]pyridazine (1.30 g, 8.47 mmol, preparation method with reference to: WO2022121914A1, page 73, synthesis of compound 25c) was added to a 100 mL single-necked bottle at room temperature, dissolved in dimethylformamide (20 mL), then cesium carbonate (11.0 g, 33.9 mmol) and compound **2-2** (5.28 g, 18.62 mmol) were added to the reaction bottle. The reaction solution was stirred at 100 °C for 6 hours. The reaction solution was poured into 50 ml of water, extracted with ethyl acetate (50 ml), the organic phase was concentrated under reduced pressure, and the residue was purified by column chromatography (ethyl acetate/petroleum ether = 0-50%) to obtain compound **2-3.** LC-MS: m/z :265.2 [M+H] ⁺.

¹H NMR (400 MHz, DMSO-*d₆*) δ 8.09 (d, *J =* 0.7 Hz, 1H), 7.96 - 7.94 (m, 2H), 6.93 - 6.92 (m, 1H), 4.20 (d, *J* = 5.3 Hz, 2H), 3.00 - 2.97 (m, 1H), 2.82 - 2.78 (m, 1H), 2.20 - 2.09 (m, 2H), 1.74 - 1.73 (m, 1H), 1.58 - 1.57 (m, 1H), 1.41 - 1.37 (m, 2H), 0.90 (t, *J*=7.2 Hz, 3H).

**Step 3):** Compound **2-3** (90.0 mg, 0.340 mmol), intermediate **M-1** (90.0 mg, 0.340 mmol), potassium phosphate (289 mg, 1.36 mmol) and dioxane (2 mL), water (0.2 mL) were added to a 25 mL three-necked bottle, stirred evenly, and XPhos Pd G3 (29 mg, 0.03 mmol) was added, and the mixture was purged with nitrogen three times. The reaction solution was stirred at 80°C for 1 hour. The reaction solution was cooled to room temperature, water (10 mL) was added, and extracted with ethyl acetate (5 mL×3). The organic phase was washed with saturated brine (5 mL), dried over anhydrous sodium sulfate, and concentrated under reduced pressure to remove the solvent. The residue was purified by column chromatography (0%-10% methanol/dichloromethane) to obtain compound **2-4.** LC-MS: m/z :449.2 [M+H] ⁺.

**Step 4):** Compound **2-4** (60 mg, 0.13 mmol) and dichloromethane (1 mL) were added to a 25 mL single-necked bottle at room temperature, stirred evenly, trifluoroacetic acid (1 mL) was added, the reaction solution was stirred at room temperature for 2 hours. Saturated sodium bicarbonate aqueous solution was added to the reaction solution and the pH was adjusted to 7-8. The mixture was extracted with dichloromethane (5 mL) three times, the organic phases were combined, washed with saturated brine (8 mL), dried over anhydrous sodium sulfate, filtered and concentrated under reduced pressure. The residue was purified by high-performance liquid chromatography (phase A is 0.01 mol per liter ammonium bicarbonate aqueous solution, phase B is acetonitrile) to obtain compound 2. LC-MS: m/z :405.1 [M+H]⁺.

¹H NMR (400 MHz, DMSO-*d₆*) δ 10.08 (s, 1H), 7.90 (d, *J =* 3.6 Hz, 1H), 7.81 (s, 1H), 7.16 (s, 1H), 7.11 (s, 1H), 6.91 (d, *J* = 3.2 Hz, 1H), 4.21 - 4.17 (m, 2H), 2.99 - 2.97 (m, 1H), 2.81 - 2.79 (m, 1H), 2.59 - 2.56 (m, 1H), 2.19 - 2.08 (m, 2H), 2.06 (s, 3H), 1.72 - 1.68 (m, 1H), 1.60 - 1.56 (m, 1H), 1.47 - 1.42 (m, 2H), 0.91 - 0.85(m, 3H).

### Example 9: Preparation of Compound 4

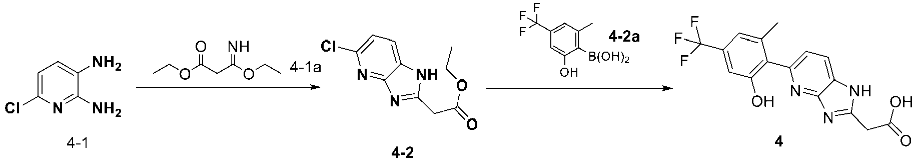

**Step 1):** Compound **4-1** (1.00 g, 6.97 mmol, Bide) and compound **4-1a** (1.50 g, 7.66 mmol, Bide) were added to ethanol (10 mL) at room temperature and refluxed with stirring overnight. The reaction system was directly concentrated, and the residue was purified by column chromatography (0-3% methanol/dichloromethane) to obtain compound **4-2.** LC-MS: m/z:240.1 [M+H]⁺.

**Step 2):** Compound **4-2a** (97.4 mg, 0.440 mmol, synthesis method with reference to: US20200361898A1, synthesis of intermediate B005 on page 39), Pd(dtbpf)Cl₂ (28.6 mg, 0.040 mmol) and potassium carbonate (122 mg, 0.890 mmol) were added to compound **4-2** in acetonitrile (0.5 mL) and water (0.5 mL) at room temperature, and the reaction was carried out in a microwave reactor at 120 °C for 40 minutes. The reaction solution was concentrated, the residue was mixed with water (5 mL), extracted 3 times with dichloromethane (5 mL), the organic phases were combined, dried over anhydrous sodium sulfate, filtered, and dried by rotary evaporation. The residue was purified by preparation column (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound 4. LC-MS: m/z 352.3 [M+H]⁺.

### Example 10: Preparation of Compound 6

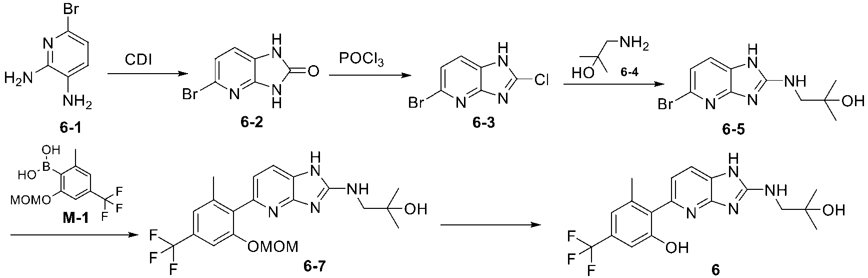

**Step 1):** *N, N*'-carbonyldiimidazole (1.29 g, 7.98 mmol) was added to compound **6-1** (1000 mg, 5.32 mmol, Bide) in tetrahydrofuran (10 mL) at room temperature. The reaction solution was stirred at 70°C for 3 hours. The mixture was filtered to obtain compound **6-2.** LC-MS: m/z :214.0 [M+H]⁺.

**Step 2):** Tetramethylammonium chloride (410 mg, 3.74 mmol) was added to compound **6-2** (400 mg, 1.87 mmol) in phosphorus oxychloride (20 mL). The reaction solution was stirred at 110 °C for 3 hours. After the reaction was completed, it was cooled to room temperature, and distillated under pressure to remove phosphorus oxychloride, saturated sodium bicarbonate solution (200 mL) was added for neutralization, extracted with ethyl acetate (200 mL×3). The organic phase was dried over anhydrous sodium sulfate and concentrated to obtain compound **6-3.** LC-MS: m/z :233.9 [M+H]⁺.

**Step 3):** Compound **6-4** (481 mg, 5.38 mmol) and N-methylmorpholine (0.35 mL, 3.23 mmol) were added to compound **6-3** (250 mg, 1.08 mmol) in N-methylpyrrolidone (4 mL). The reaction solution was stirred at 100 °C for 18 hours. The reaction solution was purified by C18 liquid chromatography (eluent: 17% acetonitrile aqueous solution) to obtain compound **6-5.** LC-MS: m/z :284.9 [M+H]⁺.

**Step 4):** Potassium carbonate (145 mg, 1.05 mmol) and 1,1'-bis(diphenylphosphino)ferrocene palladium(II) dichloride (26 mg, 0.04 mmol) were added to compound **6-5** (100 mg, 0.35 mmol) and intermediate M-1 (93 mg, 0.35 mmol) in dioxane (5 mL) and water (1 mL) at room temperature. The reaction solution was stirred at 80 °C for 2 hours under a nitrogen protection. The reaction solution was filtered and concentrated under reduced pressure, and the residue was purified by chromatography on a silica gel plate (eluent: dichloromethane/methanol = 10/1) to obtain compound **6-7.** LC-MS: m/z :425.5 [M+H]⁺.

**Step 5):** Trifluoroacetic acid (1 mL) was added to compound **6-7** (60 mg, 0.14 mmol) in dichloromethane (2 mL) at room temperature. The reaction solution was stirred at 25 °C for 1 hour. The reaction solution was concentrated under reduced pressure, and the residue was purified by high performance liquid chromatography (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound 6. LC-MS: m/z :381.1 [M+H]⁺.

¹H NMR (400 MHz, DMSO-*d₆*) δ 7.67 (s, 1H), 7.12 (s, 1H), 7.06 - 7.02 (m, 2H), 3.32 (s, 2H), 2.13 (s, 3H), 1.17 (s, 6H).

### Example 11: Preparation of Compound 13

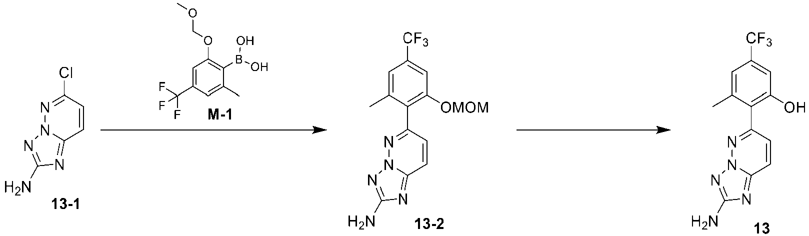

**Step 1):** Compound **13-1** (50.0 mg, 0.290 mmol), intermediate **M-1** (132 mg, 0.500 mmol), Xphos Pd G3 (49.92 mg, 0.06 mmol), potassium phosphate (250 mg, 1.18 mmol), dioxane (2 mL), and water (0.2 mL) were added to an 8 mL single-necked bottle at room temperature. The reaction was carried out under a nitrogen protection at 80 °C for 1 hour. Dichloromethane (10 mL) was added to the reaction solution, diluted with saturated sodium chloride aqueous solution (3 mL), and extracted 3 times with dichloromethane (5 mL), the organic phases were combined, dried over anhydrous sodium sulfate, filtered, and dried by rotary evaporation. The residue was purified by preparative thin layer chromatography on silica gel plates (dichloromethane/methanol = 20/1) to obtain compound **13-2.** LC-MS: m/z : 354.0 [M+H]⁺.

**Step 2):** Compound **13-2** was dissolved in dichloromethane (1 mL) in an 8 mL single-necked bottle at room temperature, and trifluoroacetic acid (1 mL) was added. The reaction was carried out at 25 °C for 0.5 hours. The reaction solution was dried by rotary evaporation. The residue was purified by high performance liquid chromatography (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **13.** LC-MS: m/z : 310.1 [M+H]⁺.

¹H NMR (400 MHz, DMSO-*d₆*) δ 10.32 (s, 1H), 7.96 (d, J = 9.1 Hz, 1H), 7.39 (d, J = 9.1 Hz, 1H), 7.17 (s, 1H), 7.09 (s, 1H), 6.35 (s, 2H), 2.15 (s, 3H).

### Example 12: Preparation of Compound 7

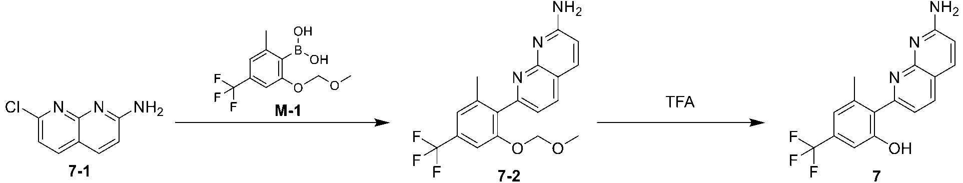

**Step 1):** Intermediate **M-1** (73.9 mg, 0.280 mmol), potassium carbonate (0.56 mL, 0.56 mmol, 1M aqueous solution), SPhos Pd G3 (24.1 mg, 0.03 mmol) were added to compound **7-1** (50.0 mg, 0.280 mmol) in dioxane/water (1.0 mL/0.2 mL) at room temperature. The system was purged with nitrogen three times, and the reaction solution was stirred at 90 °C for 3 hours. The reaction solution was diluted with water (5 mL), and extracted 3 times with ethyl acetate (5 mL), the organic phases were combined, dried over anhydrous sodium sulfate, filtered, and dried by rotary evaporation to obtain a crude compound **7-2,** which was directly used in the next step without purification.

**Step 2):** Trifluoroacetic acid (1 mL) was added to compound **7-2** (45.0 mg, 0.120 mmol) in dichloromethane (3 mL) at room temperature, and the reaction solution was stirred at room temperature for 2 hours. The reaction solution was evaporated to dryness under reduced pressure, and the residue was purified by preparation column (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **7.** LC-MS: m/z :320.1 [M+H]⁺.

### Example 13: Preparation of Compound 8

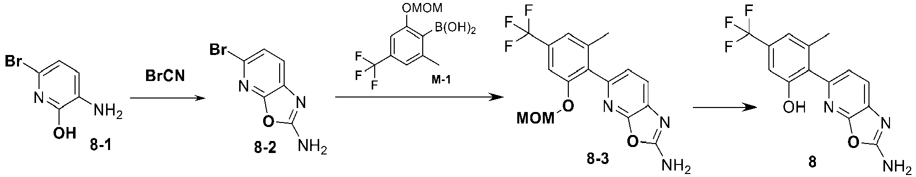

**Step 1):** Compound **8-1** (3.58 g, 18.9 mmol, Bide) was added to a single-necked bottle at room temperature, then methanol (35 mL) was added, and cyanogen bromide (2.01 g, 18.9 mmol) was added to the reaction solution with rapidly stirring. The reaction solution was allowed to react at 50 °C for 2 hours after the addition. After the reaction solution cooled to room temperature, saturated sodium bicarbonate solution (200 mL) was added to the reaction solution, and the solid was precipitated and filtered. The filter cake was washed with water (20 mL × 3) and dried to obtain compound **8-2.** LC-MS: m/z: 214.0 [M+H]⁺.

¹H NMR (400 MHz, DMSO-*d₆*) δ 7.92 (s, 2H), 7.51 (d, *J* = 8.0 Hz, 1H), 7.36 (d, *J* = 8.0 Hz, 1H).

**Step 2):** Compound **8-2** (100 mg, 0.470 mmol), intermediate M-1 (248 mg, 0.940 mmol), Pd(dppf)Cl₂ (34.4 mg, 0.050 mmol), potassium carbonate (260 mg, 1.88 mmol), dioxane (2 mL), water (0.2 mL) were added to a single-necked bottle at room temperature. The reaction was carried out under a nitrogen protection at 80 °C for 1 hour. Dichloromethane (10 mL) was added to the reaction solution, diluted with water (5 mL), separated, and extracted 3 times with dichloromethane (5 mL), the organic phases were combined, dried over anhydrous sodium sulfate, filtered, and dried by rotary evaporation to obtain crude compound **8-3.**

**Step 3):** Compound **8-3** (145 mg, 0.410 mmol), dichloromethane (3 mL), and trifluoroacetic acid (1.5 mL) were added to a single-necked bottle at room temperature. The reaction was carried out at 25 °C for 1 hour. The reaction solution was evaporated to dryness under reduced pressure. The residue was purified by preparation column (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **8.** LC-MS: m/z : 310.0 [M+H]⁺.

### Example 14: Preparation of Compound 1

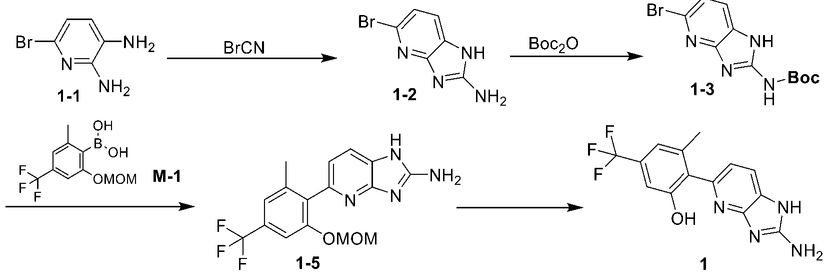

**Step 1):** At room temperature, the mixture of compound **1-1** (500 mg, 2.66 mmol), cyanogen bromide (282 mg, 2.66 mmol), methanol (5 mL) and water (5 mL) was stirred at 50 °C for 3 hours. The mixture was distilled under reduced pressure, then ethyl acetate (10 mL) and petroleum ether (10 mL) were added to the residue, stirred for 2 hours, then the mixture was filtered to obtain compound **1-2.** LC-MS: m/z:212.9 [M+H]⁺.

**Step 2):** Di-tert-butyl dicarbonate (799 mg, 3.66 mmol), triethylamine (0.78 mL, 5.63 mmol) and 4-dimethylaminopyridine (1.03 g, 8.45 mmol) were added to compound **1-2** (600 mg, 2.82 mmol) in tetrahydrofuran (3 mL) at room temperature. The reaction solution was stirred at 25 °C for 18 hours. Ethyl acetate (50 ml) and water (50 ml) were added to the reaction solution for extraction. The organic phase was washed with saturated brine (50 mL), dried over anhydrous sodium sulfate, filtered and concentrated, and the crude product was purified by silica gel chromatography (eluent: petroleum ether/ethyl acetate = 1/1) to obtain compound **1-3.** LC-MS: m/z :312.9 [M+H]⁺.

**Step 3):** Potassium carbonate (212 mg, 1.53 mmol) and 1,1'-bis(diphenylphosphino)ferrocene palladium(II) dichloride (37.4 mg, 0.050 mmol) were added to the mixture of compound 1-3 (160 mg, 0.51 mmol), intermediate M-1 (270 mg, 1.02 mmol), dioxane (3 mL) and water (0.5 mL) at room temperature. The mixture was purged with nitrogen three times and stirred at 80 °C for 18 hours. The reaction solution was filtered and concentrated under reduced pressure, and the residue was purified by silica gel chromatography (eluent: dichloromethane/methanol = 20/1) to obtain compound **1-5.** LC-MS: m/z :353.1 [M+H]⁺.

**Step 4):** Trifluoroacetic acid (1 mL) was added to compound 1-5 (80 mg, 0.23 mmol) in dichloromethane (1 mL) at room temperature. The reaction solution was stirred at 25°C for 2 hours. The reaction solution was concentrated under reduced pressure, and the residue was purified by high performance liquid chromatography (Waters-SunFire-C18-10µm-19×250mm; mobile phase: water (containing 10mmol/L formic acid) and acetonitrile, gradient ratio: acetonitrile 23%-48%, flow rate: 30mL/min) to obtain compound **1.** LC-MS: m/z :309.1 [M+H]⁺.

¹H NMR (400 MHz, DMSO-*d₆*) δ 8.16 (s, 1H), 7.43 (d, *J =* 7.9 Hz, 1H), 7.07 (s, 1H), 7.02 (s, 1H), 6.86 (d, *J* = 7.9 Hz, 1H), 6.60 (s, 2H), 2.14 (s, 3H).

### Example 15: Preparation of Compound 111

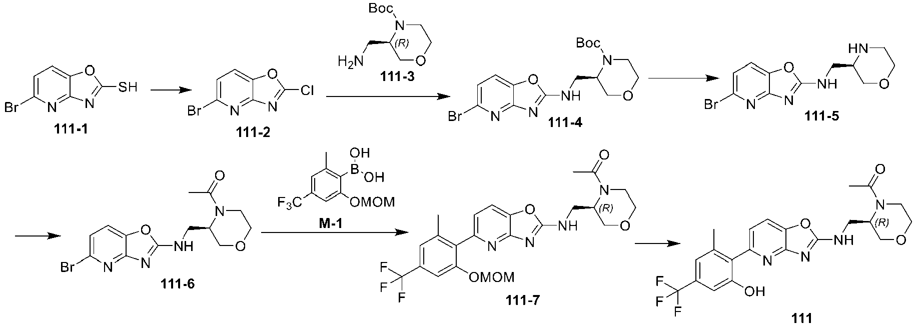

**Step 1):** Compound **111-1** (10.0 g, 43.3 mmol, prepared by the method disclosed in the intermediate step A compound on page 102 of the specification of "WO2022078971A1"), thionyl chloride (100 mL) were added to a 500 mL single-necked bottle at room temperature, with stirring, and N,N-dimethylformamide (1 mL) was slowly added dropwise, the reaction solution was stirred at 70°C for 3 hours. The reaction solution was concentrated, and water was added to the residue, extracted with ethyl acetate (100 mL×3),washed with saturated brine (50 mL×1), dried over anhydrous sodium sulfate, and concentrated under reduced pressure. The residue was purified by silica gel column chromatography with an eluent system (petroleum ether/ethyl acetate = 50/1 to 10/1) to obtain compound **111-2.**

¹H NMR (400 MHz, DMSO-*d₆*) δ 8.25 (d, *J =* 8.5 Hz, 1H), 7.75 (d, *J =* 8.5 Hz, 1H).

**Step 2):** Compound **111-3** (1.00 g, 4.28 mmol, Leyan) and compound **111-2** (923 mg, 4.28 mmol) were dissolved in acetonitrile (20 mL), potassium carbonate (1.78 g, 12.8 mmol) was added, and the reaction solution was stirred at 80 °C for 2 hours. Water (50 mL) was added to the reaction solution, and extracted with dichloromethane (50 mL×3),the organic phases were combined, and washed with saturated brine. The organic phase was dried over anhydrous sodium sulfate, filtered to remove the desiccant, and the filtrate was concentrated under reduced pressure. The residue was purified by silica gel column chromatography with an eluent system (dichloromethane/methanol = 40/1 to 15/1) to obtain compound **111-4.** MS m/z(ESI):415.1 [M+1]⁺.

**Step 3):** Compound **111-4** (1.70 g, 4.11 mmol) was dissolved in dichloromethane (10 mL), trifluoroacetic acid (10 mL) was added, the reaction solution was stirred at 25°C for 3 hours, and the reaction solution was concentrated under reduced pressure to obtain compound **111-5.** MS m/z(ESI):314.9 [M+1]⁺.

**Step 4):** Compound **111-5** (1.50 g, 4.79 mmol) was dissolved in dichloromethane (20 mL), triethylamine (3.32 mL, 23.4 mmol) was added, and the reaction was carried out with stirring at 0 °C for 10 minutes. Acetic anhydride (0.47 mL, 5.03 mmol) was added to the reaction solution, and the reaction continued at 25 °C for 15 minutes. Water (50 mL) was added to the reaction solution and extracted with dichloromethane (50 mL×3),the organic phases were combined, washed with saturated brine, dried over anhydrous sodium sulfate, and filtered to remove the desiccant, and the filtrate was concentrated under reduced pressure. The residue was purified by silica gel column chromatography with an eluent system (dichloromethane/methanol = 40/1 to 15/1) to obtain compound **111-6.** MS m/z(ESI):355.0 [M+1]⁺.

**Step 5):** Compound **111-6** (1.15 g, 3.24 mmol) and intermediate **M-1** (1.71 g, 6.48 mmol) were dissolved in dioxane (30 mL) and water (6 mL), 1,1'-bis(diphenylphosphino)ferrocene palladium(II) dichloride (236 mg, 0.320 mmol, adamas) and potassium carbonate (1.78 g, 12.6 mmol) were added. The reaction was purged with nitrogen three times, and stirred for 18 hours at 80°C under a nitrogen atmosphere. After the reaction was completed, water (50 mL) was added to the reaction solution and extracted with dichloromethane (50 mL×2). The combined organic phase was dried over anhydrous sodium sulfate, and filtered to remove the desiccant, and the filtrate was concentrated under reduced pressure. The residue was purified by silica gel column chromatography (dichloromethane/methanol = 100/1 to 5/10) to obtain compound **111-7.** MS m/z(ESI):495.3[M+1]⁺.

**Step 6):** Compound **111-7** (1.26 g, 2.29 mmol) was dissolved in the dioxane solution of hydrochloric acid (12 mL, 4M), and the reaction solution was stirred at 25°C for 2 hours. The reaction solution was filtered to obtain a filter cake, which was dissolved in methanol (3 mL) and dichloromethane (20 mL) and extracted by added saturated sodium bicarbonate solution (20 mL). The organic phase was dried over anhydrous sodium sulfate, filtered to remove the desiccant, and the filtrate was concentrated under reduced pressure. The residue was purified by silica gel column chromatography (dichloromethane/methanol = 100/1 to 5/10) to obtain compound **111.** MS m/z(ESI):451.2[M+1]⁺.

¹H NMR (400 MHz, DMSO-*d₆*) δ 9.93 (d, *J =* 3.8 Hz, 1H), 8.79 - 8.43 (m, 1H), 7.82 - 7.65 (m, 1H), 7.07 (d, *J* = 14.2 Hz, 2H), 6.98 - 6.87 (m, 1H), 4.68 - 4.00 (m, 2H), 3.94 - 3.80 (m, 2H), 3.74 - 3.61 (m, 2H), 3.56 - 3.41 (m, 2H), 3.29 - 2.95 (m, 1H), 2.12 - 1.91 (m, 6H).

### Example 16: Synthesis of Compound 247

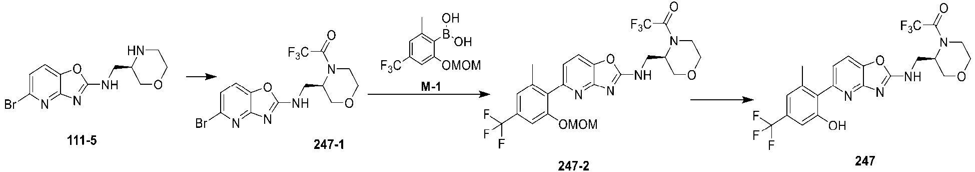

**Step 1):** Compound **111-5** (80.0 mg, 0.26 mmol) was dissolved in dichloromethane (5 mL), trifluoroacetic anhydride (82.0 mg, 0.39 mmol, Bide) and triethylamine (78.0 mg, 0.77 mmol) were added. After stirred at room temperature for 2 hours, the reaction solution was added to a saturated sodium bicarbonate solution (10 ml), and extracted with dichloromethane (10 mL×3),the organic phases were combined, dried over anhydrous sodium sulfate, and filtered to remove the desiccant, and the filtrate was concentrated under reduced pressure. The residue was purified by thin layer chromatography with an eluent system (dichloromethane/methanol = 20/1) to obtain compound **247-1.** MS m/z(ESI):408.0[M+1]+.

**Step 2):** Compound **247-1** (50.0 mg, 0.12 mmol) was dissolved in a mixed solution of 1,4-dioxane (3 mL) and water (0.6 mL), intermediate M-1 (65.0 mg, 0.24 mmol), potassium carbonate (51.0 mg, 0.37 mmol) and 1,1'-bis(diphenylphosphino)ferrocene palladium(II) dichloride (8.94 mg, 0.01 mmol) were added, and the mixture was purged with nitrogen three times. After stirred at 80 °C for 2 hours, water (15 ml) was added to the reaction solution, and extracted with ethyl acetate (10 mL×3),the organic phases were combined, dried over anhydrous sodium sulfate, and filtered to remove the desiccant, and the filtrate was concentrated under reduced pressure. The residue was purified by thin layer chromatography with an eluent system (dichloromethane/methanol = 20/1) to obtain compound **247-2.** MS m/z(ESI):547.3[M+1]+.

**Step 3):** Compound **247-2** (50.0 mg, 0.09 mmol) was dissolved in dichloromethane (3 mL), trifluoroacetic acid (3 mL) was added, and the reaction solution was stirred at room temperature for 2 hours. The reaction solution was directly concentrated under reduced pressure, and the residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min) to obtain compound **247.** MS m/z(ESI):505.3[M+1]+.

¹H NMR (400 MHz, DMSO-*d₆*) δ 9.92 (s, 1H), 8.71 - 8.63 (m, 1H), 7.77 - 7.73 (m, 1H), 7.08 (s, 1H), 7.05 (s, 1H), 6.96 - 6.92 (m, 1H), 4.63 (s, 1H), 4.10 - 4.07 (m, 1H), 4.00 - 3.93 (m, 2H), 3.87 - 3.82 (m, 2H), 3.67 - 3.56 (m, 2H), 3.49 - 3.44 (m, 1H), 2.07 (s, 3H).

### Example 17: Preparation of Compound 248

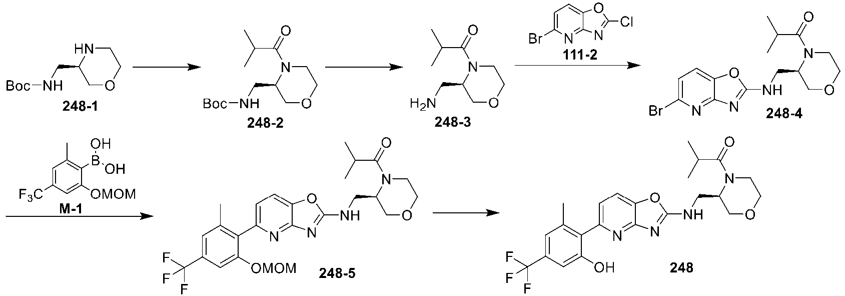

**Step 1):** Compound **248-1** (70.0 mg, 0.32 mmol) was dissolved in dichloromethane (3 mL), triethylamine (98.3 mg, 0.97 mmol) and isobutyryl chloride (44.8 mg, 0.42 mmol, Bide) were added at 0 °C, and the reaction solution was stirred at room temperature for 2 hours. The reaction mixture was added to a saturated sodium bicarbonate solution (15 ml), and extracted with dichloromethane (10 mL×3),the organic phases were combined, dried over anhydrous sodium sulfate, and filtered to remove the desiccant, and the filtrate was concentrated under reduced pressure to obtain compound **248-2,** the residue was directly used as the raw material for the next step without purification. MS m/z(ESI):231.2[M-55]+.

**Step 2):** Compound **248-2** (140 mg, 0.49 mmol) was dissolved in dichloromethane (3 mL), trifluoroacetic acid (3 mL) was added, and the reaction solution was stirred at room temperature for 1.5 hours. The reaction solution was directly concentrated under reduced pressure to obtain compound **248-3,** the residue was directly used as the raw material for the next step without purification. MS m/z(ESI):187.2[M+1]+.

**Step 3):** Compound **111-2** (110 mg, 0.47 mmol) was dissolved in acetonitrile (8 mL), compound **248-3** (87.8 mg, 0.47 mmol) and potassium carbonate (195 mg, 1.41 mmol) were added. After stirred at 80 °C for 2 hours, water (20 ml) was added to the reaction solution, and extracted with ethyl acetate (15 mL×3),the organic phases were combined, dried over anhydrous sodium sulfate, and filtered to remove the desiccant, and the filtrate was concentrated under reduced pressure. The residue was purified by silica gel column chromatography with an eluent system (dichloromethane/methanol = 100/1 to 20/1) to obtain compound **248-4.** MS m/z(ESI):383.1[M+1]+.

**Step 4):** Compound **248-4** (50.0 mg, 0.13 mmol) was dissolved in a mixed solution of 1,4-dioxane (3 mL) and water (0.6 mL), intermediate **M-1** (68.9 mg, 0.24 mmol), potassium carbonate (54.1 mg, 0.39 mmol) and 1,1'-bis(diphenylphosphino)ferrocene palladium(II) dichloride (9.55 mg, 0.01 mmol) were added, and the mixture was purged with nitrogen three times. After stirred at 80 °C for 12 hours, water (10 ml) was added to the reaction solution, and extracted with ethyl acetate (10 mL×3),the organic phases were combined, dried over anhydrous sodium sulfate, and filtered to remove the desiccant, and the filtrate was concentrated under reduced pressure. The residue was purified by thin layer chromatography with an eluent system (dichloromethane/methanol = 20/1) to obtain compound **248-5.** MS m/z(ESI):523.3[M+1]+.

**Step 5):** Compound **248-5** (50.0 mg, 0.10 mmol) was dissolved in dichloromethane (2 mL), trifluoroacetic acid (2 mL) was added, and the reaction solution was stirred at room temperature for 2 hours. The reaction solution was directly concentrated under reduced pressure, and the residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min) to obtain compound **248.** MS m/z(ESI):479.3[M+1]+.

¹H NMR (400 MHz, DMSO-*d₆*) δ 9.95 (s, 1H), 8.63 (d, *J =* 81.9 Hz, 1H), 7.78 - 7.71 (m, 1H), 7.08 (s, 1H), 7.05 (s, 1H), 6.93 (dd, *J =* 11.9, 8.0 Hz, 1H), 4.68 - 4.63 (m, 1H), 4.14 - 4.11 (m, 1H), 3.89 - 3.78 (m, 1H), 3.70 - 3.63 (m, 2H), 3.55 - 3.50 (m, 2H), 3.32 - 3.25 (m, 2H), 3.04 - 2.93 (m, 1H), 2.07 (s, 3H), 1.00 - 0.96 (m, 3H), 0.90 - 0.75 (m, 3H).

### Example 18: Preparation of Compound 249

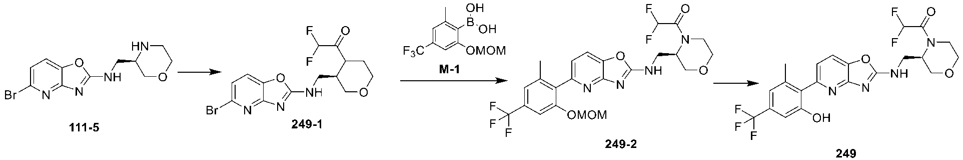

**Step 1):** Compound **111-5** (80.0 mg, 0.26 mmol) was dissolved in dichloromethane (5 mL), triethylamine (77.6 mg, 0.77 mmol) and difluoroacetic anhydride (67.9 mg, 0.39 mmol, Bide) were added at 0 °C, and stirred at room temperature for 2 hours. The reaction mixture was added to a saturated sodium bicarbonate solution (15 mL), and extracted with dichloromethane (10 mL×3), the organic phases were combined, dried over anhydrous sodium sulfate, and filtered to remove the desiccant, and the filtrate was concentrated under reduced pressure, and the residue was purified by thin layer chromatography with an eluent system (dichloromethane/methanol = 20/1) to obtain compound **249-1.** MS m/z(ESI):390.1[M+1]+.

**Step 2):** Compound **249-1** (50.0 mg, 0.13 mmol) was dissolved in a mixed solution of 1,4-dioxane (3 mL) and water (0.6 mL), intermediate **M-1** (67.5 mg, 0.26 mmol), potassium carbonate (53.0 mg, 0.38 mmol) and 1,1'-bis(diphenylphosphino)ferrocene palladium(II) dichloride (9.35 mg, 0.01 mmol) were added, and the mixture was purged with nitrogen three times. After stirred at 80°C for 2 hours, water (15 mL) was added to the reaction solution, and extracted with ethyl acetate (10 mL×3), the organic phases were combined, dried over anhydrous sodium sulfate, and filtered to remove the desiccant, and the filtrate was concentrated under reduced pressure, the residue was purified by thin layer chromatography with an eluent system (dichloromethane/methanol = 20/1) to obtain compound **249-2.** MS m/z(ESI):531.7[M+1]+.

**Step 3):** Compound **249-2** (50.0 mg, 0.09 mmol) was dissolved in dichloromethane (2 mL), trifluoroacetic acid (2 mL) was added, and the reaction solution was stirred at room temperature for 2 hours. The reaction solution was directly concentrated under reduced pressure, and the residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min) to obtain compound **249.** MS m/z(ESI):487.4[M+1]+.

¹H NMR (400 MHz, DMSO-*d₆*) δ 9.91 (s, 1H), 8.67 - 8.52 (m, 1H), 7.77 - 7.10(m, 1H), 7.07 (d, *J=* 12.2 Hz, 2H), 6.96 - 6.91 (m, 1H), 6.81 - 6.53 (m, 1H), 4.60 - 4.15 (m, 1H), 4.06 (d, *J =* 13.8 Hz, 1H), 4.00 - 3.90 (m, 2H), 3.85 - 3.70 (m, 2H), 3.64 - 3.54 (m, 2H), 3.45 - 3.40 (m, 1H), 2.07 (d, *J* = 2.8 Hz, 3H).

### Example 19: Preparation of Compound 250

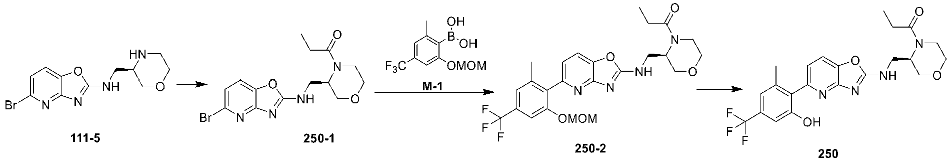

**Step 1):** Compound **111-5** (50.0 mg, 0.16 mmol) was dissolved in dichloromethane (3 mL), triethylamine (48.5 mg, 0.48 mmol) and propionyl chloride (14.8 mg, 0.16 mmol, Bide) were added at 0°C, stirred at 0 °C and allowed to react for 3 minutes. The reaction solution was added to a saturated sodium bicarbonate solution (15 ml), and extracted with dichloromethane (10 mL × 3), the organic phases were combined, dried over anhydrous sodium sulfate, and filtered to remove the desiccant, and the filtrate was concentrated under reduced pressure, the residue was purified by thin layer chromatography with an eluent system (dichloromethane/methanol = 20/1) to obtain compound **250-1.** MS m/z(ESI):371.1[M+1]+.

**Step 2):** Compound **250-1** (50.0 mg, 0.14 mmol) was dissolved in a mixed solution of 1,4-dioxane (3 mL) and water (0.6 mL), intermediate **M-1** (71.5 mg, 0.27 mmol), potassium carbonate (56.2 mg, 0.41 mmol) and 1,1'-bis(diphenylphosphino)ferrocene palladium(II) dichloride (9.91 mg, 0.01 mmol) were added, and the mixture was purged with nitrogen three times. After stirred at 80 °C for 12 hours, water (10 mL) was added to the reaction solution, and extracted with ethyl acetate (10 mL×3), the organic phases were combined, dried over anhydrous sodium sulfate, and filtered to remove the desiccant, and the filtrate was concentrated under reduced pressure, the residue was purified by thin layer chromatography with an eluent system (dichloromethane/methanol = 20/1) to obtain compound **250-2.** MS m/z(ESI):509.4[M+1]+.

**Step 3):** Compound **250-2** (50.0 mg, 0.10 mmol) was dissolved in dichloromethane (2 mL), trifluoroacetic acid (2 mL) was added, and the reaction solution was stirred at room temperature for 2 hours. The reaction solution was directly concentrated under reduced pressure, and the residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min) to obtain compound **250.** MS m/z(ESI):465.7[M+1]+.

¹H NMR (400 MHz, DMSO-*d₆*) δ 10.07 (s, 1H), 9.02 - 8.80 (m, 1H), 7.89 - 7.82 (m, 1H), 7.12 - 7.10 (m, 1H), 7.07 (s, 1H), 7.03 - 6.99 (m, 1H), 4.70 - 4.60 (m, 1H), 4.16 - 4.07 (m, 1H), 3.90 - 3.83 (m, 2H), 3.70 - 3.62 (m, 2H), 3.58 - 3.43 (m, 2H), 3.38 - 3.26 (m, 1H), 2.43 - 2.29 (m, 2H), 2.10 - 2.07 (m, 3H), 0.97 - 0.84 (m, 3H).

### Example 20: Preparation of Compound 253

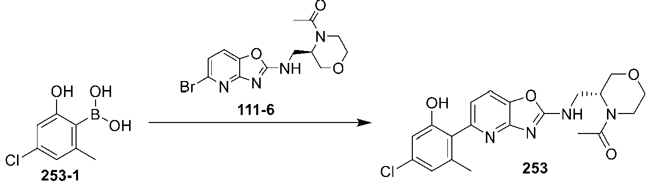

Compound **253-1** (209 mg, 1.13 mmol, Bide) was dissolved in a dioxane-water solution (5/1, 5 mL), compound **111-6** (200 mg, 0.56 mmol), 1,1'-bis(diphenylphosphino)ferrocene palladium(II) dichloride (41.2 mg, 0.06 mmol), potassium carbonate (312 mg, 2.25 mmol) were added, and the mixture was purged with nitrogen three times. The reaction solution was stirred at 80 °C for 2 hours, the reaction solution was diluted with water (5 mL), and extracted with ethyl acetate (5 mL) three times, and the organic phases were combined, dried over anhydrous sodium sulfate, filtered, and dried by rotary evaporation. The residue was purified by high performance liquid chromatography (Waters-2545, column: SharpSil-T C18, 30×150mm, 5µm; mobile phase: water (containing 10 mmol/L formic acid) and acetonitrile, gradient ratio: acetonitrile 38%-45%, flow rate: 30 mL/min) to obtain compound **253.** MS m/z(ESI):417.5[M+1]+.

¹H NMR (400 MHz, DMSO-*d₆*) δ 9.81 - 9.71 (m, 1H), 8.77 - 8.43 (m, 1H), 7.76 - 7.66 (m, 1H), 6.94 - 6.84 (m, 1H), 6.83 - 6.76 (m, 2H), 4.68 - 4.07 (m, 1H), 3.90 - 3.81 (m, 2H), 3.73 - 3.58 (m, 2H), 3.56 - 3.39 (m, 3H), 3.28 - 2.94 (m, 1H), 2.09 - 1.91 (m, 6H).

### Example 21: Preparation of Compound 254

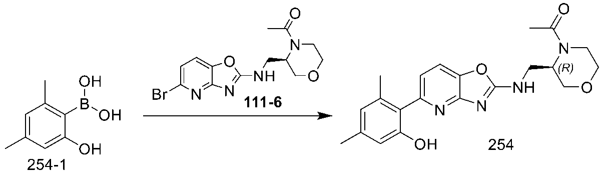

Compound **254-1** (120 mg, 0.72 mmol, Bide), compound **111-6** (154 mg, 0.43 mmol) were dissolved in dioxane (5 mL) and water (1 mL), 1,1'-bis(diphenylphosphino)ferrocene palladium(II) dichloride (52.9 mg, 0.07 mmol, adamas) and potassium carbonate (199 mg, 1.45 mmol) were added. The reaction solution was purged with nitrogen three times, stirred at 80 °C for 18 hours under a nitrogen atmosphere. After the reaction was completed, water (20 mL) was added to the reaction solution, and extracted with dichloromethane (20 mL×2). The combined organic phase was dried over anhydrous sodium sulfate, and filtered to remove the desiccant, and the filtrate was concentrated under reduced pressure. The residue was purified by high performance liquid chromatography (Gilson_306_1741, chromatographic column: Waters-SunFire-C18-10µm-19×250mm; mobile phase: water (containing 10 mmol/L formic acid) and acetonitrile, gradient ratio: acetonitrile 46%-95%, flow rate: 25 mL/min) to obtain compound **254.** MS m/z(ESI):397.3[M+1]+.

¹H NMR (400 MHz, DMSO-*d₆*) δ 9.09 (s, 1H), 8.52 - 8.13 (m, 1H), 7.67 (d, *J =* 7.9 Hz, 1H), 6.93 (d, *J* = 9.0 Hz, 1H), 6.56 (d, *J =* 6.6 Hz, 2H), 4.87 - 4.38 (m, 1H), 4.24 - 4.05 (m, 1H), 3.94 - 3.81 (m, 2H), 3.75 - 3.60 (m, 2H), 3.58 - 3.26 (m, 3H), 2.23 (s, 3H), 2.12 - 1.84 (m, 6H).

### Example 22: Preparation of Compound 252

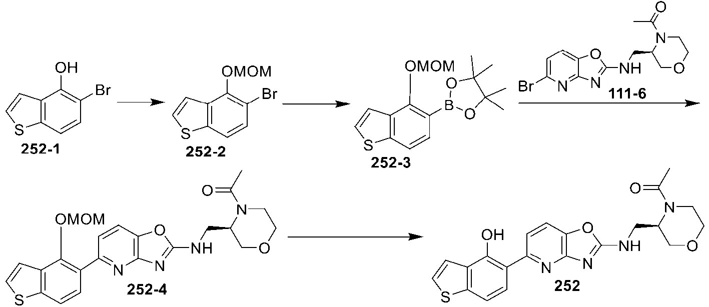

**Step 1):** Compound **252-1** (500 mg, 2.16 mmol, prepared by the intermediate 20-3 preparation method disclosed on page 66 of the specification of "WO 2022/166890Al") and chloromethyl methyl ether (0.25 mL, 3.27 mmol) were dissolved in dichloromethane (10 mL), and N, N-diisopropylethylamine (0.72 mL, 4.3 mmol) was added. The reaction solution was stirred at 25 °C for 18 hours, and water (50 mL) was added to the reaction solution, and extracted with ethyl acetate (50 mL×3), the organic phases were combined, and washed with saturated brine, and the organic phase was dried over anhydrous sodium sulfate, and filtered to remove the desiccant, and the filtrate was concentrated under reduced pressure, the residue was purified by silica gel column chromatography with an eluent system (petroleum ether/ethyl acetate = 50/1 to 10/1) to obtain compound **252-2.**

**Step 2):** Compound **252-2** (300 mg, 1.10 mmol) was dissolved in anhydrous tetrahydrofuran, and n-butyl lithium (0.57 mL, 2.33 mmol, 2.5M) was added at -40 °C. After the reaction solution was stirred at -40 °C for half an hour, 2-isopropoxy-4,4,5,5-tetramethyl-1,3,2-dioxaborolane (0.45 mL, 2.20 mmol) was added, and the reaction was continued at 25 °C for 2 hours. Ice water (20 mL) was added to the reaction solution, and extracted with ethyl acetate was (20 mL×3), the organic phases were combined, and washed with saturated brine, and the organic phase was dried over anhydrous sodium sulfate and filtered to remove the desiccant, and the filtrate was concentrated under reduced pressure, the residue was purified by silica gel column chromatography with an eluent system (petroleum ether/ethyl acetate = 50/1 to 10/1) to obtain compound **252-3.**

¹H NMR (400 MHz, CDCl₃) δ 8.09 (s, 1H), 7.53 (d, *J =* 8.3 Hz, 1H), 7.29 (t, *J =* 8.0 Hz, 1H), 6.96 (d, *J =* 7.8 Hz, 1H), 5.31 (s, 2H), 3.51 (s, 3H), 1.38 (s, 12H).

**Step** 3): Compound **252-3** (200 mg, 0.62 mmol) and compound **111-6** (155 mg, 0.44 mmol) were dissolved in dioxane (5 mL) and water (1 mL), 1,1'-bis(diphenylphosphino)ferrocene palladium(II) dichloride(45.7 mg, 0.06 mmol, adamas) and potassium carbonate (172 mg, 1.25 mmol) were added. The reaction solution was purged with nitrogen three times, stirred for 18 hours at 80 °C under a nitrogen atmosphere. After the reaction was completed, water (50 mL) was added to the reaction solution, and extracted with ethyl acetate (50 mL×2). The combined organic phase was dried over anhydrous sodium sulfate, and filtered to remove the desiccant, and the filtrate was concentrated under reduced pressure. The residue was purified by silica gel column chromatography (dichloromethane/methanol = 100/1 to 20/1) to obtain compound **252-4.** MS m/z(ESI):469.2[M+1]+.

**Step 4):** Compound **252-4** (80 mg, 0.17 mmol) was dissolved in dichloromethane (4 mL), and trifluoroacetic acid (1 mL) was added. The reaction solution was stirred at 25 °C for 2 hours. The reaction solution was concentrated under reduced pressure after completed, and the residue was purified by high performance liquid chromatography (Gilson _306_1741, chromatographic column: Waters-SunFire-C18-10µm-19×250mm; mobile phase: water (containing 10 mmol/L formic acid) and acetonitrile, gradient ratio: acetonitrile 46%-95%, flow rate: 25 mL/min) to obtain compound **252.** MS m/z(ESI):425.2[M+1]+.

¹H NMR (400 MHz, DMSO-*d₆*) δ 9.80 (s, 1H), 8.70 - 8.40 (m, 1H), 8.00 (s, 1H), 7.76 - 7.69 (m, 1H), 7.65 - 7.57 (m, 1H), 7.35 (d, *J =* 8.0 Hz, 1H), 7.16 (t, *J =* 7.9 Hz, 1H), 6.74 (d, *J =* 7.7 Hz, 1H), 4.79 - 4.53 (m, 1H), 4.19 - 4.05 (m, 1H), 3.93 - 3.84 (m, 2H), 3.76 - 3.63 (m, 2H), 3.62 - 3.46 (m, 2H), 3.42 - 3.30 (m, 1H), 2.01 (s, 3H).

### Example 23: Preparation of Compound 251

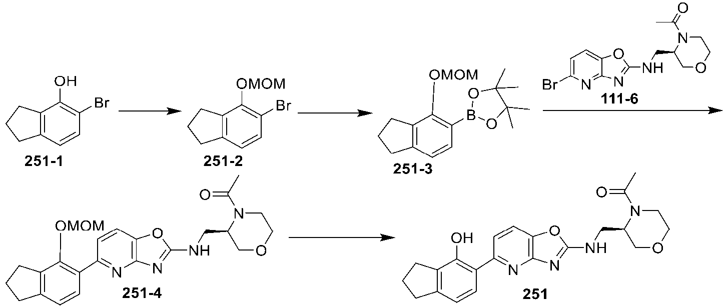

**Step 1):** Compound **251-1** (500 mg, 2.35 mmol, Bide) and chloromethyl methyl ether (0.23 mL, 3.05 mmol) were dissolved in dichloromethane (10 mL), and N, N-diisopropylethylamine (0.78 mL, 4.69 mmol) was added. The reaction solution was stirred at 25 °C for 18 hours, and water (50 mL) was added to the reaction solution, and extracted with ethyl acetate (50 mL×3), the organic phases were combined, and washed with saturated brine. The organic phase was dried over anhydrous sodium sulfate, and filtered to remove the desiccant, and the filtrate was concentrated under reduced pressure. The residue was purified by silica gel column chromatography with an eluent system (petroleum ether/ethyl acetate = 50/1 to 10/1) to obtain compound **251-2.**

**Step 2):** Compound **251-2** (300 mg, 1.17 mmol) was dissolved in anhydrous tetrahydrofuran, and n-butyl lithium (0.93 mL, 2.33 mmol, 2.5M hexane solution) was added at -78 °C. After the reaction solution was stirred at -78 °C for 1 hour, 2-isopropoxy-4,4,5,5-tetramethyl-1,3,2-dioxaborolane (0.38 mL, 1.87 mmol) was added and the reaction continued at -78 °C for 1 hour. Ice water (20 mL) was added to the reaction solution, and extracted with ethyl acetate (20 mL×3), the organic phases were combined, and washed with saturated brine, and the organic phase was dried over anhydrous sodium sulfate, and filtered to remove the desiccant, and the filtrate was concentrated under reduced pressure. The residue was purified by silica gel column chromatography with an eluent system (petroleum ether/ethyl acetate = 50/1 to 10/1) to obtain compound **251-3.**

¹H NMR (400 MHz, CDCl₃) δ 7.57 (d, *J=* 7.4 Hz, 1H), 7.01 (d, *J=* 7.4 Hz, 1H), 5.09 (s, 2H), 3.56 (s, 3H), 2.99 (t, *J=* 7.4 Hz, 2H), 2.93 (t, *J =* 7.5 Hz, 2H), 2.10 - 2.00 (m, 2H), 1.33 (s, 12H).

**Step 3):** Compound **251-3** (230 mg, 0.76 mmol) and compound **111-6** (188 mg, 0.53 mmol) were dissolved in dioxane (5 mL) and water (1 mL), 1,1'-bis(diphenylphosphino)ferrocene palladium(II) dichloride (55.3 mg, 0.08 mmol, adamas) and potassium carbonate (209 mg, 1.51 mmol) were added. The reaction solution was purged with nitrogen three times, and stirred for 18 hours at 80 °C under a nitrogen atmosphere. After the reaction was completed, water (50 mL) was added to the reaction solution, and extracted with ethyl acetate (50 mL×2). The combined organic phase was dried over anhydrous sodium sulfate, and filtered to remove the desiccant, and the filtrate was concentrated under reduced pressure. The residue was purified by silica gel column chromatography (dichloromethane/methanol = 100/1 to 20/1) to obtain compound **251-4.** MS m/z(ESI):453.6[M+1]+.

**Step 4):** Compound **251-4** (130 mg, 0.29 mmol) was dissolved in dichloromethane (2 mL), and trifluoroacetic acid (2 mL) was added. The reaction solution was stirred at 25 °C for 2 hours. The reaction solution was concentrated under reduced pressure after completed, and the residue was purified by high performance liquid chromatography (Gilson _306_1741, chromatographic column: Waters-SunFire-C18-10µm-19×250mm; mobile phase: water (containing 10 mmol/L formic acid) and acetonitrile, gradient ratio: acetonitrile 46%-95%, flow rate: 25 mL/min) to obtain compound **251.** MS m/z(ESI):409.2[M+1]+.

¹H NMR (400 MHz, DMSO-*d₆*) δ 13.56 (s, 1H), 8.84 - 8.41 (m, 1H), 7.88 - 7.78 (m, 1H), 7.70 - 7.64 (m, 2H), 6.78 (d, *J* = 8.0 Hz, 1H), 4.79 - 4.01 (m, 2H), 3.93 - 3.83 (m, 2H), 3.77 - 3.63 (m, 2H), 3.55 - 3.37 (m, 3H), 2.90 - 2.85(m, 4H), 2.12 - 1.88 (m, 5H).

### Example 24: Preparation of compound 255

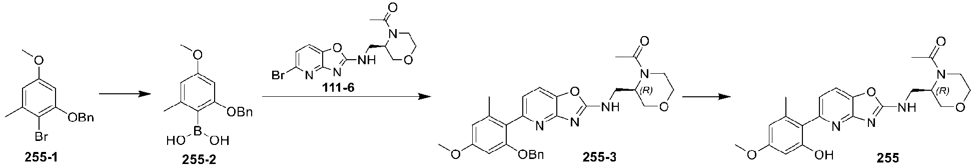

**Step 1):** Compound **255-1** (1.00 g, 3.26 mmol, prepared by the method disclosed in Journal of Agricultural and Food Chemistry (1995), 43(3), 804-8) was added to tetrahydrofuran (15 mL), and stirred evenly, the reaction solution was purged with nitrogen three times, and cooled to -70°C, and n-butyl lithium (1.60 mL, 3.91 mmol, 2.5 M tetrahydrofuran solution) was slowly added, and the temperature was controlled below -65°C. The reaction solution was stirred for 30 minutes, and tri-isopropyl borate (0.92 g, 4.88 mmol) was slowly added. After the addition was completed, the reaction solution was slowly heated to room temperature and stirred at room temperature for 18 hours. The reaction solution was slowly added to ice water (20 mL), extracted with ethyl acetate (8 mL×3), and the organic phase was dried with saturated brine and anhydrous sodium sulfate, and the solvent was removed under reduced pressure to obtain compound **255-2,** the residue was directly used in the next step without purification. MS m/z (ESI): 273.1 [M+1]+.

**Step 2):** Compound **255-2** (500 mg, 0.92 mmol) and compound **111-6** (100 mg, 0.28 mmol) were dissolved in dioxane (5 mL) and water (1 mL), and 1,1'-bis(diphenylphosphino)ferrocene palladium(II) dichloride (67.2 mg, 0.09 mmol, adamas) and potassium carbonate (254 mg, 1.84 mmol) were added. The reaction solution was purged with nitrogen three times, stirred for 18 hours at 80 °C under a nitrogen atmosphere. After the reaction was completed, water (50 mL) was added to the reaction solution, and extracted with ethyl acetate (50 mL×2). The organic phases were combined, dried over anhydrous sodium sulfate, and filtered to remove the desiccant, and the filtrate was concentrated under reduced pressure. The residue was purified by silica gel column chromatography (dichloromethane/methanol = 100/1 to 20/1) to obtain compound **255-3.** MS m/z (ESI):503.8[M+1]+.

**Step 3):** Compound **255-3** (160 mg, 0.22 mmol) was dissolved in methanol (15 mL) and 10% wet palladium carbon (20 mg, adamas) was added. The reaction solution was stirred for 18 hours under a hydrogen atmosphere. After the reaction was completed, the reaction solution was filtered to remove the palladium carbon, and the filtrate was concentrated under reduced pressure, and the residue was purified by high performance liquid chromatography (Gilson_306_1741, chromatographic column: Waters-SunFire-C18-10µm-19×250mm; mobile phase: water (containing 10 mmol/L formic acid) and acetonitrile, gradient ratio: acetonitrile 46%-95%, flow rate: 25 mL/min) to obtain compound **255.** MS m/z (ESI):413.3[M+1]+.

¹H NMR (400 MHz, DMSO-*d₆*) δ 9.59 - 9.40 (m, 1H), 8.72 - 8.36 (m, 1H), 7.77 - 7.59 (m, 1H), 6.97 - 6.83 (m, 1H), 6.32 (s, 2H), 4.67 - 4.08 (m, 1H), 4.07 - 3.80 (m, 3H), 3.72 (s, 3H), 3.69 - 3.57 (m, 2H), 3.56 - 3.43 (m, 2H), 3.20 - 2.87 (m, 1H), 2.07 - 1.92 (m, 6H).

### Example 25: Preparation of compound 257

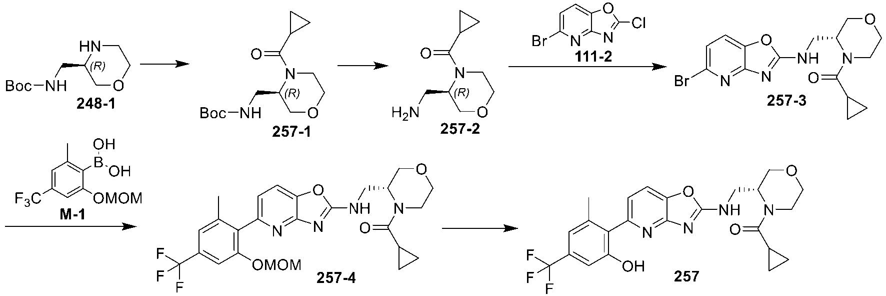

**Step 1):** Cyclopropylcarbonyl chloride (126 µL, 1.39 mmol) was added to a solution containing compound **248-1** (150 mg, 0.69 mmol, Bid), triethylamine (192 µL, 1.39 mmol) and dichloromethane (1 mL) at 0°C. The reaction solution was stirred at 25°C and allowed to react for 0.5 hours, then water (20 mL) and ethyl acetate (20 mL×2) were added for extraction. The organic phases were combined, washed with saturated brine, and the organic phase was dried over anhydrous sodium sulfate, and filtered to remove the desiccant, and the filtrate was concentrated under reduced pressure to obtain compound **257-1.** MS m/z(ESI):307.2[M+23]+.

**Step 2):** Compound **257-1** (190 mg, 0.67 mmol) was dissolved in dichloromethane (2 mL), trifluoroacetic acid (1 mL) was added, the reaction solution was stirred at 25°C and allowed to react for 1 hour, and the reaction solution was concentrated under reduced pressure to obtain compound **257-2.** MS m/z (ESI): 185.0 [M+1]+.

**Step 3):** Potassium carbonate (277 mg, 2.00 mmol) was added to a solution of compound **111-2** (156 mg, 0.67 mmol), compound **257-2** (123 mg, 0.67 mmol), and acetonitrile (3 mL). The reaction solution was stirred at 25°C for 18 hours. Then water (20 mL) was added, and extracted with ethyl acetate (20 mL×3), the organic phases were combined, washed with saturated brine, dried over anhydrous sodium sulfate, and filtered to remove the desiccant, and the filtrate was concentrated under reduced pressure. The residue was purified by silica gel column chromatography with an eluent system (dichloromethane/methanol = 40/1 to 15/1) to obtain compound **257-3.** MS m/z (ESI): 383.0 [M+1]+.

**Step 4):** 1,1'-bis(diphenylphosphino)ferrocene palladium(II) dichloride (28.8 mg, 0.04 mmol) was added to a solution of compound **257-3** (150 mg, 0.39 mmol), intermediate **M-1** (156 mg, 0.59 mmol), potassium carbonate (109 mg, 0.79 mmol), water (0.5 mL) and dioxane (2.5 mL). The reaction solution was purged with nitrogen three times, and stirred at 80°C for 2 hours. Water (20 mL) was added to the reaction solution, and extracted with ethyl acetate (20 mL×3). The organic phases were combined, washed with saturated brine, and the organic phase was dried over anhydrous sodium sulfate, and filtered to remove the desiccant, the filtrate was concentrated under reduced pressure. The residue was purified by silica gel column chromatography with an eluent system (dichloromethane/methanol = 40/1 to 15/1) to obtain compound **257-4.** MS m/z (ESI): 521.6 [M+1]+.

**Step 5):** Trifluoroacetic acid (1 mL) was added to a solution of compound **257-4** (100 mg, 0.19 mmol) and dichloromethane (3 mL). The reaction solution was stirred at 25°C for 18 hours. The reaction solution was concentrated under reduced pressure and purified by high performance liquid chromatography (Waters-2545, chromatographic column: Waters-SunFire-C18-10µm-19×250mm; mobile phase: water (containing 10 mmol/L formic acid) and acetonitrile, gradient ratio: acetonitrile 42%-52%, flow rate: 25 mL/min) to obtain compound **257.** MS m/z (ESI): 477.0 [M+1]+.

¹H NMR (400 MHz, DMSO-*d₆*) δ 10.12 - 9.87 (m, 1H), 8.89 - 8.54 (m, 1H), 7.84 - 7.70 (m, 1H), 7.16 - 7.01 (m, 2H), 6.99 - 6.89 (m, 1H), 4.70 - 4.46 (m, 1H), 4.13 - 3.85 (m, 3H), 3.67 - 3.59 (m, 2H), 3.47 - 3.30 (m, 2H), 3.18 - 2.94 (m, 1H), 2.15 - 2.01 (m, 3H), 2.00 - 1.81 (m, 1H), 0.77 - 0.29 (m, 4H).

### Example 26: Preparation of compound 258

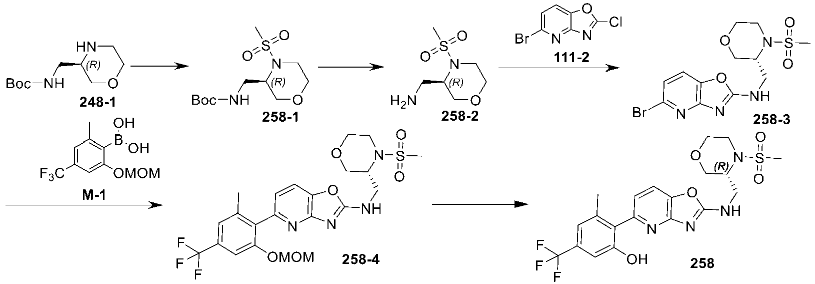

**Step 1):** At room temperature, compound **248-1** (80.0 mg, 0.37 mmol, Leyan) and triethylamine (110 mg, 1.09 mmol) were added to dichloromethane (2 mL) and stirred evenly. The reaction solution was cooled to 0°C in an ice bath, and methylsulfonyl chloride (64.0 mg, 0.56 mmol) was slowly added, and the temperature was slowly raised to room temperature, and the reaction was carried out for 2 hours. Water (10 mL) was added to the reaction solution, and extracted with dichloromethane (5 mL×3), the organic phase was dried with saturated brine and anhydrous sodium sulfate, and the solvent was removed under reduced pressure to obtain compound **258-1,** the residue was directly used in the next step without purification. MS m/z (ESI): 195.0 [M-100]+.

**Step 2):** At room temperature, compound **258-1** (120 mg, 0.41 mmol) was added to dichloromethane (1 mL) and stirred evenly, trifluoroacetic acid (1 mL) was slowly added, and the reaction was carried out at room temperature for 1 hour. The solvent was removed under reduced pressure to obtain compound **258-2,** the residue was directly used in the next step without purification. MS m/z (ESI): 194.9 [M+1]⁺.

**Step 3):** At room temperature, compound **258-2** (119 mg, 0.52 mmol), compound **111-2** (100 mg, 0.43 mmol) and potassium carbonate (178 mg, 1.29 mmol) were added to acetonitrile (3 mL), stirred evenly, and the reaction solution was heated to 70 °C and allowed to react for 2 hours. The reaction solution was cooled to room temperature, and filtered, and the filter cake was washed with dichloromethane (5 mL×3), and the solvent was removed under reduced pressure. The residue was purified by silica gel column chromatography (petroleum ether/ethyl acetate = 100/1 to 1/1) to obtain compound **258-3.** MS m/z (ESI): 392.6 [M+1]+.

**Step 4):** At room temperature, compound **258-3** (110 mg, 0.28 mmol), intermediate **M-1** (124 mg, 0.47 mmol), potassium carbonate (118 mg, 0.85 mmol) and 1,1'-bis(diphenylphosphino)ferrocene palladium(II) dichloride (41.0 mg, 0.06 mmol) were added to dioxane (3 mL) and water (0.3 mL) and stirred evenly. The reaction solution was purged with nitrogen three times, heated to 80°C and allowed to react for 2 hours. The reaction solution was cooled to room temperature, then the reaction solution was added to water (20 mL), and extracted with ethyl acetate (6 mL×3), and the organic phase was dried with saturated brine and anhydrous sodium sulfate, the solvent was removed under reduced pressure. The residue was purified by silica gel column chromatography (petroleum ether/ethyl acetate = 100/1 to 1/1) to obtain compound **258-4.** MS m/z (ESI): 531.2 [M+1]+.

**Step 5):** At room temperature, compound **258-4** (50.0 mg, 0.09 mmol) was added to dichloromethane (1 mL) and stirred evenly, trifluoroacetic acid (1 mL) was slowly added, and allowed to react at room temperature for 1 hour. The solvent was removed under reduced pressure, and the residue was purified by high performance liquid chromatography (Gilson _306_1741, chromatographic column: Waters-SunFire-C18-10µm-19×250mm; mobile phase: water (containing 0.1% formic acid) and acetonitrile, gradient ratio: acetonitrile 33%-67%, flow rate: 25mL/min) to obtain compound **258.** MS m/z(ESI):487.2[M+1]+.

¹H NMR (400 MHz, DMSO-*d₆*) δ 9.98 (s, 1H), 8.71 (s, 1H), 7.77 (d, J = 8.0 Hz, 1H), 7.09 (s, 1H), 7.05 (s, 1H), 6.94 (d, J = 8.1 Hz, 1H), 3.99 - 3.97 (m, 1H), 3.86 - 3.78 (m, 2H), 3.71 - 3.65 (m, 2H), 3.60 - 3.57 (m, 1H), 3.45 - 3.40 (m, 3H), 3.04 (s, 3H), 2.08 (s, 3H).

### Example 27: Preparation of compound 260

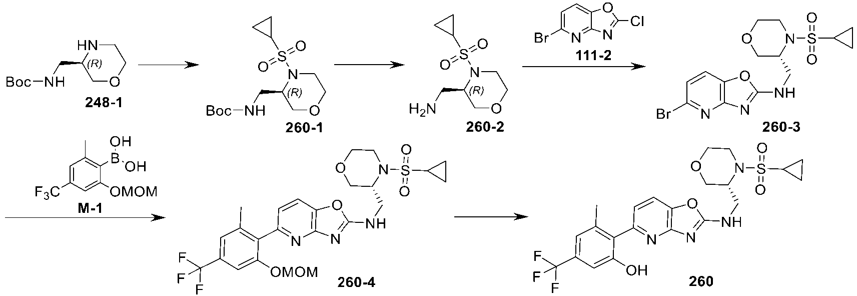

**Step 1):** At room temperature, compound **248-1** (100 mg, 0.46 mmol, Bid) and triethylamine (0.200 mL, 1.39 mmol) were added to dichloromethane (2 mL) and stirred evenly. The mixture was cooled to 0°C in an ice bath, and cyclopropanesulfonyl chloride (0.10 mL, 0.92 mmol, Bid) was slowly added. After the addition, the mixture was slowly heated to room temperature and allowed to react for 18 hours. Water (10 mL) was added to the reaction solution, and extracted with dichloromethane (5 mL×3). The organic phase was dried with saturated brine and anhydrous sodium sulfate, and the solvent was removed under reduced pressure to obtain compound **260-1,** the residue was directly used in the next step without purification. MS m/z (ESI): 221.0 [M-100]+.

**Step 2):** At room temperature, compound **260-1** (130 mg, 0.41 mmol) was added to dichloromethane (1 mL) and stirred evenly, then trifluoroacetic acid (1 mL) was added slowly, and allowed to react at room temperature for 1 hour. The solvent was removed under reduced pressure to obtain compound **260-2,** the residue was directly used in the next step without purification. MS m/z (ESI): 221.0 [M + 1]+.

**Step 3):** At room temperature, compound **111-2** (75 mg, 0.32 mmol), compound **260-2** (85.0 mg, 0.39 mmol) and potassium carbonate (133 mg, 0.96 mmol) were added to acetonitrile (3 mL), stirred evenly, then the reaction solution was heated to 70 °C, and allowed to react for 2 hours. The reaction solution was cooled and filtered, the filter cake was washed with dichloromethane (5mL×3), and the solvent was removed under reduced pressure to obtain compound **260-3,** the residue was directly used in the next step without purification. MS m/z (ESI):419.0[M+1]+.

**Step 4):** At room temperature, compound **260-3** (60.0 mg, 0.14 mmol), intermediate **M-1** (62 mg, 0.23 mmol), potassium carbonate (60 mg, 0.43 mmol) and 1,1'-bis(diphenylphosphino)ferrocene palladium(II) dichloride (21 mg, 0.03 mmol) were added to dioxane (2 mL) and water (0.2 mL) and stirred evenly, the reaction solution was purged with nitrogen three times, and heated to 80°C for 2 hours. The reaction solution was cooled to room temperature, then added to water (20 mL), extracted with ethyl acetate (6 mL×3), and the organic phase was dried with saturated brine and anhydrous sodium sulfate, and the solvent was removed under reduced pressure. The residue was purified by silica gel column chromatography (petroleum ether/ethyl acetate = 100/1 to 1/1) to obtain compound **260-4.** MS m/z (ESI): 555.2[M-1]-.

**Step 5):** At room temperature, compound **260-4** (50 mg, 0.09 mmol) was added to dichloromethane (1 mL) and stirred evenly, trifluoroacetic acid (1 mL) was slowly added, and allowed to react at room temperature for 1 hour. The solvent was removed under reduced pressure, and the residue was purified by high-performance liquid chromatography (Gilson _306_1741, chromatographic column: Waters-SunFire-C18-10µm-19×250mm; mobile phase: water (containing 0.1% formic acid) and acetonitrile, gradient ratio: acetonitrile 35%-65%, flow rate: 25mL/min) to obtain compound **260.** MS m/z(ESI):513.0[M+1]+.

¹H NMR (400 MHz, DMSO-*d₆*) δ 9.96 (s, 1H), 8.68 (s, 1H), 7.75 (d, J = 8.0 Hz, 1H), 7.09 (s, 1H), 7.05 (s, 1H), 6.93 (d, J = 8.0 Hz, 1H), 4.02 - 3.95 (m, 1H), 3.88 - 3.81 (m, 2H), 3.74 - 3.66 (m, 2H), 3.64 - 3.60 (m, 1H), 3.49 - 3.47 (m, 3H), 2.76 - 2.73 (m, 1H), 2.07 (s, 3H), 1.00 - 0.90 (m, 4H).

### Example 28: Preparation of compound 259

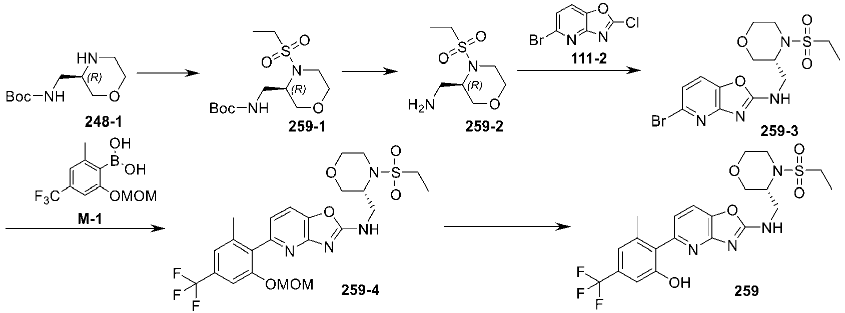

**Step 1):** Ethylsulfonyl chloride (44.0 µL, 0.46 mmol, Leyan) was added to a solution containing compound **248-1** (50.0 mg, 0.23 mmol, Bid), triethylamine (64.0 µL, 0.46 mmol) and dichloromethane (1 mL) at 0°C. The reaction solution was stirred at 25°C for 0.5 hours, then water (20 mL) was added, and extracted with ethyl acetate (20 mL×2), the organic phases were combined, washed with saturated brine, dried over anhydrous sodium sulfate, and filtered to remove the desiccant, and the filtrate was concentrated under reduced pressure to obtain compound **259-1.** MS m/z(ESI):331.0[M+23]+.

**Step 2):** Compound **259-1** (70 mg, 0.23 mmol) was dissolved in dichloromethane (2 mL), trifluoroacetic acid (0.5 mL) was added, stirred at 25°C for 1 hour, and the reaction solution was concentrated under reduced pressure to obtain compound **259-2.** MS m/z (ESI): 209.0 [M+1] +.

**Step 3):** Potassium carbonate (145 mg, 1.05 mmol) was added to a solution of compound **111-2** (82.0 mg, 0.35 mmol), compound **259-2** (73.0 mg, 0.35 mmol), and acetonitrile (5 mL). The reaction solution was stirred at 25°C for 18 hours. Then water (20 mL) was added, and extracted with ethyl acetate (20 mL×3). The organic phases were combined, washed with saturated brine, dried over anhydrous sodium sulfate, and filtered to remove the desiccant, the filtrate was concentrated under reduced pressure. The residue was purified by silica gel column chromatography with an eluent system (dichloromethane/methanol = 40/1 to 15/1) to obtain compound **259-3.** MS m/z (ESI): 407.0 [M+1]+.

**Step 4):** 1,1'-bis(diphenylphosphino)ferrocene palladium(II) dichloride (18.0 mg, 0.02 mmol) was added to a solution of compound **259-3** (100 mg, 0.25 mmol), intermediate **M-1** (130 mg, 0.49 mmol), potassium carbonate (68.0 mg, 0.49 mmol), water (0.5 mL) and dioxane (2.5 mL). The reaction solution was purged with nitrogen three times, and stirred at 80°C for 2 hours. Water (20 mL) was added to the reaction solution, and extracted with ethyl acetate (20 mL×3). The organic phases were combined, washed with saturated brine, dried over anhydrous sodium sulfate, and filtered to remove the desiccant, and the filtrate was concentrated under reduced pressure. The residue was purified by silica gel column chromatography with an eluent system (dichloromethane/methanol = 40/1 to 15/1) to obtain compound **259-4.** MS m/z(ESI):545.7[M+1]+.

**Step 5):** Trifluoroacetic acid (1 mL) was added to a solution of compound **259-4** (80.0 mg, 0.15 mmol) and dichloromethane (3 mL). The reaction solution was stirred at 25°C for 18 hours. The reaction solution was concentrated under reduced pressure and purified by high performance liquid chromatography (Waters-2545, column: Waters-SunFire-C18-10µm-19*250mm; mobile phase: water (containing 10 mmol/L formic acid) and acetonitrile, gradient ratio: acetonitrile 45%-55%, flow rate: 25 mL/min) to obtain compound **259.** MS m/z(ESI):501.0[M+1]+.

¹H NMR (400 MHz, DMSO-*d₆*) δ 9.99 (s, 1H), 8.72 (s, 1H), 7.78 (d, J = 8.0 Hz, 1H), 7.09 (s, 1H), 7.05 (s, 1H), 6.95 (d, J = 8.0 Hz, 1H), 3.98 - 3.91 (m, 1H), 3.89 - 3.80 (m, 2H), 3.77 - 3.64 (m, 3H), 3.58 - 3.53 (m, 3H), 3.19 - 3.10 (m, 2H), 2.08 (s, 3H), 1.16 (t, J = 7.2 Hz, 3H).

### Example 29: Preparation of compound 420

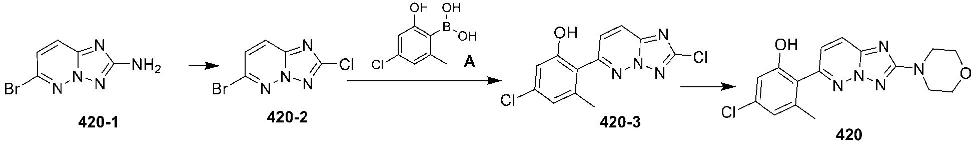

**Step 1):** Compound **420-1** (1.00 g, 4.67 mmol, prepared by the method of intermediate 33 preparation disclosed on page 84 of the specification of "WO2014198594A1") and cuprous chloride (0.320 g, 3.27 mmol, Bid) were dissolved in concentrated hydrochloric acid (12 mL), then sodium nitrite (0.520 g, 7.48 mmol, Bid) in water (1 mL) was added at 5°C and stirred at 5°C for 1 hour, then the reaction solution was slowly poured into a saturated aqueous sodium bicarbonate solution (50 mL), and the mixture was diluted with dichloromethane (20 mL). The aqueous phase was separated, and extracted with dichloromethane (30 mL×3), the organic phases were combined, dried over anhydrous sodium sulfate, and filtered to remove the desiccant, and the filtrate was concentrated under reduced pressure. The residue was purified by silica gel column chromatography with an eluent system (dichloromethane/methanol = 100/1 to 40/1) to obtain compound **420-2.** MS m/z (ESI): 232.9, 234.9 [M+H]+.

¹H NMR (400 MHz, DMSO-*d*₆): δ 8.41 (d, J *=* 9.5 Hz, 1H), 8.01 (d, J = 9.5 Hz, 1H).

**Step 2):** Compound **420-2** (100 mg, 0.430 mmol), compound A (104 mg, 0.560 mmol), potassium carbonate (178 mg, 1.29 mmol) and 1,1-bis(diphenylphosphino)ferrocene palladium(II) dichloride(31.5 mg, 40 µmol, Bid) were dissolved in dioxane (2.5 mL) and water (0.5 mL). The reaction solution was heated to 80°C under a nitrogen atmosphere and allowed to react for 2 hours. The reaction solution was diluted with dichloromethane (5 mL) and water (5 mL), and the aqueous phase was separated, and extracted with dichloromethane (5 mL×3). The organic phases was combined, dried over anhydrous sodium sulfate, and filtered to remove the desiccant, and the filtrate was concentrated under reduced pressure. The residue was purified by silica gel column chromatography with an eluent system (dichloromethane/methanol = 100/1 to 40/1) to obtain compound **420-3.** MS m/z(ESI):294.9[M+H]+.

**Step 3):** Compound **420-3** (100 mg, 0.430 mmol) was dissolved in morpholine (1 mL). The mixture was heated to 200°C by microwave and allowed to react at this temperature for 30 minutes. The reaction solution was poured into water (5 mL). The aqueous phase was extracted with dichloromethane (5 mL×3). The organic phases were combined, washed with saturated brine (5 mL), dried over anhydrous sodium sulfate, and filtered to remove the desiccant, and the filtrate was concentrated under reduced pressure. The residue was purified by high performance liquid chromatography (chromatographic column: Waters-SunFire-C18-10µm-19×250mm; mobile phase: water (containing 0.1% formic acid) and acetonitrile, gradient ratio: acetonitrile 32%-48%, flow rate: 25 mL/min) to obtain compound **420.** MS m/z(ESI):346.1[M+H]+.

¹H NMR (400 MHz, DMSO-*d*₆): δ 10.22 (s, 1H), 8.07 (d, J = 9.2 Hz, 1H), 7.46 (d, J = 9.2 Hz, 1H), 6.87 (d, J = 13.3 Hz, 2H), 3.77 - 3.67 (m, 4H), 3.55 - 3.48 (m, 4H), 2.07 (s, 3H).

### Example 30: Preparation of compound 419

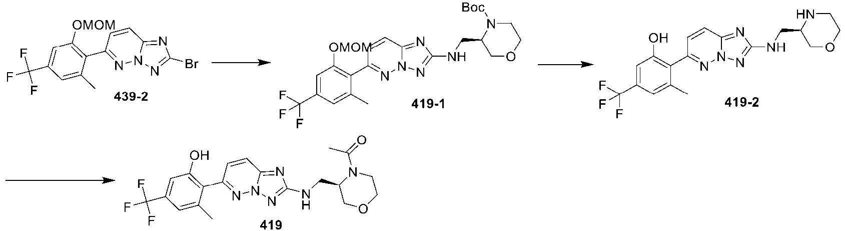

**Step 1):** Compound **439-2** (150 mg, 360 µmol) was dissolved in dioxane (6 mL), and (R)-4-Boc-3-morpholinemethylamine (156 mg, 720 µmol, Leyan), 4,5-bis(diphenylphosphino)-9,9-dimethyloxanthene (41.6 mg, 72.0 µmol), sodium tert-butoxide (69.1 mg, 720 µmol) and tris(dibenzylideneacetone)dipalladium (65.9 mg, 72.0 µmol) were added, and the reaction solution was purged with nitrogen three times, and stirred at 100°C for 2 hours, then the reaction solution was poured into water (20 mL), and extracted with ethyl acetate (20 mL×3), and the organic phases were combined, dried over anhydrous sodium sulfate, and filtered, and the filtrate was concentrated under reduced pressure. The residue was purified by silica gel column chromatography with an eluent system (dichloromethane/methanol = 100/1 to 20/1) to obtain compound **419-1.** MS m/z (ESI): 453.6 [M-Boc+1]+.

**Step 2):** Compound **419-1** (100 mg, 181 µmol) was dissolved in dichloromethane (2 mL), and the dioxane solution of hydrogen chloride (4 mL, 4 M) was added. The reaction solution was stirred at room temperature for 1 hour, then the reaction solution was directly concentrated under reduced pressure to obtain compound **419-2.** The residue was directly used as the raw material for the next step without purification. MS m/z (ESI): 409.3 [M+1]+.

**Step 3):** Compound **419-2** (60 mg, 147 µmol) was dissolved in dichloromethane (5 mL), triethylamine (44.6 mg, 441 µmol) and acetic anhydride (15.0 mg, 147 µmol) were added at 0°C. The reaction solution was stirred at 0°C for 30 minutes, and the reaction solution was mixed with sodium bicarbonate aqueous solution (10 mL), and extracted with dichloromethane (20 mL×3), and the organic phases were combined, dried over anhydrous sodium sulfate, and filtered, and the filtrate was concentrated under reduced pressure. The residue was purified by high performance liquid chromatography (Waters-2545, chromatographic column: SharpSil-TC18, 30×150 mm, 5 µm; mobile phase: water (containing 10 mmol/L formic acid) and acetonitrile, gradient ratio: acetonitrile 38%-45%, flow rate: 30 mL/min) to obtain compound **419.** MS m/z(ESI):451.2[M+1]+.

¹H NMR (400 MHz, DMSO-*d*₆): δ 10.38 (s, 1H), 8.08 - 7.90 (m, 1H), 7.52 - 7.38 (m, 1H), 7.33 - 6.86 (m, 3H), 4.75 - 3.75 (m, 4H), 3.70 - 3.35 (m, 4H), 3.30 - 2.93 (m, 1H), 2.20 - 2.09 (m, 3H), 2.05 - 1.89 (m, 3H).

### Example 31: Preparation of compound 425

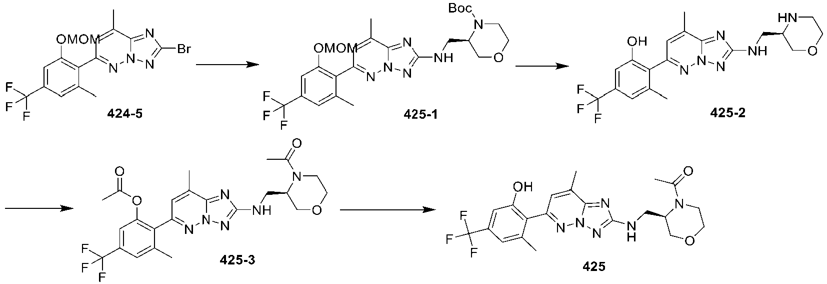

**Step 1):** At room temperature, compound **424-5** (50 mg, 120 µmol), (R)-4-Boc-3-morpholinemethylamine (26 mg, 120 µmol, Leyan), tris(dibenzylideneacetone)dipalladium (11 mg, 12.0 µmol), sodium tert-butoxide (34 mg, 360 µmol) and 4,5-bis(diphenylphosphino)-9,9-dimethylxanthene (7.0 mg, 12.0 µmol) were added to dioxane (2 mL) and stirred evenly. The reaction solution was purged with nitrogen three times and heated to 80°C for 3 hours. The reaction solution was diluted with water (20 mL) and extracted with ethyl acetate (6 mL×3). The organic phases were combined, washed with brine (20 mL), dried over anhydrous sodium sulfate, and filtered, and the filtrate was concentrated under reduced pressure. The residue was purified by silica gel column chromatography (petroleum ether/ethyl acetate = 100/1 to 1/1) to obtain compound **425-1.** MS m/z(ESI):467.3[M-Boc+1]+.

**Step 2):** Trifluoroacetic acid (1 mL) was added to a solution containing compound **425-1** (50 mg, 180 µmol) and dichloromethane (2 mL) and stirred at 25°C for 1 hour. The reaction solution was concentrated under reduced pressure to obtain compound **425-2,** which was used directly in the next step without purification. MS m/z(ESI):423.4[M+1]+.

**Step 3):** Compound **425-2** (30 mg, 71 µmol), triethylamine (8.0 mg, 79 µmol) and acetic anhydride (7.0 mg, 69 µmol) were dissolved in dichloromethane (2 mL) and stirred at 25°C for 2 hours, and the reaction solution was mixed with dichloromethane (10 mL) and water (5 mL). The aqueous phase was separated, and extracted with dichloromethane (5 mL×3). The organic phases were combined, dried over anhydrous sodium sulfate, and filtered, and the filtrate was concentrated under reduced pressure. The residue was purified by silica gel column chromatography with an eluent system (dichloromethane/methanol = 100/1 to 30/1) to obtain compound **425-3.** MS m/z (ESI): 507.3 [M+1]+.

**Step 4):** Compound **425-3** (40 mg, 79 µmol) and potassium carbonate (17 mg, 180 µmol) were dissolved in methanol (2 mL), and the resulting mixture was heated to 60°C and stirred for 2 hours. The reaction solution was mixed with dichloromethane (10 mL) and water (5 mL). The aqueous phase was separated, and extracted with dichloromethane (5 mL×3). The organic phases were combined, dried over anhydrous sodium sulfate, and filtered, and the filtrate was concentrated under reduced pressure. The residue was purified by high performance liquid chromatography (A: 0.1% formic acid/water, B: acetonitrile, chromatographic column: Waters-SunFire-C18-10µm-19×250mm; mobile phase: water (containing 0.1% formic acid) and acetonitrile, gradient ratio: acetonitrile 32%-48%, flow rate: 25 mL/min) to obtain compound **425.** MS m/z(ESI):465.3[M+1]+.

¹H NMR (400 MHz, DMSO-*d₆*) δ 10.44 (s, 1H), 7.30 - 7.22 (m, 2H), 7.15 - 7.11 (m, 1H), 6.89 - 6.56 (m, 1H), 5.37 - 5.30 (m, 1H), 4.60 - 4.00 (m, 2H), 3.89 - 3.81 (m, 2H), 3.58 - 3.51 (m, 2H), 3.18 - 2.87 (m, 1H), 2.82 - 2.59 (m, 1H), 2.13 - 1.97 (m, 8H), 1.48 - 1.44 (m, 1H).

### Example 32: Preparation of compound 451

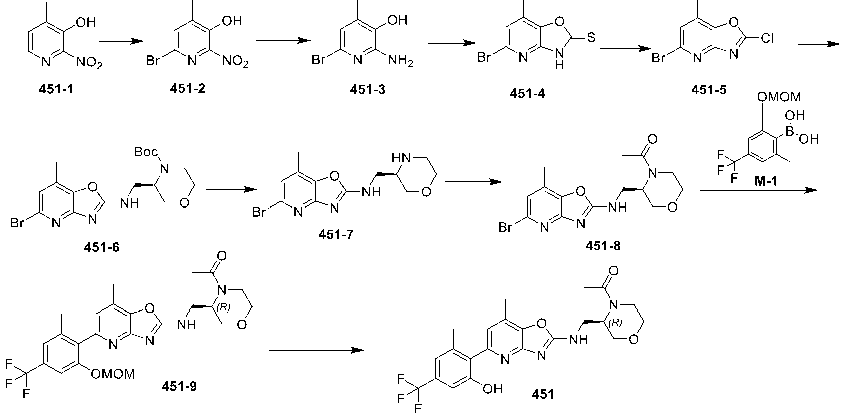

**Step 1):** Sodium methoxide in methanol solution (12 mL, 30% methanol solution) was added to compound 451-1 (10.0 g, 64.8 mmol, Anaiji) in methanol (220 mL), and the mixture was stirred for 30 minutes under a nitrogen atmosphere at 25 °C. The mixture was cooled to 0 °C, and liquid bromine (3.33 mL, 64.8 mmol) was added. The reaction solution was stirred for 2 hours under a nitrogen atmosphere at 0 °C. The reaction solution was concentrated under reduced pressure, the residue was mixed with water (100 mL), stirred, and filtered, and the filter cake was collected and vacuum dried to obtain compound **451-2.** MS m/z (ESI): 233.0 [M+1]+.

**Step 2):** Raney nickel (3.00 g) was added to compound **451-2** (6.00 g, 25.7 mmol) in methanol (60 mL), and the mixture was stirred for 3 hours at 25 °C under hydrogen (1 atmosphere) atmosphere, and filtered, and the filtrate was concentrated to obtain compound **451-3.** MS m/z (ESI): 204.9 [M+1]⁺.

**Step 3):** Potassium hydroxide (330 mg, 5.91 mmol) and carbon disulfide (7 mL) were added to compound **451-3** (1.00 g, 4.93 mmol) in ethanol (10 mL), and the mixture was stirred at 80 °C under a nitrogen atmosphere for 24 hours. The mixture was concentrated under reduced pressure, and the residue was purified by silica gel chromatography (dichloromethane/methanol = 100/1 to 10/1) to obtain compound **451-4.** MS m/z (ESI): 245.0 [M+1]⁺.

**Step 4):** Under a nitrogen atmosphere at 0°C, thionyl chloride (0.90 mL, 12.4 mmol) and *N, N-*dimethylformamide (0.80 mL, 10.3 mmol) were added to compound **451-4** (1.00 g, 4.08 mmol) in dichloromethane (15 mL), and the mixture was stirred at 25°C under a nitrogen atmosphere for 1 hour. The reaction solution was diluted with ethyl acetate (50 mL) and poured into water (20 mL), and the organic phase was washed with brine (20 mL×2), dried over anhydrous sodium sulfate, and filtered, and the filtrate was concentrated under reduced pressure to obtain compound **451-5.** MS m/z (ESI): 247.0 [M+1]+.

**Step 5):** Compound (R)-4-Boc-3-morpholinemethylamine (577 mg, 2.67 mmol, Leyan) and compound **451-5** (440 mg, 1.78 mmol) were dissolved in acetonitrile (5 mL), potassium carbonate (246 mg, 1.78 mmol) was added, and the mixture was stirred at 80°C for 2 hours. Water (30 mL) was added to the reaction solution, and extracted with dichloromethane (30 mL×3). The organic phases were combined and washed with saturated brine (30 mL). The organic phase was dried over anhydrous sodium sulfate, and filtered to remove the desiccant, and the filtrate was concentrated under reduced pressure. The residue was purified by silica gel column chromatography (dichloromethane/methanol = 40/1 to 15/1) to obtain compound **451-6.** MS m/z (ESI): 429.2 [M+1]+.

**Step 6):** Compound **451-6** (430 mg, 1.01 mmol) was dissolved in dioxane (4 mL), and the dioxane solution of hydrogen chloride (4 mL, 4M) was added. The reaction solution was stirred at 25°C for 3 hours, and the reaction solution was concentrated under reduced pressure to obtain compound **451-7.** MS m/z (ESI): 328.8 [M+1]+.

**Step 7):** Compound **451-7** (430 mg, 1.31 mmol) was dissolved in dichloromethane (10 mL), triethylamine (0.55 mL, 3.94 mmol) was added, and the mixture was stirred at 0°C for 10 minutes. Acetic anhydride (0.13 mL, 1.38 mmol) was added to the reaction solution, and the reaction continued at 25°C for 15 minutes. Water (50 mL) was added to the reaction solution, and extracted with dichloromethane (50 mL×3). The organic phases were combined and washed with saturated brine (50 mL), dried over anhydrous sodium sulfate, and filtered to remove the desiccant, and the filtrate was concentrated under reduced pressure. The residue was purified by silica gel column chromatography with an eluent system (dichloromethane/methanol = 40/1 to 15/1) to obtain compound **451-8.** MS m/z(ESI): 371.1 [M+1]+.

**Step 8):** Compound **451-8** (130 mg, 0.35 mmol) and intermediate **M-1** (167 mg, 0.63 mmol) were dissolved in dioxane (5 mL) and water (0.5 mL), and 1,1-bis(diphenylphosphino)ferrocene palladium(II) dichloride (25.7 mg, 0.035 mmol, adamas) and potassium carbonate (146 mg, 1.06 mmol) were added. The reaction was purged with nitrogen three times, and stirred at 80°C for 18 hours under a nitrogen atmosphere. After the reaction was completed, water (20 mL) was added to the reaction solution, and extracted with dichloromethane (20 mL×2). The organic phases were combined, dried over anhydrous sodium sulfate, and filtered to remove the desiccant, and the filtrate was concentrated under reduced pressure. The residue was purified by silica gel column chromatography (dichloromethane/methanol = 100/1 to 20/1) to obtain compound **451-9.** MS m/z (ESI): 509.4 [M+1]+.

**Step 9):** Compound **451-9** (150 mg, 0.29 mmol) was dissolved in the dioxane solution of hydrogen chloride (4 mL, 4M), and the reaction solution was stirred at 25°C for 2 hours. The reaction solution was concentrated under reduced pressure, and the residue was purified by high performance liquid chromatography (A: 0.1% formic acid/water, B: acetonitrile, chromatographic column: Waters-SunFire-C18-10µm-19×250mm; mobile phase: water (containing 0.1% formic acid) and acetonitrile, gradient ratio: acetonitrile 32%-48%, flow rate: 25 mL/min) to obtain compound **451.** MS m/z (ESI): 465.7 [M+1]+.

¹H NMR (400 MHz, DMSO-*d₆*) δ 9.94 (s, 1H), 8.75 - 8.41 (m, 1H), 7.06 (d, J = 11.5 Hz, 2H), 6.84 - 6.76 (m, 1H), 4.67 - 4.08 (m, 1H), 4.07 - 3.69 (m, 3H), 3.68 - 3.42 (m, 4H), 3.29 - 2.93 (m, 1H), 2.44 - 2.36 (m, 3H), 2.11 - 1.93 (m, 6H).

### Example 33: Preparation of compound 452

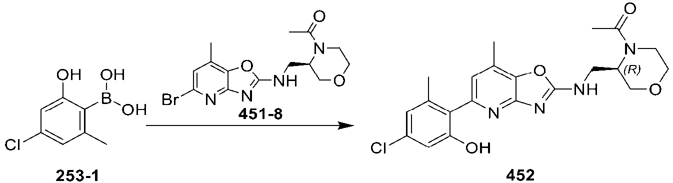

Compound **451-8** (130 mg, 0.352 mmol) and compound **253-1** (164 mg, 0.882 mmol, Bid) were dissolved in dioxane (5 mL) and water (1 mL), and 1,1-bis(diphenylphosphino)ferrocene palladium(II) dichloride (25.7 mg, 0.04 mmol, adamas) and potassium carbonate (195 mg, 1.41 mmol) were added. The reaction was purged with nitrogen three times, and stirred at 80°C for 18 hours in a nitrogen atmosphere. After the reaction was completed, water (20 mL) was added to the reaction solution, and extracted with dichloromethane (20 mL×2). The organic phases were combined, dried over anhydrous sodium sulfate, and filtered to remove the desiccant, and the filtrate was concentrated under reduced pressure. The residue was purified by HPLC (Gilson_306_1741, chromatographic column: Waters-SunFire-C18-10µm-19×250mm; mobile phase: water (containing 10 mmol/L formic acid) and acetonitrile, gradient ratio: acetonitrile 46%-95%, flow rate: 25 mL/min) to obtain compound **452.** MS m/z(ESI):431.3[M+1]+.

¹H NMR (400 MHz, DMSO-*d₆*) δ 9.79 - 9.70 (m, 1H), 8.72 - 8.36 (m, 1H), 6.83 - 6.72 (m, 3H), 4.67 - 4.08 (m, 1H), 4.07 - 3.68 (m, 3H), 3.67 - 3.50 (m, 3H), 3.47 - 3.35 (m, 1H), 3.30 - 2.94 (m, 1H), 2.43 - 2.36 (m, 3H), 2.08 - 1.93 (m, 6H).

### Example 34: Preparation of compound 453

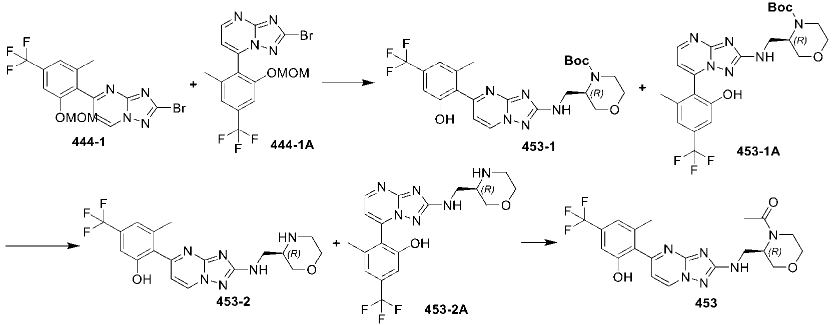

**Step 1):** At room temperature, the mixture of compound **444-1** and compound **444-1A** (200 mg, 479 µmol) was added to (R)-4-Boc-3-morpholinemethylamine (1.08 g, 5.0 mmol, Leyan), heated to 190°C by microwave, and allowed to react for 30 minutes. The reaction solution was cooled to room temperature, water (15 mL) was added, and extracted with dichloromethane/methanol (10/1, 5 mL×3), the organic phase was washed with saturated brine (10 mL), dried over anhydrous sodium sulfate, and filtered, and the filtrate was concentrated under reduced pressure to obtain a mixture of compound **453-1** and compound **453-1A.** MS m/z (ESI): 509.8 [M+1]+.

**Step 2):** At room temperature, the mixture of compound **453-1** and compound **453-1A** (240 mg, 434 µmol) was added to the dioxane solution of hydrogen chloride (4 mL, 4M), and allowed to react at room temperature for 1 hour. The solvent was removed under reduced pressure, then dichloromethane (15 mL) and water (5 mL) were added, and a saturated sodium bicarbonate solution (5 mL) was added and stirred. The reaction solution was left for separation, and the aqueous layer was extracted with dichloromethane (5 mL×3). The organic phases were combined, dried over anhydrous sodium sulfate, and filtered, and the filtrate was concentrated under reduced pressure. The residue was purified by silica gel column chromatography (methanol/dichloromethane = 1/100 to 1/10) to obtain a mixture of compound **453-2** and compound **453-2A.** MS m/z (ESI): 409.7 [M+1] +.

**Step 3):** At room temperature, the mixture of compound **453-2** and compound **453-2A** (150 mg, 367 µmol) was added to dichloromethane (3 mL), then triethylamine (148 mg, 1.47 mmol) was added, stirred evenly, and acetic anhydride (45.0 mg, 440 µmol) was slowly added, the mixture was allowed to react at room temperature for 3 hours. The solvent was removed under reduced pressure, dichloromethane (15 mL) and water (5 mL) were added, and aqueous sodium bicarbonate solution (5 mL) was added, stirred for 5 minutes, the reaction solution was left for separation. The aqueous phase was extracted with dichloromethane (5 mL×3). The organic phases were combined, dried over anhydrous sodium sulfate, and filtered, and the filtrate was concentrated under reduced pressure. The residue was purified by high performance liquid chromatography (Gilson_306_1741, chromatographic column: Waters-SunFire-C18-10µm-19×250mm; mobile phase: water (containing 10 mmol/L formic acid) and acetonitrile, gradient ratio: acetonitrile 30%-70%, flow rate: 25 mL/min) to obtain compound **453.** MS m/z(ESI):451.6[M+1]+.

¹H NMR (400 MHz, DMSO-*d₆*) δ 10.39 (s, 1H), 9.06 - 9.03 (m, 1H), 7.35 - 6.98 (m, 4H), 4.60 - 4.08 (m, 1H), 3.99 - 3.82 (m, 3H), 3.61 - 3.38 (m, 4H), 3.27 - 2.95 (m, 1H), 2.16 (s, 3H), 2.02 - 1.98 (m, 3H).

### Example 35: Preparation of compound 454

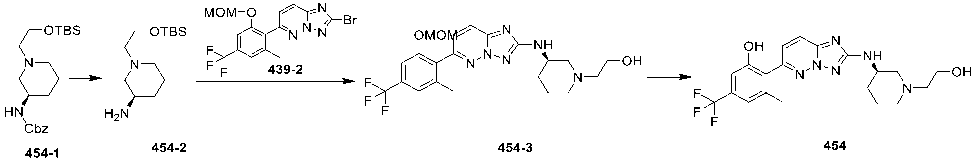

**Step 1):** Compound **454-1** (16.0 g, 40.7 mmol, prepared by the method disclosed in the third step: intermediate preparation on page 94 of the specification of "WO2023159148 A2") was dissolved in methanol (60 mL), 10% wet palladium carbon (500 mg) was added, and the mixture was stirred under a hydrogen (1 atmosphere) atmosphere for 18 hours, and filtered, and the filtrate was concentrated to obtain compound **454-2.** MS m/z (ESI): 259.6 [M+1] +.

**Step 2):** Compound **439-2** (100 mg, 0.241 mmol), compound **454-2** (93 mg, 0.361 mmol), tris(dibenzylideneacetone)dipalladium (21.9 mg, 0.025 mmol), 4,5-bis(diphenylphosphino)-9,9-dimethylxanthene (27.7 mg, 0.051 mmol) and sodium tert-butoxide (46.7 mg, 0.481 mmol, Bid) were mixed with dioxane (3 mL). The mixture was heated to 100 °C under a nitrogen atmosphere, allowed to react for 2 hours, and cooled, and the mixture was mixed with dichloromethane (5 mL) and water (5 mL). The reaction solution was left for separation, the aqueous phase was extracted with dichloromethane (5 mL×3), and the organic phases were combined, dried over anhydrous sodium sulfate, and filtered, and the filtrate was concentrated under reduced pressure, and the residue was purified by silica gel column chromatography (dichloromethane/methanol = 100/1 to 30/1) to obtain compound **454-3.** MS m/z (ESI): 481.3 [M+1] +.

**Step 3):** Compound **454-3** (100 mg, 0.21 mmol) was dissolved in the dioxane solution of hydrogen chloride (4 mL, 4M), stirred at 25°C for 2 hours. The reaction solution was concentrated under reduced pressure, and the residue was purified by high performance liquid chromatography (A: 0.1% formic acid/water, B: acetonitrile, chromatographic column: Waters-SunFire-C18-10µm-19×250mm; mobile phase: water (containing 0.1% formic acid) and acetonitrile, gradient ratio: acetonitrile 32%-48%, flow rate: 25 mL/min) to obtain compound **454.** MS m/z(ESI):437.7[M+1]+.

¹H NMR (400 MHz, DMSO-*d₆*) δ 8.18 (s, 1H), 8.00 (d, J = 9.1 Hz, 1H), 7.42 (d, J *=* 9.1 Hz, 1H), 7.17 (s, 1H), 7.11 (s, 1H), 6.87 (d, J = 8.3 Hz, 1H), 4.52 - 4.12 (m, 1H), 3.81 - 3.72 (m, 1H), 3.50 (t, J = 6.1 Hz, 2H), 3.03 - 2.95 (m, 1H), 2.76 - 2.65 (m, 1H), 2.44 (t, J = 6.1 Hz, 2H), 2.16 (s, 3H), 2.14 - 2.01 (m, 2H), 1.92 - 1.82 (m, 1H), 1.75 - 1.66 (m, 1H), 1.57 - 1.46 (m, 1H), 1.41 - 1.30 (m, 1H).

### Example 36: Preparation of compound 455

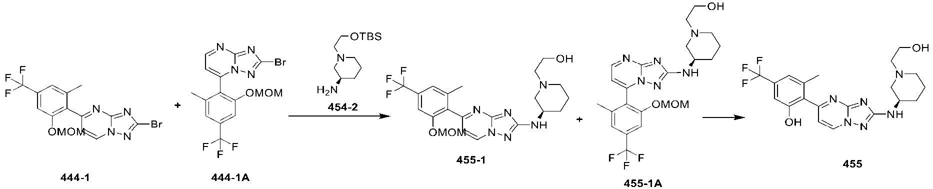

**Step 1):** At room temperature, the mixture of compound **444-1** and compound **444-1A** (100 mg, 240 µmol) was added to compound **454-2** (1.5 mL), heated to 190 °C by microwave, and allowed to react for 1 hour. After cooling to room temperature, water (15 mL) was added, and extracted with dichloromethane/methanol (10/1, 5 mL×3). The organic phase was washed with saturated brine (15 mL), dried over anhydrous sodium sulfate, and filtered, and the filtrate was concentrated under reduced pressure. The residue was purified by silica gel column chromatography (dichloromethane/methanol = 100/1 to 10/1) to obtain the mixture of compound **455-1** and compound **455-1A.** MS m/z (ESI): 481.4 [M+1]+.

**Step 2):** At room temperature, the mixture of compound **455-1** and compound **455-1A** (100 mg, 208 µmol) was added to the dioxane solution of hydrogen chloride (2 mL, 4M) and allowed to react at room temperature for 1 hour. The mixture was concentrated under reduced pressure to remove the solvent. The residue was purified by high performance liquid chromatography (Gilson_306_1741, column: Waters-SunFire-C18-10µm-19×250mm; mobile phase: water (containing 10mmol/L formic acid) and acetonitrile, gradient ratio: acetonitrile 20%-80%, flow rate: 25mL/min) to obtain compound **455.** MS m/z(ESI):437.4[M+1]+.

¹H NMR (400 MHz, DMSO-*d*₆): δ 10.29 (s, 1H), 9.05 (d, J = 6.7 Hz, 1H), 7.14 (s, 1H), 7.09 (s, 1H), 7.02 (d, J = 6.7 Hz, 1H), 6.94 (d, J = 8.4 Hz, 1H), 4.50 - 4.35 (m, 1H), 3.75 - 3.73 (m, 1H), 3.52 - 3.49 (m, 2H), 3.00 - 2.98 (m, 1H), 2.74 - 2.68 (m, 1H), 2.47 - 2.44 (m, 2H), 2.16 (s, 3H), 2.11 - 2.07 (m, 2H), 1.88 - 1.85 (m, 1H), 1.72 - 1.68 (m, 1H), 1.56 - 1.47 (m, 1H), 1.39 - 1.34 (m, 1H).

### Example 37: Preparation of compound 456

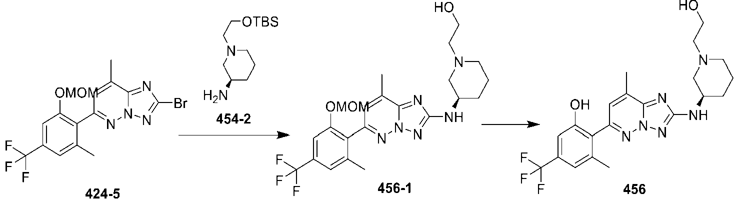

**Step 1):** At room temperature, compound **424-5** (150 mg, 350 µmol), compound **454-2** (90 mg, 350 µmol), tris(dibenzylideneacetone)dipalladium (32 mg, 30.0 µmol), sodium tert-butoxide (100 mg, 1.04mmol) and 4,5-bis(diphenylphosphino)-9,9-dimethylxanthene (21 mg, 30.0 µmol) were added to dioxane (2 mL) and stirred evenly. The reaction was purged with nitrogen three times. The mixture was heated to 80°C for 3 hours. The mixture was then mixed with water (20 mL) and extracted with ethyl acetate (6 mL×3). the organic phases were combined, washed with saturated brine (10 mL), dried over anhydrous sodium sulfate, and filtered, and the filtrate was concentrated under reduced pressure. The residue was purified by silica gel column chromatography (petroleum ether/ethyl acetate = 100/1 to 1/1) to obtain compound **456-1.** MS m/z(ESI):495.3[M+H]+.

**Step 2):** Compound **456-1** (20 mg, 30.0 µmol) was added to a 50 mL single-necked bottle at room temperature, and the dioxane solution of hydrogen chloride (2 mL, 4 M) was added and stirred. The reaction solution was stirred at room temperature for 1.5 hours, concentrated under reduced pressure, and the residue was purified by high performance liquid chromatography (A: 0.1% formic acid/water, B: acetonitrile, chromatographic column: Waters-SunFire-C18-10µm-19×250mm; mobile phase: water (containing 0.1% formic acid) and acetonitrile, gradient ratio: acetonitrile 32%-48%, flow rate: 25 mL/min) to obtain compound **456.** MS m/z(ESI):451.2[M+1]+.

¹H NMR (400 MHz, DMSO-*d₆*) δ 10.29 (s, 1H), 7.28 (s, 1H), 7.17 (s, 1H), 7.10 (s, 1H), 6.86 - 6.65 (m, 1H), 5.38 - 5.28 (m, 1H), 4.53 - 3.34 (m, 2H), 3.24 - 2.55 (m, 2H), 2.33 (s, 1H), 2.15 (s, 3H), 2.09 - 1.95 (m, 3H), 1.91 - 1.82 (m, 1H), 1.76 - 1.67 (m, 1H), 1.61 - 1.42 (m, 2H), 1.30 - 1.25 (m, 4H).

### Example 38: Preparation of compound 457

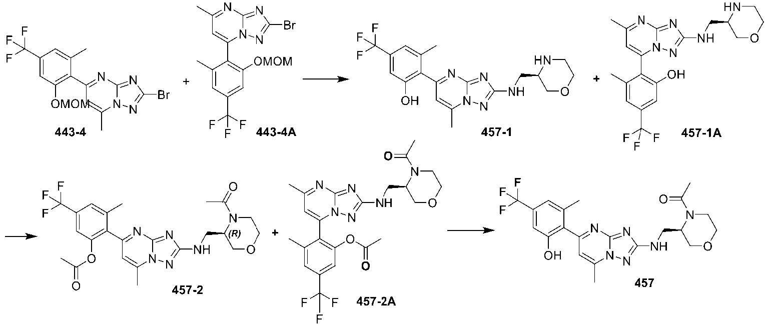

**Step 1):** The mixture of compound **443-4** and compound **443-4A** (200 mg, 0.460 mmol) and (R)-4-Boc-3-morpholinemethylamine (1.00 g, 4.64 mmol, Bid) were mixed, and the reaction solution was stirred and allowed to react for 1 hour at 170°C in a microwave. The reaction solution was mixed with dichloromethane (10 mL) and water (2 mL), and the aqueous phase was separated, and extracted with dichloromethane (2 mL×3). The organic phases were combined, dried over anhydrous sodium sulfate, and filtered, and the filtrate was concentrated under reduced pressure. The residue was purified by silica gel column chromatography (dichloromethane/methanol = 100/1 to 15/1) to obtain the mixture of compound **457-1** and compound **457-1A.** MS m/z(ESI):423.3[M+H]+.

**Step 2):** The mixture of compound **457-1** and compound **457-1A** (164 mg, 0.390 mmol) and triethylamine (0.161 mL, 1.16 mmol) were dissolved in dichloromethane (2 mL). Acetyl chloride (79.3 mg, 0.780 mmol) was slowly added dropwise to the reaction solution at 0°C. The reaction solution was allowed to react at this temperature for 1 hour. The reaction solution was concentrated under reduced pressure to obtain a mixture of compound **457-2** and compound **457-2A.** MS m/z (ESI): 507.5 [M+H] +.

**Step 3):** The mixture of compound **457-2** and compound **457-2A** (197 mg, 0.390 mmol) was dissolved in methanol (3 mL) and water (0.3 mL), and potassium carbonate (107 mg, 0.780 mmol) was added. The reaction solution was allowed to react at 25°C for 1 hour. The reaction solution was concentrated under reduced pressure to remove the solvent. The residue was adjusted to pH 7-8 with dilute hydrochloric acid (1.0 M), then extracted with dichloromethane (5 mL×3), and the organic phases were combined, then dried over anhydrous sodium sulfate, and filtered, and the filtrate was concentrated under reduced pressure. The residue was purified by high performance liquid chromatography (A: 0.1% formic acid/water, B: acetonitrile, chromatographic column: Waters-SunFire-C18-10µm-19×250mm; mobile phase: water (containing 0.1% formic acid) and acetonitrile, gradient ratio: acetonitrile 32%-48%, flow rate: 25 mL/min) to obtain a crude product, which was then purified by chiral separation (chromatographic column: ChiralCel OX, 250×30mm I.D., 5µm; mobile phase: A: carbon dioxide B: ethanol; gradient: 35%B; flow rate: 150 mL/min) to obtain compound **457.** MS m/z(ESI):465.3[M+H]+.

¹H NMR (400 MHz, DMSO-*d*₆): δ 10.34 - 10.14 (m, 1H), 7.37 - 7.33 (s, 1H), 7.12 (s, 1H), 7.07 (s, 1H), 6.99 (s, 1H), 4.14 - 3.97 (m, 2H), 3.91 - 3.84 (m, 2H), 3.61 - 3.46 (m, 4H), 3.10 - 2.83 (m, 1H), 2.66 (s, 3H), 2.13 (s, 3H), 2.09 (s, 3H).

### Biological Test

### 1. NLPR3 binding assay

This experiment uses fluorescence polarization technology to detect and evaluate the binding assay.

The fluorescent probe used in this assay is the compound NP3-301 in the Journal of Molecular Biology (2021) 433, 167309, which was synthesized with reference to this literature.

His6-Flag-TEV-NLRP3 (134-1036) protein (SEQ NO.1) was purchased from Kaihuiruizhi Biotech (Shanghai) Co., Ltd. (ChemPartner), and was customed for expression.

Prepare 1× assay buffer: 50 mM Hepes, pH 7.4, 150 mM NaCl, 2.5 mM MgCl₂, 0.005% Tween-20, 1 mM TCEP, 100 µM ATP.

First, 2.5 µL of 4× compound solution was added to a black shallow 384-well plate (Greiner, 784076), the shallow well plate was sealed, and centrifuged at 1000 rpm for 1 min; then 2× NLPR3 protein solution (final concentration of 30 nM) was added, the shallow well plate was sealed, centrifuged at 1000 rpm for 1 min, and incubated at room temperature for 60 min. Finally, 2.5 µL of 4× NP3-301 (final concentration of 0.5 nM) was added, the shallow well plate was sealed, centrifuged at 1000 rpm for 1 min, and incubated at room temperature for 30 min. Envision was used to read the fluorescence polarization values of λex = 485 ± 20 nm and λem = 535 ± 25 nm. GraphPad Prism 9.5.0 was used to analyze the data, and the IC50 value of the competition between the compound and NP3-301 for binding to His6-Flag-TEV-NLRP3 (134-1036) was fitted by four-parameter logistic. The compounds of the present invention have good NLRP3 binding activity, and the test results of some representative compounds are shown in Table 1.

**Table 1**

| Compound No. | FP Binding Assay IC₅₀(nM) | Compound No. | FP Binding Assay IC₅₀(nM) | Compound No. | FP Binding Assay IC₅₀(nM) |
|---|---|---|---|---|---|
| 1 | B | 4 | C | 9 | A |
| 10 | A | 5 | A | 6 | B |
| 11 | A | 11-a | A | 11-b | B |
| 12 | A | 12-a | A | 7 | B |
| 13 | C | 14 | A | 61 | A |
| 81 | B | 82 | A | 111 | A |
| 247 | A | 248 | A | 249 | A |
| 250 | A | 253 | A | 254 | B |
| 255 | C | 257 | A | 258 | A |
| 259 | A | 260 | A | 271 | B |
| 272 | A | 273 | B | 121 | D |
| 123 | C | 124 | B | 125 | B |
| 357 | B | 358 | B | 359 | A |
| 360 | A | 361 | B | 362 | A |
| 363 | A | 364 | A | 365 | B |
| 366 | B | 367 | B | 368 | A |
| 369 | B | 370 | C | 371 | A |
| 372 | A | 373 | B | 374 | A |
| 375 | A | 376 | A | 377 | A |
| 378 | B | 379 | B | 380 | B |
| 381 | A | 382 | B | 384 | A |
| 385 | B | 386 | A | 387 | A |
| 388 | A | 389 | A | 390 | A |
| 391 | A | 392 | A | 393 | B |
| 394 | A | 395 | A | 396 | A |
| 397 | A | 398 | A | 399 | A |
| 400 | A | 401 | A | 402 | A |
| 403 | A | 404 | A | 405 | A |
| 406 | A | 407 | A | 408 | A |
| 409 | B | 410 | B | 411 | B |
| 412 | B | 413 | A | 415 | A |
| 420 | A | 419 | B | 421 | C |
| 422 | A | 423 | A | 424 | A |
| 425 | B | 426 | A | 436 | B |
| 438 | A | 439 | A | 440 | A |
| 441 | A | 442 | A | 443 | A |
| 444 | A | 445 | A | 446 | A |
| 447 | A | 448 | A | 449 | A |
| 450 | A | 451 | A | 452 | A |
| 453 | C | 454 | A | 455 | A |
| 456 | A | 457 | B | 458 | A |
| 459 | D | 460 | A | 461 | B |
| 462 | A | | | | |

In Table 1, A represents IC50 ≤ 100 nM, B represents 100 nM < IC50 ≤ 500 nM, C represents 500 nM < IC50 ≤ 1000 nM, and D represents IC50 > 1000 nM.

### 2. Experiment to detect the effect of NLRP3 inhibitor on IL-1β secretion of THP-1 cells

This experiment used the PerkinElmer AlphaLISA_human_IL1β kit (AL220 C/F). The detection buffer was RPMI-1640+10FBS. AlphaLISA Immunoassay Buffer, AlphaLISA Anti-IL1β Acceptor beads, Streptavidin (SA)-coated Donor beads, and Biotinylated Antibody Anti-IL1β were all included in the kit.

The density of THP1 cells was adjusted to 4×10⁵ cells/mL, PMA (final concentration 50nM) was added, inoculated into 96-well flat-bottom plates at 200µL/well, and stimulated overnight at 37°C, 5% CO₂ (try to <16h). The next day, the supernatant was discarded, and the flat washed twice with DPBS (200µL/time). Then LPS solution (final concentration of 100ng/ml) was added at 198µL/well , incubated at 37°C, 5% CO₂ for 3h. 1µL of the corresponding concentration of compounds was added to each well according to the experimental MAP, incubated at 37°C, 5% CO₂ for 1h. 1µL Nigericin (final concentration of 10µM) was added to each well, incubated overnight at 5% CO₂ (>18h). On the third day, 5µL of supernatant was taken, diluted 20 times, and the content of IL-Iβ in the supernatant was detected by Human Interleukin 1 beta (IL1β) Kit. Envision was used to read the AlphaLISA test value of Ex = 680/ Em = 570. GraphPad Prism 9.5.0 was used to analyze the data, and the IC50 value of the compound for IL-Iβ antagonism was fitted by four-parameter Logistic. It was found that the compounds of the present invention have good inhibitory activity on the maturation and secretion of IL-1β in THP-1 cells. The results of some representative compounds of the present invention are shown in Table 2.

**Table 2: Results of IC50 experiments using THP-1 cells to detect NLRP3 inhibitors**

| Compound No. | IC50(nM) | Compound No. | IC50(nM) | Compound No. | IC50(nM) |
|---|---|---|---|---|---|
| 9 | B | 10 | C | 5 | C |
| 6 | C | 11 | A | 11-a | A |
| 12 | C | 12-a | A | 14 | B |
| 111 | A | 247 | B | 248 | C |
| 249 | C | 250 | C | 253 | A |
| 257 | A | 260 | A | 371 | B |
| 376 | A | 381 | C | 384 | A |
| 387 | B | 388 | B | 390 | A |
| 392 | A | 394 | B | 397 | C |
| 399 | A | 400 | A | 404 | A |
| 413 | B | 422 | C | 423 | C |
| 424 | A | 426 | A | 439 | A |
| 440 | B | 441 | A | 442 | A |
| 443 | A | 444 | A | 445 | A |
| 446 | A | 447 | A | 448 | A |
| 451 | B | 456 | A | 460 | A |
| 462 | A | | | | |

In Table 2, A represents IC50 < 50 nM, B represents 50 nM ≤ IC50 < 100 nM, C represents 100 nM ≤IC50 < 500 nM, and D represents IC50 ≥ 500 nM.

### 3. Liver microsome metabolic stability experiment

Purpose: This experiment investigates the metabolic stability of the compound in liver microsomes of different species such as humans, mice and rats, and the residual content of the test compound after incubation with liver microsomes of different species such as humans, mice and rats.

### Experimental method

### (1) Solution preparation

Preparation of 500 µM solution of target compound and positive control: 5 µL of each 10 mM stock solution was taken, added to 95 µL acetonitrile, and mixed for 30 s;

Preparation of 6 mM NADPH solution: 100 mM Stock aliquot liquid was diluted with Mg-Mg-K-Buffer.

### (2) Sample preparation

### 2.1 Reaction solution preparation

2.1.1 Liver microsome solution: 18.75 µL of 20 mg/mL HLM, RLM, MLM, DLM, MOLM (final concentration: 0.5 mg/ml) were taken and placed them in each well of a 96-well plate;
2.1.2 1.5 µL of 500 µM solution of each compound was added respectively;
2.1.3 479.8 µL of phosphate buffer was taken, then added to each compound well, and mixed at 1000 rpm in an oscillating mixer for 10 s.

### 2.2. Sample distribution

30 µL of the above samples were taken and aliquoted to a new 96-well plates respectively (including NADPH group, n=2, and no NADPH group at 0 and 45 min, n=1), marked as 0, 5, 15, 30 and 45 min;

### 2.3. Incubation

After the above samples were pre-incubated at 37°C for 5 min, 15 µL of 6 mM NADPH, which was preheated at 37°C, was added in the order of 45, 30, 15 and 5 min samples to start the reaction, 15 µL of Mg-K-Buffer solution was added to the NADPH-free group, mixed at 1000 rpm in an oscillating mixer for 10 s and continue incubation (cover the lid without sealing the plate);

### 3. Sample post-processing

For the sample at 0 min, 200 µL of acetonitrile internal standard was added, followed by addition of NADPH, the plate was sealed and shaken for 1 min, stored at 4°C until centrifugation;

After incubation of the samples at other time points have completed, add 200 µL acetonitrile internal standard respectively, seal the plate and shake for 1 min, store at 4°C until centrifugation;

After incubation was completed, vortex mix for 1 min, centrifuge at 4000 rpm for 50 min, add 80 µL of the supernatant to the 96-well plate containing 160 µL of pure water, mix for 30 s, and centrifuge at 4000 rpm for 5 min for testing.

### (3) Data analysis

The slope was calculated based on the LN value of the compound concentration percentage and time as the coordinate axis, and the residual content of the compound after incubation with liver microsomes was calculated based on the slope and formula. It was found that the compounds of the present invention have good stability in liver microsomes of different species. The test results of representative compounds (the residual content of the test compound after incubation with liver microsomes for 45 minutes) are shown in Table 3.

**Table 3**

| Compound No. | LMS (h,r,m) (% rem, 45 min) |
|---|---|
| 111 | 93.24(h)/82.45(r)/91.34(m) |

### 4. In vitro evaluation of CYP450 enzyme inhibition in human liver microsomes

### (1) Purpose:

This experiment incubates the compound, liver microsomes and different CYP subtype enzyme substrates in a 37°C water bath to measure the amount of substrate metabolites produced and evaluate the inhibitory effect of the compound of the present invention on CYP enzymes based on the IC50 value.

### (2) Experimental method

i. The compound was diluted to 0, 0.02 mM, 0.06 mM, 0.2 mM, 0.6 mM, 2 mM and 6 mM concentrations with DMSO (Aladdin, catalog number: D103273-500ml). The positive reference inhibitors of each CYP subtype and their concentrations are shown in Table 4.

**Table 4: Positive reference inhibitors of CYP subtype enzymes and their concentrations**

| **CYP Isoform** | **Inhibitor** | **Work solution Concentration (µM)** |
|---|---|---|
| CYP1A2 | α-naphthoflavone | 100, 30, 10,3, 1, 0.3, 0 |
| CYP2C9 | Sulfaphenazole | 1000, 300, 100, 30, 10, 3, 0 |
| CYP2C19 | S-(+)-N-3-benzyl-nirvanol | 1000, 300, 100, 30, 10, 3, 0 |
| CYP2D6 | Quinidine | 200, 60, 20,6, 2, 0.6, 0 |
| CYP 3A4 | Ketoconazole | 100, 30, 10,3, 1, 0.3, 0 |

ii. The enzyme substrate solutions of each CYP subtype were prepared by phosphate buffer (sigma: V900050-500G). The enzyme substrates and concentrations of each CYP subtype are shown in Table 5. Table 5: CYP subtype enzyme substrates and their concentrations

| CYP Isoform | Substrate | Conc. of stock solution (mM) |
|---|---|---|
| CYP1A2 | Phenacetin | 80 |
| CYP2C9 | Diclofenac Sodium | 16 |
| CYP2C 19 | Mephenytoin | 40 |
| CYP2D6 | Dextromethorphan | 40 |
| CYP3A4-Testosterone | Testosterone | 8 |
| CYP3A4-Midazolam | Midazolam | 4 |

iii. Sample incubation. 1 µL of each concentration of compound and CYP subtype enzyme positive reference inhibitor were added in a 96-deep well plate (A-gen: P-1.0-RD-96-S), 169 µL of 0.05 mg/mL liver microsome (BioIVT, IHG) phosphate buffer solution (CYP2C19 uses 0.2 mg/mL liver microsome) was added, mixed well and placed in a 37°C shaking water bath (Shanghai Jinghong Experimental Equipment Co., Ltd., DK-500) for preincubation for 15 min. 10 µL substrate (1 µL substrate was added to CYP3A4-Testosterone) and 20 µL 10 mM NADPH phosphate buffer solution (MCE, HYF003/CS-4998) were added, mixed well and incubated in a shaking water bath for the following time: CYP1A2: 20 min, CYP2C9: 10 min, CYP2C19: 10 min, CYP2D6: 10 min, CYP3A4-Testosterone: 10 min, CYP3A4-Midazolam: 5 min. 400 µL of glacial acetonitrile/methanol (v/v, 1:1) containing internal standards (500 nM tolbutamide, 5 nM terfenadine) was added to terminate the reaction. After centrifugation at 4000 rpm and 4°C for 40 min, the supernatant was diluted with pure water according to the response of the substrate metabolites and then detected by UPLC-MS/MS (Sciex, Q-Trap 4500). The C18 column was Kinetex 2.6u C18 100A (50mm×3mm).

### (3) Data analysis

The inhibitory effect of the compound on each P450 enzyme in human liver microsomes was calculated as the percentage of reduced metabolite formation compared with the non-inhibitory control (i.e., blank DMSO). The IC50 value for each P450 enzyme in human liver microsomes was calculated using the logarithm of the remaining activity and the inhibitor concentration using Graphpad Prism software. The calculation formula is as follows:$Area Ratio = Peak Area Analyte / Peak Area Internal Standard$ $Remaining Activity \left(%\right) = Area Ratio test compound / Area Ratio vehicle \times 100 %$

The compounds of the present invention have weak inhibitory effects on the enzyme activities of CYP1A2, CYP2C9, CYP2C19, CYP2D6, and CYP3A4. The test results of representative compounds are shown in Table 6.

**Table 6: Inhibitory activity data**

| | IC₅₀ (µM) | | | | | |
|---|---|---|---|---|---|---|
| | CYP1A2 | CYP2C9 | CYP2C19 | CYP2D6 | CYP3A4-T | CYP3A4-M |
| Compound 111 | >30.000 | >30.000 | >30.000 | >30.000 | >30.000 | 28.680 |

### 5. Evaluation of the inhibitory effect of hERG ion channels

### (1) Purpose:

This experiment used manual patch clamp electrophysiology to detect the inhibitory effect of compounds on hERG ion channels, and evaluated cardiac safety based on the single concentration inhibition rate or IC50 size of the compounds.

### (2) Experimental methods

The cells used in this experiment are HEK293 cell lines transfected with hERG cDNA and stably expressing hERG channels (#60187, provided by BPS, P3-P23 generations are used for experimental research). Cells were cultured in a medium containing the following components: MEM medium, 10% (v/v) inactivated fetal bovine serum, 1 mM sodium pyruvate, 500 µg geneticin, 0.1 mM non-essential amino acids, and 100 U penicillin-streptomycin. HEK293 hERG cells were grown in culture flasks containing the above culture medium and cultured in an incubator at 37°C and 5% CO₂. The cells were passaged approximately three times a week and the cell fusion rate was maintained between 40% and 80%. 24 to 48 hours before electrophysiological experiments, HEK293 hERG cells were transferred to 0.05 mg/ml poly-lysine (PDL) pre-treated glass slides, plated at 1×10⁴ cells in 48-well plates, and grown in the same culture medium and conditions. The density of HEK293 hERG cells per slide should be sufficient to ensure that most cells are independent and single.

The extracellular solution used in the hERG experiment contains the following components (mM): 145 NaCl, 4 KCl, 2 CaCl₂, 1 MgCl₂, 10 glucose, and 10 HEPES (pH adjusted to 7.40 with NaOH). The intracellular solution contains the following components (mM): 130 KCl, 2 MgCl₂, 5 EGTA, 10 HEPES and 5 Na₂ATP (pH adjusted to 7.25 with KOH).

To obtain the IC50 of the compounds, the following concentrations (30, 10, 3, 1, 0.3 and 0.1 µM) were tested. Prior to the test, the stock solutions were first diluted with DMSO in a serial dilution manner to 10, 3, 1, 0.3 and 0.1 mM, and then diluted with extracellular fluid to the final µM test concentration. The final DMSO concentration in each concentration of the compound solution ranged from 0.1% to 0.3%. All compound solutions were routinely sonicated for 5 to 10 minutes and fully shaken to ensure complete dissolution of the compound, and all test solutions were mixed by rotation for at least 10 minutes.

The electrophysiological experiments were performed by a manual patch clamp system (HEKA EPC-10 signal amplifier and digital conversion system, purchased from HEKA Electronics, Germany) to record the whole-cell current. The specific test method is described as follows: A round glass slide with HEK293 cells stably expressing hERG channels growing on the surface was placed in an electrophysiological recording tank under an inverted microscope. The recording tank was continuously perfused with extracellular fluid (approximately 1 mL per minute). The experimental process used conventional whole-cell patch clamp current recording technology. Unless otherwise specified, the experiments were performed at normal room temperature (~25°C). The cells were clamped at a voltage of -80 mV. The cell clamping voltage was depolarized to +30 mV to activate the hERG potassium channel, and then clamped to -50 mV after 5 seconds to eliminate inactivation and generate tail current. The peak value of the tail current was used as the value of the hERG current size. After the hERG current recorded in the above steps reaches stability under continuous perfusion of extracellular fluid in the recording tank, different concentrations of the test compound can be perfused until the inhibitory effect of the drug on the hERG current reaches a stable state.

### (3) Data analysis

Excel software was used to calculate the hERG ion channel inhibition rate of the compound at each concentration. Graphpad Prism 9.0 software was used to plot the dose-response curve of the test compound with %hERG inhibition rate as the vertical axis and the concentration of the test compound as the horizontal axis. The IC50 was calculated using the log(inhibitor) vs. response -- Variable slope (four parameters) parameter for curve fitting. The specific equation parameters are as follows: Y=Bottom + (Top-Bottom)/(1+10^((LogIC50-X)*HillSlope)). The compounds of the present invention have a small inhibitory effect on the hERG ion channel. The test results of some representative compounds are shown in Table 7 below.

**Table 7**

| Compound No. | hERG(IC50, manupatch) |
|---|---|
| 111 | >30µM |
| 384 | >30µM |

### 6. Pharmacokinetic evaluation of the compound in mice

Purpose: ICR male mice were selected as test animals. The LC-MS/MS method was used to quantitatively determine the drug concentration in the plasma of mice at different time points after intravenous injection or oral administration of the test compound to evaluate the pharmacokinetic characteristics of the test drug in mice.

Experimental materials: ICR mice (male, 15-35g, 5-10 weeks old, Shanghai Xipu-Bikai Experimental Animal Co., Ltd.)

### Experimental procedure A:

The clear solution of the test compound was injected into ICR mice via the tail vein (without fasting, vehicle: 5% DMSO/10% Solutol/85% Saline) and administered to ICR mice by gavage (fasting for 10-14h). For intravenous administration, blood was collected from the submandibular venous plexus at 0.033, 0.083, 0.25, 0.5, 1, 2, 4, 8, and 24 hrs after administration, placed in an EDTA-K2 anticoagulant tube (Jiangsu Kangjian Medical Supplies Co., Ltd.), mixed, and centrifuged at 2-8°C, 6800 g for 6 minutes to obtain plasma; for oral gavage administration, blood was collected from the submandibular venous plexus at 0.083, 0.25, 0.5, 1, 2, 4, 6, 8, and 24 hrs after administration, placed in an EDTA-K2 anticoagulant tube (Jiangsu Kangjian Medical Supplies Co., Ltd.), mixed, and centrifuged at 2-8°C, 6800 g for 6 minutes to obtain plasma. The blood drug concentration was determined by LC-MS/MS, and the pharmacokinetic software Phoenix WinNonlinTM Version 7.0 (Pharsight, USA) was used to calculate the relevant pharmacokinetic parameters by the non-compartmental linear logarithmic trapezoidal method. The compounds of the present invention have good pharmacokinetic characteristics in mice, and the test results of representative compounds are shown in Table 8.

**Table 8**

| Compound No. | Dosage IV/PO (mg/kg) | Cmax (ng/mL) | CL (mL/min/kg) | Vdss (L/kg) | T1/2 (IV, h) | AUC₀₋ₗₐₛₜ PO (ng.h/mL) | AUC₀₋ₗₐₛₜ **IV** (ng.h/mL) | F (%) |
|---|---|---|---|---|---|---|---|---|
| 111 | 1/3 | 1546.36 | 19.25 | 1.926 | 2.12 | 3435.47 | 834.17 | 137.28 |

### 7. Pharmacokinetic evaluation of the compound in rats

Purpose: The test animals were SD male rats. The LC-MS/MS method was used to quantitatively determine the drug concentration in the plasma of rats at different time points after intravenous injection or oral administration of the test compound to evaluate the pharmacokinetic characteristics of the test drug in rats.

### Experimental materials:

SD rats (male, 160-300 g, 5-10 weeks old, Shanghai Xipu-Bikai Experimental Animal Co., Ltd.)

### Experimental procedure A:

The clear solution of the test compound was injected into SD rats via the tail vein (without fasting) (vehicle: 5% DMSO/10% Solutol/85% Saline), and the clear solution of the test compound was administered to SD rats by gavage (fasting for 10-14 h, free access to water) (vehicle: 5% DMSO/10% Solutol/85% Saline). For intravenous administration, blood was collected from the jugular vein at 0.033, 0.083, 0.25, 0.5, 1, 2, 4, 8, and 24 hrs after administration, placed in an EDTA-K2 anticoagulant tube (Jiangsu Kangjian Medical Supplies Co., Ltd.), mixed, and centrifuged at 2-8°C, 6800 g for 6 minutes to obtain plasma; for oral gavage administration, blood was collected from the jugular vein at 0.083, 0.25, 0.5, 1, 2, 4, 6, 8, and 24 hrs after administration, placed in an EDTA-K2 anticoagulant tube (Jiangsu Kangjian Medical Supplies Co., Ltd.), mixed, and centrifuged at 2-8°C, 6800 g for 6 minutes to obtain plasma. The plasma concentration was determined by LC-MS/MS, and the relevant pharmacokinetic parameters were calculated by the non-compartmental linear logarithmic trapezoidal method using Phoenix WinNonlinTM Version 7.0 (Pharsight, USA) pharmacokinetic software. The compounds of the present invention have good pharmacokinetic characteristics in rats. The test results of representative compounds are shown in Table 9.

**Table 9**

| Compound No. | Dosage IV/PO (mg/kg) | Cmax (ng/mL) | CL (mL/min/kg) | Vdss (L/kg) | T1/2 (IV, h) | AUC₀₋ₗₐₛₜ PO (ng.h/mL) | AUC₀₋ₗₐₛₜ IV (ng.h/mL) | F (%) |
|---|---|---|---|---|---|---|---|---|
| 111 | 1/3 | 1394.18 | 5.78 | 1.018 | 3.36 | 8121.53 | 2880.04 | 94% |

### 8. Study on the efficacy of compounds on iodoacetic acid-induced SD rat knee osteoarthritis model

Purpose: Evaluate the efficacy of MCC950 (Positive reference compound) and compound 111 of the present invention on iodoacetic acid (MIA)-induced SD rat knee osteoarthritis model.

Experimental materials: Experimental male SD rats, 4 weeks old.

### Experimental methods:

Model induction began after the experimental animals were adapted to the animal environment for 7 days. Day 0, the rats were anesthetized, the hair around the left knee joint was removed, and the skin was disinfected. The left leg was flexed 90° to expose the patellar platform. The depression in the middle of the platform was touched by hand to find the femoral condyle, patella and ligament. The syringe needle was inserted from the bottom of the patella on both sides of the patellar ligament to the direction of the femoral condyle. When a clear sense of empty space was felt, 50µL of iodoacetic acid solution was injected. Day 7 Grouping was performed according to the width of the knee joint on the induced side. The groups were: normal control group (healthy control without modeling, 5% DMSO+10% Solutol+85% Saline), model blank control group (5% DMSO+10% Solutol+85% Saline), MCC950 30mg/kg, and compound 111 of the present invention 30mg/kg. Starting from Day 7, the normal control group and the model blank control group were given the solvent (5% DMSO+10% Solutol+85% Saline) orally once a day, and the other groups were given the corresponding compounds orally once a day until the end of the administration on Day 42. The knee joint width and body weight of the rats were measured once on Day 7, 14, 21, 28, 35, and 42.

### Experimental conclusion:

This experiment tested the efficacy of MCC950 (Positive compound) and compound 111 of the present invention on the SD rat knee osteoarthritis model induced by iodoacetic acid (MIA), see Figures 1 and 2. The results of oral administration of the compounds once a day showed that compared with the model blank control group, MCC950 (Positive compound) and the compound 111 of the present invention could significantly inhibit the widening of the knee joints of rats with knee osteoarthritis model from 21 days to 42 days after modeling without affecting the body weight of the rats.

The technical solution of the present invention is not limited to the above-mentioned specific embodiments. All technical variations made according to the technical solution of the present invention fall within the protection scope of the present invention.

## Claims

1. A compound represented by Formula I, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof, 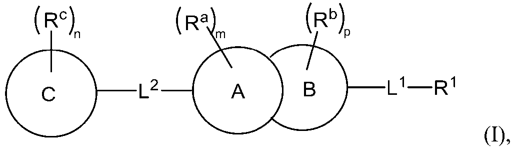
wherein, 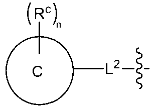 is connected to ring A, 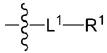 is connected to ring B;
ring A and ring B are each independently selected from: 5-6 membered heteroaryl, 5-6 membered heterocyclyl, and phenyl;
ring C is selected from: 6-10 membered aryl, 5-10 membered heteroaryl, phenyl fused 4-7 membered cycloalkyl, phenyl fused 4-7 membered heterocyclyl, 5-6 membered heteroaryl fused 4-7 membered cycloalkyl, 5-6 membered heteroaryl fused 4-7 membered heterocyclyl, 4-7 membered cycloalkyl, and 4-7 membered heterocyclyl;
each R^{c} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, R^{c1}S(O)₂-, -(CH₂)ₛ-N(R^{c1})(R^{c2}), 3-6 membered cycloalkyl, 3-6 membered heterocyclyl, phenyl, and 5-6 membered heteroaryl; the 3-6 membered cycloalkyl, 3-6 membered heterocyclyl, phenyl, and 5-6 membered heteroaryl are optionally substituted with 1, 2, or 3 substituents selected from: deuterium, halogen, -OH, -NH₂, -CN, C₁₋₆ alkyl, C₁₋₆ haloalkyl, and C₁₋₆ alkoxy; or
two R^{c} together with the C atom to which they are attached may form a 3-6 membered cycloalkyl, 4-7 membered heterocyclyl, 6 membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
each R^{a} and R^{b} are independently selected from: H, deuterium, halogen, -OH, -NH₂, -CN, oxo, C₁₋₆ alkyl, C₁₋₆ haloalkyl, C₁₋₆ alkoxy, C₁₋₆ alkoxy-C₁₋₆ alkyl, C₁₋₆ haloalkoxy, hydroxyl C₁₋₆ alkyl, -NH(C₁₋₆ alkyl), -N(C₁₋₆ alkyl)(C₁₋₆ alkyl), -C(=O)-(C₁₋₆ alkyl), and 3-6 membered heterocyclyl optionally substituted by hydroxyl;
L¹ is selected from: bond, C₃₋₆ cycloalkylene, -NR²-, -O-, -S-, -C(=O)-, -(CR³R⁴)ₐ-, -NR²-(CR³R⁴)ₐ-, - (CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, - (CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, - NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, -C(=O)-(CR³R⁴)ₐ-NR²-, -NR²-(CR³R⁴)ₐ-C(=O)-NR⁵-, -NR²-C(=O)-(CR³R⁴)ₐ-NR⁵-, -C(=S)-NR²-, and -NR²-C(=S)-;
L² is selected from: bond, -NR⁶-, -O-, -S-, -NR⁶-(CR⁷R⁸)b-, -(CR⁷R⁸)b-NR⁶-, -O-(CR⁷R⁸)_{b}-, -(CR⁷R⁸)b-O-, - S-(CR⁷R⁸)_{b}-, and -(CR⁷R⁸)_{b}-S-;
each R², R⁵, R⁶, R⁹ and R¹⁰ are each independently selected from: H, deuterium, and C₁₋₆ alkyl;
each R³, R⁴, R⁷, and R⁸ are each independently selected from: H, deuterium, halogen, oxo, -OH, -NH₂, C₁₋₆ alkyl;
R¹ is selected from: H, -OH, -NH₂, -COOH, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the -NH₂, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: H, deuterium, halogen, -OH, oxo, -CN, C₁₋₆ alkyl, hydroxyl C₁₋₆ alkyl, C₁₋₆ haloalkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, 5-6 membered heteroaryl, -C(=O)-R^{c3}, -S(O)-R^{c3}, -S(O)₂-R^{c3}, -C(=O)O-R^{c3}, -CH₂-C(=O)O-R^{c4}, -N(R^{c4})-C(=O)-R^{c3}, -CH₂-N(R^{c4})-C(=O)-R^{c4}, -C(=O)-N(R^{c6})(R^{c5}), -CH₂-C(=O)-N(R^{c4})(R^{c4}), -N(R^{c4})-C(=O)-N(R^{c6})(R^{c5}), -P(=O)R^{c5}R^{c6}, -CR^{c7}R^{c8}-CN, and -(CH₂)ₛ-R^{c9};
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: H, deuterium, C₁₋₆ alkyl, C₁₋₆ haloalkyl, and C₃₋₆ cycloalkyl, wherein the C₁₋₆ alkyl and C₃₋₆ cycloalkyl are optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and - N(C₁₋₆ alkyl)(C₁₋₆ alkyl);
R^{c4} is selected from: H, deuterium, and C₁₋₆ alkyl;
R^{c5} and R^{c6} are selected from: H, deuterium, C₁₋₆ alkyl, and C₃₋₆ cycloalkyl, or R^{c5}, R^{c6} together with the N atom to which they are attached form a 4-6 membered heterocyclyl; wherein the C₁₋₆ alkyl, C₃₋₆ cycloalkyl, and 4-6 membered heterocyclyl are optionally substituted with 1-3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
R^{c7} and R^{c8} are selected from: H, deuterium, and C₁₋₆ alkyl, or R^{c7}, R^{c8} together with the C atom to which they are attached form a C₃₋₆ cycloalkyl;
R^{c9} is selected from 3-6 membered heterocyclyl, wherein the heterocyclyl is optionally substituted with 1, 2 or 3 substituents selected from: H, deuterium, and C₁₋₆ alkyl;
R¹¹ is selected from: H, deuterium, C₁₋₆ alkyl, and 3-6 monocyclic heterocyclyl;
each a and b are each independently 1, 2, 3, or 4;
each s, n, m, and p are each independently selected from: 0, 1, 2, 3, and 4;
provided that, 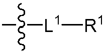 is not H, C₁₋₆ alkyl.

2. The compound according to claim 1, wherein, 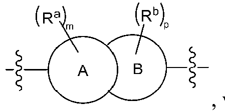 which relates to ring A and ring B, is selected from the following groups: 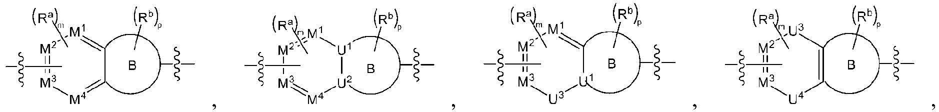 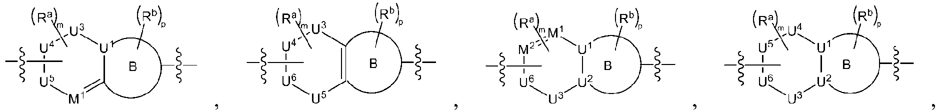 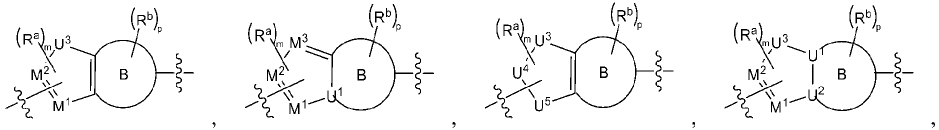 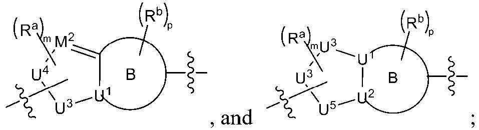
each M¹, M², M³, M⁴, U¹, and U² are each independently selected from: CH and N;
each U³, U⁴, U⁵, and U⁶ are each independently selected from: CH₂, NH, O and S.

3. The compound according to claim 1, wherein, 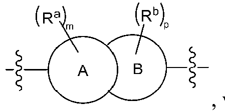 which relates to ring A and ring B, is selected from the following groups: 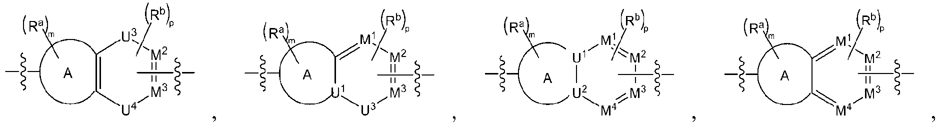 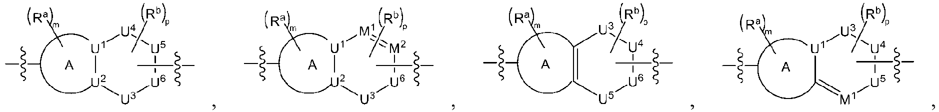 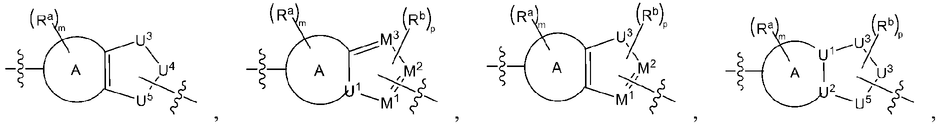 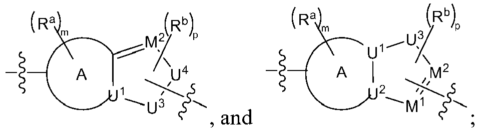
each M¹, M², M³, M⁴, U¹, and U² are each independently selected from: CH and N;
each U³, U⁴, U⁵, and U⁶ are each independently selected from: CH₂, NH, O and S.

4. The compound according to claim 1, wherein, 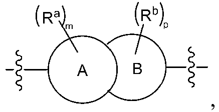 which relates to ring A and ring B is selected from the following groups: 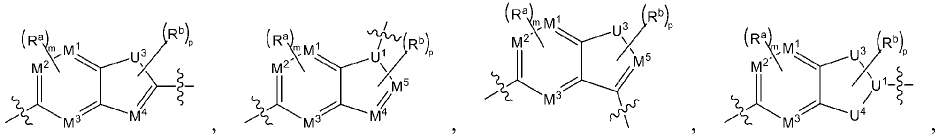 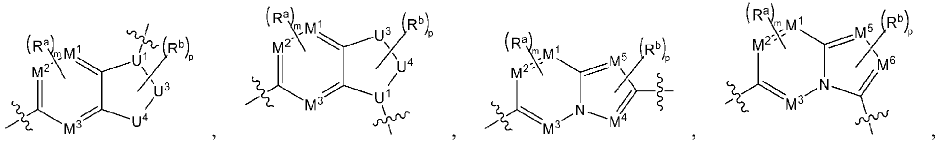 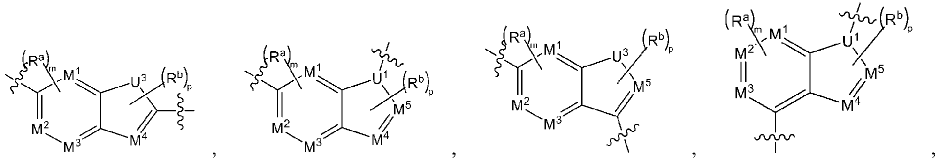 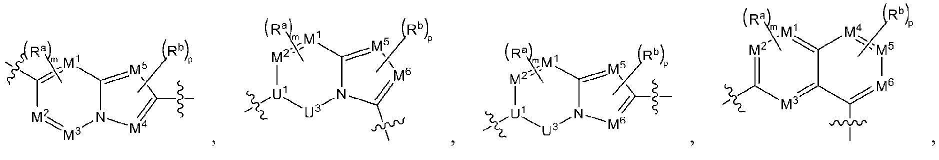 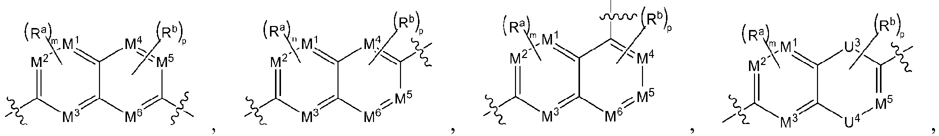 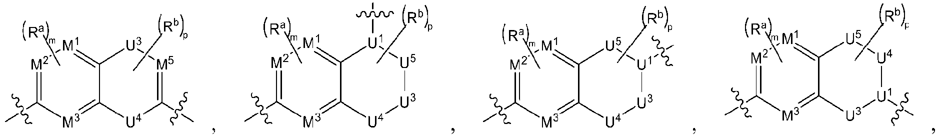 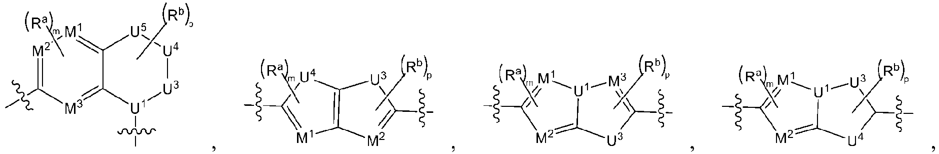 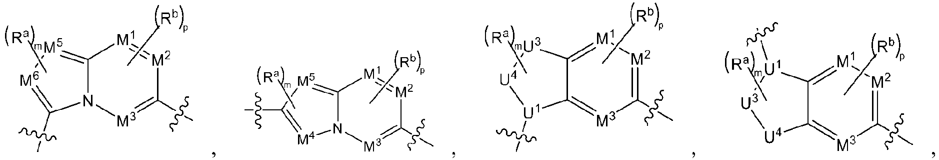 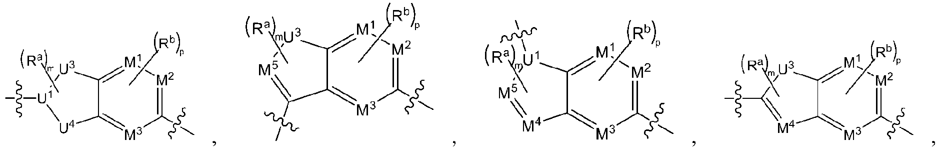 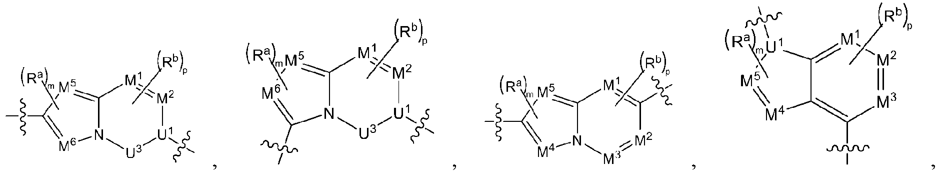 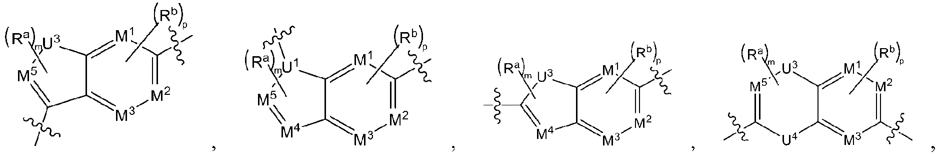 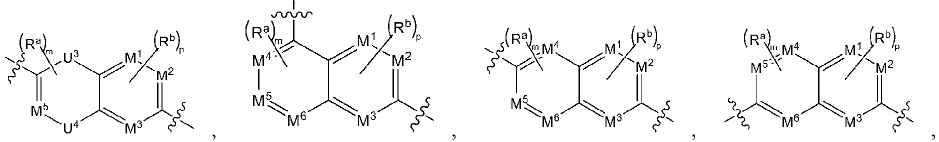 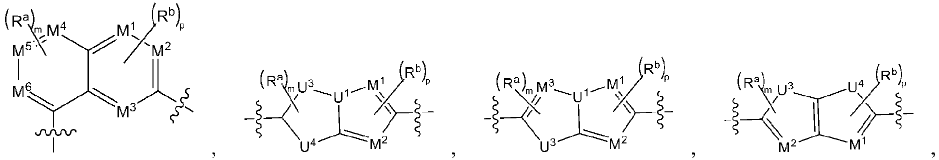 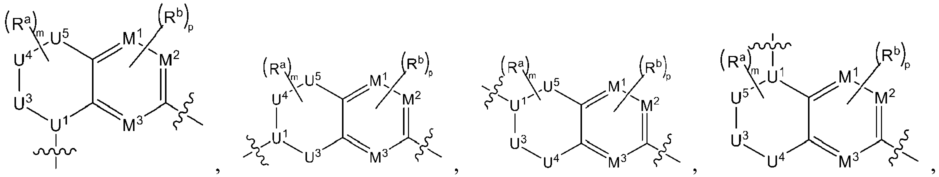 and 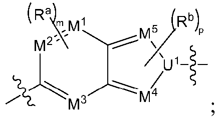
each M¹, M², M³, M⁴, M⁵, M⁶, U¹, and U² are each independently selected from: CH and N;
each U³, U⁴, U⁵, and U⁶ are each independently selected from: CH₂, NH, O and S.

5. The compound according to any one of claims 1-4, wherein, the compound has the structure represented by Formula VIII-3 or VIII-4: 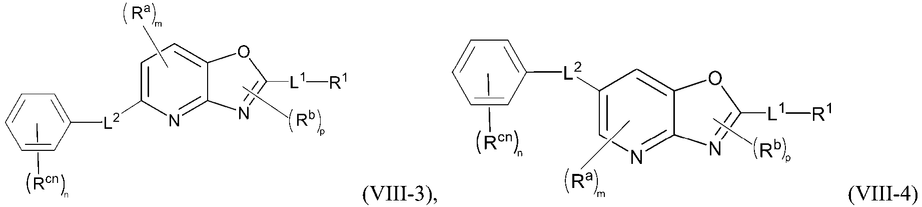
wherein, R¹ is selected from: -OH, -NH₂, -COOH, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the -NH₂, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: H, deuterium, halogen, -OH, oxo, -CN, C₁₋₆ alkyl, hydroxyl C₁₋₆ alkyl, C₁₋₆ haloalkyl, C₁₋₆ alkoxy, -(CH₂)ₛ-O(R^{c1}), - (CH₂)ₛ-N(R^{c1})(R^{c2}), C₃₋₆ cycloalkyl, 3-6 membered monocyclic heterocyclyl, 5-6 membered heteroaryl, -CR^{c7}R^{c8}-CN, and -(CH₂)ₛ-R^{c9};
R^{cn} is defined as R^{c} in claim 1;
provided that the following 1)-45) are met at the same time:
1) when 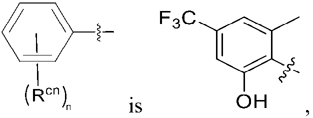 L² is bond, m=p=0 or R^{a}=R^{b}=H, and L¹ is -NH-, R¹ is not selected from the following groups: 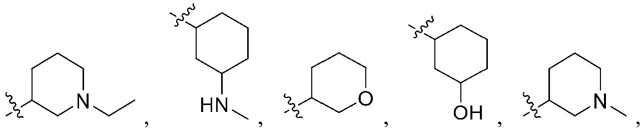 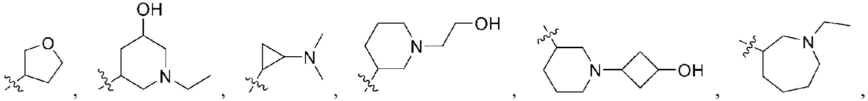 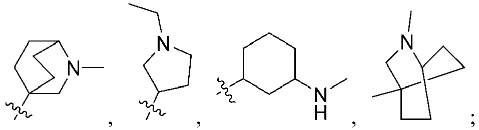
2) when 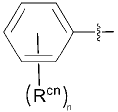 is 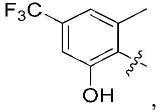 L² is bond, m=p=0 or R^{a}=R^{b}=H, and L¹ is -NH-CH₂-, R¹ is not selected from the following groups: 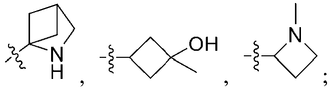
3) when 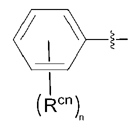 is 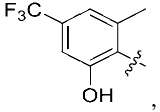 L² is bond, m=p=0 or R^{a}=R^{b}=H, and L¹ is bond, R¹ is not selected from the following groups: 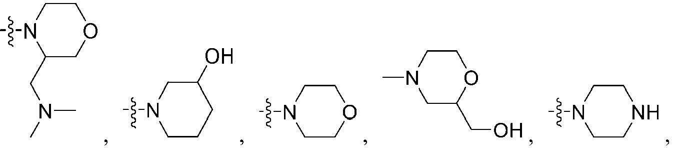 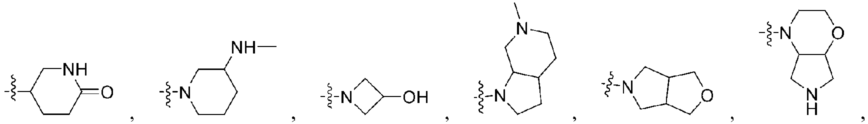 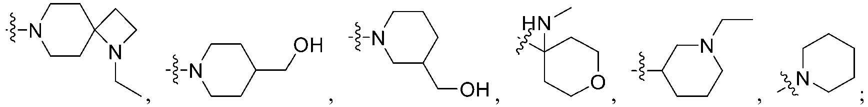
4) when 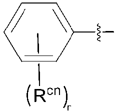 is 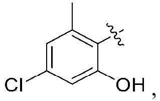 L² is bond, m=p=0 or R^{a}=R^{b}=H, and L¹ is -NH-, R¹ is not selected from the following groups: 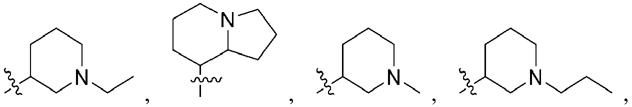 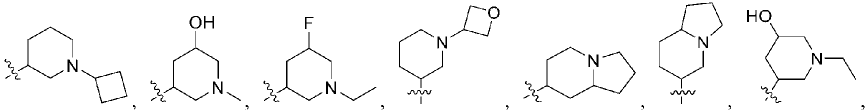 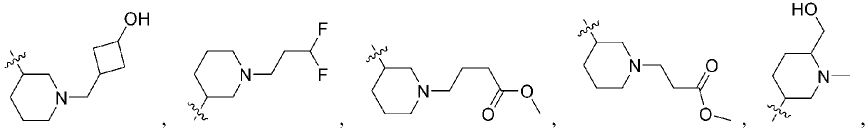 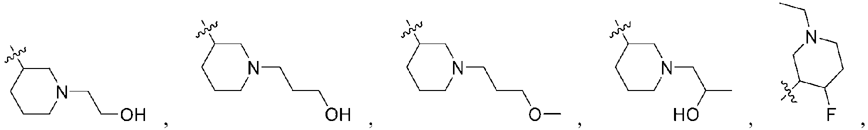 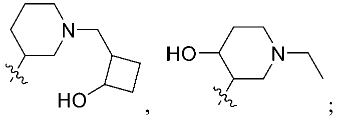
5) when 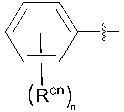 is 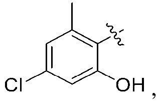 L² is bond, m=p=0 or R^{a}=R^{b}=H, and L¹ is bond, R¹ is not selected from the following groups: 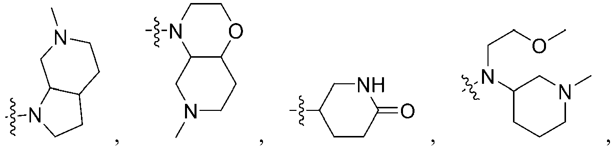 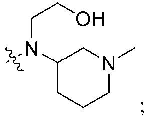
6) when 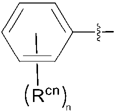 is 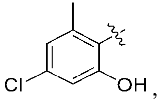 L² is bond, m=p=0 or R^{a}=R^{b}=H, and L¹ is -NH-CH₂-, R¹ is not selected from the following groups: 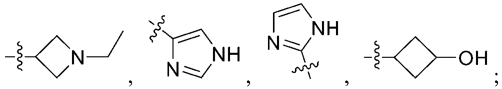
7) when 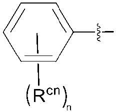 is 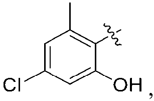 L² is bond, m=p=0 or R^{a}=R^{b}=H, and L¹ is -CH₂-, R¹ is not selected from the following groups: 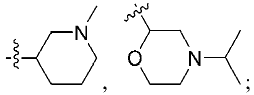
8) when 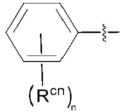 is 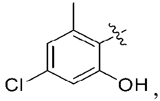 L² is bond, 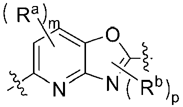 is 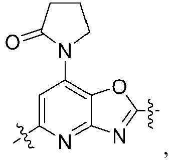 and L¹ is -NH-, R¹ is not 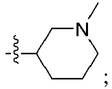
9) when 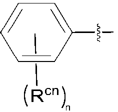 is 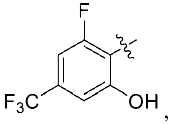 L² is bond, m=p=0 or R^{a}=R^{b}=H, and L¹ is -NH-, R¹ is not 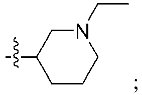
10) when 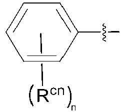 is 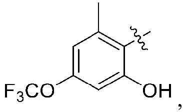 L² is bond, m=p=0 or R^{a}=R^{b}=H, and L¹ is -NH-, R¹ is not 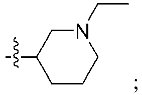
11) when 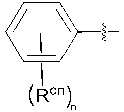 is 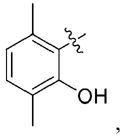 L² is bond, m=p=0 or R^{a}=R^{b}=H, and L¹ is -NH-, R¹ is not 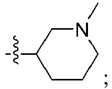
12) when 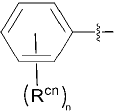 is 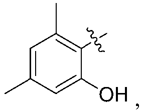 L² is bond, m=p=0 or R^{a}=R^{b}=H, and L¹ is -NH-, R¹ is not selected from the following groups: 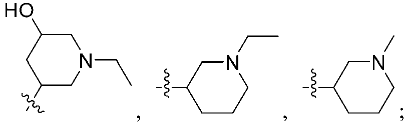
13) when 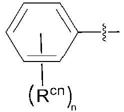 is 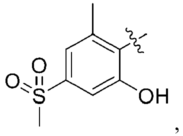 L² is bond, m=p=0 or R^{a}=R^{b}=H, and L¹ is -NH-, R¹ is not 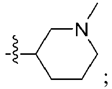
14) when 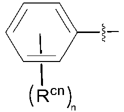 is 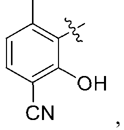 L² is bond, m=p=0 or R^{a}=R^{b}=H, and L¹ is -NH-, R¹ is not 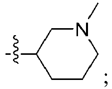
15) when 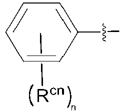 is 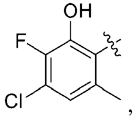 L² is bond, m=p=0 or R^{a}=R^{b}=H, and L¹ is -NH-, R¹ is not 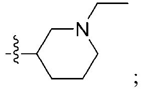
16) when 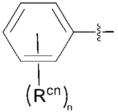 is 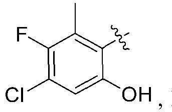 L² is bond, m=p=0 or R^{a}=R^{b}=H, and L¹ is -NH-, R¹ is not 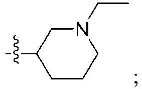
17) when 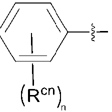 is 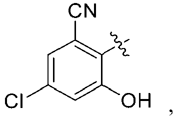 L² is bond, m=p=0 or R^{a}=R^{b}=H, and L¹ is -NH-, R¹ is not 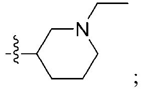
18) when 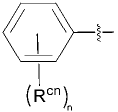 is 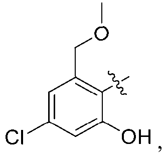 L² is bond, m=p=0 or R^{a}=R^{b}=H, and L¹ is -NH-, R¹ is not selected from the following groups: 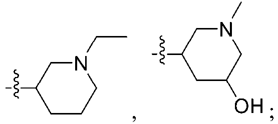
19) when 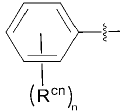 is 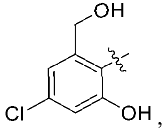 L² is bond, m=p=0 or R^{a}=R^{b}=H, and L¹ is -NH-, R¹ is not 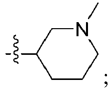
20) when 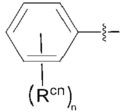 is 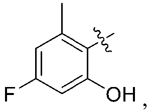 L² is bond, m=p=0 or R^{a}=R^{b}=H, and L¹ is -NH-, R¹ is not selected from the following groups: 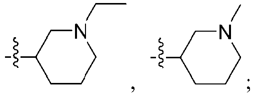
21) when 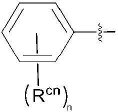 is 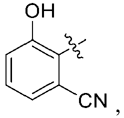 L² is bond, m=p=0 or R^{a}=R^{b}=H, and L¹ is -NH-, R¹ is not 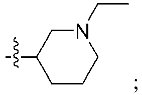
22) when 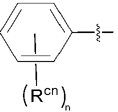 is 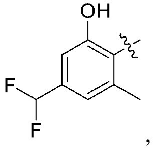 L² is bond, m=p=0 or R^{a}=R^{b}=H, and L¹ is -NH-, R¹ is not selected from the following groups: 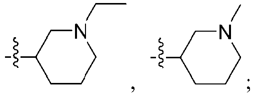
23) when 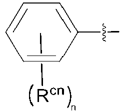 is 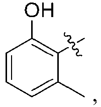 L² is bond, m=p=0 or R^{a}=R^{b}=H, and L¹ is -NH-, R¹ is not 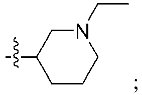
24) when 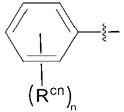 is 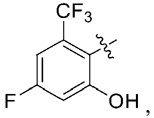 L² is bond, m=p=0 or R^{a}=R^{b}=H, and L¹ is -NH-, R¹ is not 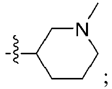
25) when 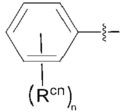 is 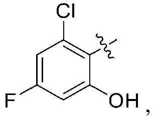 L² is bond, m=p=0 or R^{a}=R^{b}=H, and L¹ is -NH-, R¹ is not 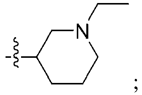
26) when 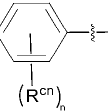 is 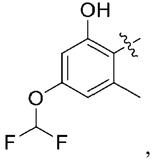 L² is bond, m=p=0 or R^{a}=R^{b}=H, and L¹ is -NH-, R¹ is not 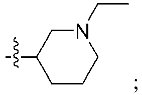
27) when 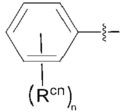 is 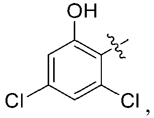 L² is bond, m=p=0 or R^{a}=R^{b}=H, and L¹ is -NH-, R¹ is not 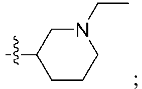
28) when 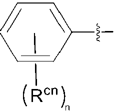 is 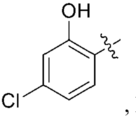 L² is bond, 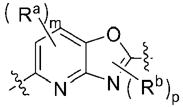 is 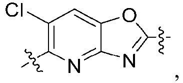 and L¹ is -NH-, R¹ is not 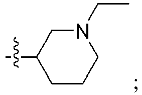
29) when 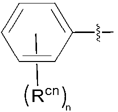 is 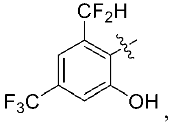 L² is bond, m=p=0 or R^{a}=R^{b}=H, and L¹ is -NH-, R¹ is not 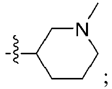
30) when 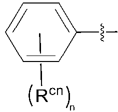 is 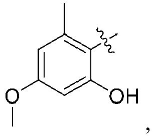 L² is bond, m=p=0 or R^{a}=R^{b}=H, and L¹ is -NH-, R¹ is not 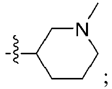
31) when 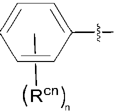 is 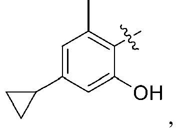 L² is bond, m=p=0 or R^{a}=R^{b}=H, and L¹ is -NH-, R¹ is not 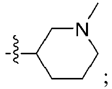
32) when 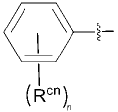 is 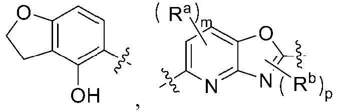 is 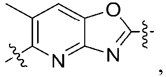 and L¹ is -NH-, R¹ is not 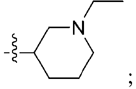
33) when 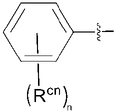 is 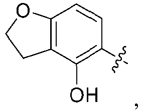 L² is bond, m=p=0 or R^{a}=R^{b}=H, and L¹ is -NH-, R¹ is not 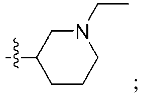
34) when 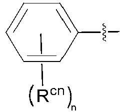 is 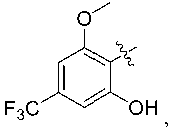 L² is bond, m=p=0 or R^{a}=R^{b}=H, and L¹ is -NH-, R¹ is not 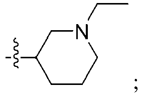
35) when 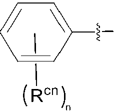 is 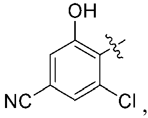 L² is bond, m=p=0 or R^{a}=R^{b}=H, and L¹ is -NH-, R¹ is not 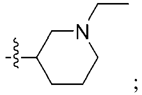
36) when 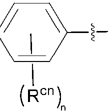 is 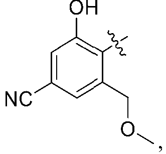 L² is bond, m=p=0 or R^{a}=R^{b}=H, and L¹ is -NH-, R¹ is not 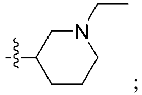
37) when 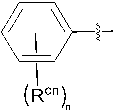 is 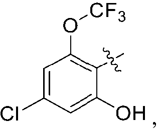 L² is bond, m=p=0 or R^{a}=R^{b}=H, and L¹ is -NH-, R¹ is not 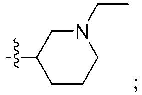
38) when 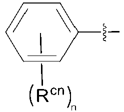 is 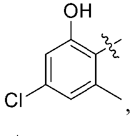 L² is bond, m=p=0 or R^{a}=R^{b}=H, and L¹ is -NH-, R¹ is not selected from the following groups: 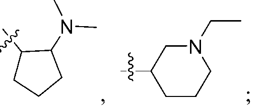
39) when 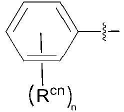 is 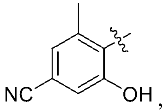 L² is bond, m=p=0 or R^{a}=R^{b}=H, and L¹ is -NH-, R¹ is not selected from the following groups: 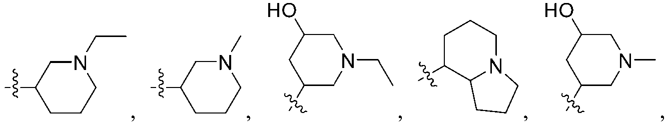 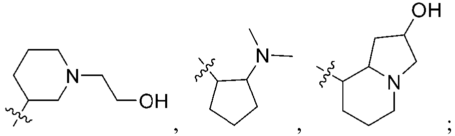
40) when 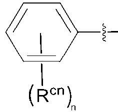 is 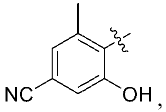 L² is bond, m=p=0 or R^{a}=R^{b}=H, and L¹ is bond, R¹ is not selected from the following groups: 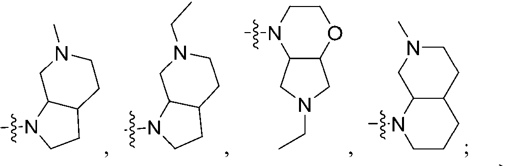
41) when 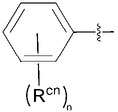 is 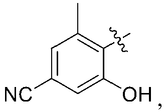 L² is bond, 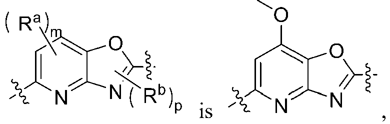 and L¹ is bond, R¹ is not 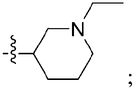
42) when 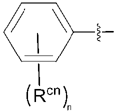 is 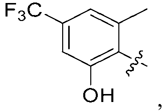 L² is bond, L¹ is -NH-, and R¹ is 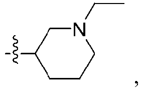 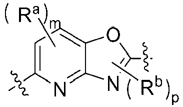 is not selected from the following groups: 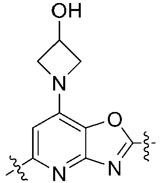 , 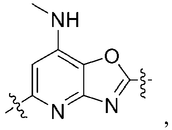 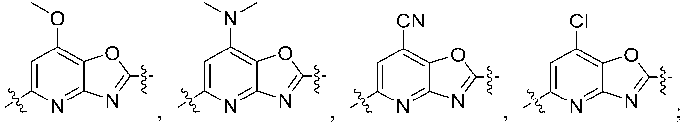
43) when 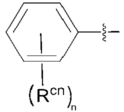 is 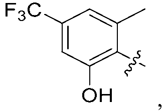 L² is bond, L¹ is -NH-, and R¹ is 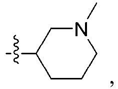 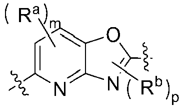 is not selected from the following groups: 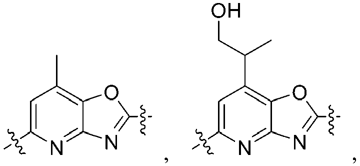 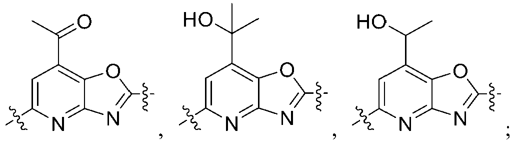
44) when 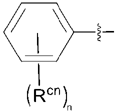 is 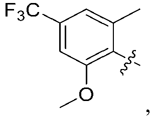 L² is bond, m=p=0 or R^{a}=R^{b}=H, and L¹ is bond, R¹ is not selected from the following groups: 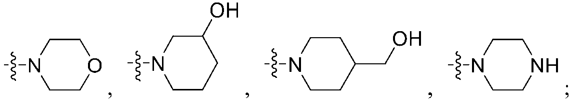
45) the compound is not 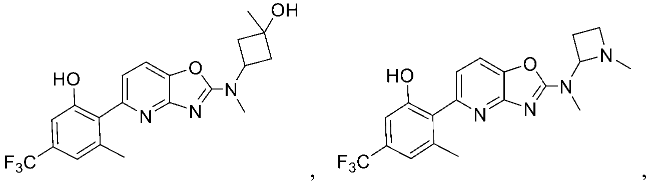 or

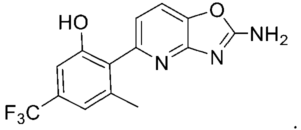

6. The compound according to claim 1, wherein, 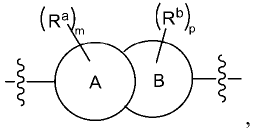 which relates to ring A and ring B, is selected from the following groups, wherein, the following bicyclic groups represent ring A and ring B from left to right or ring A and ring B from right to left; 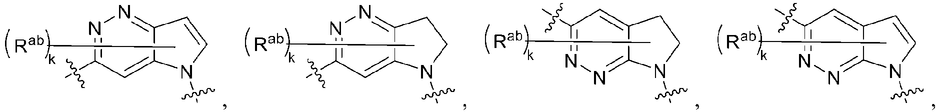 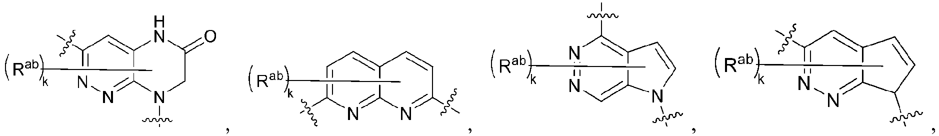 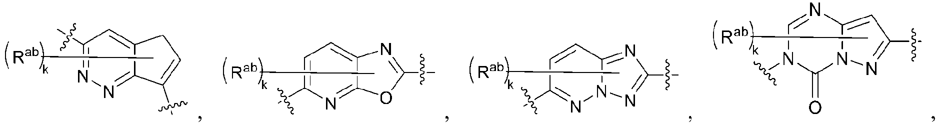 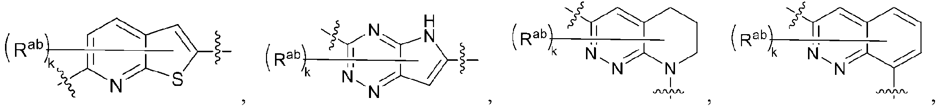 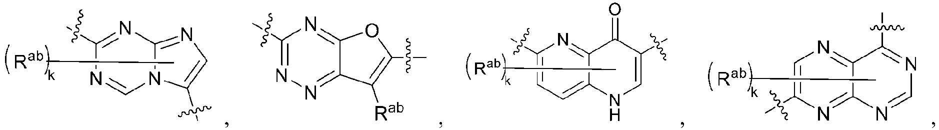 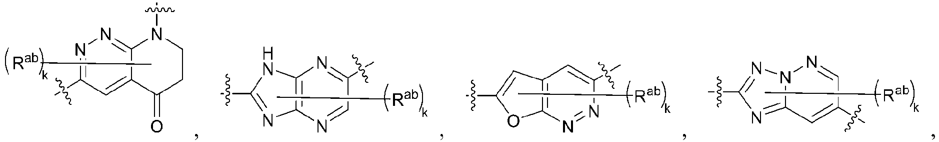 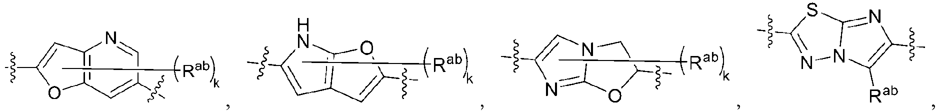 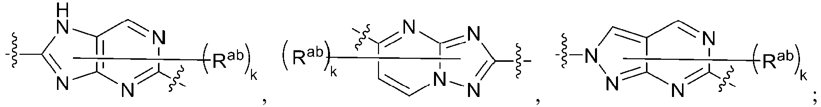 wherein,
each R^{ab} is independently selected from: H, deuterium, halogen, -OH, -NH₂, -CN, oxo, methyl, ethyl, propyl, isopropyl, butyl;
each k is independently selected from: 0, 1, 2, 3, and 4.

7. The compound according to claim 1, wherein, 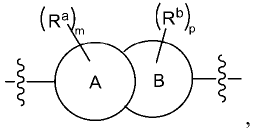 which relates to ring A and ring B, is selected from the following groups, wherein, the following bicyclic groups represent ring A and ring B from left to right or ring A and ring B from right to left; 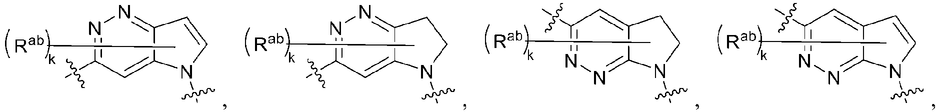 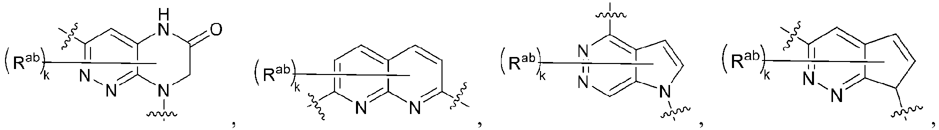 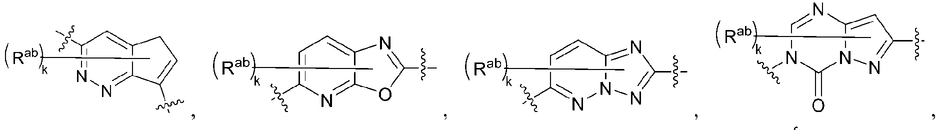 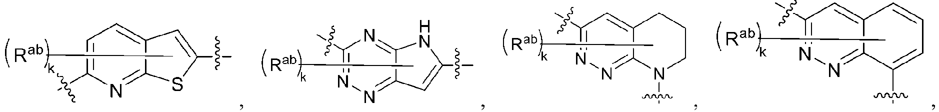 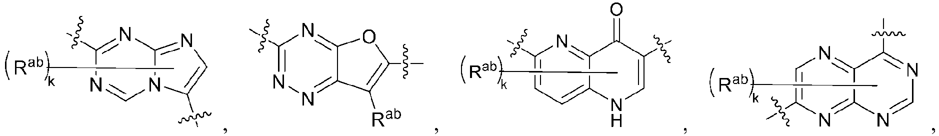 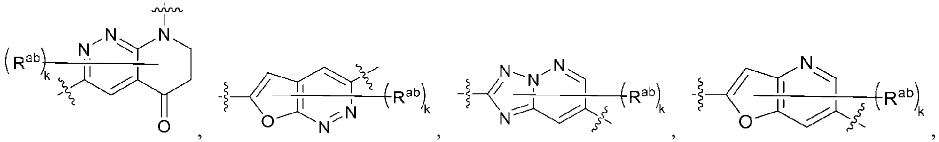 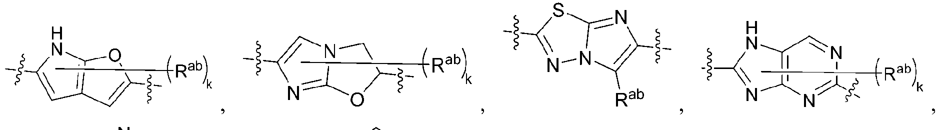 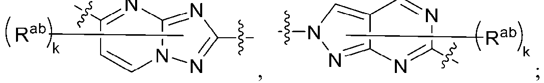
wherein, each R^{ab} is independently selected from: H, deuterium, halogen, -OH, -NH₂, -CN, oxo, methyl, ethyl, propyl, isopropyl, butyl;
each k is independently selected from: 0, 1, 2, 3, and 4.

8. The compound according to claim 1, wherein, 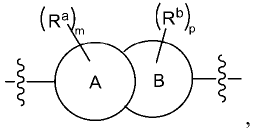 which relates to ring A and ring B, is selected from the following groups, wherein, the following bicyclic groups represent ring A and ring B from left to right or ring A and ring B from right to left;

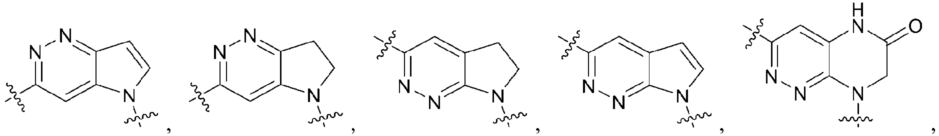

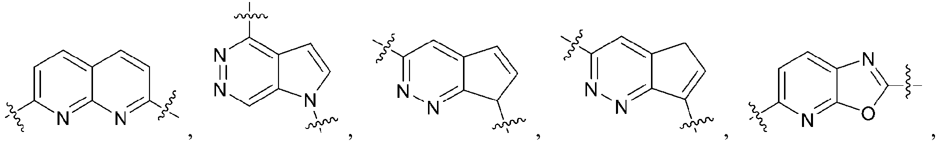

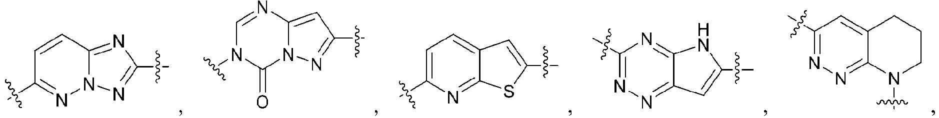

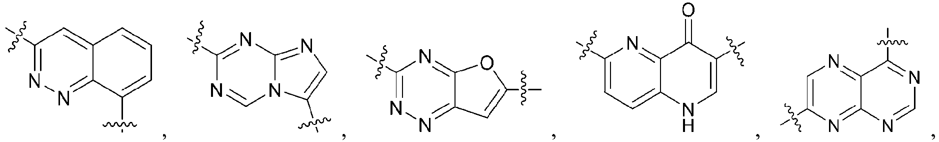

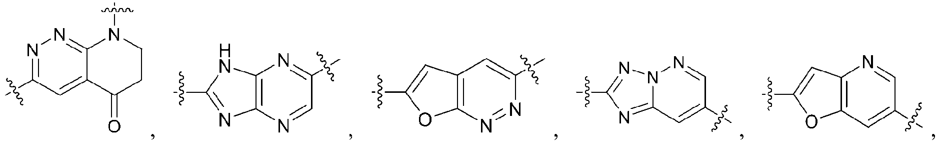

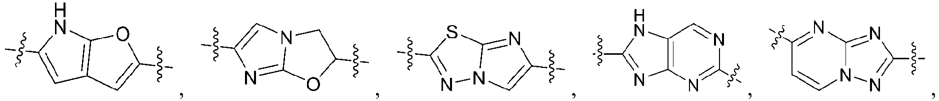

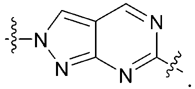

9. The compound according to claim 1, wherein, 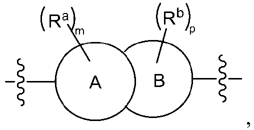 which relates to ring A and ring B, is selected from the following groups, wherein, the following bicyclic groups represent ring A and ring B from left to right or ring A and ring B from right to left;

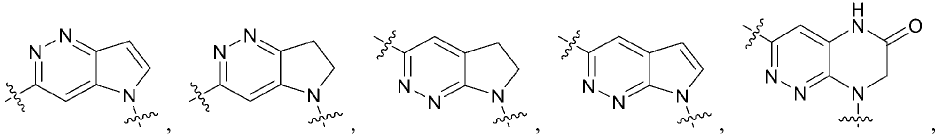

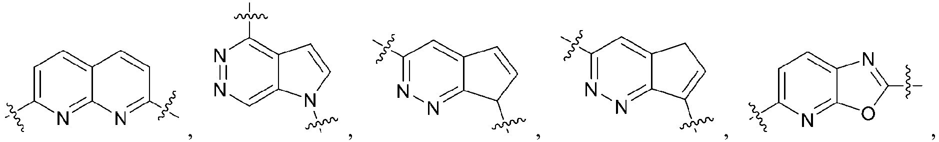

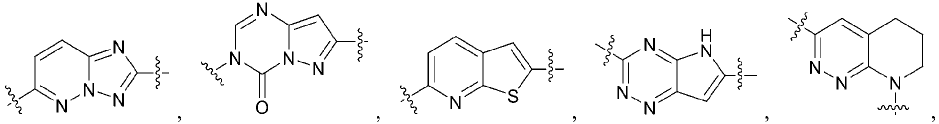

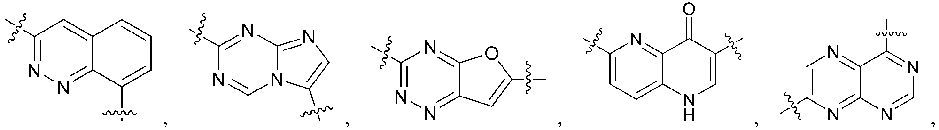

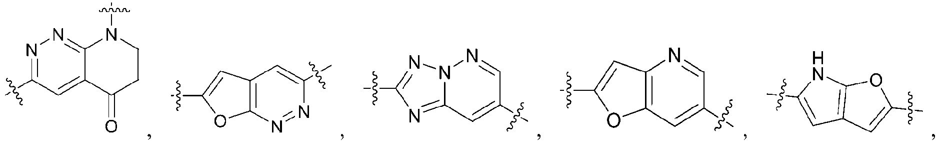

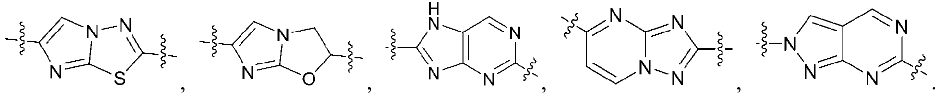

10. The compound according to any one of claims 1-9, wherein,
ring C is selected from: 6-10 membered aryl, 5-6 membered heteroaryl, phenyl fused 5-6 membered heteroaryl, phenyl fused 4-6 membered cycloalkyl, phenyl fused 4-6 membered heterocyclyl, 5-6 membered heteroaryl fused 5-6 membered heteroaryl, 5-6 membered heteroaryl fused 4-6 membered cycloalkyl, 5-6 membered heteroaryl fused 4-6 membered heterocyclyl, 5-6 membered cycloalkyl, and 5-6 membered heterocyclyl.

11. The compound according to any one of claims 1-10, wherein,
ring C is selected from: 6-10 membered aryl, 5-6 membered heteroaryl, phenyl fused 5-6 membered heteroaryl, phenyl fused 4-6 membered cycloalkyl, phenyl fused 4-6 membered heterocyclyl, nitrogen-containing 6-membered heteroaryl fused 5-6 membered heteroaryl, nitrogen-containing 6-membered heteroaryl fused 4-6 membered cycloalkyl, nitrogen-containing 6-membered heteroaryl fused 4-6 membered heterocyclyl, 5-6 membered cycloalkyl, and 5-6 membered heterocyclyl.

12. The compound according to any one of claims 1-11, wherein, 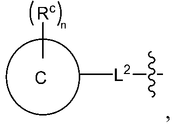 which relates to ring C, is selected from the following groups: 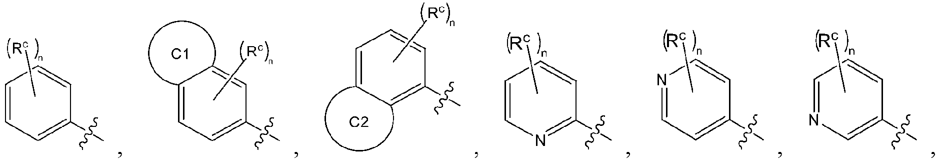 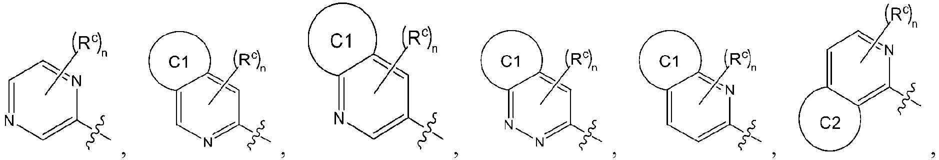 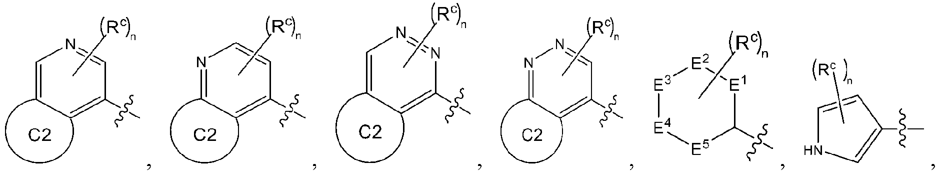 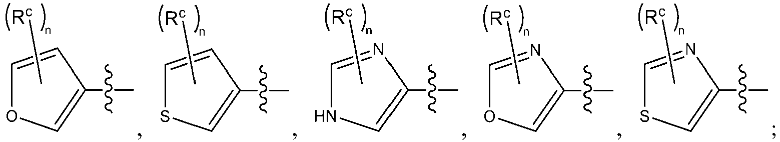 wherein,
ring C1 and ring C2 are each independently selected from: phenyl, 5-membered heteroaryl, 6-membered heteroaryl, 4-membered heterocyclyl, 5-membered heterocyclyl, 6-membered heterocyclyl, 4-membered cycloalkyl, 5-membered cycloalkyl, 6-membered cycloalkyl; preferably, ring C1 and ring C2 are each independently selected from: phenyl, pyridyl, pyrimidinyl, pyrazinyl, pyrrolyl, furyl, thienyl, imidazolyl, oxazolyl, thiazolyl, triazolyl, 2,3-dihydrofuranyl, 2,3-dihydrothienyl, 2,3-dihydropyrrolyl, 2,3-dihydrodioxinyl, cyclobutyl, cyclopentyl, cyclohexyl;
E¹, E², E³, E⁴, and E⁵ are each independently selected from: CH₂, O, S, and NH, provides that, at least one of E¹, E², E³, E⁴, and E⁵ is NH, and they form a stable ring.

13. The compound according to any one of claims 1-12, wherein, 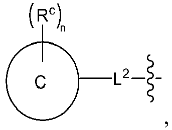 which relates to ring C, is selected from the following groups:

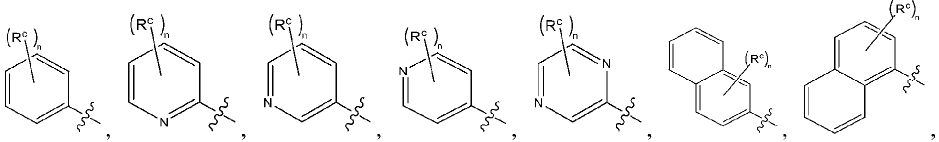

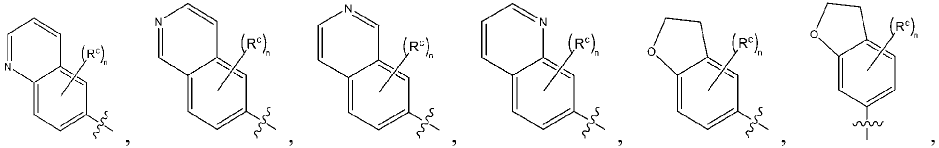

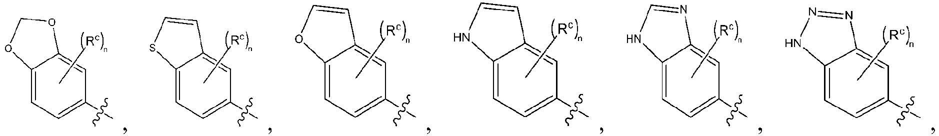

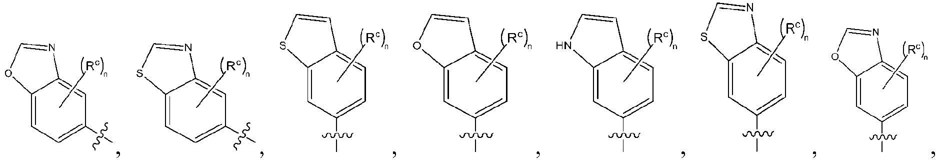

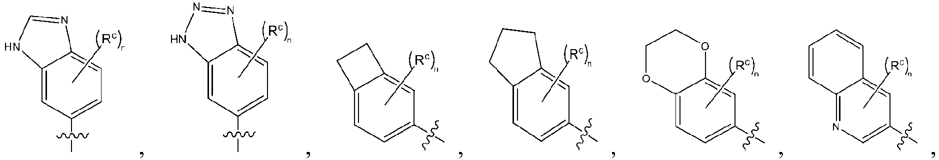

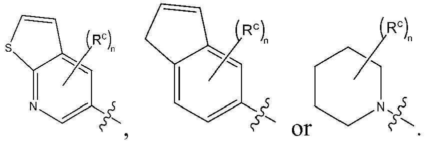

14. The compound according to any one of claims 1-13, wherein, 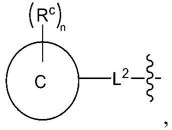 which relates to ring C, is selected from the following groups:

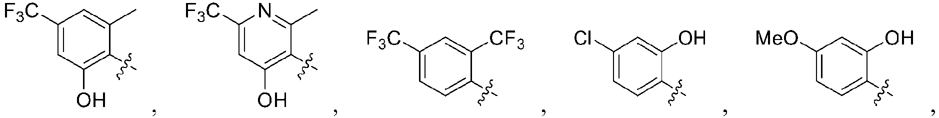

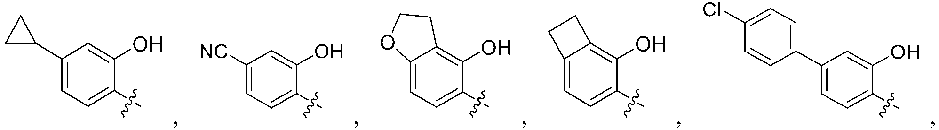

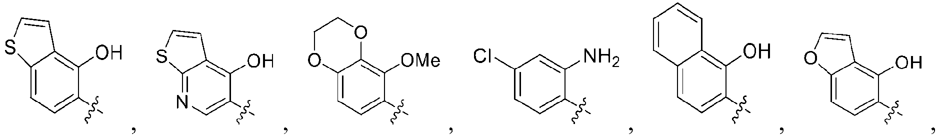

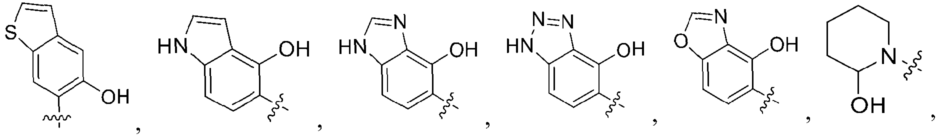

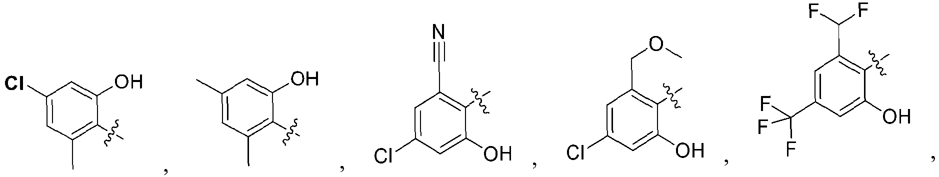

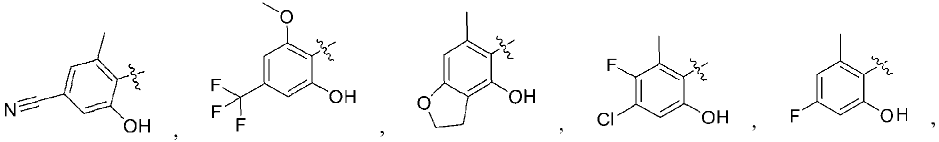

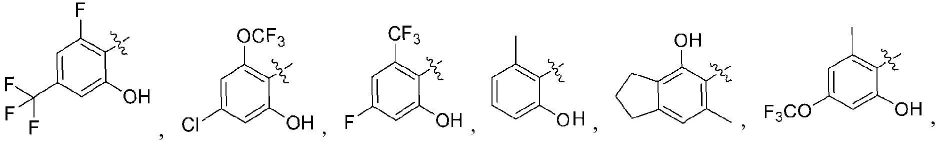

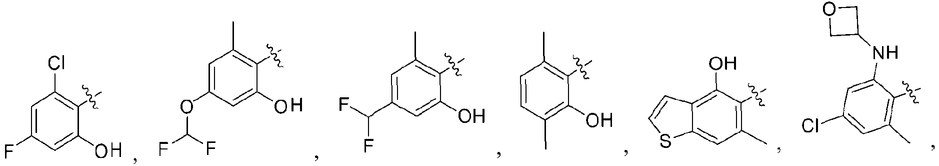

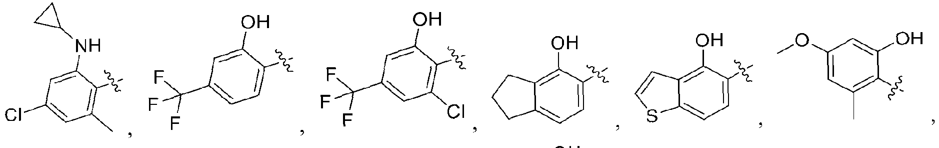

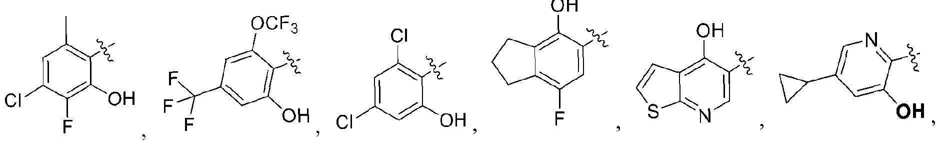

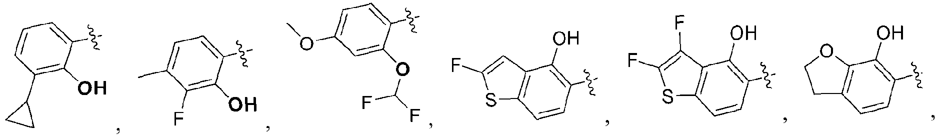

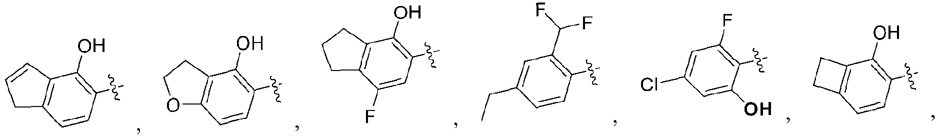

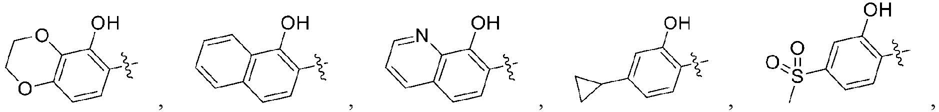

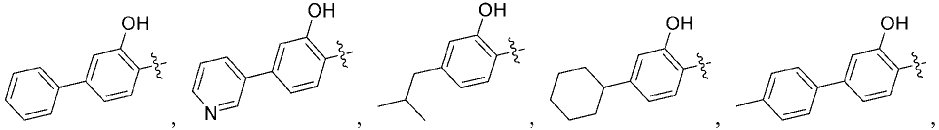

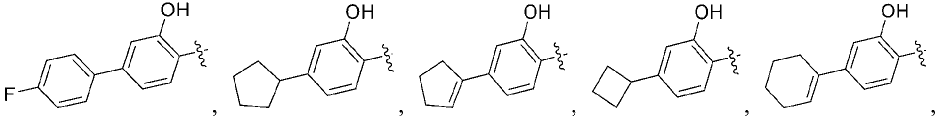

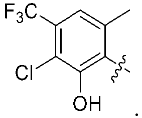

15. The compound according to any one of claims 1-5 and 10-14, wherein, each R^{a} and R^{b} are independently selected from: H, deuterium, F, Cl, Br, -OH, -NH₂, -CN, oxo, methyl, ethyl, trifluoromethyl, methoxy, n-propyl, isopropyl, dimethylamino, and acetyl.

16. The compound according to any one of claims 1-13 and 15, wherein,
each R^{c} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, hydroxyl C₁₋₄ alkyl, C₁₋₄ alkoxy-C₁₋₂ alkyl, R^{c1}S(O)₂-, -(CH₂)ₛ-N(R^{c1})(R^{c2}), 3-6 membered cycloalkyl, 3-6 membered heterocyclyl, and phenyl, the phenyl is optionally substituted with 1, 2, or 3 substituents selected from: deuterium, halogen, -OH, -NH₂, -CN, C₁₋₆ alkyl, C₁₋₆ haloalkyl, C₁₋₆ alkoxy; or
two R^{c} together with the C atom to which they are attached may form a 3-6 membered cycloalkyl, 4-7 membered heterocyclyl, 6 membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, C₁₋₆ haloalkyl;
optionally, each R^{c} is independently selected from: F, Cl, Br, -OH, -NH₂, -CN, methoxy, ethoxy, isopropoxy, methylamino, ethylamino, isopropylamino, methyl, ethyl, n-propyl, isopropyl, n-butyl, isobutyl, trifluoromethyl, difluoromethyl, 2,2,2-trifluoroethyl, pentafluoroethyl, trifluoromethoxy, difluoromethoxy, hydroxymethyl, aminoethyl, methoxymethyl, methoxyethyl, ethoxymethyl, cyclopropyl, cyclobutyl, cyclopentyl, cyclohexyl, cyclopentenyl, cyclobutenyl, cyclohexenyl, oxetanyl, phenyl, 4-chloro-phenyl, 2-chloro-4-fluoro-phenyl, 4-hydroxyphenyl, 4-methyl-phenyl, 4-fluoro-phenyl, pyridyl, cyclobutylamino, 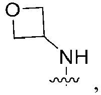 and methanesulfonyl; or two R^{c} together with the C atom to which they are attached form a cyclopentenyl, 2,3-dihydrofuranyl, cyclobutenyl, thienyl, 1,4-dioxanyl, pyridyl, cyclopenta-1,3-dienyl, phenyl, or dioxenyl.

17. The compound according to any one of claims 1-16, wherein,
each R², R⁵, and R⁶ are each independently selected from: H, deuterium, methyl, ethyl, propyl, and isopropyl;
each R³, R⁴, R⁷, and R⁸ are each independently selected from: H, deuterium, oxo, -OH, -NH₂, methyl, ethyl, propyl, and isopropyl;
each a and b are each independently 1 or 2.

18. The compound according to any one of claims 1-17, wherein,
L¹ is selected from: bond, -NR²-, -O-, -S-, -C(=O)-, -NR²-(CR³R⁴)ₐ-, -(CR³R⁴)ₐ-NR²-, -O-(CR³R⁴)ₐ-, - (CR³R⁴)ₐ-O-, -S-(CR³R⁴)ₐ-, and -(CR³R⁴)ₐ-S-; wherein a is 1 or 2;
L² is selected from: bond, -NR⁶-, -NR⁶-(CR⁷R⁸)_{b}-, -O-, and -S-.

19. The compound according to any one of claims 1-18, wherein,
each s, n, m, and p are each independently selected from: 0, 1, 2, and 3.

20. The compound according to any one of claims 1-4 and 6-19, wherein,
R¹ is selected from: H, -OH, -NH₂, -COOH, C₁₋₄ alkyl, C₁₋₄ alkoxy, NHR¹¹-C(=O)-, C₁₋₄ alkyl-C(=O)-O-, C_{1 4} alkyl-O-C(=O)-, 3-6 membered cycloalkyl, 3-6 membered monocyclic heterocyclyl, 6-11 membered spiro bicyclic heterocyclyl, 6-11 membered bridged bicyclic heterocyclyl, 6-11 membered fused bicyclic heterocyclyl, phenyl, naphthyl, and 5-10 membered heteroaryl; wherein the -NH₂, C₁₋₄ alkyl, C₁₋₄ alkoxy, NHR¹¹-C(=O)-, C₁₋₄ alkyl-C(=O)-O-, C₁₋₄ alkyl-O-C(=O)-, 3-6 membered cycloalkyl, 3-6 membered monocyclic heterocyclyl, 6-11 membered spiro bicyclic heterocyclyl, 6-11 membered bridged bicyclic heterocyclyl, 6-11 membered fused bicyclic heterocyclyl, phenyl, naphthyl, and 5-10 membered heteroaryl are each independently optionally further substituted with 1, 2, or 3 substituents selected from: H, deuterium, halogen, -OH, -NH₂, oxo, C₁₋₄ alkyl, hydroxyl C₁₋₄ alkyl, C₁₋₄ haloalkyl, C₁₋₄ alkoxy, C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -CH₂-C(=O)O-R^{c4}, -N(R^{c4})-C(=O)-R^{c3}, -CH₂-N(R^{c4})-C(=O)-R^{c4}, -C(=O)-N(R^{c6})(R^{c5}), -CH₂-C(=O)-N(R^{c4})(R^{c4}), -N(R^{c4})-C(=O)-N(R^{c6})(R^{c5}), -P(=O)R^{c5}R^{c6}, -CR^{c7}R^{c8}-CN, and -(CH₂)ₛ-R^{c9};
R¹¹ is selected from: H, deuterium, C₁₋₄ alkyl, and 3-6 membered monocyclic heterocyclyl.

21. The compound according to any one of claims 1-4 and 6-20, wherein,
R¹ is selected from: H, -OH, -NH₂, -COOH, methyl, ethyl, n-propyl, isopropyl, methoxy, ethoxy, n-propoxy, isopropoxy, NH₂-C(=O)-, CH₃NH-C(=O)-, CH₃CH₂NH-C(=O)-, CH₃-C(=O)-O-, CH₃CH₂-C(=O)-O-, CH₃-O-C(=O)-, CH₃CH₂-O-C(=O)-, cyclopropyl, cyclobutyl, cyclopentyl, cyclohexyl, azetidinyl, oxetanyl, tetrahydrofuranyl, pyrrolidinyl, piperidinyl, piperazinyl, morpholinyl, 1,4-dioxanyl, tetrahydro-2H-pyranyl, phenyl, naphthyl, pyridyl, pyrimidinyl, pyrazinyl, pyrrolyl, furyl, thienyl, imidazolyl, pyrazolyl, oxazolyl, isoxazolyl, thiazolyl, isothiazolyl, thiadiazolyl, triazolyl, quinolyl, and isoquinolyl, or selected from the following groups: 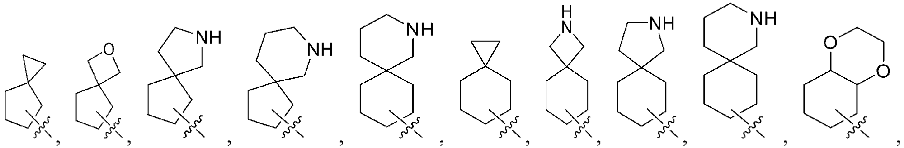 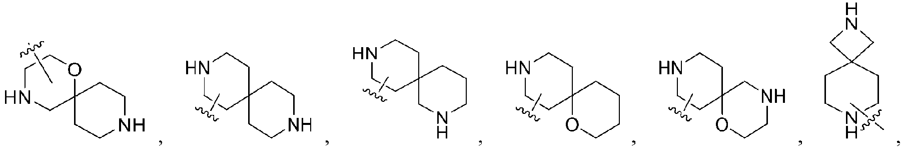 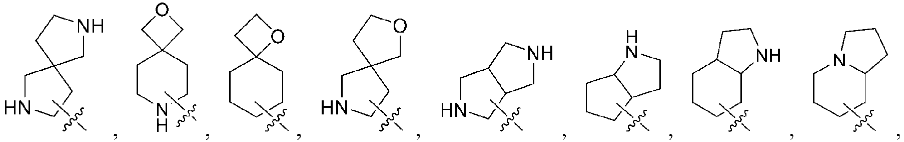 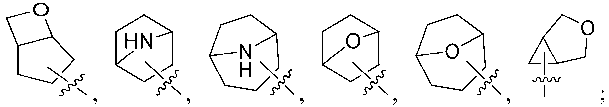
wherein, the methyl, ethyl, n-propyl, isopropyl, methoxy, ethoxy, n-propoxy, isopropoxy, NH₂-C(=O)-, CH₃NH-C(=O)-, CH₃CH₂NH-C(=O)-, CH₃-C(=O)-O-, CH₃CH₂-C(=O)-O-, CH₃-O-C(=O)-, CH₃CH₂-O-C(=O)-, cyclopropyl, cyclobutyl, cyclopentyl, cyclohexyl, azetidinyl, oxetanyl, tetrahydrofuranyl, pyrrolidinyl, piperidinyl, piperazinyl, morpholinyl, 1,4-dioxanyl, tetrahydro-2H-pyranyl, phenyl, naphthyl, pyridyl, pyrimidinyl, pyrazinyl, pyrrolyl, furyl, thienyl, imidazolyl, pyrazolyl, oxazolyl, isoxazolyl, thiazolyl, isothiazolyl, thiadiazolyl, triazolyl, quinolyl, isoquinolyl, 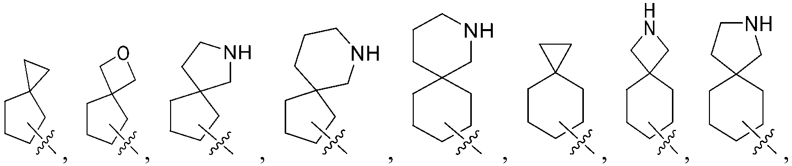 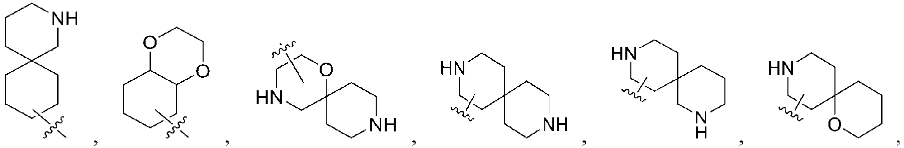 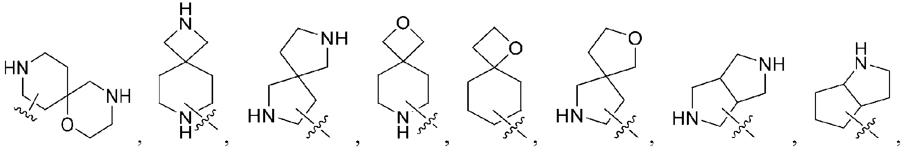 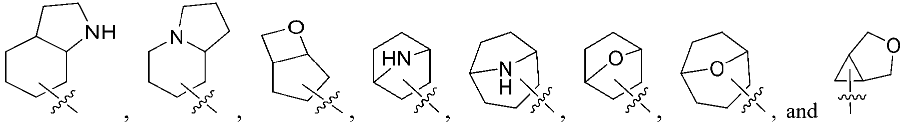 are each independently optionally substituted with 1, 2, or 3 substituents selected from: H, deuterium, halogen, -OH, -NH₂, oxo, methyl, ethyl, n-propyl, isopropyl, 3-6 membered monocyclic heterocyclyl, -C(=O)-R^{c3}, -S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R ^{c6})(R^{c5}), -N(R^{c4})-C(=O)-N(R^{c6})(R^{c5}), -P(=O)R^{c5}R^{c6}, and -CR^{c7}R^{c8}-CN.

22. The compound according to any one of claims 1-19, wherein,
R¹ is selected from: 3-6 membered cycloalkyl, 3-6 membered monocyclic heterocyclyl, and 6-11 membered bicyclic heterocyclyl; wherein the 3-6 membered cycloalkyl, 3-6 membered monocyclic heterocyclyl, and 6-11 membered bicyclic heterocyclyl are substituted with 1 or 2 substituents selected from: -C(=O)-R^{c3}, -S(O)-R^{c3}, - S(O)₂-R^{c3}, -C(=O)-N(R^{c6})(R^{c5}), and -P(=O)R^{c5}R^{c6}; and the 3-6 membered cycloalkyl, 3-6 membered monocyclic heterocyclyl, and 6-11 membered bicyclic heterocyclyl are optionally substituted with 1, 2 or 3 substituents selected from: H, deuterium, halogen, -OH, oxo, C₁₋₆ alkyl, C₁₋₆ alkoxy, C₁₋₆ alkoxy-C₁₋₄ alkyl, -(CH₂)ₛ-N(R^{c1})(R^{c2}), C₃₋₆ cycloalkyl, and 3-6 membered monocyclic heterocyclyl;
R^{c3} is selected from: C₁₋₆ alkyl and C₃₋₆ cycloalkyl, wherein the C₁₋₆ alkyl and C₃₋₆ cycloalkyl are further optionally substituted with 1-3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
R^{c5} and R^{c6} are selected from: H, deuterium, C₁₋₆ alkyl, and C₃₋₆ cycloalkyl; wherein the C₁₋₆ alkyl and C₃₋₆ cycloalkyl are further optionally substituted with 1-3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and - N(C₁₋₆ alkyl)(C₁₋₆ alkyl).

23. The compound according to any one of claims 1-19, wherein,
R¹ is selected from: cyclopentyl, cyclohexyl, azetidinyl, tetrahydropyrrolyl, piperidinyl, morpholinyl, piperazinyl, 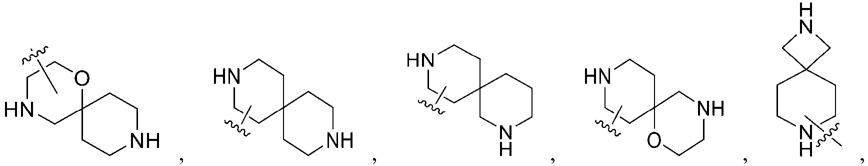 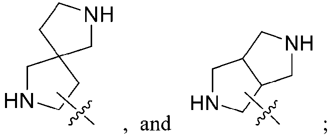 the cyclopentyl, cyclohexyl, azetidinyl, tetrahydropyrrolyl, piperidinyl, morpholinyl, piperazinyl, 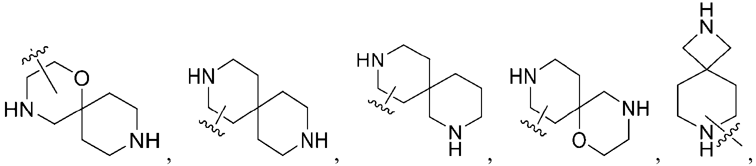 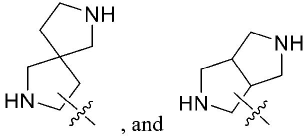 are substituted with 1 or 2 substituents selected from: -C(=O)-R^{c3}, -S(O)-R^{c3}, -S(O)₂-Rc³, -C(=O)-N(R^{c6})(R^{c5}), and -P(=O)R^{c5}R^{c6}, and the cyclopentyl, cyclohexyl, azetidinyl, tetrahydropyrrolyl, piperidinyl, morpholinyl, piperazinyl, 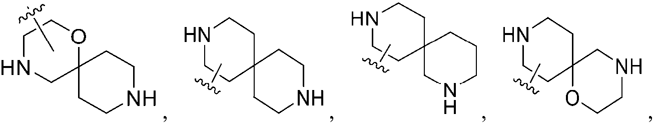 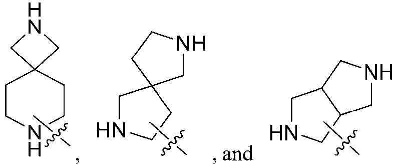 are further optionally substituted with 1, 2 or 3 substituents selected from: H, deuterium, halogen, -OH, oxo, C₁₋₆ alkyl, C₁₋₆ alkoxy, C₁₋₆ alkoxy-C₁₋₄ alkyl, -(CH₂)ₛ-N(R^{c1})(R^{c2}), C₃₋₆ cycloalkyl, and 3-6 membered monocyclic heterocyclyl;
preferably, R¹ is selected from: tetrahydropyrrolyl, piperidinyl, morpholinyl, piperazinyl, 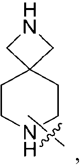 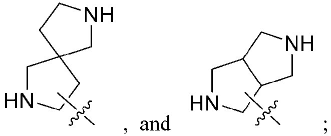 the tetrahydropyrrolyl, piperidinyl, morpholinyl, piperazinyl, 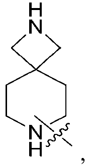 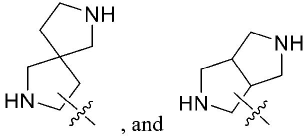 are substituted with 1 or 2 substituents selected from: -C(=O)-R^{c3}, -S(O)-R^{c3}, -S(O)₂-R^{c3}, -C(=O)-N(R^{c6})(R^{c5}), and -P(=O)R^{c5}R^{c6}, and the tetrahydropyrrolyl, piperidinyl, morpholinyl, piperazinyl, 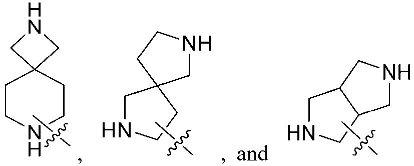 are further optionally substituted with 1, 2 or 3 substituents selected from: H, deuterium, halogen, -OH, oxo, C₁₋₆ alkyl, C₁₋₆ alkoxy, -(CH₂)ₛ-N(R^{c1})(R^{c2}), C₃₋₆ cycloalkyl, C₁₋₆ alkoxy-C₁₋₄ alkyl, and 3-6 membered monocyclic heterocyclyl;
more preferably, R¹ is selected from: tetrahydropyrrolyl, piperidinyl, morpholinyl, and piperazinyl; wherein the tetrahydropyrrolyl, piperidinyl, morpholinyl, and piperazinyl are substituted with 1 substituent selected from: -C(=O)-R^{c3}, -S(O)₂-R^{c3}, and -P(=O)R^{c5}R^{c6}, and the tetrahydropyrrolyl, piperidinyl, morpholinyl, and piperazinyl are further optionally substituted with 1 or 2 substituents selected from: deuterium, halogen, -OH, C₁₋₆ alkyl, C₁₋₆ alkoxy, and C₁₋₆ alkoxy-C₁₋₄ alkyl.

24. The compound according to any one of claims 1-19, wherein, R¹ is selected from:

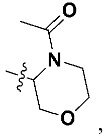

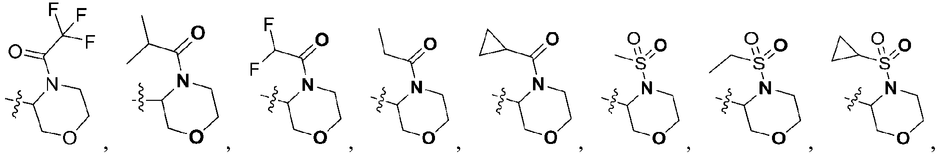

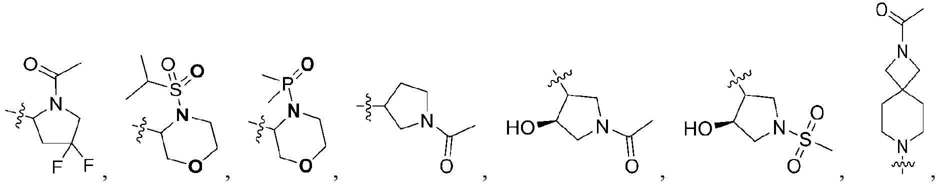

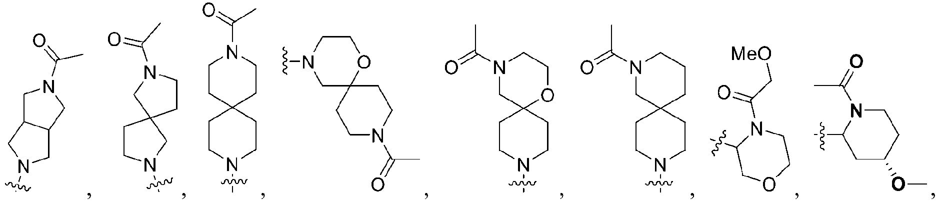

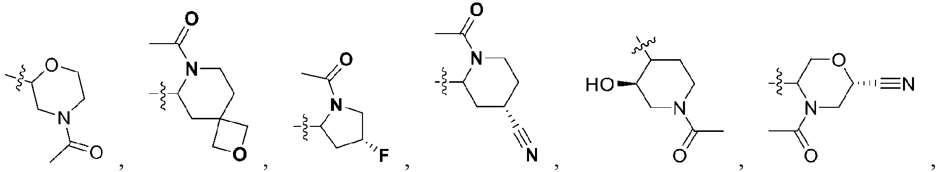

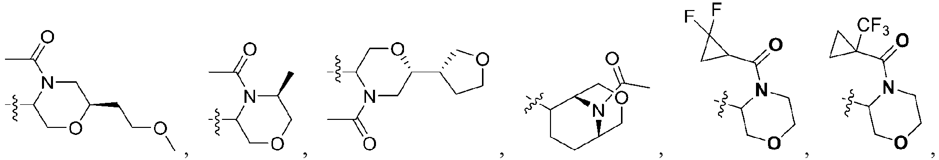

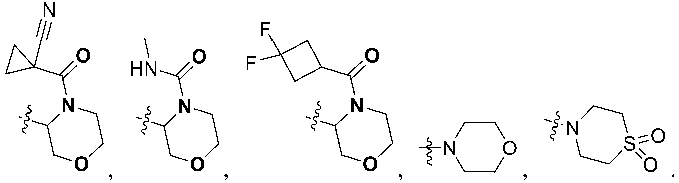

25. The compound according to any one of claims 1-4 and 6-24, wherein, the compound has the structure represented by Formula II,

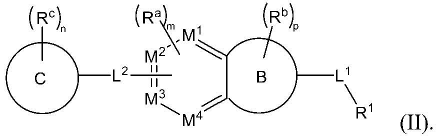

26. The compound according to any one of claims 1-4 and 6-25, wherein, the compound has the structure represented by Formula III,

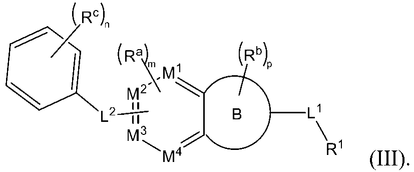

27. The compound according to any one of claims 1-4 and 6-26, wherein, the compound has the structure represented by Formula IV-1, IV-2, IV-3, IV-4, IV-5, IV-6, IV-7, IV-8, IV-9 or IV-10,

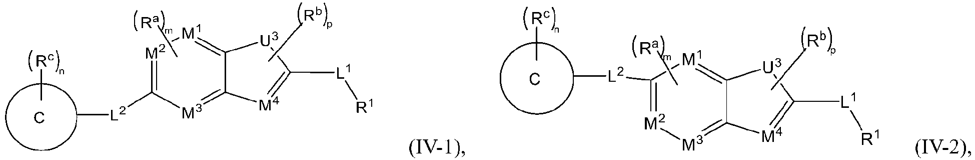

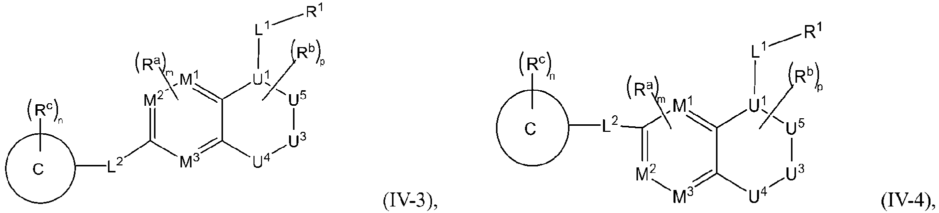

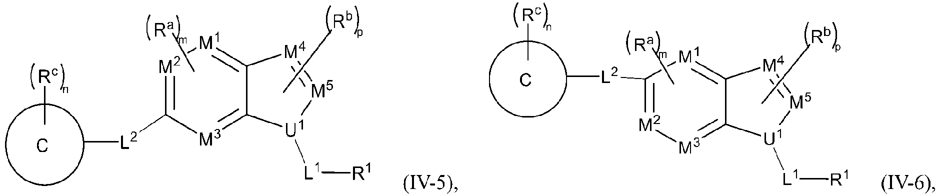

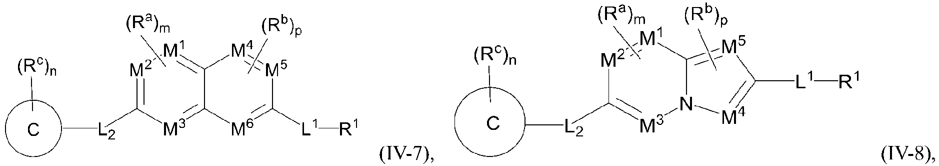

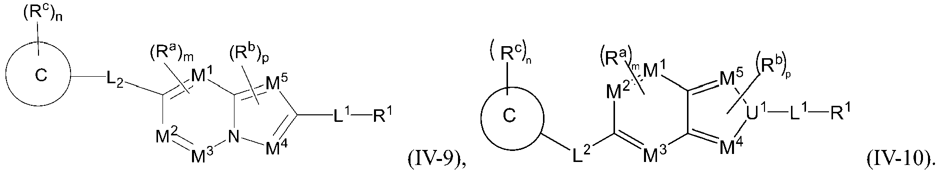

28. The compound according to any one of claims 1-4 and 6-27, wherein, the compound has the structure represented by Formula V-1, V-2, V-3, V-4, V-5, V-6, V-7, V-8, V-9 or V-10,

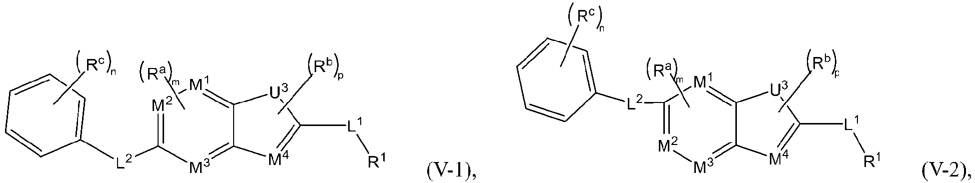

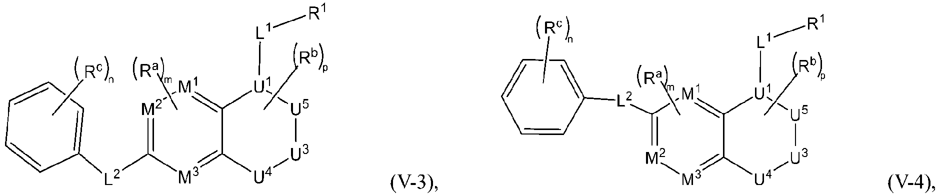

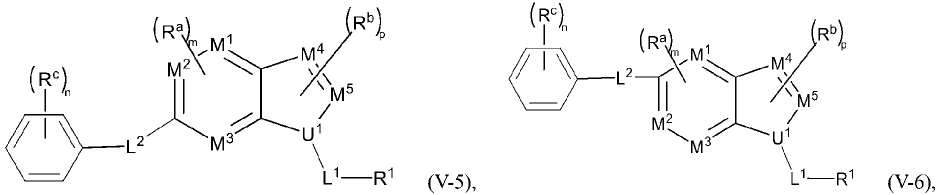

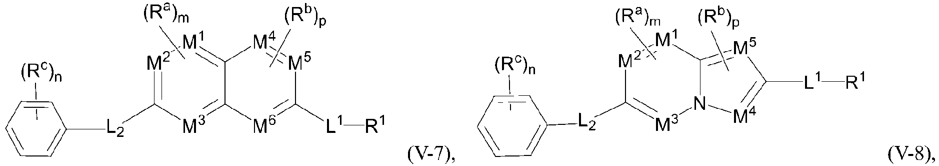

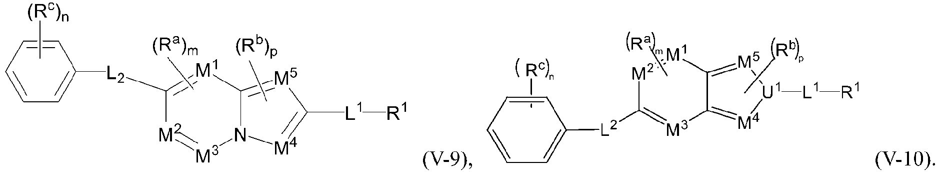

29. The compound according to any one of claims 1-4 and 6-28, wherein, the compound has the structure represented by Formula VI-1, VI-2, VI-3, VI-4, VI-5, VI-6, VI-7, VI-8, VI-9 or VI-10, 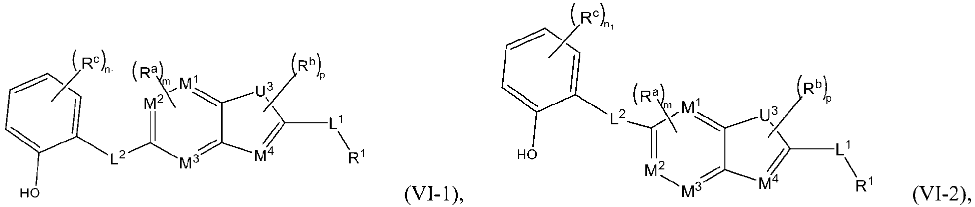 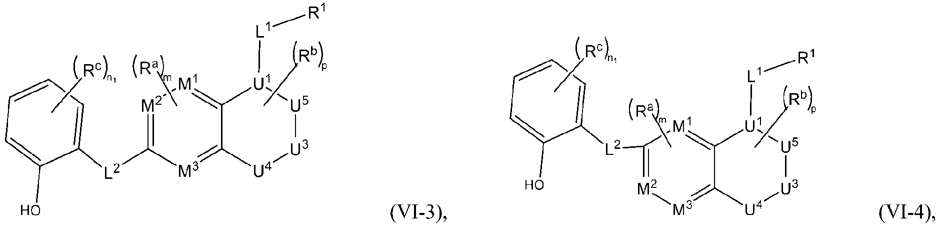 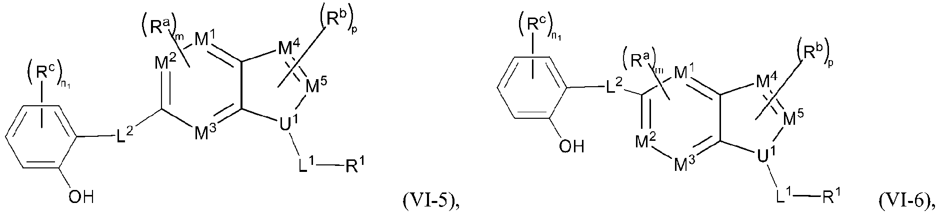 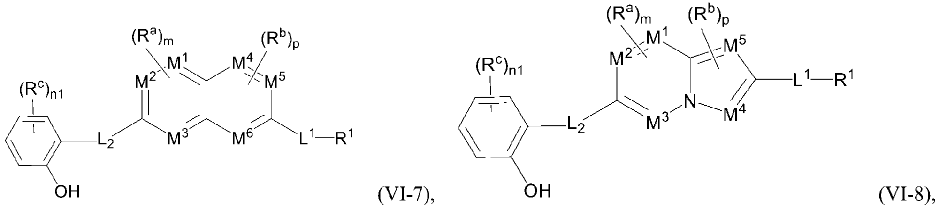 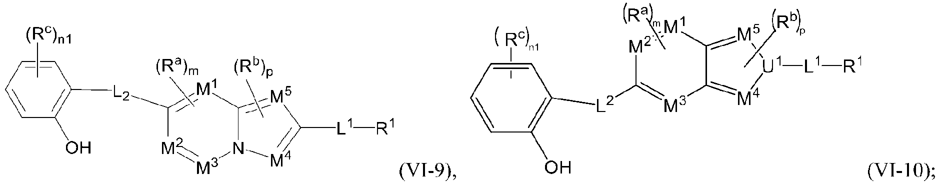 wherein, n₁ is 0, 1, 2 or 3.

30. The compound according to any one of claims 1-4 and 6-29, wherein, the compound has the structure represented by Formula VII-1, VII-2, VII-3, VII-4, VII-5, VII-6, VII-7, VII-8, VII-9 or VII-10, 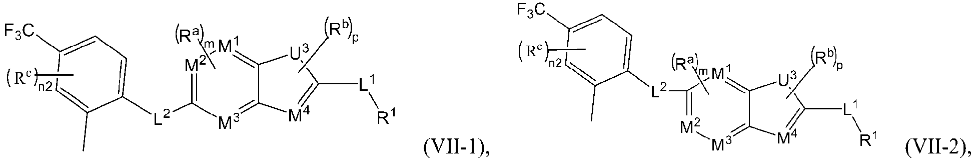 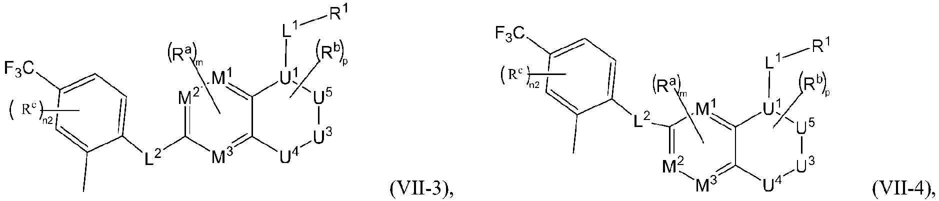 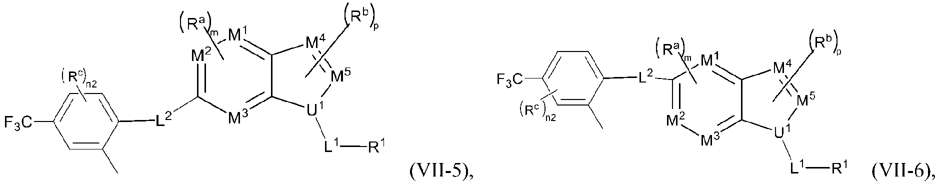 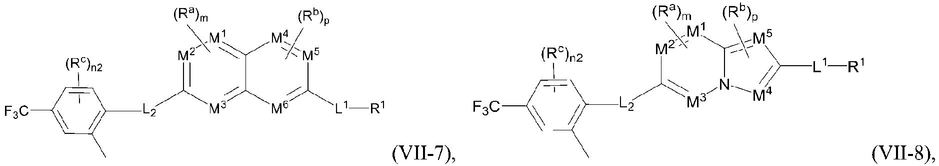 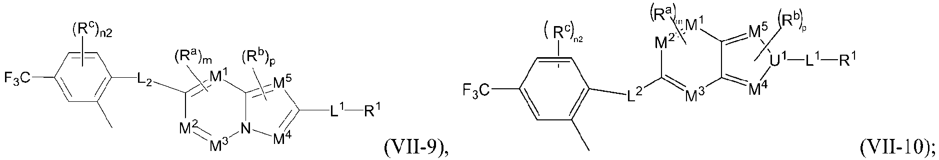 wherein, n₂ is 0, 1 or 2.

31. The compound according to any one of claims 1-4 and 6-30, wherein, the compound has the structure represented by Formula VIII-1, VIII-2, VIII-3, VIII-4, VIII-5, VIII-6, VIII-7, VIII-8, VIII-9, VIII-10, VIII-11 or VIII-12,

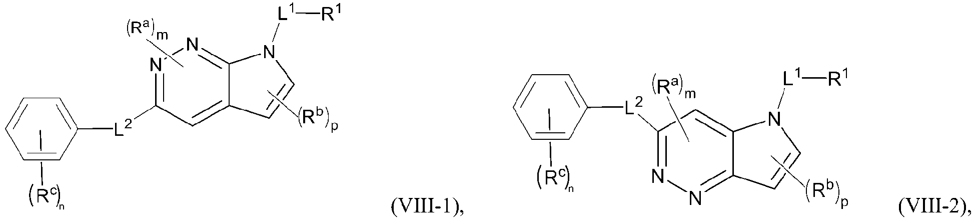

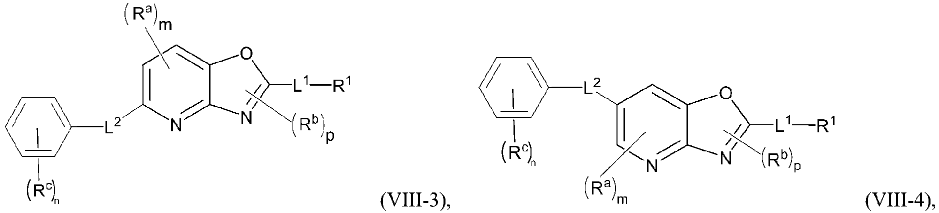

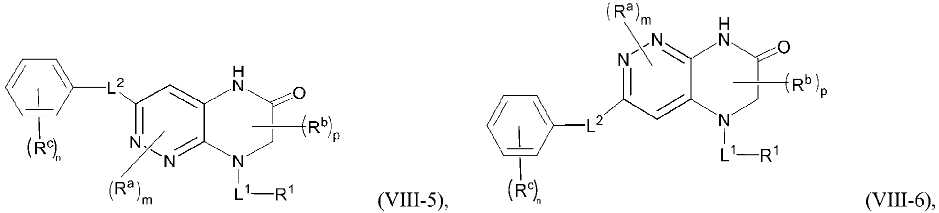

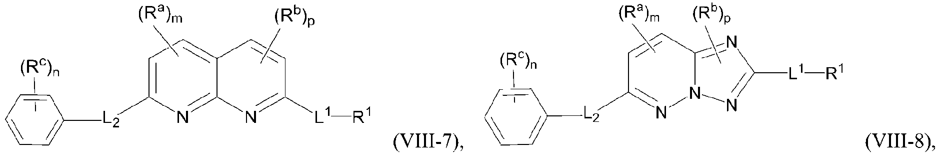

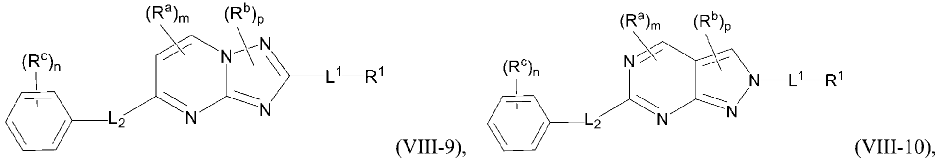

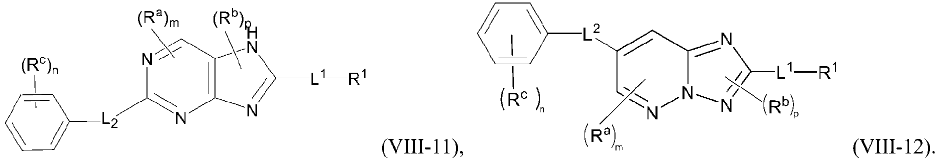

32. The compound according to any one of claims 1-4 and 6-31, wherein, the compound has the structure represented by Formula IX-1, IX-2, IX-3, IX-4, IX-5 or IX-6,

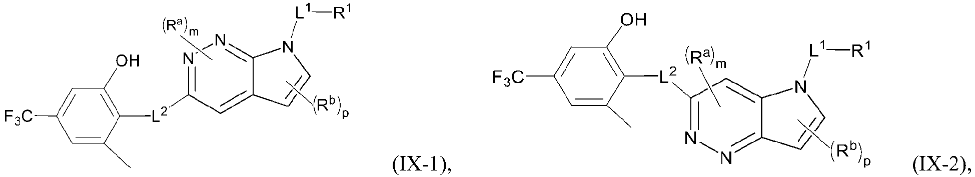

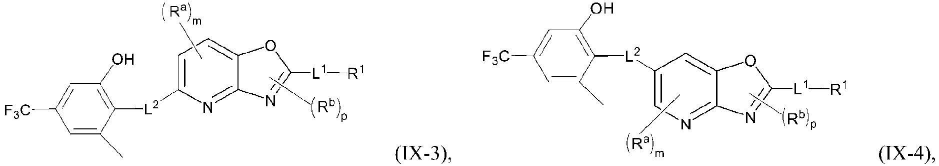

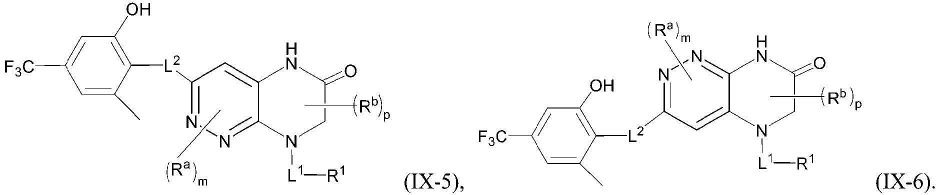

33. A compound represented by Formula VIII-1 or VIII-2, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof, 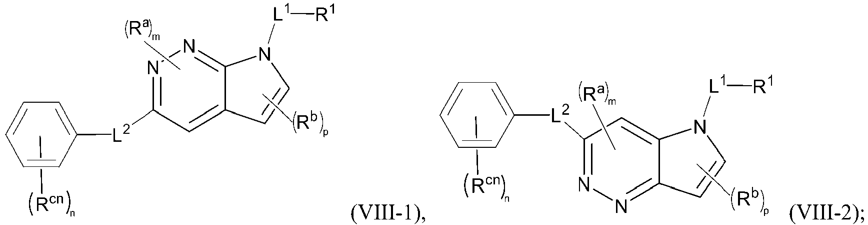 wherein,
each R^{cn} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, -(CH₂)ₛ-N(R^{c1})(R^{c2}), 3-6 membered cycloalkyl, 3-6 membered heterocyclyl, phenyl, and 5-6 membered heteroaryl; wherein the 3-6 membered cycloalkyl, 3-6 membered heterocyclyl, phenyl, and 5-6 membered heteroaryl are optionally substituted with 1, 2 or 3 substituents selected from: deuterium, halogen, -OH, -NH₂, -CN, C₁₋₆ alkyl, C₁₋₆ haloalkyl, and C₁₋₆ alkoxy; or two R^{cn} together with the C atom to which they are attached may form a 3-6 membered cycloalkyl, 4-7 membered heterocyclyl, 6-membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
each R^{a} and R^{b} are independently selected from: H, deuterium, halogen, -OH, -NH₂, -CN, C₁₋₆ alkyl, C₁₋₆ haloalkyl, C₁₋₆ alkoxy, C₁₋₆ alkoxy-C₁₋₆ alkyl, C₁₋₆ haloalkoxy, hydroxyl C₁₋₆ alkyl, -NH(C₁₋₆ alkyl), -N(C₁₋₆ alkyl)(C₁₋₆ alkyl), -C(=O)-(C₁₋₆ alkyl), and 3-6 membered heterocyclyl optionally substituted by hydroxyl;
L¹ is selected from: bond, C₃₋₆ cycloalkylene, -NR²-, -O-, -S-, -C(=O)-, -(CR³R⁴)ₐ-, -NR²-(CR³R⁴)ₐ-, - (CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, - (CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, - NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, -C(=O)-(CR³R⁴)ₐ-NR²-, -NR²-(CR³R⁴)ₐ C(=O)-NR⁵-, -NR²-C(=O)-(CR³R⁴)ₐ-NR⁵-, -C(=S)-NR²-, and -NR²-C(=S)-;
L² is selected from: bond, -NR⁶-, -O-, -S-, -NR⁶-(CR⁷R⁸)_{b}-, -(CR⁷R⁸)_{b}-NR⁶-, -O-(CR⁷R⁸)_{b}-, -(CR⁷R⁸)_{b}-O-, - S-(CR⁷R⁸)_{b}-, and -(CR⁷R⁸)_{b}-S-;
each R², R⁵, R⁶, R⁹ and R¹⁰ are each independently selected from: H, deuterium, and C₁₋₆ alkyl;
each R³, R⁴, R⁷, and R⁸ are each independently selected from: H, deuterium, halogen, oxo, -OH, -NH₂, and C₁₋₆ alkyl;
R¹ is selected from: H, -OH, -NH₂, -COOH, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the -NH₂, C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl are each independently optionally substituted with 1, 2, or 3 substituents selected from: H, deuterium, halogen, -OH, oxo, - CN, C₁₋₆ alkyl, hydroxyl C₁₋₆ alkyl, C₁₋₆ haloalkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, 5-6 membered heteroaryl, -C(=O)-R^{c3}, -S(O)-R^{c3}, -S(O)₂-R^{c3}, -C(=O)O-R^{c3}, -N(R^{c4})-C(=O)-R^{c3} -C(=O)-N(R^{c6})(R^{c5}), -CH₂-C(=O)-N(R^{c4})(R^{c4}), and -N(R^{e4})-C(=O)-N(R^{c6})(R^{c5});
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: H, deuterium, C₁₋₆ alkyl, C₁₋₆ haloalkyl, and C₃₋₆ cycloalkyl, wherein the C₃₋₆ cycloalkyl is optionally substituted with 1, 2, or 3 substituents selected from: deuterium, -OH, halogen, and C₁₋₆ alkyl;
R^{c4} is selected from: H, deuterium, and C₁₋₆ alkyl;
R^{c5} and R^{c6} are selected from: H, deuterium, C₁₋₆ alkyl, C₁₋₆ haloalkyl, and C₃₋₆ cycloalkyl, or R^{c5}, R^{c6} together with the N atom to which they are attached form a 4-6 membered heterocyclyl; wherein the C₃₋₆ cycloalkyl and 4-6 membered heterocyclyl are optionally substituted with 1-3 substituents selected from: deuterium, -OH, halogen, and C₁₋₆ alkyl;
R¹¹ is selected from: H, deuterium, C₁₋₆ alkyl, and 3-6 monocyclic heterocyclyl;
each a and b are each independently 1, 2, 3, or 4;
each m is 0 or 1;
each p is 0, 1 or 2;
each s and n are each independently selected from: 0, 1, 2, 3, and 4;
provided that both 1) and 2) below are met:
1) 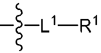 is not H, methyl, ethyl, CHF₂ or Boc;
2) the compound is not

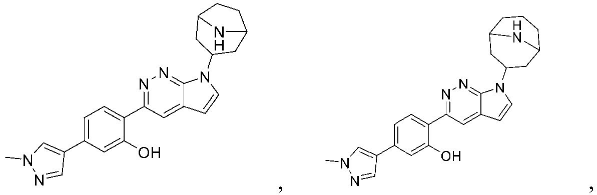

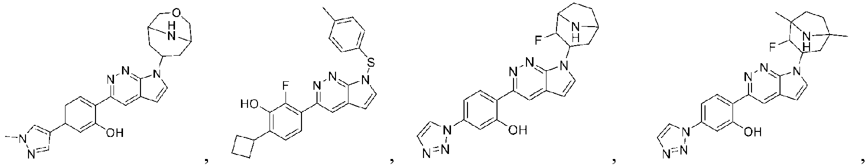

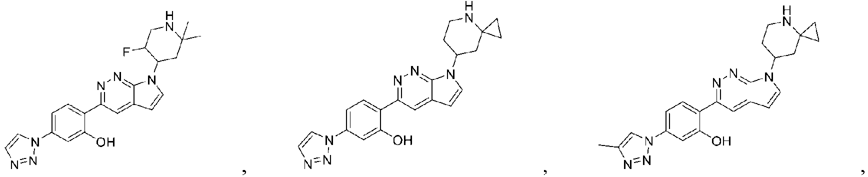

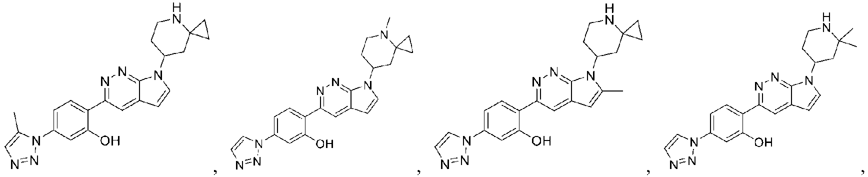

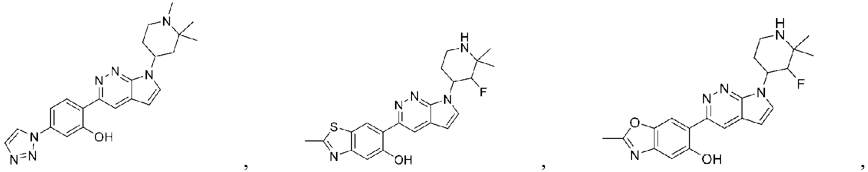

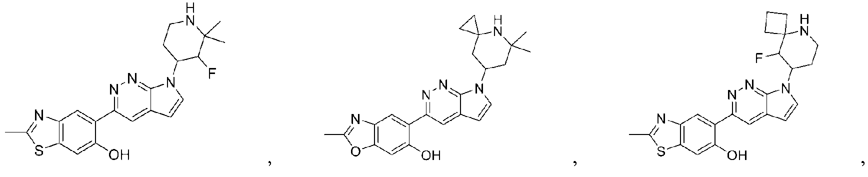

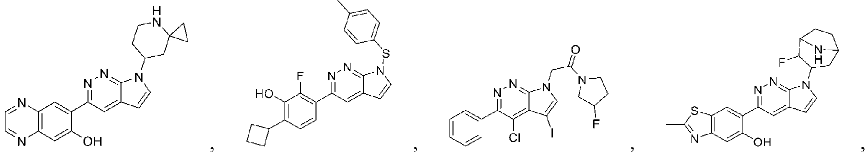

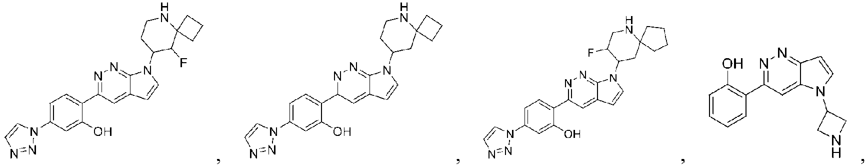

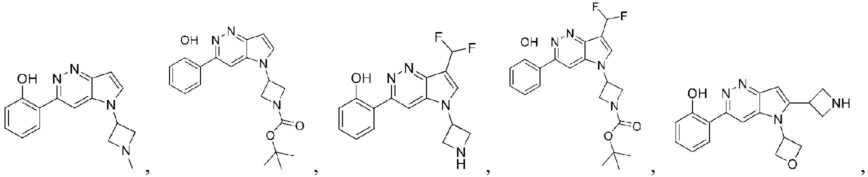

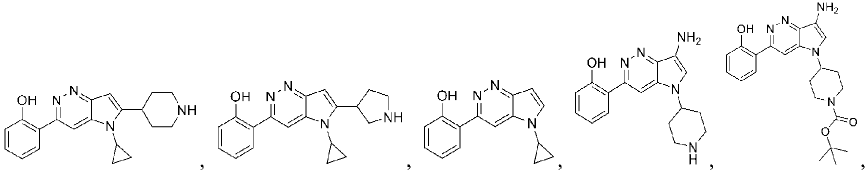

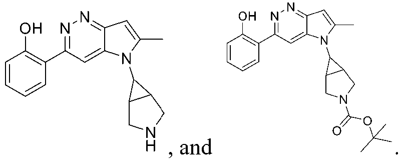

34. The compound according to claim 33, wherein, the compound has the structure represented by Formula VIII-1,

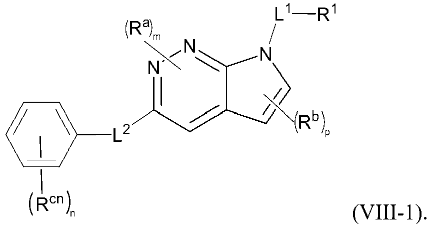

35. The compound according to claim 33 or 34, wherein, L² is bond.

36. The compound according to any one of claims 33-35, wherein, R^{a} and R^{b} are each independently selected from: H, deuterium, F, Cl, Br, -CN, methyl, ethyl, trifluoromethyl, methoxy, dimethylamino, and acetyl; preferably, R^{a} and R^{b} are each independently selected from: H, deuterium, F, Cl, Br, methyl, and ethyl; more preferably, R^{a} and R^{b} are each independently selected from: H, deuterium, and methyl.

37. The compound according to any one of claims 33-36, wherein, each R^{cn} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, hydroxyl C₁₋₄ alkyl, C₁₋₄ alkoxy-C₁₋₂ alkyl, -(CH₂)ₛ-N(R^{c1})(R^{c2}), 3-6 membered cycloalkyl, 3-6 membered heterocyclyl, and phenyl; the 3-6 membered cycloalkyl, 3-6 membered heterocyclyl, and phenyl are optionally substituted with 1, 2 or 3 substituents selected from: deuterium, halogen, -OH, -NH₂, -CN, C₁₋₆ alkyl, C₁₋₆ haloalkyl, and C₁₋₆ alkoxy; or two R^{cn} together with the C atom to which they are attached may form a 3-6 membered cycloalkyl, 4-7 membered heterocyclyl, or 5-6 membered heteroaryl;
preferably, each R^{cn} is independently selected from: F, Cl, Br, -OH, -NH₂, -CN, methoxy, ethoxy, isopropoxy, methylamino, ethylamino, isopropylamino, methyl, ethyl, n-propyl, isopropyl, trifluoromethyl, difluoromethyl, 2,2,2-trifluoroethyl, pentafluoroethyl, trifluoromethoxy, difluoromethoxy, hydroxymethyl, aminoethyl, methoxymethyl, methoxyethyl, ethoxymethyl, cyclopropyl, cyclobutyl, cyclopentyl, oxetanyl, phenyl, 4-chloro-phenyl, 2-chloro-4-fluoro-phenyl, 4-hydroxyphenyl, cyclobutylamino, and 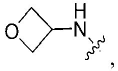 or two R^{cn} together with the C atom to which they are attached form a cyclopentyl or tetrahydrofuranyl;
more preferably, each R^{cn} is independently selected from: F, Cl, Br, -OH, methoxy, ethoxy, methyl, ethyl, trifluoromethyl, difluoromethyl, trifluoromethoxy, and difluoromethoxy.

38. The compound according to any one of claims 33-37, wherein, 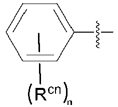 is selected from: 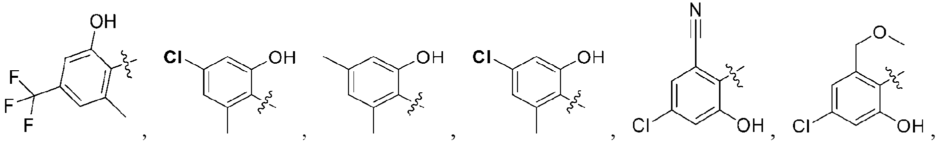 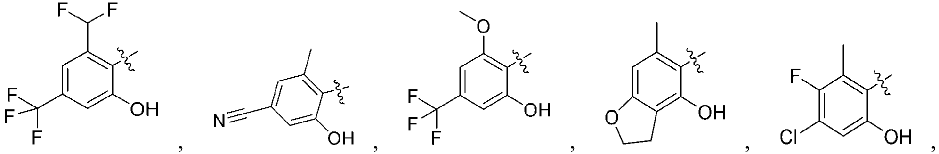 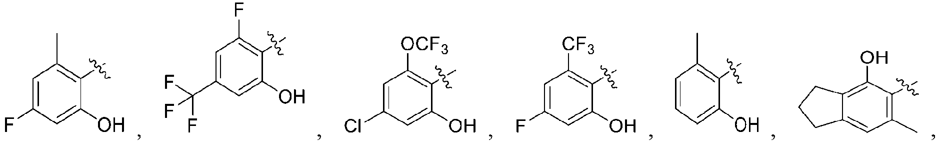 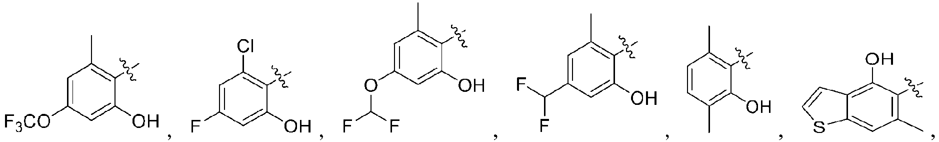 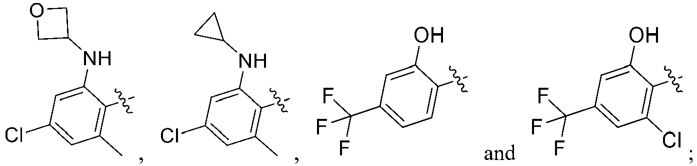 preferably, wherein 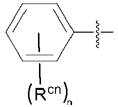 is selected from: 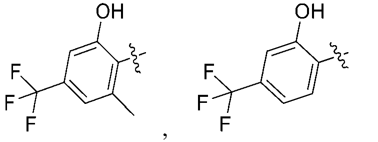 and

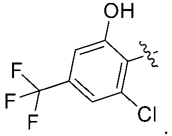

39. The compound according to any one of claims 33-38, wherein, L¹ is selected from: bond, C₃₋₆ cycloalkylene, -NR²-, -O-, -S-, -C(=O)-, -(CR³R⁴)ₐ-, -NR²-(CR³R⁴)ₐ-, -(CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, -(CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, -NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, and -C(=O)-(CR³R⁴)ₐ-NR²-;
preferably, L¹ is selected from: bond, C₃₋₆ cycloalkylene, -C(=O)-, -(CR³R⁴)ₐ-, -(CR³R⁴)ₐ-(CR⁹=CR¹⁰)-, - (CR³R⁴)ₐ-C(=O)-NR²-, and -(CR³R⁴)ₐ-NR²-C(=O)-.

40. The compound according to claim 39, wherein,
each R² is each independently selected from: H, deuterium, and C₁₋₄ alkyl;
each R³, and R⁴ are each independently selected from: H, deuterium, halogen, -OH, and C₁₋₄ alkyl.

41. The compound according to any one of claims 33-40, wherein, L¹ is selected from: bond, methylene, - C(=O)-, cyclopropylene, 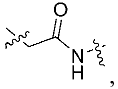 n-propylene, n-butylene, ethylene, 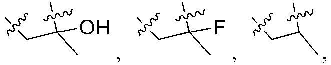 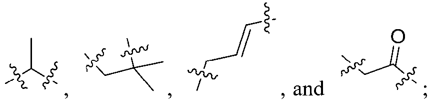 preferably, L¹ is selected from: bond, methylene, -C(=O)-, and cyclopropylene.

42. The compound according to any one of claims 33-41, wherein, R¹ is selected from: C₁₋₆ alkyl, C₁₋₆ alkoxy, NHR¹¹-C(=O)-, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the C₁₋₆ alkyl, C₁₋₆ alkoxy, C₁₋₆ alkyl-C(=O)-O-, C₁₋₆ alkyl-O-C(=O)-, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: H, deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -N(R^{c4})-C(=O)-R^{c3} - C(=O)-N(R^{c6})(R^{c5}), and -N(R^{c4})-C(=O)-N(R^{c6})(R^{c5});
preferably, R¹ is selected from: C₁₋₃ alkyl, NHR¹¹-C(=O)-, 3-10 membered cycloalkyl, 3-6 membered monocyclic heterocyclyl, 6-11 membered bicyclic heterocyclyl, 6-10 membered aryl, and 5-10 membered heteroaryl; wherein the C₁₋₃ alkyl, 3-10 membered cycloalkyl, 3-6 membered monocyclic heterocyclyl, 6-11 membered bicyclic heterocyclyl, 6-10 membered aryl, and 5-10 membered heteroaryl are each independently optionally further substituted with 1, 2, or 3 substituents selected from: deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, - C(=O)-R^{c3}, -S(O)₂-R^{c3}, -C(=O)O-R^{c3}, -N(R^{c4})-C(=O)-R^{c3}, -C(=O)-N(R^{c6})(R^{c5}), and -N(R^{c4})-C(=O)-N(R^{c6})(R^{c5});
more preferably, R¹ is selected from: methyl, ethyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, triazolyl, -C(=O)-NH₂, 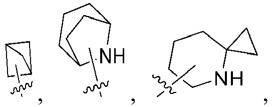 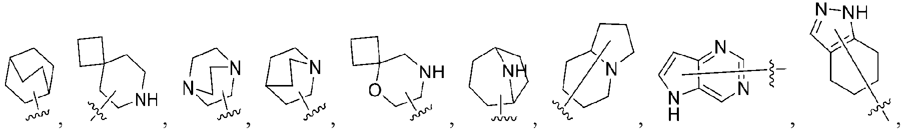 and 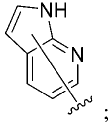 wherein the methyl, ethyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, triazolyl, 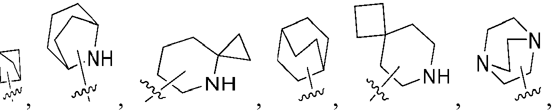 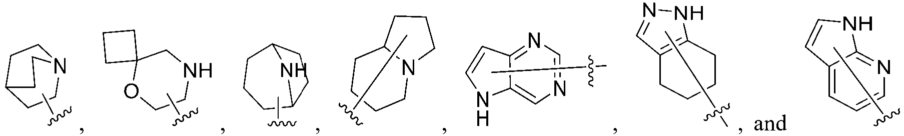 are each independently optionally further substituted with 1, 2, or 3 substituents selected from: deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, -C(=O)-R^{c3}, -S(O)₂-R^{c3}, -C(=O)O-R^{c3}, -N(R^{c4})-C(=O)-R^{c3}, -C(=O)-N(R^{c6})(R^{c5}), and -N(R^{c4})-C(=O)-N(R^{c6})(R^{c5});
more preferably, R¹ is selected from: methyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, and triazolyl; wherein, the methyl, isopropyl, pyrrolidinyl, tetrahydrofuranyl, tetrahydrothienyl, tetrahydrothiopyranyl, tetrahydropyranyl, thietanyl, oxetanyl, azetidinyl, piperidinyl, morpholinyl, piperazinyl, cyclobutyl, cyclopentyl, cyclohexyl, n-heptyl, phenyl, pyridyl, pyrimidyl, pyrrolyl, imidazolyl, oxazolyl, isoxazolyl, thiazolyl, pyrazolyl, furyl, and triazolyl are each independently optionally further substituted with 1, 2, or 3 substituents selected from: deuterium, halogen, -OH, oxo, -CN, C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, -C(=O)-R^{c3}, -S(O)₂-R^{c3}, -C(=O)O-R^{c3}, -N(R^{c4})-C(=O)-R^{c3} -C(=O)-N(R^{c6})(R^{c5}), and -N(R^{c4})-C(=O)-N(R^{c6})(R^{c5}).

43. The compound according to any one of claims 33-42, wherein,
-L¹-R¹ is selected from: 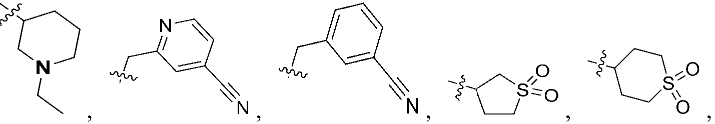 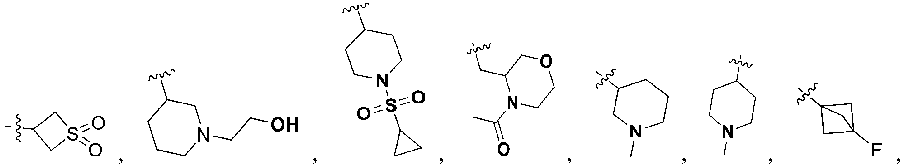 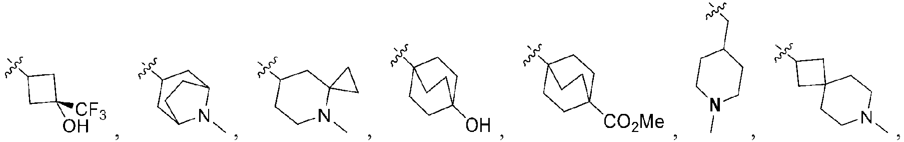 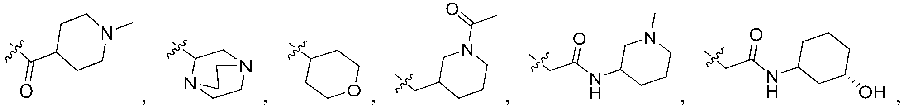 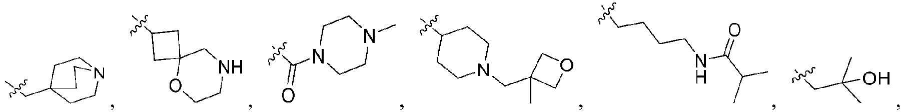 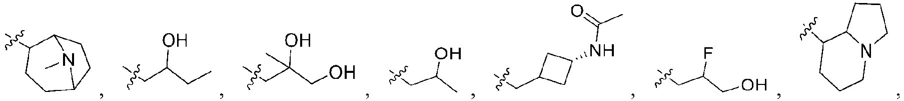 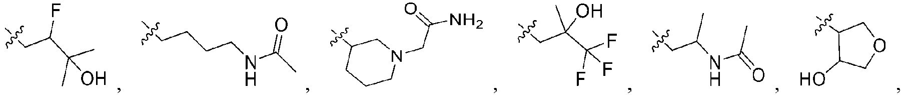 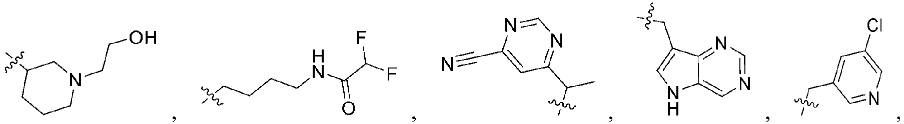 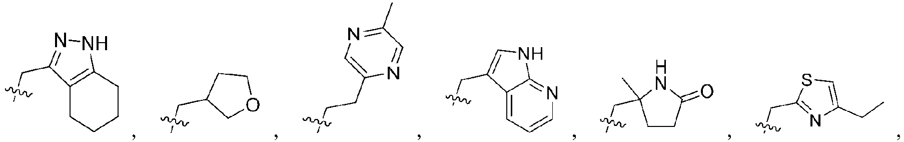 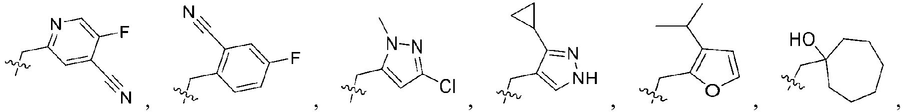 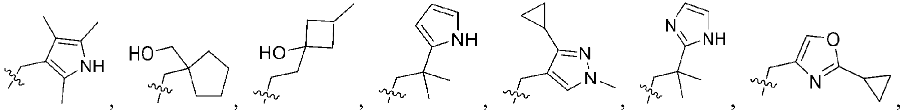 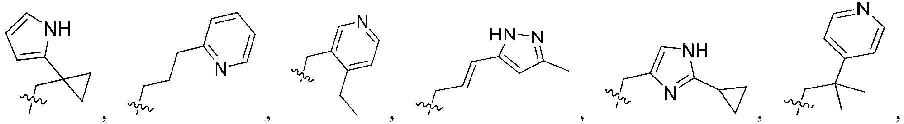 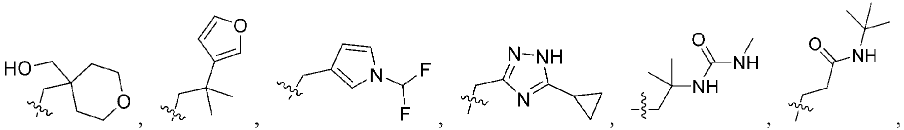 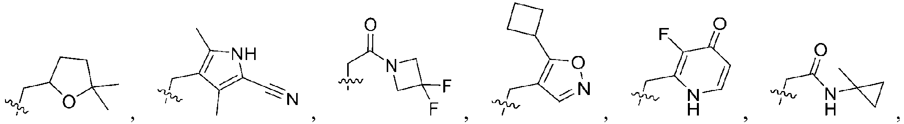 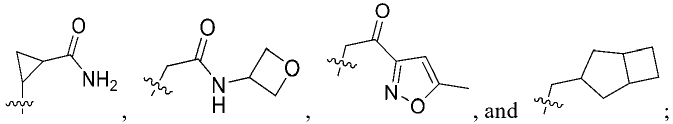
preferably, -L¹-R¹ is selected from: 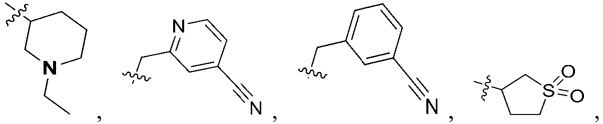 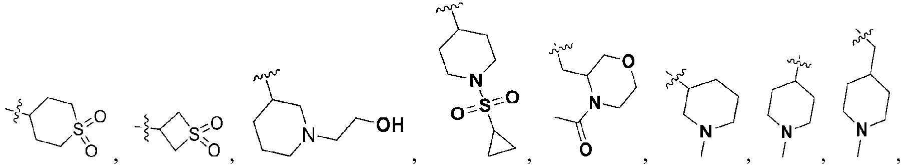 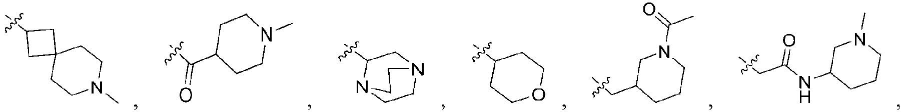 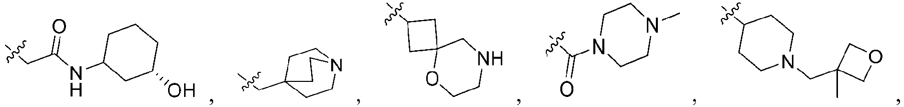 and 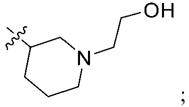
more preferably, -L¹-R¹ is selected from:

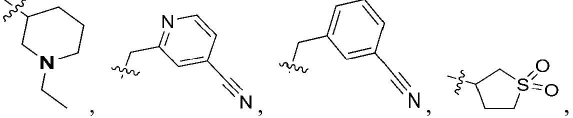

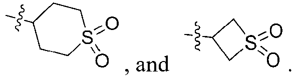

44. A compound represented by Formula X-1 or X-2, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof, 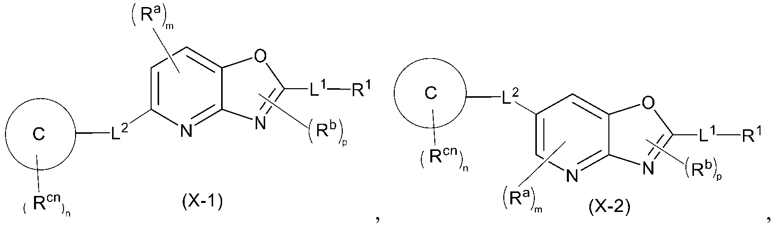 wherein,
ring C selected from: 6-10 membered aryl, 5-10 membered heteroaryl, phenyl fused 4-7 membered cycloalkyl, phenyl fused 4-7 membered heterocyclyl, 5-6 membered heteroaryl fused 4-7 membered cycloalkyl, 5-6 membered heteroaryl fused 4-7 membered heterocyclyl, 4-7 membered cycloalkyl, and 4-7 membered heterocyclyl;
each R^{cn} is independently selected from: H, deuterium, halogen, -OH, -NH₂, -CN, C₁₋₆ alkoxy, C₁₋₆ alkyl, C₁₋₆ haloalkyl, C₁₋₆ haloalkoxy, hydroxyl C₁₋₆ alkyl, C₁₋₆ alkoxy-C₁₋₆ alkyl, R^{c1}S(O)₂-, -(CH₂)ₛ-N(R^{c1})(R^{c2}), 3-6 membered cycloalkyl, 3-6 membered heterocyclyl, phenyl, and 5-6 membered heteroaryl; wherein the 3-6 membered cycloalkyl, 3-6 membered heterocyclyl, phenyl, and 5-6 membered heteroaryl are optionally substituted with 1, 2 or 3 substituents selected from: deuterium, halogen, -OH, -NH₂, -CN, C₁₋₆ alkyl, C₁₋₆ haloalkyl, and C₁₋₆ alkoxy; or
two R^{cn} together with the C atom to which they are attached may form a 3-6 membered cycloalkyl, 4-7 membered heterocyclyl, 6-membered aryl, or 5-6 membered heteroaryl, the 3-6 membered cycloalkyl, 4-7 membered heterocyclyl, 6 membered aryl, and 5-6 membered heteroaryl may be optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
each R^{a} and R^{b} are independently selected from: H, deuterium, halogen, -OH, -NH₂, -CN, C₁₋₆ alkyl, C₁₋₆ haloalkyl, C₁₋₆ alkoxy, C₁₋₆ alkoxy-C₁₋₆ alkyl, C₁₋₆ haloalkoxy, hydroxyl C₁₋₆ alkyl, -NH(C₁₋₆ alkyl), -NH(C₁₋₆ alkyl)(C₁₋₆ alkyl), -C(=O)-(C₁₋₆ alkyl), and 3-6 membered heterocycloalkyl optionally substituted by hydroxyl;
L¹ is selected from: bond, C₃₋₆ cycloalkylene, -NR²-, -O-, -S-, -C(=O)-, -(CR³R⁴)ₐ-, -NR²-(CR³R⁴)ₐ-, - (CR³R⁴)ₐ-NR²-, -O-(CR³R⁴)ₐ-, -(CR³R⁴)ₐ-O-, -S-(CR³R⁴)ₐ-, -(CR³R⁴)ₐ-S-, -C(=O)-(CR³R⁴)ₐ-, -(CR³R⁴)ₐ-C(=O)-, - (CR³R⁴)ₐ-(CR⁹=CR¹⁰)-, -(CR⁹=CR¹⁰)-(CR³R⁴)ₐ-, -C(=O)-O-, -O-C(=O)-, -C(=O)-NR²-, -(CR³R⁴)ₐ-C(=O)-NR²-, - NR²-C(=O)-, -(CR³R⁴)ₐ-NR²-C(=O)-, -S(=O)₂-NR²-, -NR²-S(=O)₂-, -NR²-(CR³R⁴)ₐ-C(=O)-, -C(=O)-(CR³R⁴)ₐ-NR²-, -NR²-(CR³R⁴)ₐ-C(=O)-NR⁵-, -NR²-C(=O)-(CR³R⁴)ₐ-NR⁵-, -C(=S)-NR²-, and -NR²-C(=S)-;
L² is selected from: bond, -NR⁶-, -O-, -S-, -NR⁶-(CR⁷R⁸)_{b}-, -(CR⁷R⁸)_{b}-NR⁶-, -O-(CR⁷R⁸)_{b}-, -(CR⁷R⁸)_{b}-O-, - S-(CR⁷R⁸)_{b}-, and -(CR⁷R⁸)_{b}-S-;
each R², R⁵, R⁶, R⁹ and R¹⁰ are each independently selected from: H, deuterium, and C₁₋₆ alkyl;
each R³, R⁴, R⁷, and R⁸ are each independently selected from: H, deuterium, halogen, oxo, -OH, -NH₂, C₁₋₆ alkyl;
R¹ is selected from: 3-10 membered cycloalkyl, 3-6 membered monocyclic heterocyclyl, 6-11 membered bicyclic heterocyclyl, 6-10 membered aryl, and 5-10 membered heteroaryl; wherein the 3-10 membered cycloalkyl, 3-6 membered monocyclic heterocyclyl, 6-11 membered bicyclic heterocyclyl, 6-10 membered aryl, and 5-10 membered heteroaryl are substituted with 1 or 2 substituents selected from: -C(=O)-R^{c3}, -S(O)-R^{c3}, -S(O)₂-R^{c3}, - C(=O)-N(R^{c5})(R^{c6}), and -P(=O)R^{c5}R^{c6}; and the 3-10 membered cycloalkyl, 3-6 membered monocyclic heterocyclyl, 6-11 membered bicyclic heterocyclyl, 6-10 membered aryl, and 5-10 membered heteroaryl are each optionally further substituted with 1, 2, or 3 substituents selected from: H, deuterium, halogen, -OH, oxo, -CN, C₁₋₆ alkyl, hydroxyl C₁₋₆ alkyl, C₁₋₆ haloalkyl, C₁₋₆ alkoxy, C₁₋₆ alkoxy-C₁₋₆ alkyl, -(CH₂)ₛ-N(R^{c1})(R^{c2}), C₃₋₆ cycloalkyl, and 3-6 membered monocyclic heterocyclyl;
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: C₁₋₆ alkyl, and C₃₋₆ cycloalkyl, wherein the C₁₋₆ alkyl and C₃₋₆ cycloalkyl are further optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
R^{c5} and R^{c6} are selected from: H, deuterium, C₁₋₆ alkyl, and C₃₋₆ cycloalkyl, or R^{c5}, R^{c6} together with the N atom to which they are attached form a 4-6 membered heterocyclyl; wherein the C₁₋₆ alkyl, C₃₋₆ cycloalkyl, and 4-6 membered heterocyclyl are optionally substituted with 1, 2, or 3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
each a and b are each independently 1, 2, 3, or 4;
each m is 0 or 1;
each p is 0, 1 or 2;
each s and n are each independently selected from: 0, 1, 2, 3, and 4;
provided that, the compound is not

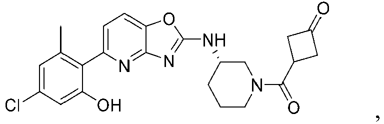

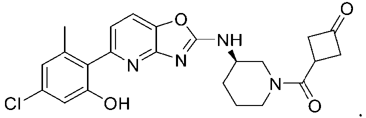

45. The compound according to claim 44, wherein, the compound has the structure represented by Formula VIII-3 or VIII-4,

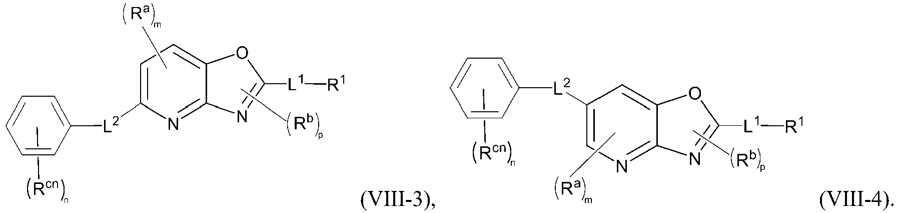

46. The compound according to claim 44 or 45, wherein the compound has the structure represented by Formula VIII-3:

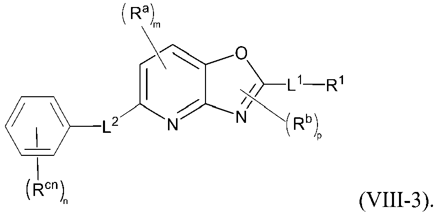

47. The compound according to any one of claims 44-46, wherein, R¹ is selected from: 3-6 membered cycloalkyl, 3-6 membered monocyclic heterocyclyl, and 6-11 membered bicyclic heterocyclyl; wherein the 3-6 membered cycloalkyl, 3-6 membered monocyclic heterocyclyl, and 6-11 membered bicyclic heterocyclyl are substituted with 1 or 2 substituents selected from: -C(=O)-R^{c3}, -S(O)-R^{c3}, -S(O)₂-R^{c3}, -C(=O)-N(R^{c5})(R^{c6}), and - P(=O)R^{c5}R^{c6}; and the 3-6 membered cycloalkyl, 3-6 membered monocyclic heterocyclyl, and 6-11 membered bicyclic heterocyclyl are each optionally substituted with 1, 2, or 3 substituents selected from: H, deuterium, halogen, -OH, oxo, -CN, C₁₋₆ alkyl, C₁₋₆ haloalkyl, C₁₋₆ alkoxy, C₁₋₆ alkoxy-C₁₋₆ alkyl, -(CH₂)ₛ-N(R^{c1})(R^{c2}), C₃₋₆ cycloalkyl, and 3-6 membered monocyclic heterocyclyl;
each R^{c1} and R^{c2} are independently selected from: H, C₁₋₆ alkyl, C₁₋₆ haloalkoxy, C₃₋₆ cycloalkyl, and 4-7 membered heterocyclyl;
R^{c3} is selected from: C₁₋₆ alkyl, and C₃₋₆ cycloalkyl, wherein the C₁₋₆ alkyl and C₃₋₆ cycloalkyl are further optionally substituted with 1-3 substituents selected from: deuterium, halogen, -OH, -NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl); R^{c5} and R^{c6} are selected from: H, deuterium, C₁₋₆ alkyl, and C₃₋₆ cycloalkyl, or R^{c5}, R^{c6} together with the N atom to which they are attached form a 4-6 membered heterocyclyl; wherein the C₁₋₆ alkyl, C₃₋₆ cycloalkyl, and 4-6 membered heterocyclyl are optionally substituted with 1-3 substituents selected from: deuterium, halogen, -OH, - NH₂, -CN, oxo, C₁₋₆ alkyl, C₁₋₆ alkoxy, C₁₋₆ haloalkyl, C₁₋₆ haloalkoxy, C₁₋₆ alkoxy-C₁₋₆ alkyl, -NH(C₁₋₆ alkyl), and -N(C₁₋₆ alkyl)(C₁₋₆ alkyl);
each a and b are each independently 1, 2, 3, and 4;
each m is 0 or 1;
each p is 0, 1 or 2;
each s and n are each independently selected from: 0, 1, 2, 3, and 4.

48. The compound according to any one of claims 44-47, wherein, L² is bond.

49. The compound according to any one of claims 44-48, wherein, R^{a} and R^{b} are each independently selected from: H, deuterium, F, Cl, Br, -CN, methyl, ethyl, trifluoromethyl, methoxy, 1-hydroxyethyl-1-yl, methylamino, dimethylamino, acetyl, and 3-hydroxyazetidinyl; preferably, R^{a} and R^{b} are each independently selected from: H, deuterium, F, Cl, Br, methyl, and ethyl; more preferably, R^{a} and R^{b} are each independently selected from: H, deuterium, and methyl.

50. The compound according to any one of claims 44-49, wherein, each R^{cn} is independently selected from: H, deuterium, F, Cl, Br, -OH, -NH₂, -CN, C₁₋₄ alkoxy, C₁₋₄ haloalkoxy, C₁₋₄ alkyl, C₁₋₄ haloalkyl, hydroxyl C₁₋₄ alkyl, C₁₋₄ alkoxy-C₁₋₂ alkyl, R^{c1}S(O)₂-, -(CH₂)ₛ-N(R^{c1})(R^{c2}), 3-6 membered cycloalkyl, 3-6 membered heterocyclyl, phenyl and 5-6 membered heteroaryl; wherein the 3-6 membered cycloalkyl, 3-6 membered heterocyclyl, phenyl and 5-6 membered heteroaryl are optionally substituted with 1-3 substituents selected from: deuterium, halogen, -OH, -NH₂, -CN, C₁₋₆ alkyl, C₁₋₆ haloalkyl, and C₁₋₆ alkoxy; or
two R^{cn} together with the C atom to which they are attached may form a 3-6 membered cycloalkyl, 4-7 membered heterocyclyl, 6 membered aryl or 5-6 membered heteroaryl; wherein the 3-6 membered cycloalkyl, 4-7 membered heterocyclyl, 6 membered aryl or 5-6 membered heteroaryl are optionally substituted with 1-3 substituents selected from: deuterium, halogen, -OH, -NH₂, -CN, C₁₋₆ alkoxy, C₁₋₆ alkyl, and C₁₋₆ haloalkyl;
preferably, each R^{cn} is independently selected from: F, Cl, Br, -OH, -NH₂, -CN, methoxy, ethoxy, isopropoxy, methylamino, ethylamino, isopropylamino, methyl, ethyl, n-propyl, isopropyl, n-butyl, isobutyl, trifluoromethyl, difluoromethyl, 2,2,2-trifluoroethyl, pentafluoroethyl, trifluoromethoxy, difluoromethoxy, hydroxymethyl, aminoethyl, methoxymethyl, methoxyethyl, ethoxymethyl, cyclopropyl, cyclobutyl, cyclopentyl, cyclohexyl, cyclopentenyl, cyclobutenyl, cyclohexenyl, oxetanyl, phenyl, 4-chloro-phenyl, 2-chloro-4-fluoro-phenyl, 4-hydroxyphenyl, 4-methyl-phenyl, 4-fluoro-phenyl, pyridyl, cyclobutylamino, and 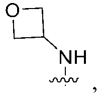 or two R^{cn} together with the C atom to which they are attached form a cyclopentenyl, 2,3-dihydrofuranyl, cyclobutenyl, thienyl, 1,4-dioxanyl, pyridyl, cyclopenta-1,3-dienyl, phenyl, or 1,4-dioxa-2-hexenyl;
more preferably, each R^{cn} is independently selected from: F, Cl, Br, -OH, -CN, methoxy, ethoxy, methyl, ethyl, trifluoromethyl, difluoromethyl, trifluoromethoxy, and difluoromethoxy.

51. The compound according to any one of claims 44-50, wherein, 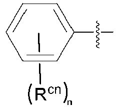 is selected from: 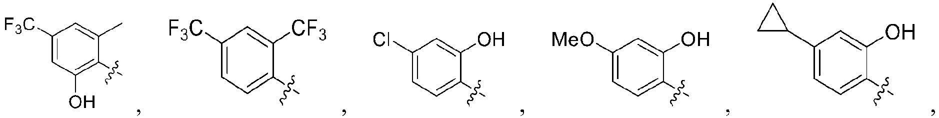 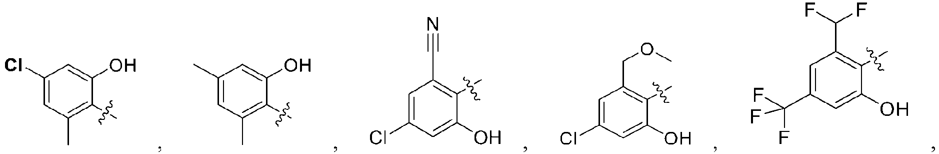 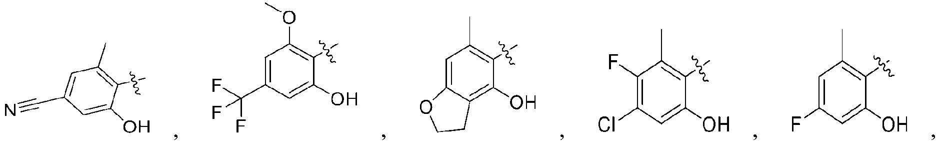 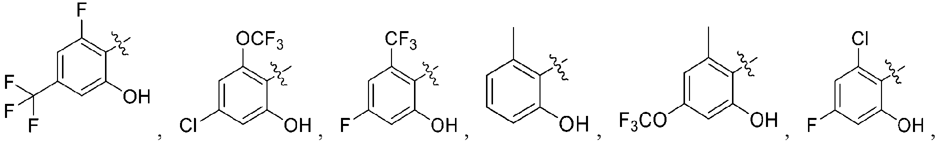 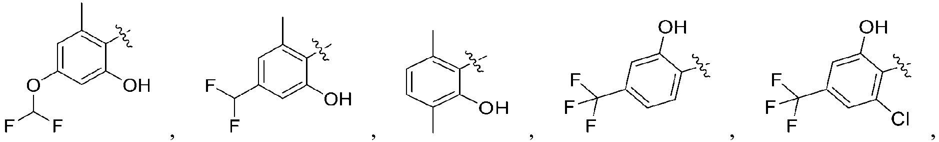 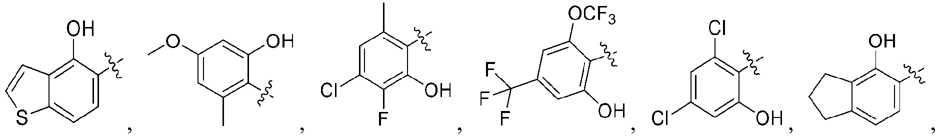 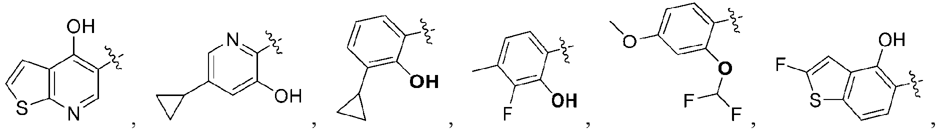 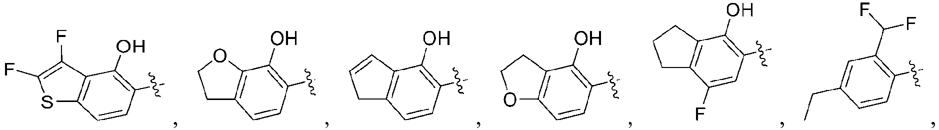 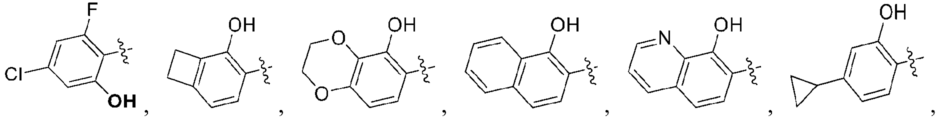 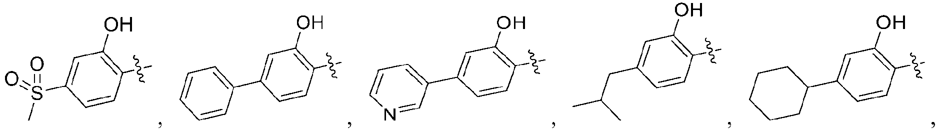 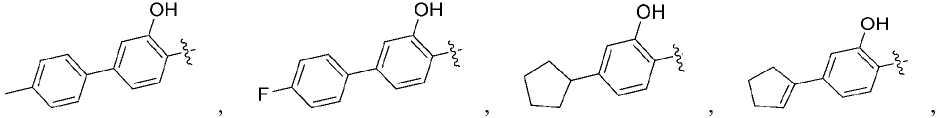 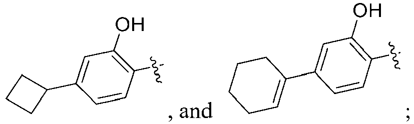 preferably, wherein 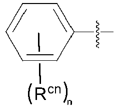 is selected from:

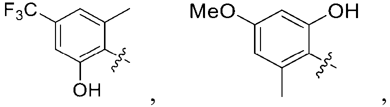

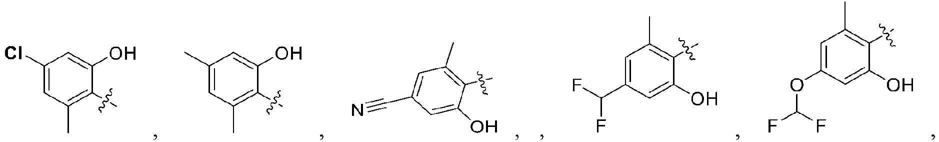

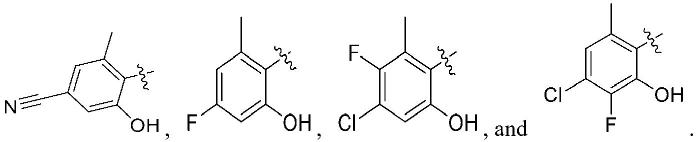

52. The compound according to any one of claims 44-51, wherein, L² is selected from: bond, -NR⁶-, -O-, -S-, -NR⁶-(CR⁷R⁸)b₁-, -(CR⁷R⁸)_{b1}-NR⁶-, -O-(CR⁷R⁸)_{b1}-, -(CR⁷R⁸)_{b1}-O-, -S-(CR⁷R⁸)_{b1}-, and -(CR⁷R⁸)_{b1}-S-; wherein b1 is 1 or 2;
preferably, L² is selected from: bond, -NR⁶-, -NR⁶-(CR⁷R⁸)b₁-, and -(CR⁷R⁸)_{b1}-NR⁶-; wherein b1 is 1 or 2;
more preferably, L² is selected from: -NR⁶-, -NR⁶-(CR⁷R⁸)b₁-, and -(CR⁷R⁸)_{b1}-NR⁶-; wherein b1 is 1;
further more preferably, L² is selected from: -NH- and -NH-(CR⁷R⁸)-;
further more preferably, L² is selected from: -NH- and -NH-CH₂-.

53. The compound according to any one of claims 44-52, wherein, R¹ is selected from: cyclopentyl, cyclohexyl, azetidinyl, tetrahydropyrrolyl, piperidinyl, morpholinyl, piperazinyl, 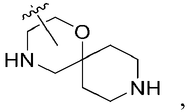 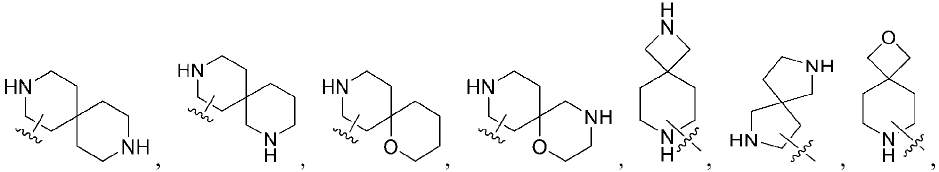 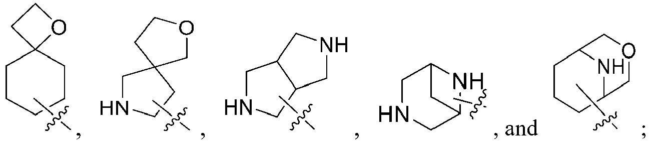 the cyclopentyl, cyclohexyl, azetidinyl, tetrahydropyrrolyl, piperidinyl, morpholinyl, piperazinyl, 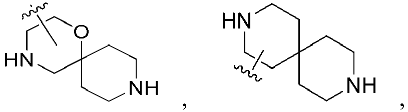 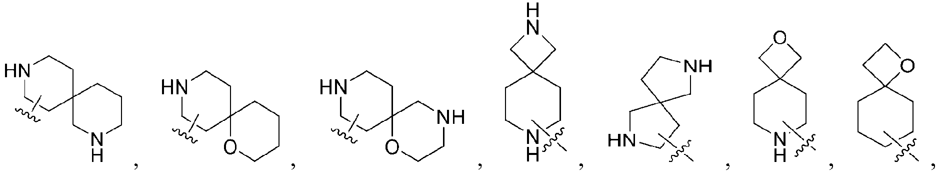 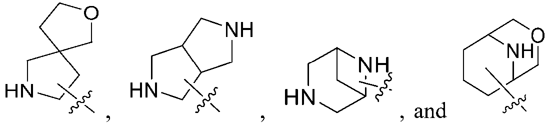 are substituted with 1 or 2 substituents selected from: -C(=O)-R^{c3}, -S(O)-R^{c3}, -S(O)₂-R^{c3}, -C(=O)-N(R^{c5})(R^{c6}), and -P(=O)R^{c5}R^{c6}; and the cyclopentyl, cyclohexyl, azetidinyl, tetrahydropyrrolyl, piperidinyl, morpholinyl, piperazinyl, 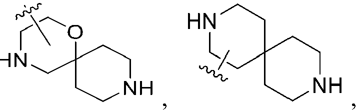 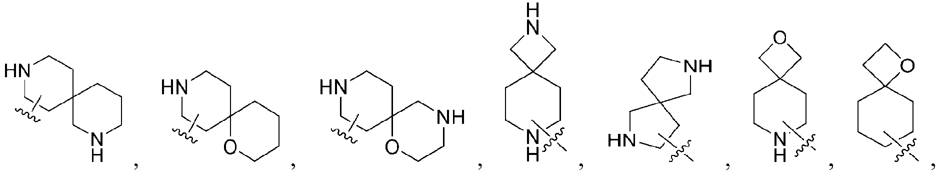 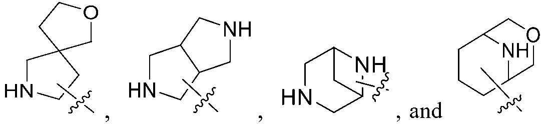 are further optionally substituted with 1, 2, or 3 substituents selected from: H, deuterium, halogen, -OH, oxo, C₁₋₆ alkyl, C₁₋₆ alkoxy, C₁₋₆ alkoxy-C₁₋₆ alkyl, -(CH₂)ₛ-N(R^{c1})(R^{c2}), C₃₋₆ cycloalkyl, and 3-6 membered monocyclic heterocyclyl;
preferably, R¹ is selected from: tetrahydropyrrolyl, piperidinyl, morpholinyl, piperazinyl, 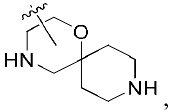 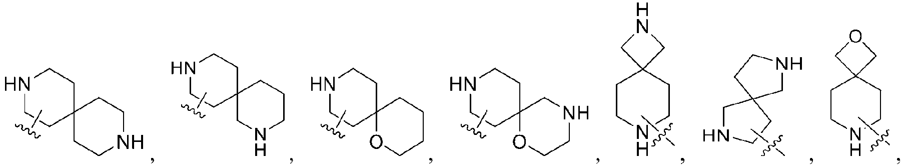 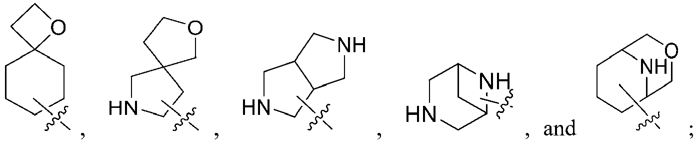 wherein the tetrahydropyrrolyl, piperidinyl, morpholinyl, piperazinyl, 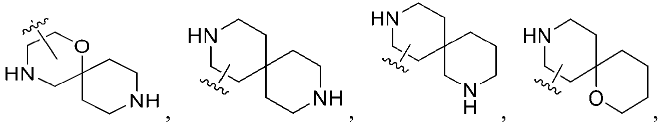 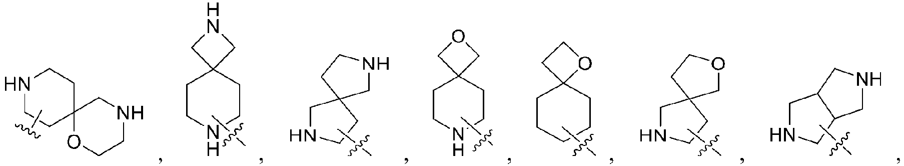 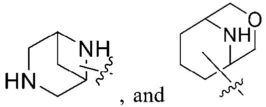 are substituted with 1 or 2 substituents selected from: -C(=O)-R^{c3}, -S(O)-R^{c3}, - S(O)₂-R^{c3}, -C(=O)-N(R^{c5})(R^{c6}), and -P(=O)R^{c5}R^{c6}; and the tetrahydropyrrolyl, piperidinyl, morpholinyl, piperazinyl, 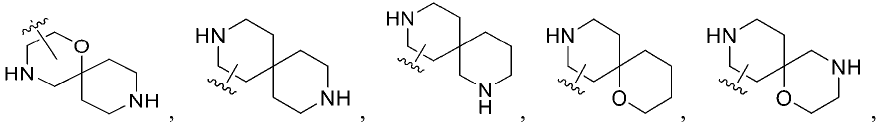 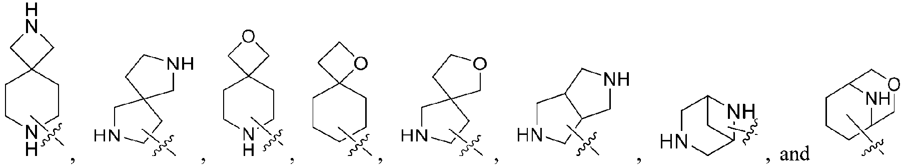 are further optionally substituted with 1, 2, or 3 substituents selected from: H, deuterium, halogen, -OH, oxo, C₁₋₆ alkyl, C₁₋₆ alkoxy, -(CH₂)ₛ-N(R^{c1})(R^{c2}), C₃₋₆ cycloalkyl, C₁₋₆ alkoxy-C₁₋₆ alkyl, and 3-6 membered monocyclic heterocyclyl;
more preferably, R¹ is selected from: tetrahydropyrrolyl, piperidinyl, morpholinyl, piperazinyl, 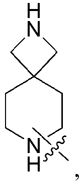 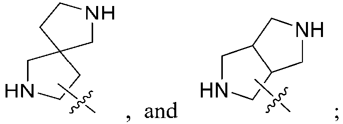 wherein the tetrahydropyrrolyl, piperidinyl, morpholinyl, piperazinyl, 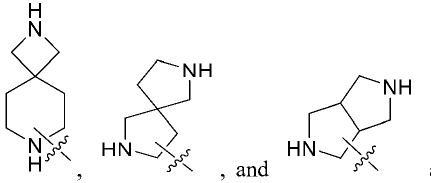 are substituted with 1 substituent selected from: -C(=O)-R^{c3}, - S(O)₂-R^{c3}, and -P(=O)R^{c5}R^{c6}; and the tetrahydropyrrolyl, piperidinyl, morpholinyl, piperazinyl, 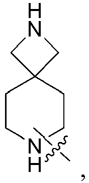 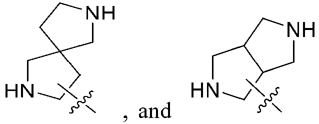 are further optionally substituted with 1 or 2 substituents selected from: deuterium, halogen, -OH, C₁₋₆ alkyl, C₁₋₆ alkoxy, and C₁₋₆ alkoxy-C₁₋₆ alkyl;
more preferably, R¹ is selected from: 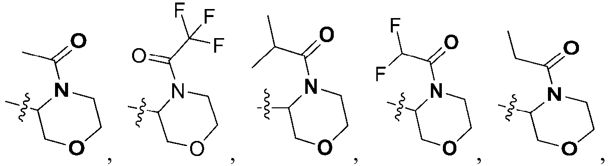 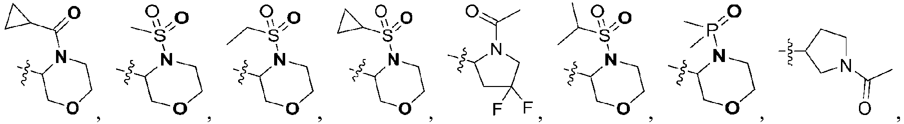 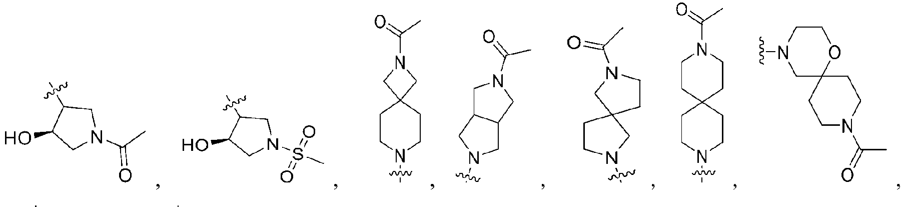 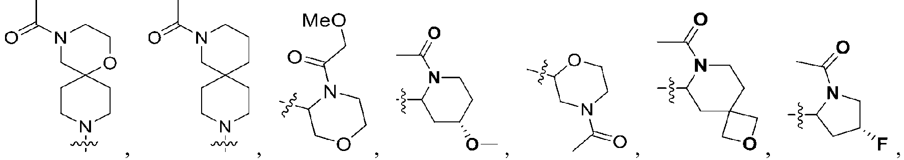 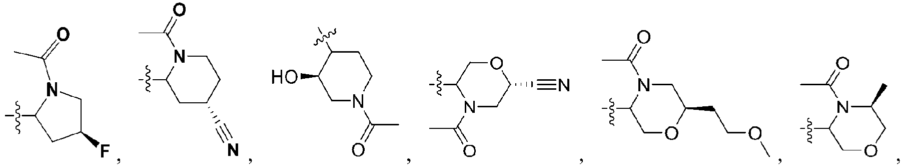 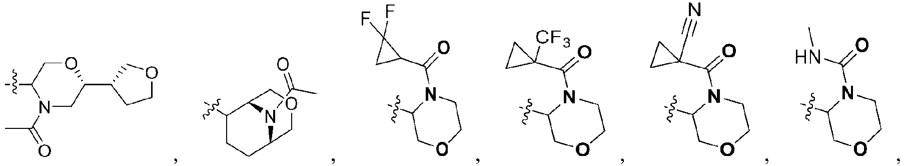 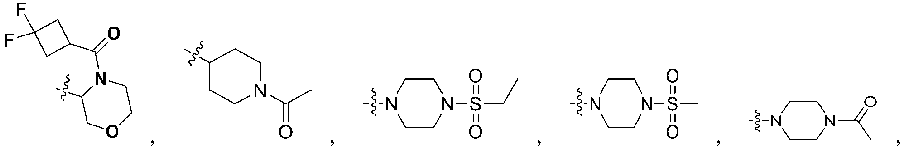 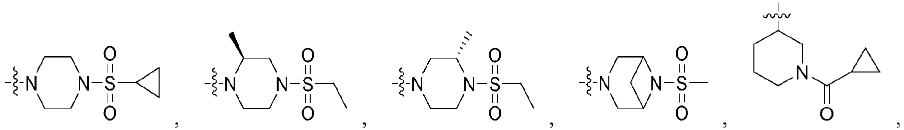 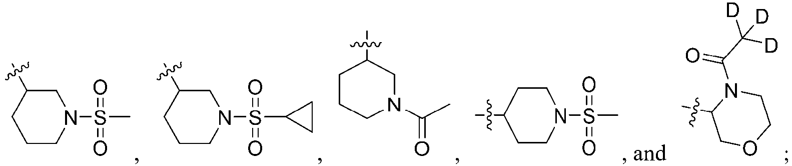
more preferably, R¹ is selected from:

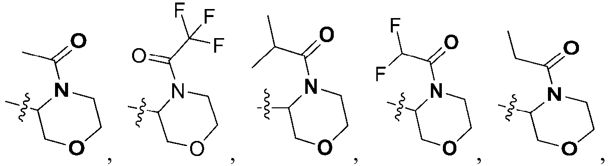

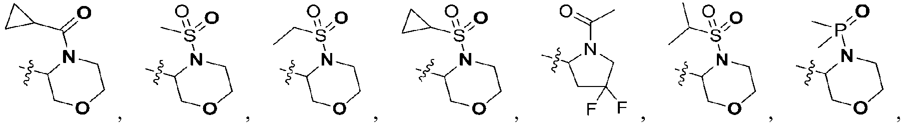

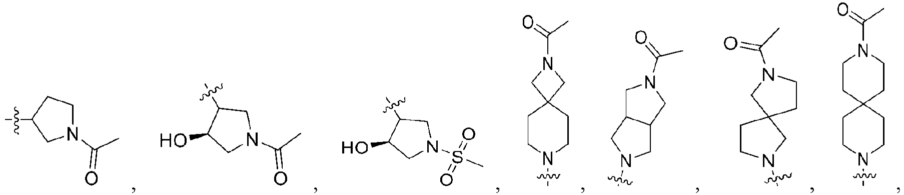

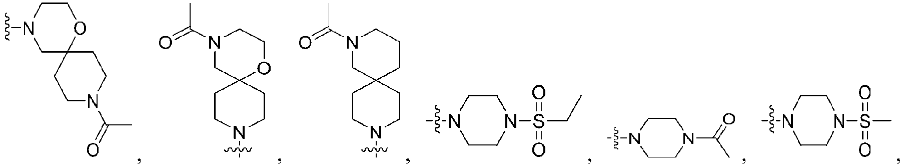

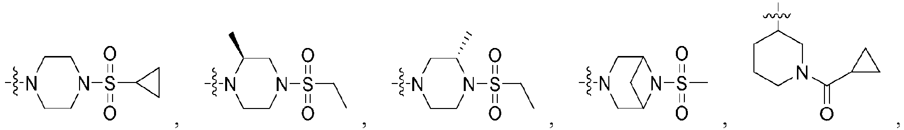

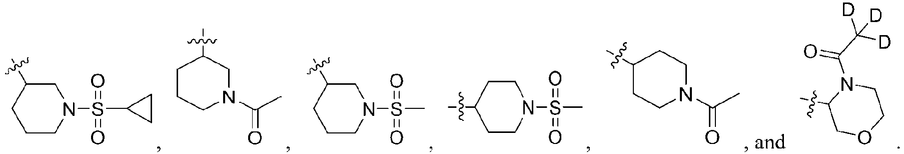

54. The compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof, according to any one of claims 1-4 and 6-27, wherein, the compound has the structure represented by Formula VIII-8 or VIII-12: 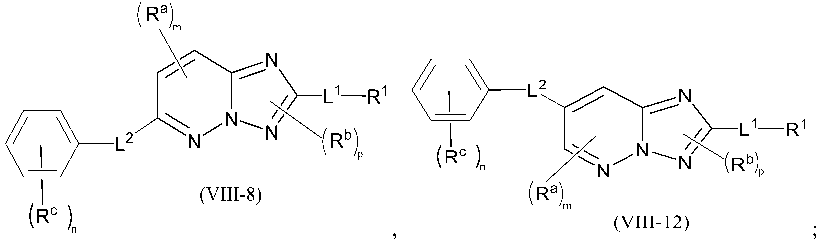 wherein,
each R^{c} is independently selected from: H, deuterium, halogen, -OH, -CN, C₁₋₆ alkoxy, C₁₋₆ alkyl, C₁₋₆ haloalkyl, C₁₋₆ haloalkoxy, C₁₋₆ alkoxy-C₁₋₆ alkyl, R^{c1}S(O)₂-, 3-6 membered cycloalkyl, 3-6 membered heterocyclyl, phenyl, and 5-6 membered heteroaryl; the 3-6 membered cycloalkyl, 3-6 membered heterocyclyl, phenyl, and 5-6 membered heteroaryl are optionally substituted with 1, 2, or 3 substituents selected from: deuterium, halogen, - OH, -NH₂, -CN, C₁₋₆ alkyl, C₁₋₆ haloalkyl, and C₁₋₆ alkoxy; or
two R^{c} together with the C atom to which they are attached may form a 3-6 membered cycloalkyl, 4-7 membered heterocyclyl, 6 membered aryl, or 5-6 membered heteroaryl, the 3-6 membered cycloalkyl, 4-7 membered heterocyclyl, 6 membered aryl, and 5-6 membered heteroaryl are optionally substituted with 1, 2, or 3 substituents selected from: deuterium, halogen, -OH, -NH₂, -CN, C₁₋₆ alkoxy, C₁₋₆ alkyl, and C₁₋₆ haloalkyl;
L² is selected from: bond, -NR⁶-, -O-, and -S-;
R^{a}, R^{b}, R^{c}, R^{c1}, R⁶, R¹, L¹, n, m, and p are defined as claim 1.

55. The compound according to claim 54, wherein, the compound has the structure represented by Formula VIII-8:

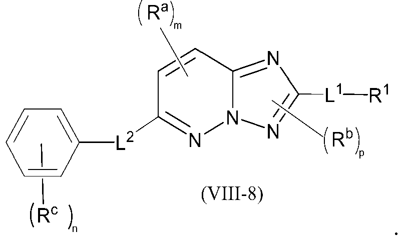

56. The compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof, according to claim 54 or 55, wherein,
R¹ is 3-6 membered monocyclic heterocyclyl, the 3-6 membered monocyclic heterocyclyl is optionally further substituted with 1, 2, or 3 substituents selected from: deuterium, halogen, -OH, oxo, -CN, C₁₋₆ alkyl, C₁₋₆ haloalkyl, hydroxyl C₁₋₆ alkyl, C₁₋₆ alkoxy, -(CH₂)ₛ-O(R^{c1}), -(CH₂)ₛ-N(R^{c1})(R^{c2}), C₃₋₆ cycloalkyl, 3-6 membered monocyclic heterocyclyl, 5-6 membered heteroaryl, -C(=O)-R^{c3}, -S(O)-R^{c3}, -S(O)₂-R^{c3}, -C(=O)O-R^{c3}, -N(R^{c4})-C(=O)-R^{c3} - CH₂-N(R^{c4})-C(=O)-R^{c4}, -C(=O)-N(R^{c6})(R^{c5}), -N(R^{C4})-C(=O)-N(R^{c6})(R^{c5}), and -P(=O)R^{c5}R^{c6};
preferably, R¹ is selected from: tetrahydropyrrolyl, piperidinyl, morpholinyl, piperazinyl, and 1,4-oxathianyl;
the tetrahydropyrrolyl, piperidinyl, morpholinyl, piperazinyl, and 1,4-oxathianyl are each independently optionally further substituted with 1, 2, or 3 substituents selected from: deuterium, halogen, -OH, -NH₂, oxo, C₁₋₄ alkyl, C₁₋₄ haloalkyl, hydroxyl C₁₋₄ alkyl, C₁₋₄ alkoxy, C₃₋₆ cycloalkyl, 3-6 membered monocyclic heterocyclyl, -C(=O)-R^{c3}, - S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6};
preferably, R¹ is selected from morpholinyl, the morpholinyl is optionally further substituted with 1, 2, or 3 substituents selected from: deuterium, halogen, -OH, -NH₂, oxo, C₁₋₄ alkyl, C₁₋₄ alkoxy, C₃₋₆ cycloalkyl, 3-6 membered monocyclic heterocyclyl, -C(=O)-R^{c3}, -S(O)-R^{c3}, -S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6};
more preferably, R¹ is selected from:

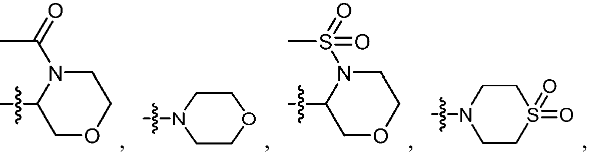

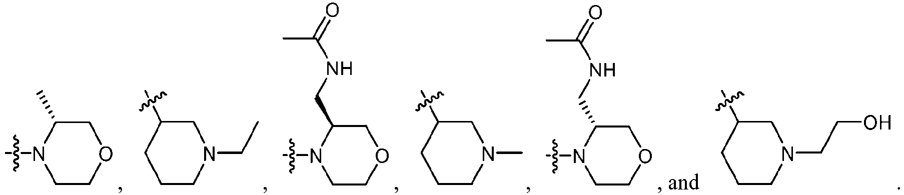

57. The compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof, according to any one of claims 1-4 and 6-27, wherein, the compound has the structure represented by Formula VIII-9, 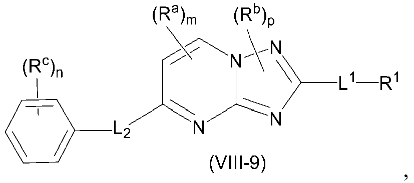 wherein,
R¹ is 3-6 membered monocyclic heterocyclyl, the 3-6 membered monocyclic heterocyclyl is optionally further substituted with **1,** 2, or 3 substituents selected from: deuterium, halogen, -OH, -CN, C₁₋₆ alkyl, C₁₋₆ haloalkyl, hydroxyl C₁₋₆ alkyl, C₁₋₆ alkoxy, -(CH₂)ₛ-N(R^{c1})(R^{c2}), C₃₋₆ cycloalkyl, 3-6 membered monocyclic heterocyclyl, - C(=O)-R^{c3}, -S(O)-R^{c3}, -S(O)₂-R^{c3}, -C(=O)O-R^{c3}, -N(R^{c4})-C(=O)-R^{c3} -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6};
preferably, R¹ is selected from: morpholinyl and piperidinyl, the morpholinyl and piperidinyl are each independently optionally further substituted with 1, 2, or 3 substituents selected from: deuterium, halogen, -OH, - NH₂, C₁₋₄ alkyl, C₁₋₄ alkoxy, C₃₋₆ cycloalkyl, 3-6 membered monocyclic heterocyclyl, -C(=O)-R^{c3}, -S(O)-R^{c3}, - S(O)₂-R^{c3}, -C(=O)O-R^{c3}, -N(R^{c4})-C(=O)-R^{c3}, -CH₂-N(R^{c4})-C(=O)-R^{c4}, -C(=O)-N(R^{c6})(R^{c5}), -N(R^{c4})-C(=O)-N(R^{c6})(R^{c5}), and -P(=O)R^{c5}R^{c6};
more preferably, the R¹ is selected from: 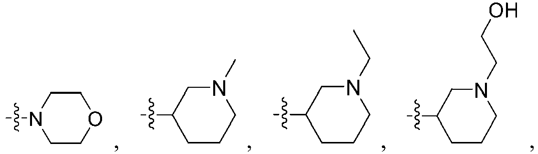 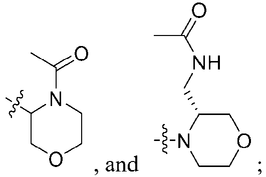
R^{a}, R^{b}, R^{c}, R^{c1}, R^{c2}, R^{c3}, R^{c4}, R^{c5}, R^{c6}, R, L², L¹, s, n, m, and p are defined as claim 1;
provided that, the compound is not

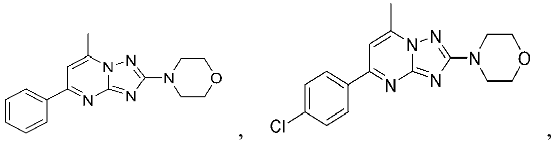

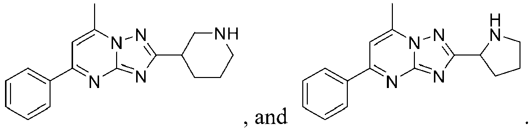

58. The compound according to any one of claims 1-57, wherein, the compound has the following structures:

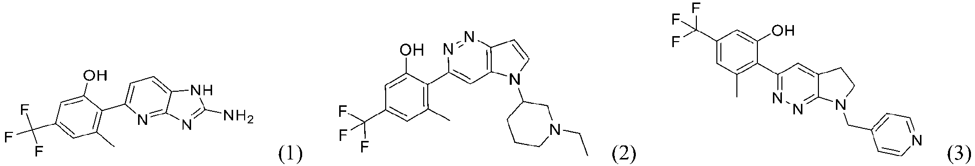

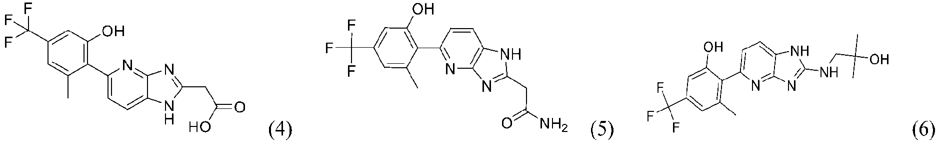

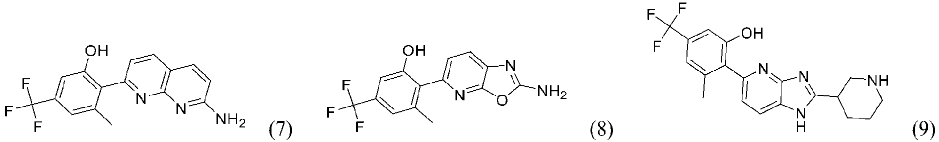

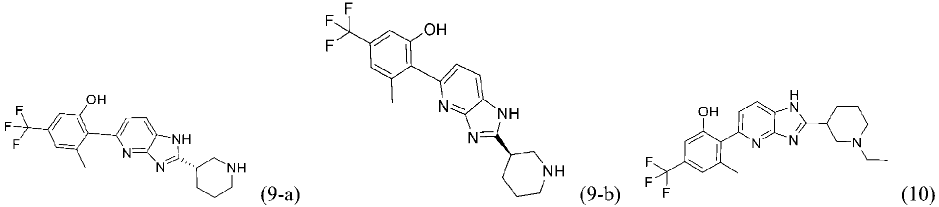

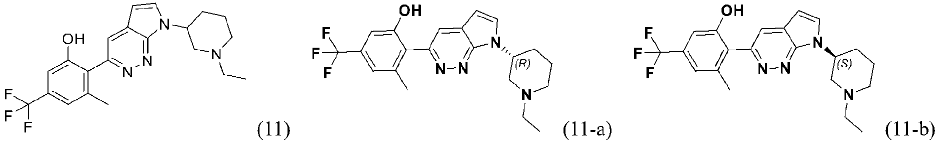

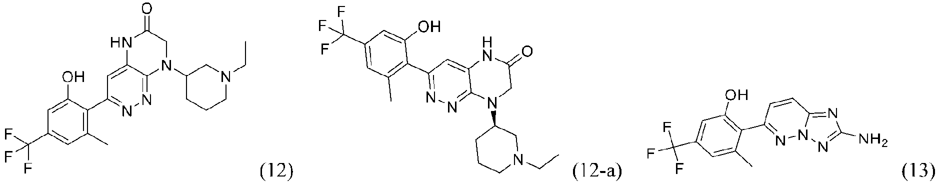

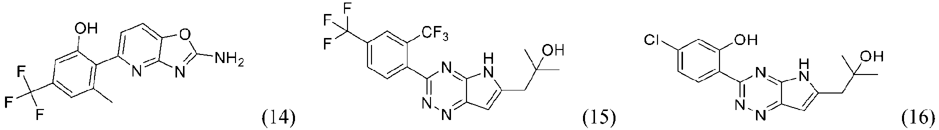

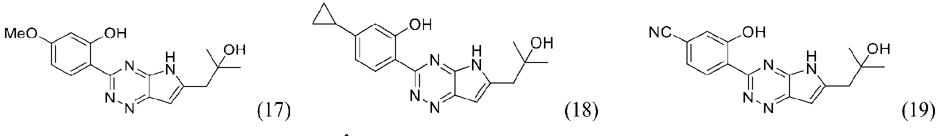

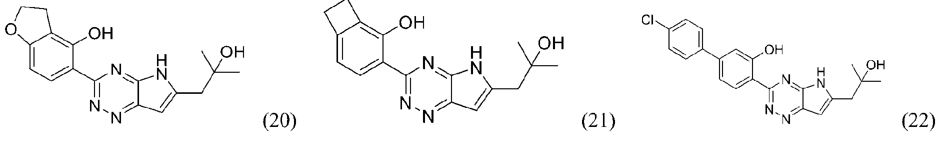

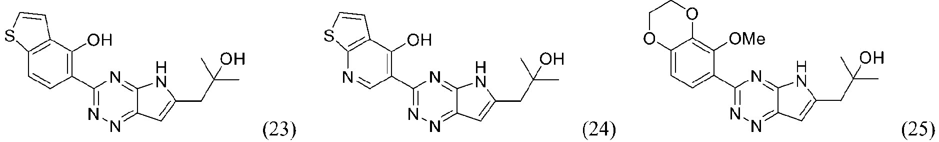

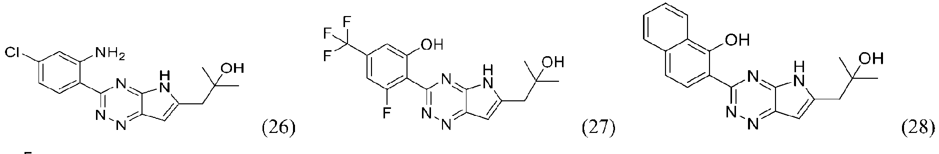

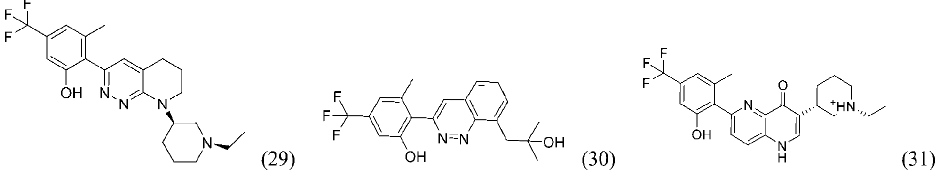

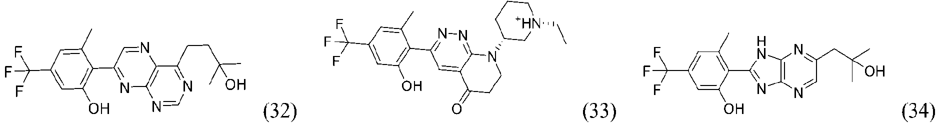

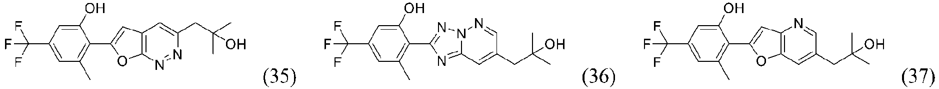

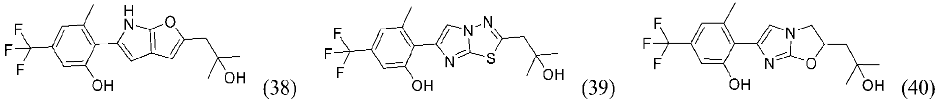

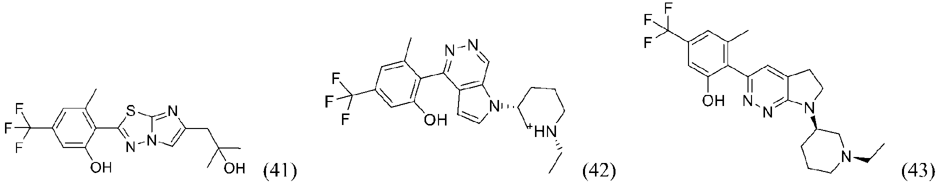

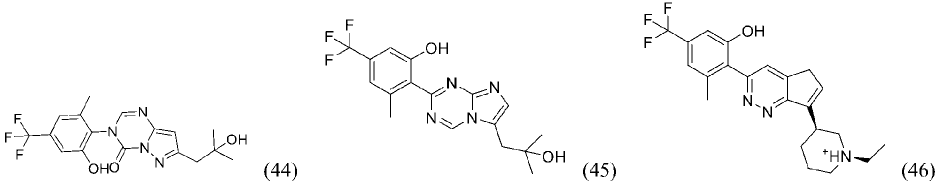

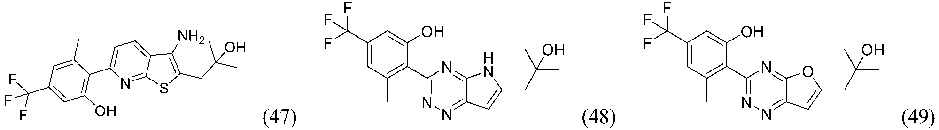

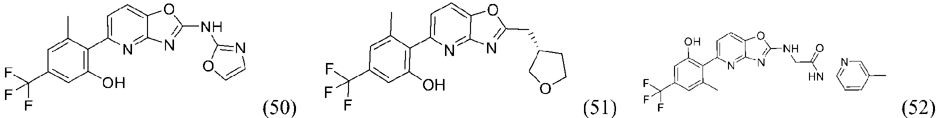

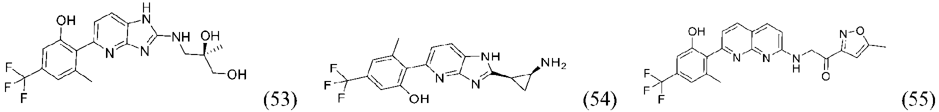

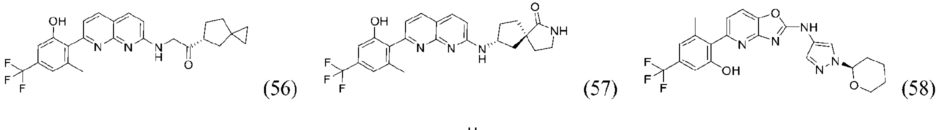

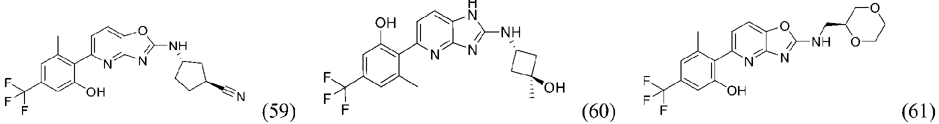

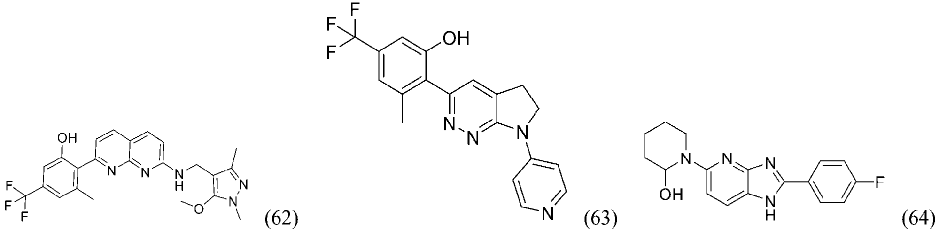

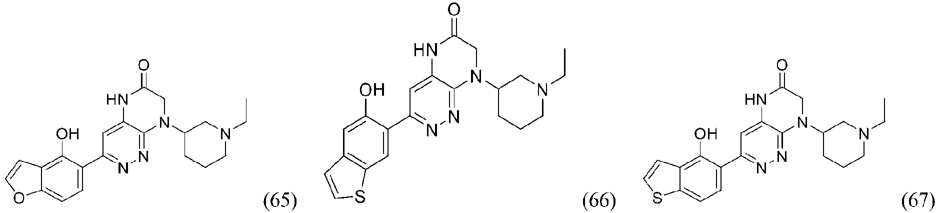

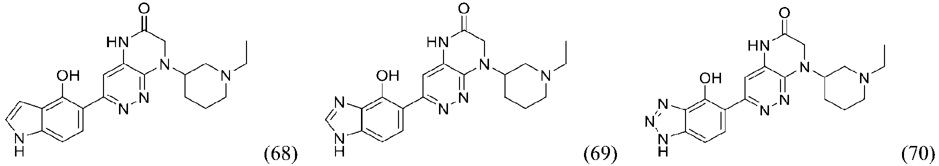

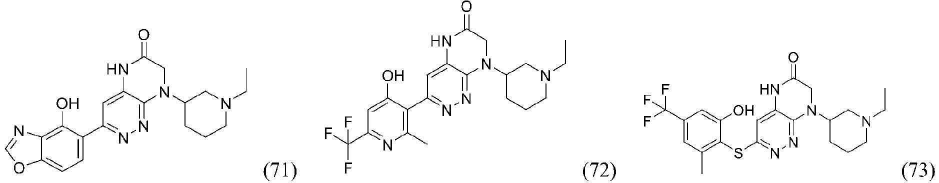

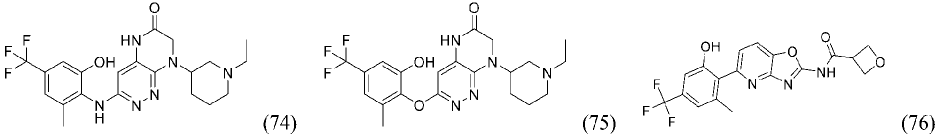

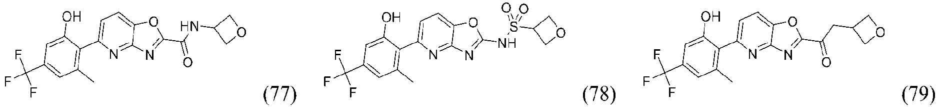

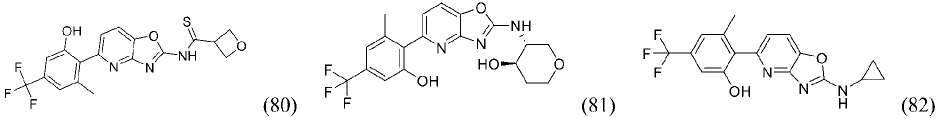

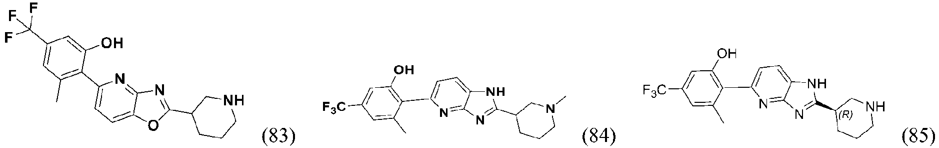

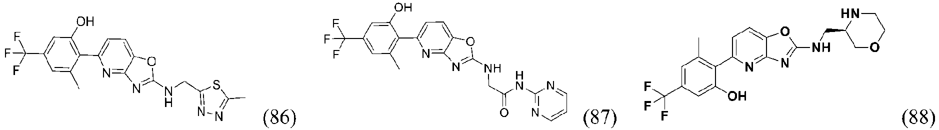

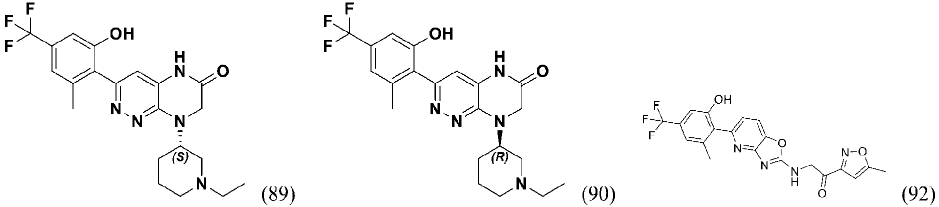

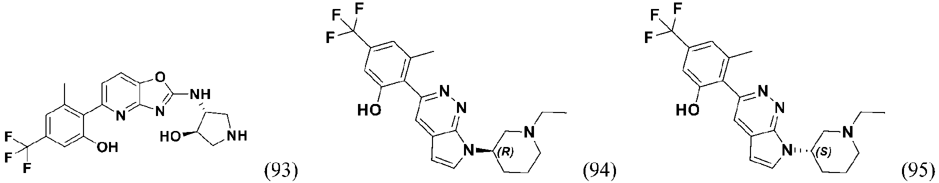

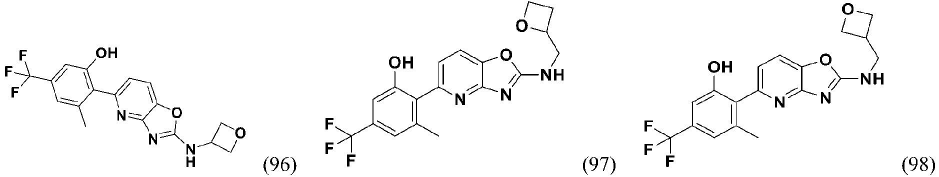

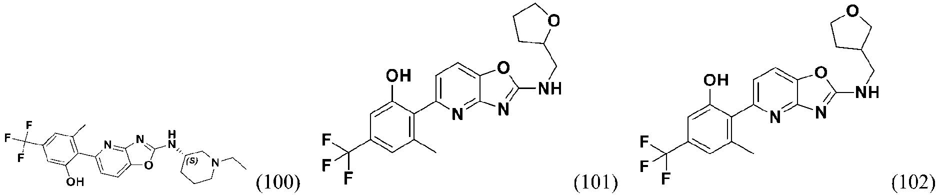

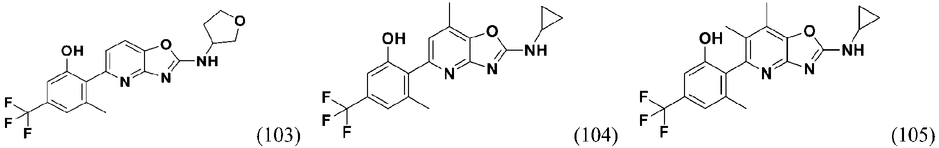

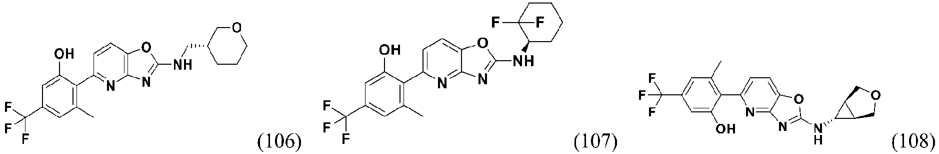

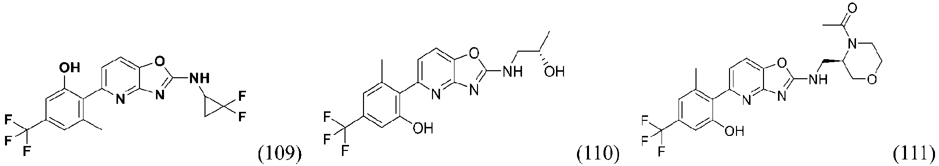

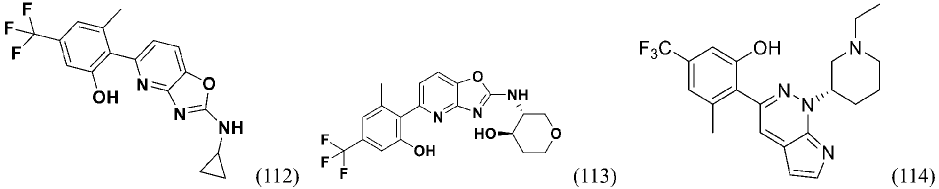

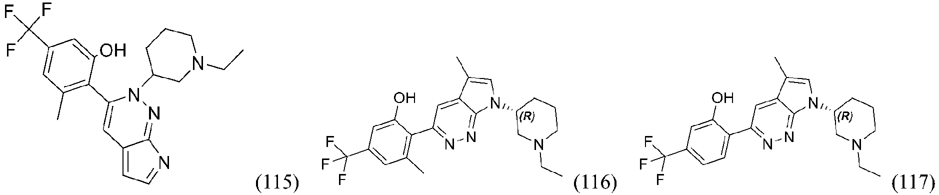

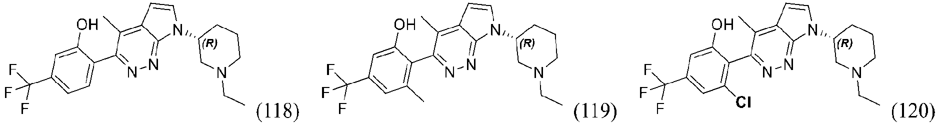

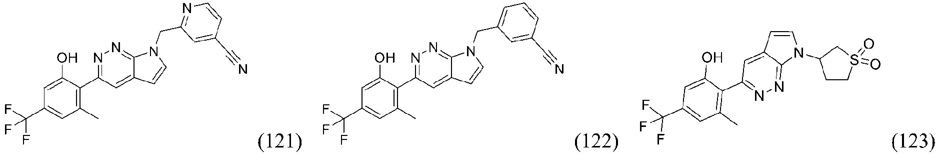

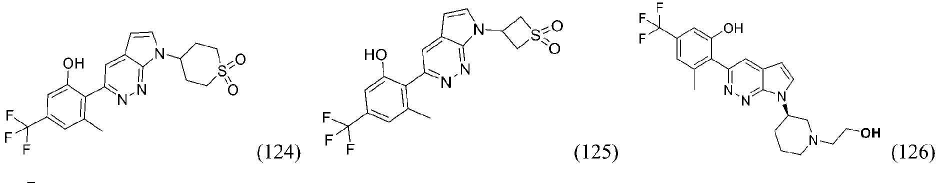

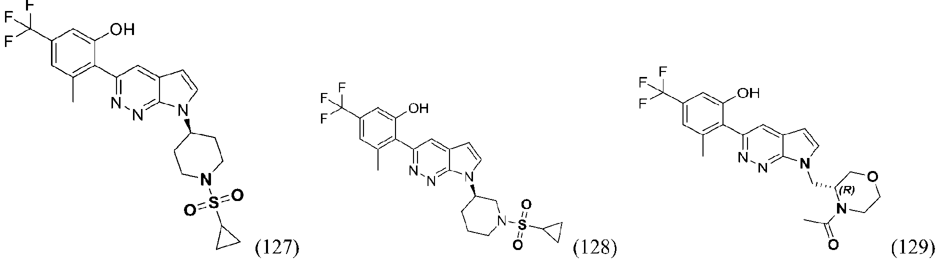

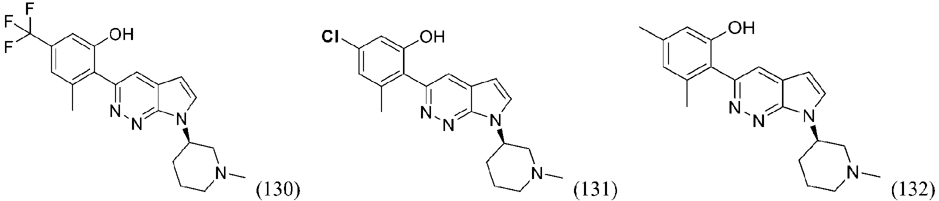

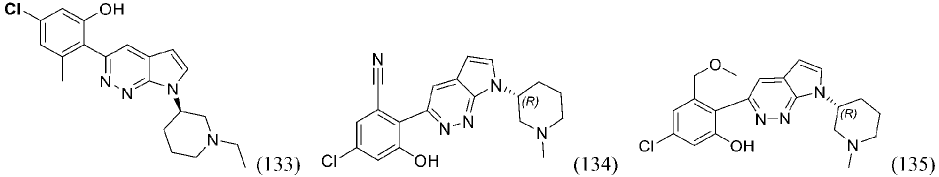

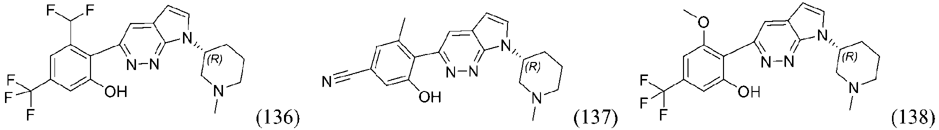

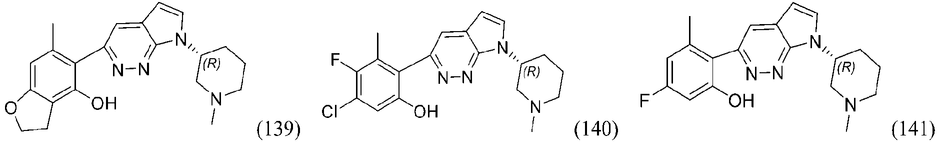

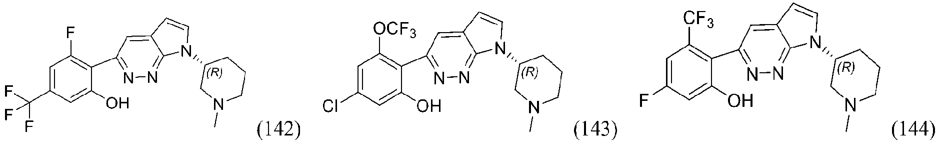

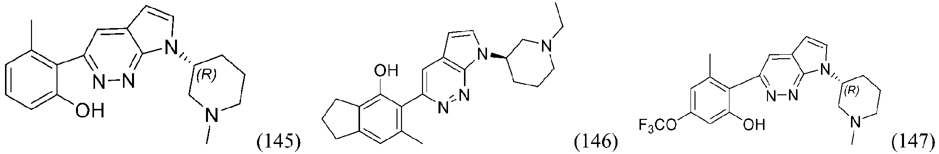

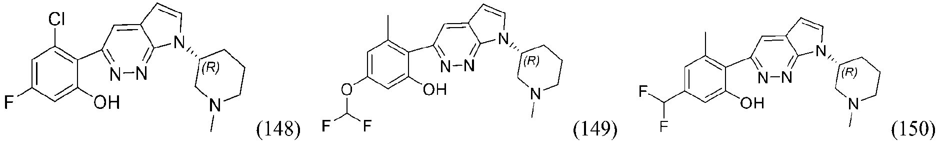

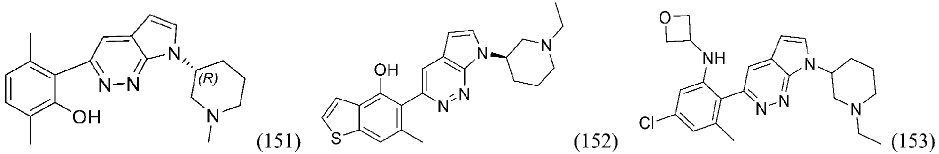

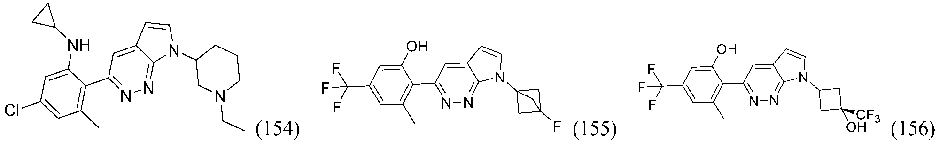

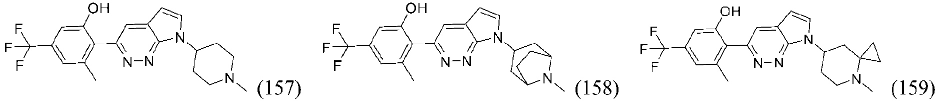

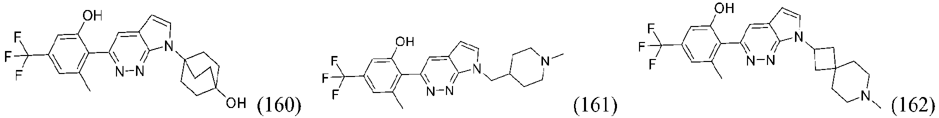

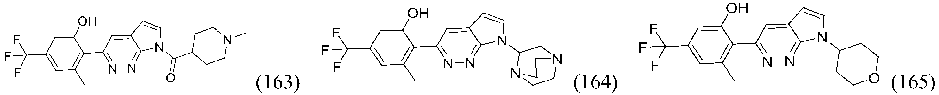

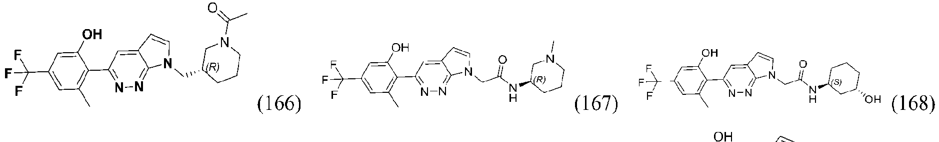

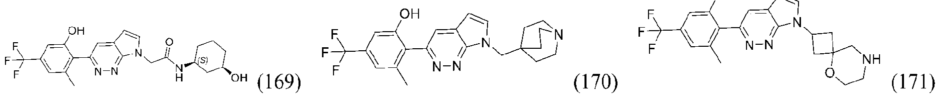

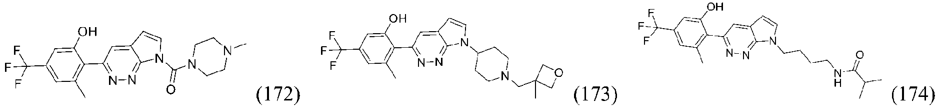

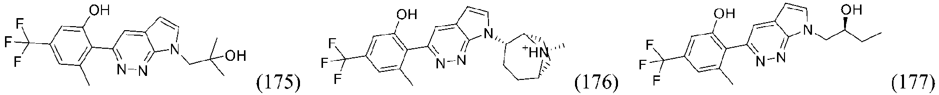

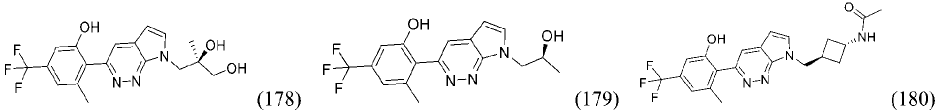

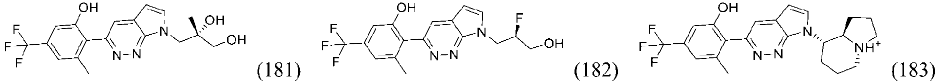

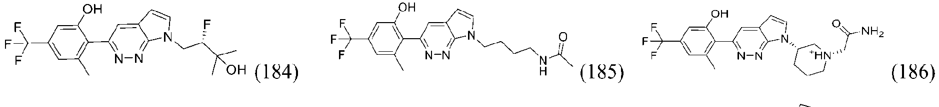

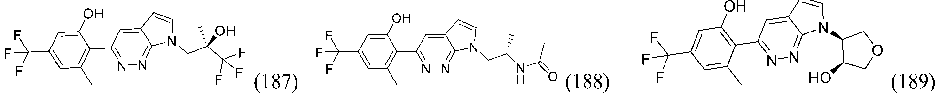

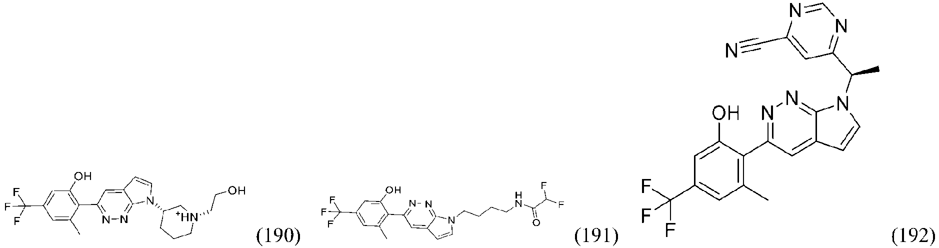

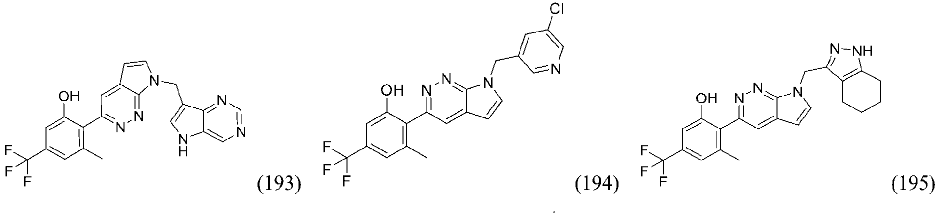

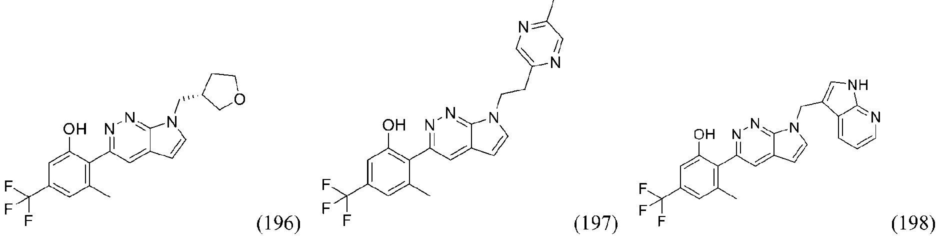

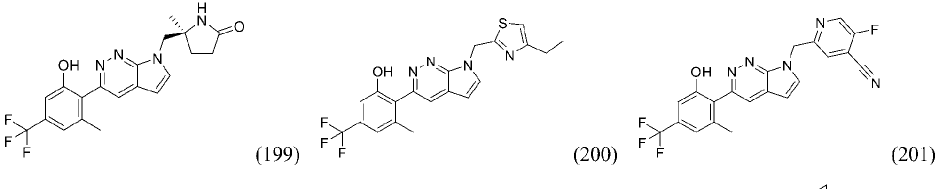

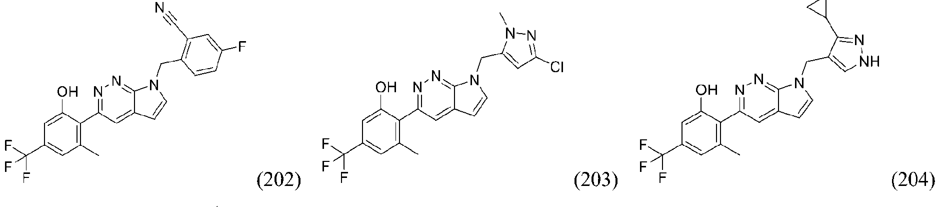

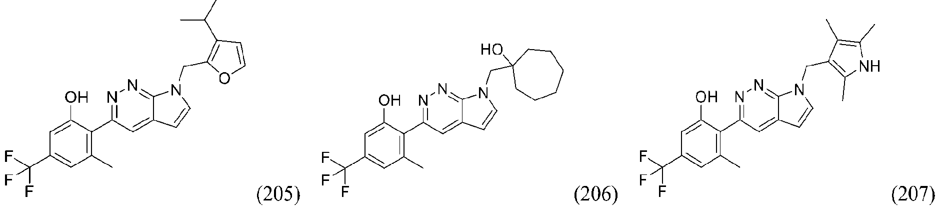

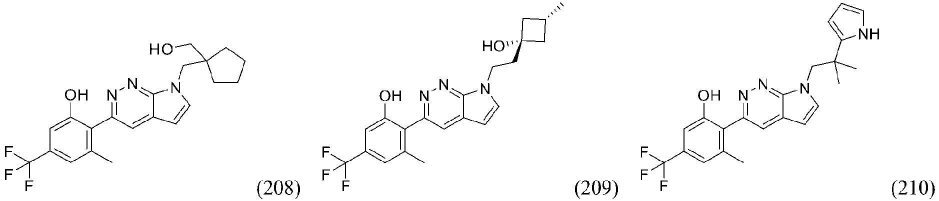

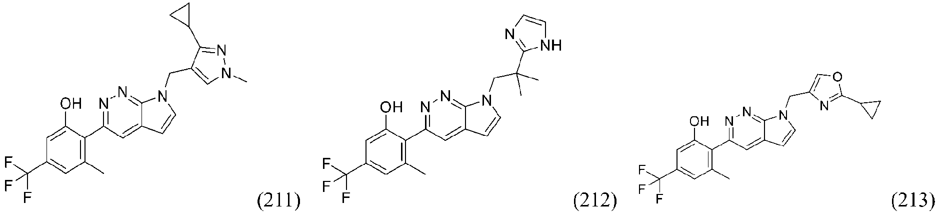

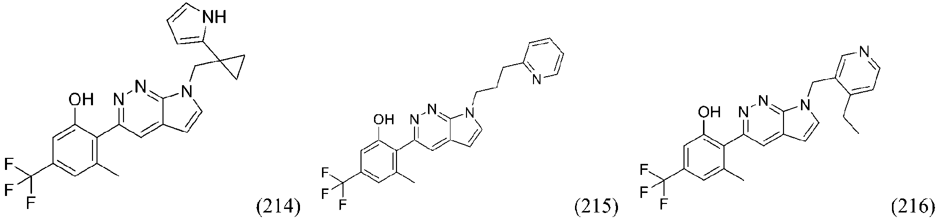

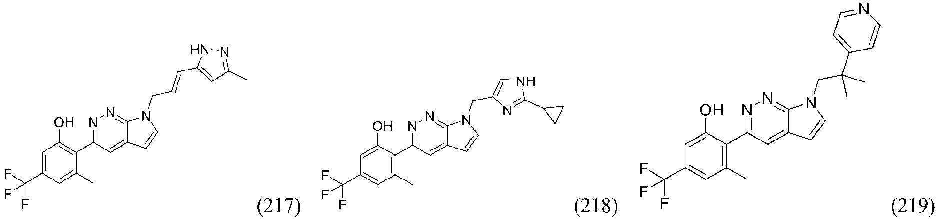

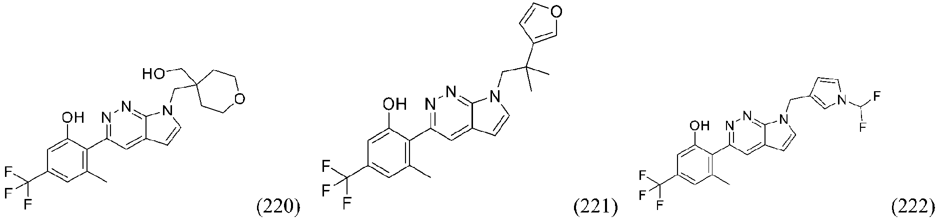

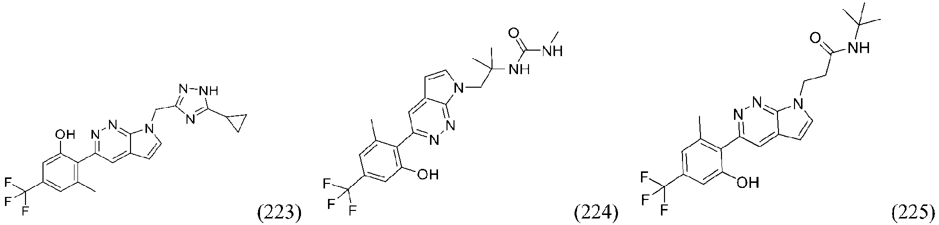

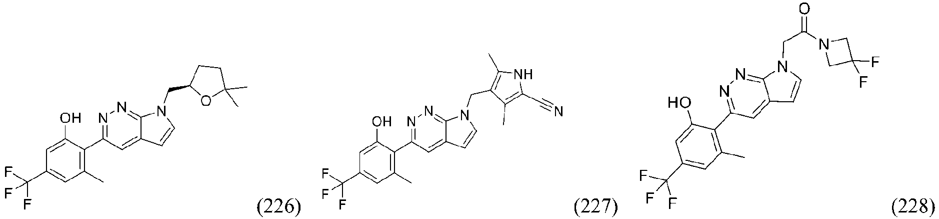

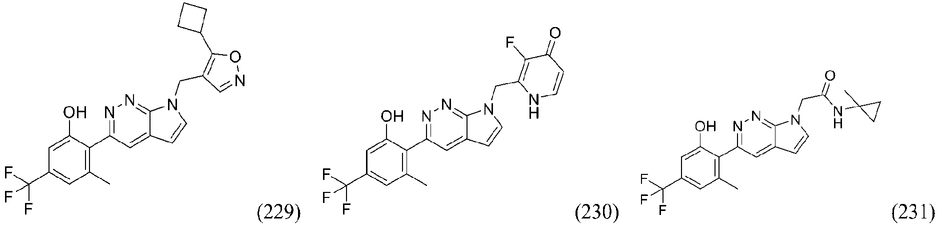

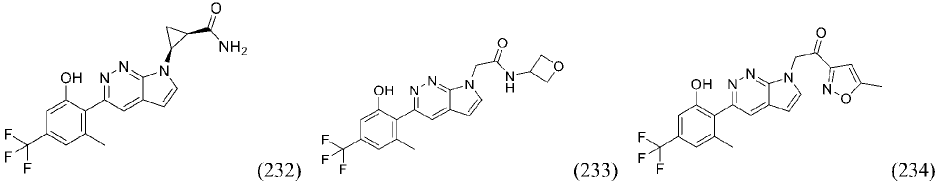

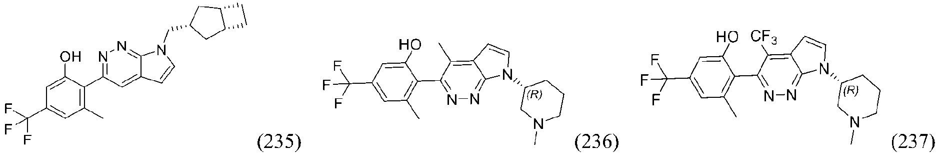

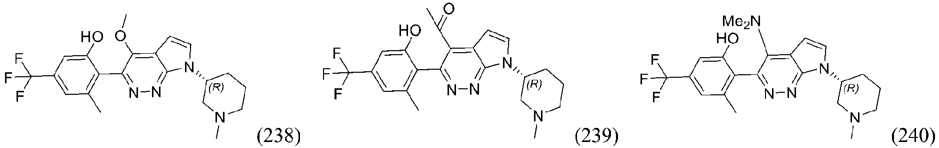

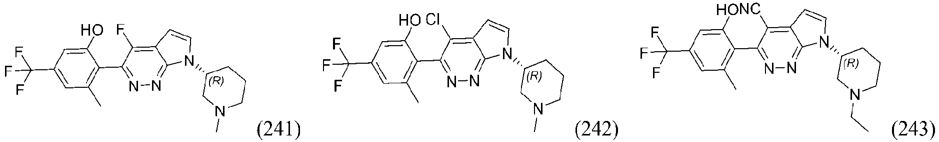

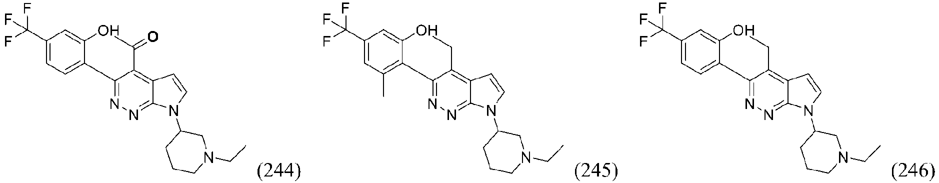

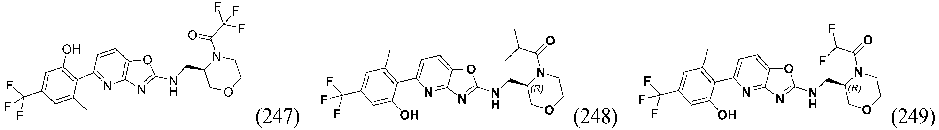

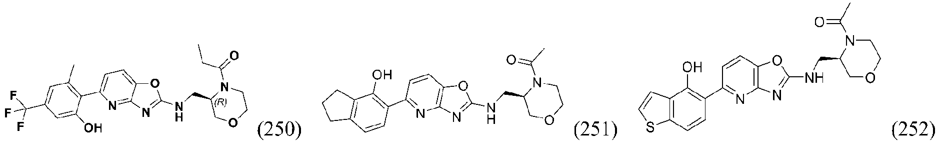

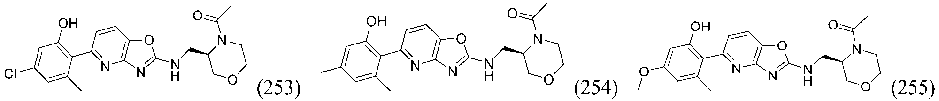

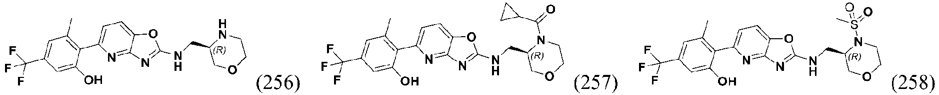

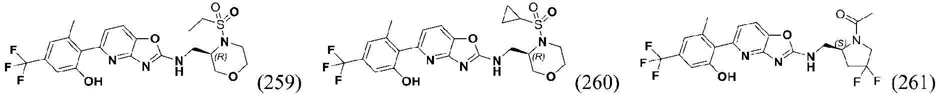

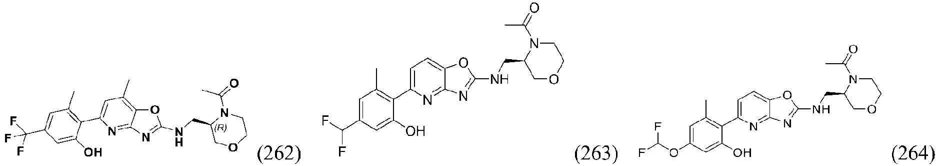

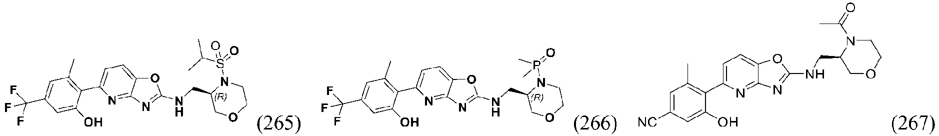

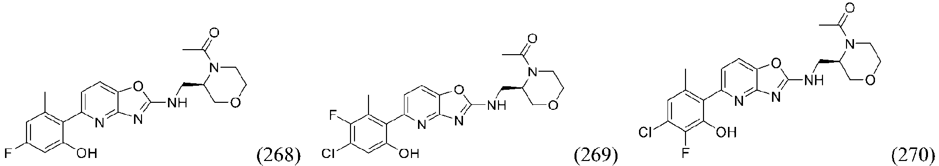

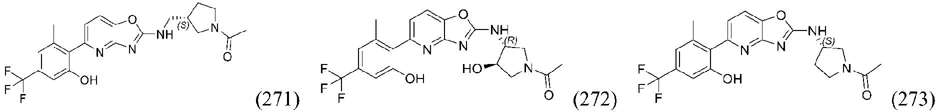

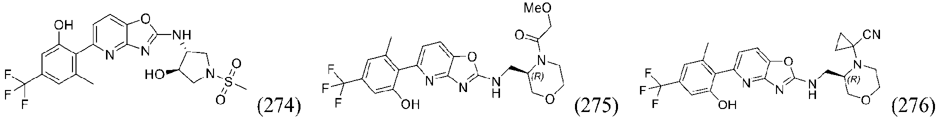

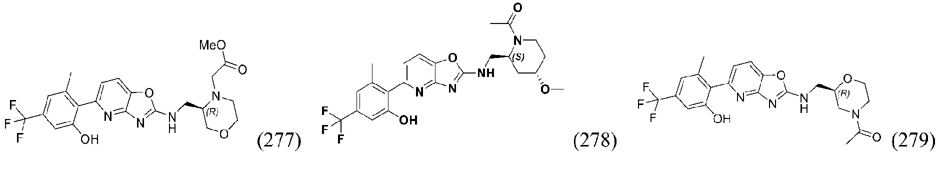

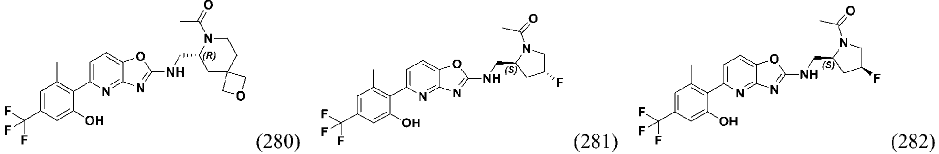

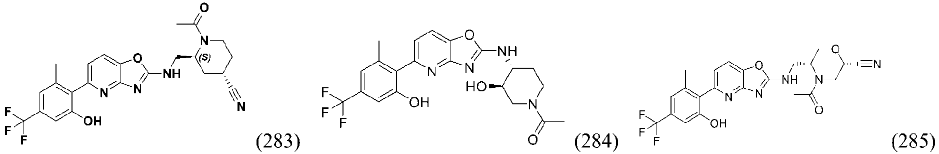

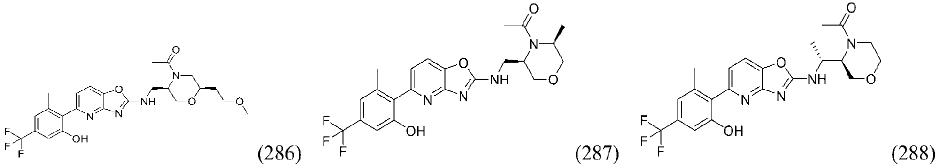

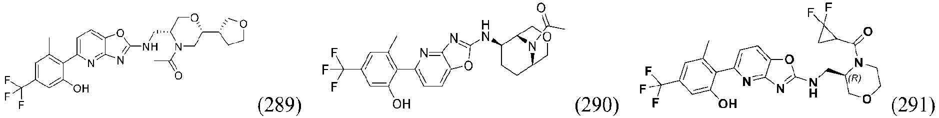

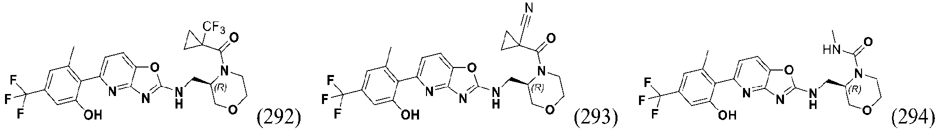

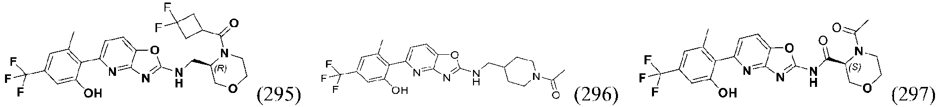

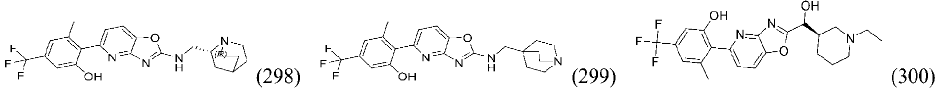

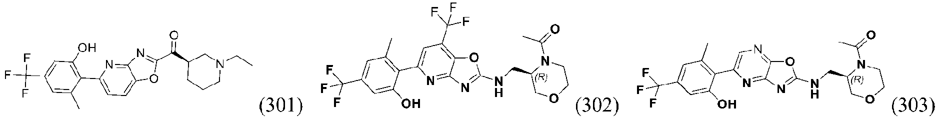

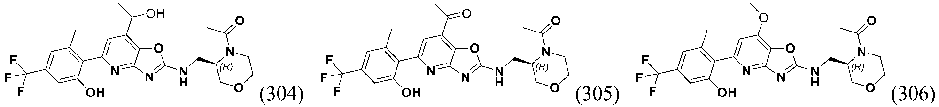

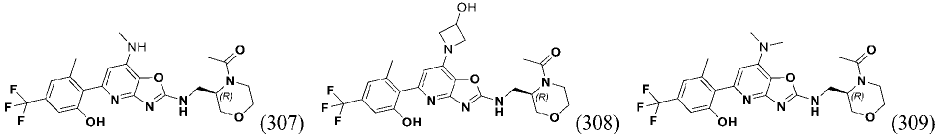

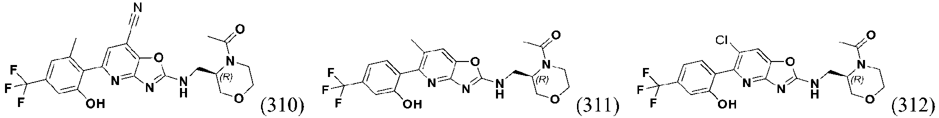

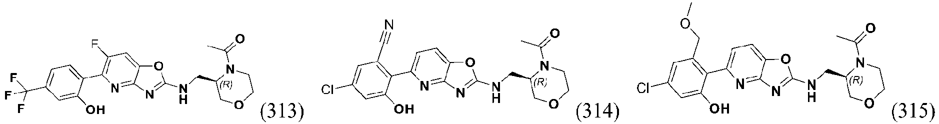

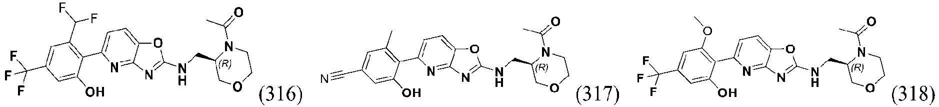

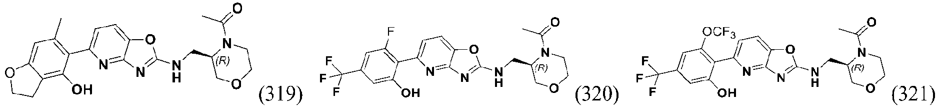

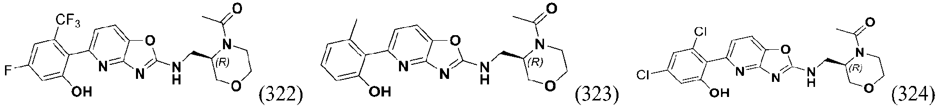

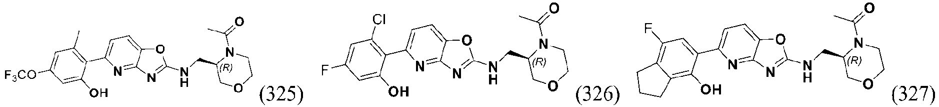

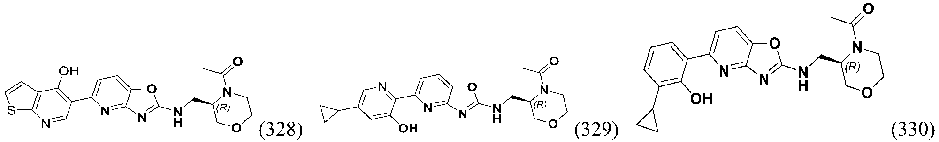

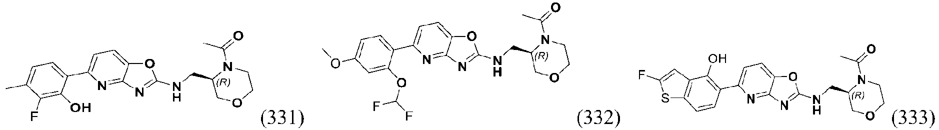

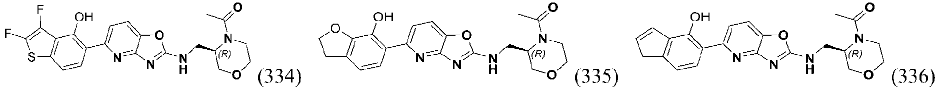

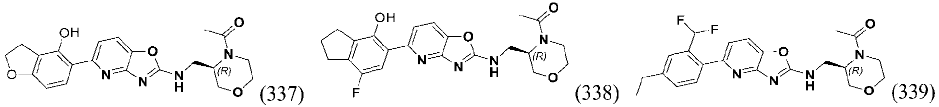

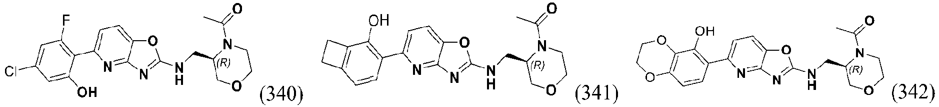

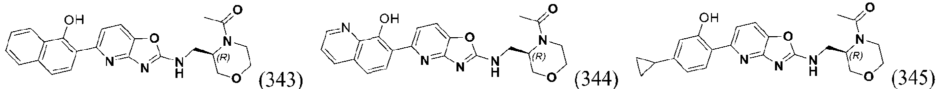

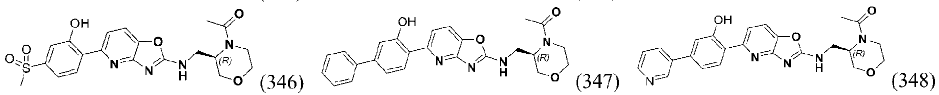

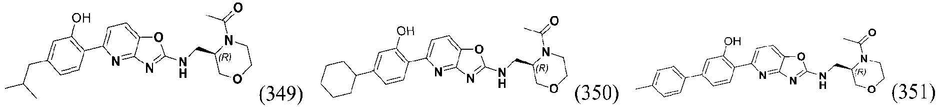

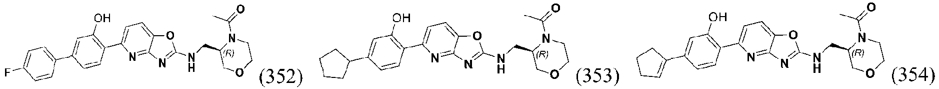

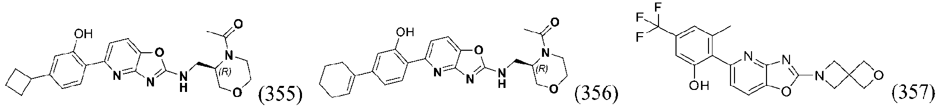

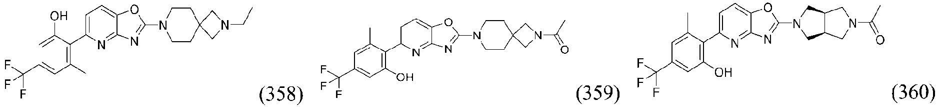

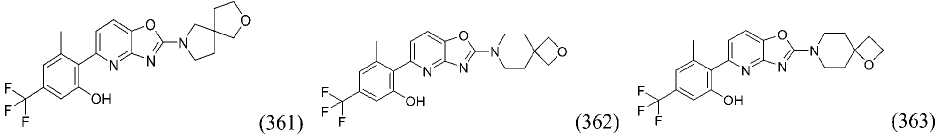

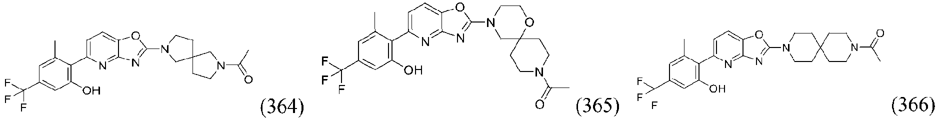

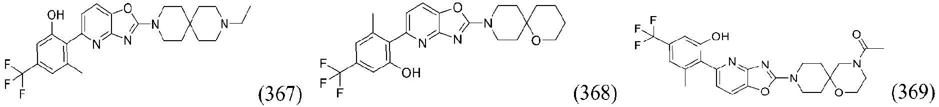

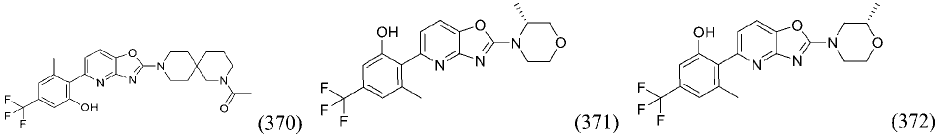

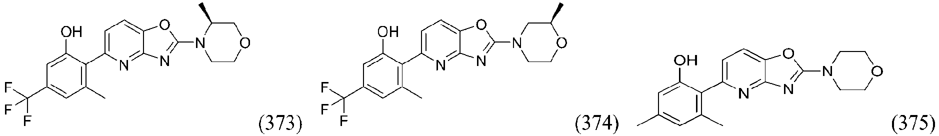

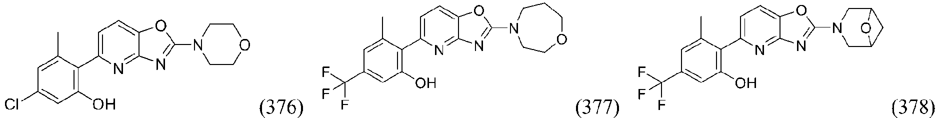

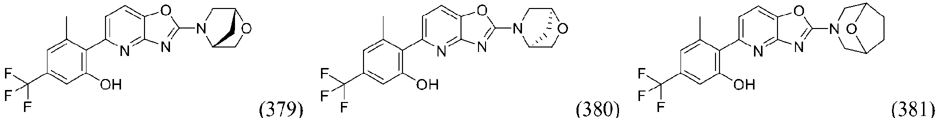

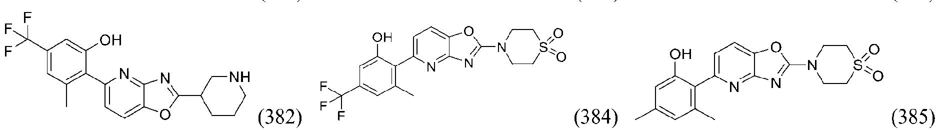

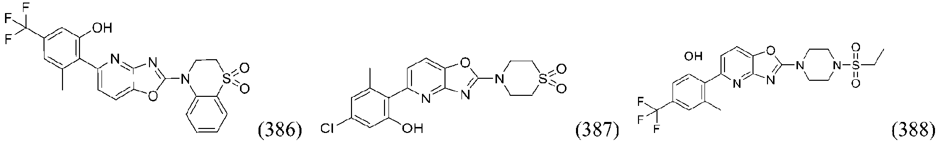

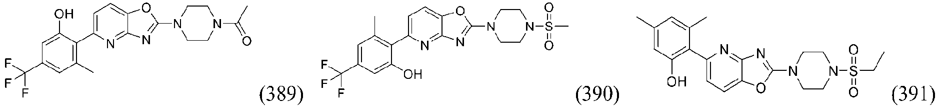

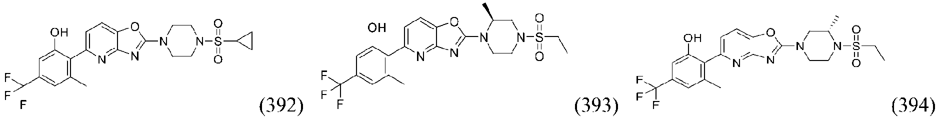

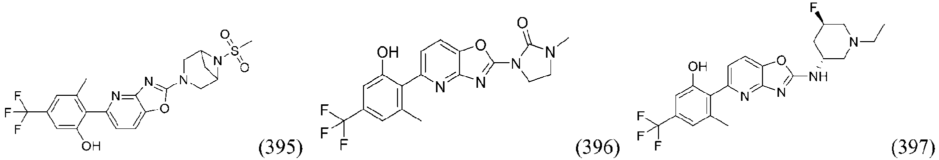

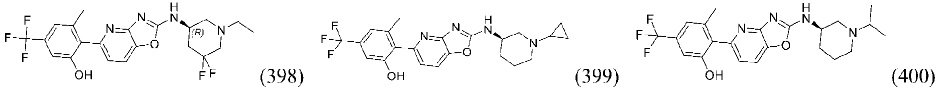

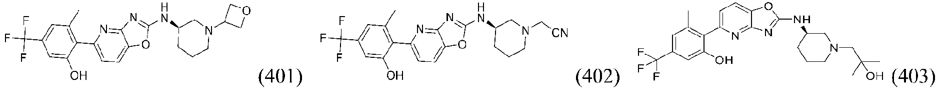

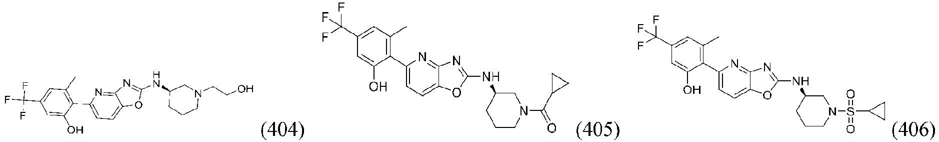

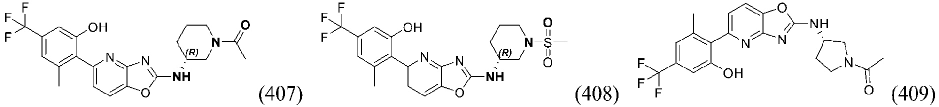

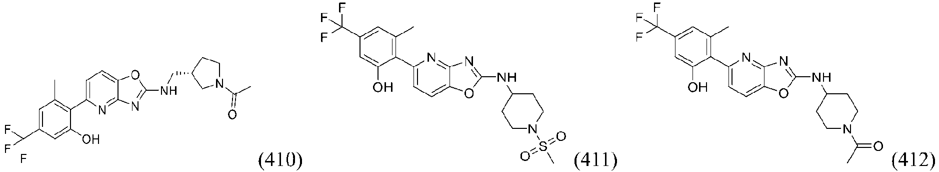

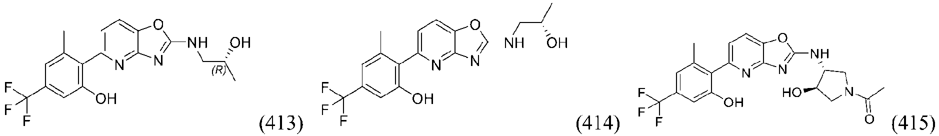

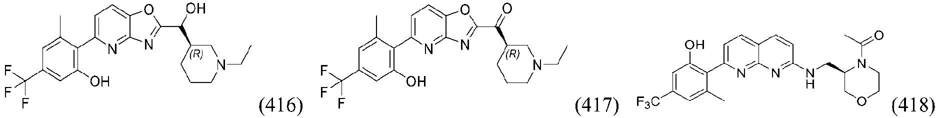

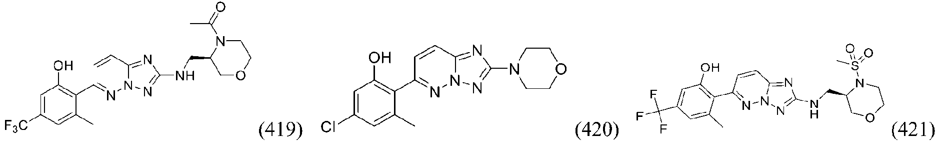

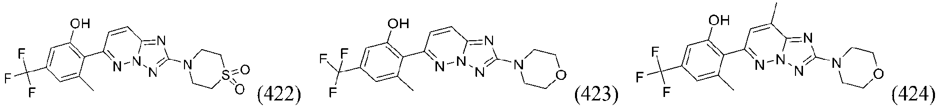

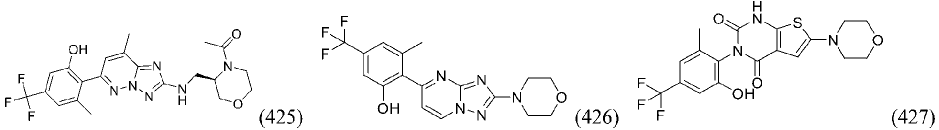

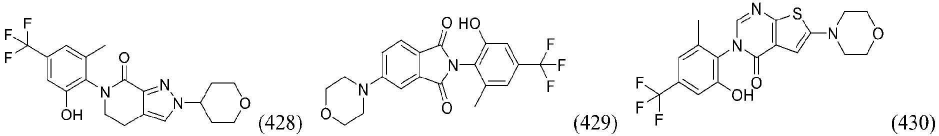

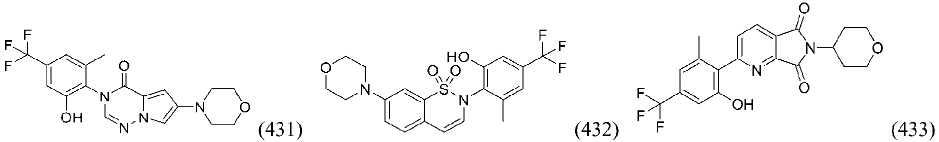

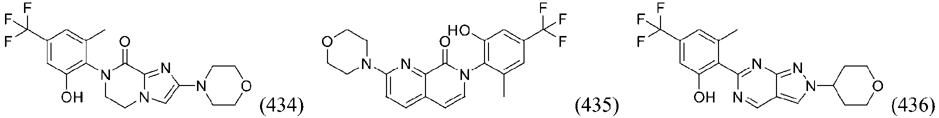

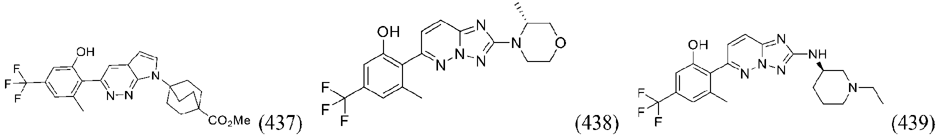

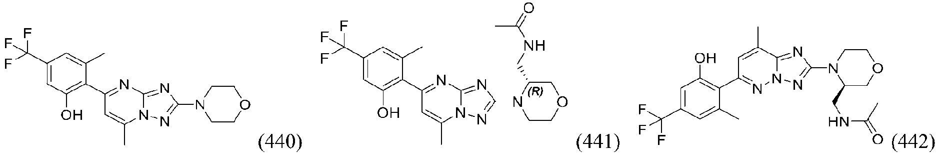

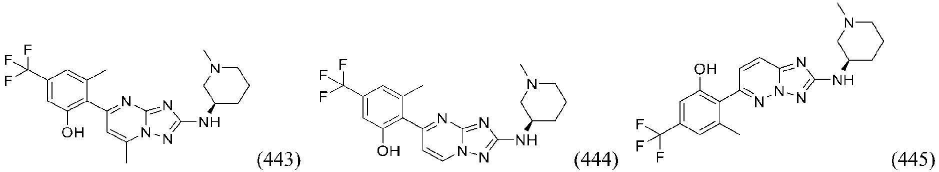

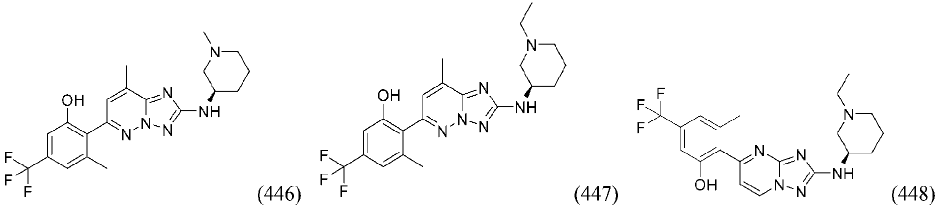

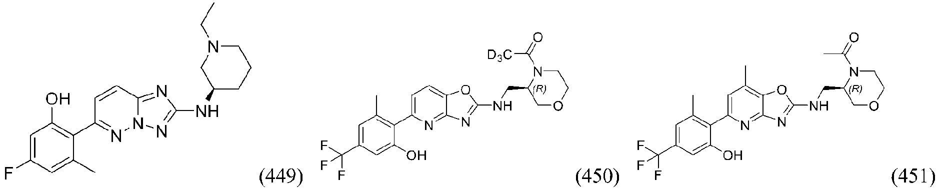

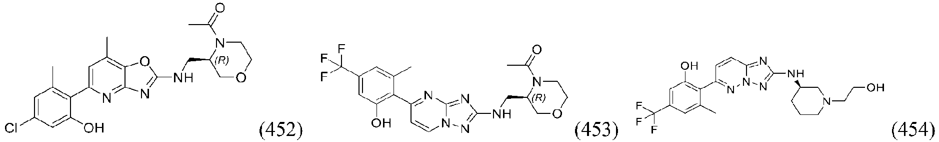

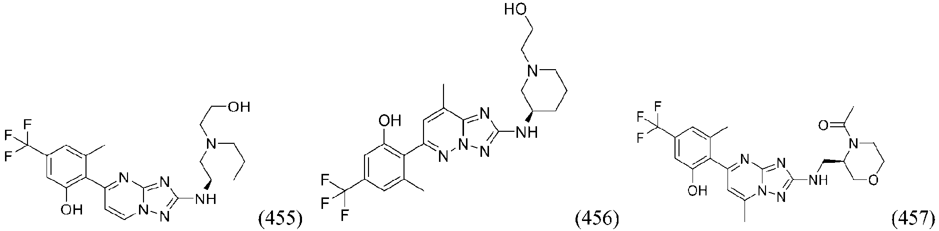

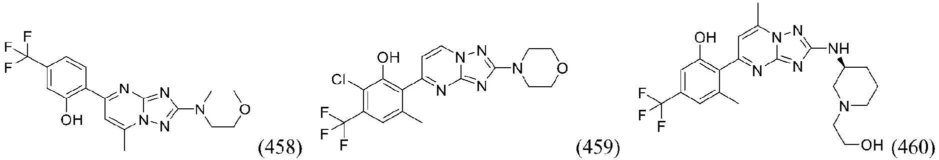

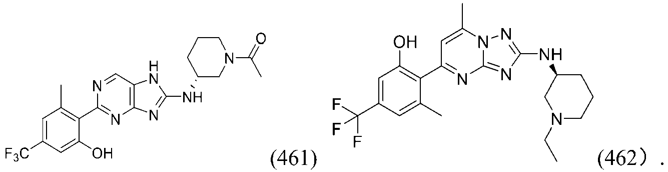

59. A pharmaceutical composition, which comprises a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to any one of claims 1-58.

60. A combination, which comprises a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to any one of claims 1-58, and one or more therapeutic agents.

61. The combination according to claim 60, the one or more therapeutic agents are independently selected from famesoid X receptor (FXR) agonists; anti-steatosis agents; anti-fibrotic agents; JAK inhibitors; checkpoint inhibitors; chemotherapy, radiation therapy and surgery; urate-lowering therapy; anabolic agents and cartilage regeneration therapy; blockers of IL 17; complement inhibitors; Bruton's tyrosine kinase inhibitors (BTK inhibitors); Toll-like receptor inhibitors (TLR7/8 inhibitors); CAR T therapy; antihypertensive agents; cholesterol-lowering agents; leukotriene A4 hydrolase (LTAH4) inhibitors; SGLT2 inhibitors; β2 agonists; anti-inflammatory agents; nonsteroidal anti-inflammatory drugs ("NSAIDs"); acetylsalicylic acid drugs (ASA); regenerative therapy treatments; cystic fibrosis treatments; and atherosclerosis treatments.

62. The compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to any one of claims 1-58, or the combination according to claim 60 or 61, for use as a medicament.

63. The compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to any one of claims 1-58, for use in the treatment of the following diseases or disorders, in which NLRP3 signaling contributes to the pathology, and/or symptoms, and/or progression of the disease or disorder.

64. A method of treating a disease or disorder in which NLRP3 signaling contributes to the pathology, and/or symptoms, and/or progression of the disease or disorder, comprises administering a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to any one of claims 1-58.

65. Use of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to any one of claims 1-58, in the manufacture of the medicament for the treatment of a disease or disorder in which NLRP3 signaling contributes to the pathology, and/or symptoms, and/or progression of the disease or disorder.

66. The compound for use according to claim 62 or 63, the treatment method according to claim 64, or the use according to claim 65, wherein the disease or disorder is selected from: inflammasome-related disease/disorder, immune disease, inflammatory disease, autoimmune disease, or auto inflammatory disease, for example, auto inflammatory fever syndrome (e.g., cryptopyrin-associated periodic syndrome), liver-related disease/disorder (e.g., chronic liver disease, viral hepatitis, nonalcoholic steatohepatitis (NASH), alcoholic steatohepatitis, and alcoholic liver disease), inflammatory arthritis-related disorder (e.g., gout, pseudogout (chondrocalcinosis), osteoarthritis, rheumatoid arthritis, joint diseases such as acute and chronic joint diseases), kidney-related disease (e.g., hyperoxaluria, lupus nephritis, type I/type II diabetes and related complications (e.g., nephropathy, retinopathy, hypertensive nephropathy, hemodialysis-related inflammation), neuroinflammation-related diseases (e.g., multiple sclerosis, brain infection, acute injury, neurodegenerative diseases, Alzheimer's disease), cardiovascular/metabolic diseases/disorders (e.g., cardiovascular risk reduction (CvRR), hypertension, atherosclerosis, type I and type II diabetes and related complications, peripheral arterial disease (PAD), acute heart failure), inflammatory skin diseases (e.g., hidradenitis suppurativa, acne), wound healing and scarring, asthma, sarcoidosis, age-related macular degeneration, and cancer-related diseases/disorders (e.g., colon cancer, lung cancer, myeloproliferative neoplasms, leukemia, myelodysplastic syndrome (MDS), myelofibrosis).

67. A method of inhibiting NLRP3 inflammasome activity in a subject in need thereof, comprises administering to a subject in need thereof a therapeutically effective amount of the compound, or an enantiomer, diastereomer, racemate, tautomer, stereoisomer, geometric isomer, nitrogen oxide, metabolite thereof, or a pharmaceutically acceptable salt, ester, solvate, hydrate, isotope-labeled (preferably deuterated) compound or prodrug thereof according to any one of claims 1-58.
